# EUROPEAN PATENT APPLICATION

(11) **EP 4 545 641 A1**
(43) Date of publication of application: **30.04.2025**
(21) Application number: 23830270.7
(22) Date of filing: 27.06.2023
(51) Int. Cl.: C12N 15/113, A61P 3/06

(54) **SIRNA FOR INHIBITING APOLIPOPROTEIN C3 EXPRESSION**

(30) Priority: 27.06.2022 CN 202210744263; 05.05.2023 CN 202310504968
(71) Applicant: Rona Bioscience, Limited, Admiralty, Hong Kong (HK)
(72) Inventor: HUANG, Jinyu, Shanghai 201315 (CN); GUO, Hongli, Shanghai 201315 (CN); CAI, Guoqing, Shanghai 201315 (CN); ZOU, Hao, Shanghai 201315 (CN)
(74) Representative: Cabinet Becker et Associés
(86) International application number: PCT/CN2023/102861
(87) International publication number: WO 2024/002093

(57) **Abstract**

Provided in an siRNA for inhibiting apolipoprotein C3 expression. Provided are a small interfering RNA (siRNA) for inhibiting APOC3 expression in cells, vectors and cells containing a nucleotide encoding the siRNA, and a method for treating diseases or symptoms related to APOC3 expression in subjects by using the siRNA, vectors or cells.

## Description

### Field of the Invention

The present invention relates to the field of RNA interference and also to the treatment of hypercholesterolemia and related diseases thereof.

### Background of the Invention

Lipoproteins are globular, micelle-like particles that consist of a non-polar core of acylglycerols and cholesteryl esters surrounded by an amphiphilic coating of protein, phospholipid, and cholesterol. Lipoproteins have been classified into five broad categories on the basis of their functional and physical properties: chylomicrons, very low density lipoproteins (VLDLs), intermediate density lipoproteins (IDLs), low density lipoproteins (LDLs), and high density lipoproteins (HDLs). Chylomicrons transport dietary lipids from the intestine to tissues. VLDLs, IDLs, and LDLs all transport triacylglycerols and cholesterol from the liver to tissues. HDLs transport endogenous cholesterol from tissues to the liver.

Apolipoprotein C3 (APOC3) is an important regulator of lipoprotein metabolism. In humans, APOC3 is expressed in the liver and to a lesser extent in the intestine. APOC3 is first expressed as a protein consisting of 99 amino acids, and after removal of the 20-amino-acid signal peptide in the endoplasmic reticulum, a mature ApoC3 protein of 79 amino acids is formed.

The primary role of APOC3 is as a regulator of lipolysis through non-competitive inhibition of endothelial-bound lipoprotein lipase (LPL). LPL hydrolyses triacylglycerols in triacylglycerol (triglyceride)-rich lipoproteins (TRLs), releasing fatty acids into the plasma and transforming large triacylglycerol-rich particles into smaller triacylglycerol-depleted remnant lipoproteins. Individuals lacking APOC3 have low TRL levels, coupled with highly efficient lipolysis of triacylglycerols.

APOC3 also inhibits hepatic lipase (HL), a lipolytic enzyme with triacylglycerol lipase and phospholipase A1 activity that is synthesized in the liver. The inhibitory effect of APOC3 on HL further reduces the lipolysis and uptake of TRL remnants by the liver.

APOC3 has been shown to inhibit lipolysis by both inhibiting lipoprotein lipase and interfering with lipoprotein binding to the cell surface glycosaminoglycan matrix. An increase in APOC3 levels induces the development of hypertriglyceridemia or high blood levels of triglycerides. Elevated levels of triglycerides are associated with a variety of diseases, including cardiovascular disease, atherosclerosis, non-alcoholic fatty liver disease, non-alcoholic steatohepatitis, polycystic ovary syndrome, kidney disease, obesity, type 2 diabetes mellitus (insulin resistance), hypertension, and skin lesions (xanthomas). Very high triglyceride levels also increase the risk of acute pancreatitis.

There is a need in the art for regulators of APOC3 expression for treating apolipoprotein C3-associated disorders, such as hypertriglyceridemia.

In recent years, inhibitors targeting APOC3 have emerged as a new therapeutic approach to managing these diseases. siRNA has great potential for development as a new treatment method. It acts on mRNA in cells and can directly silence target genes compared with traditional small molecule drugs, so it can fundamentally and more efficiently prevent the occurrence and development of diseases. However, due to its poor stability, siRNA is easily degraded by nucleases *in vivo,* not easily absorbed by tissues, difficult to be taken up by cells, and easy to produce off-target effects, which limits its clinical application. There is an urgent need for a siRNA that can effectively inhibit APOC3 gene expression in cells.

### Summary of the Invention

The present invention relates to small interfering RNA (siRNA) for inhibiting the expression of apolipoprotein C3 (APOC3) in cells and methods of using the siRNA to treat diseases.

In a first aspect, the present invention provides a small interfering RNA (siRNA) for inhibiting the expression of apolipoprotein C3 (APOC3) in cells, the siRNA comprising a sense strand and an antisense strand forming a double-stranded region, wherein the length of the sense strand and the antisense strand is each independently 15-30 nucleotides, and the antisense strand comprises at least 15 contiguous nucleotides from a nucleotide sequence as set forth in any one of SEQ ID NOs: 414-826. In some embodiments, the sense strand comprises at least 15 contiguous nucleotides from a nucleotide sequence as set forth in any one of SEQ ID NOs: 1-413.

In some embodiments, the length of the sense strand and the antisense strand is each independently 17-27 nucleotides, preferably 19-25 nucleotides, more preferably 21-23 nucleotides.

In some embodiments, the double-stranded region is 15-25 base pairs in length, preferably 17-21 base pairs, more preferably 19 base pairs.

In some embodiments, one or both of the sense strand and the antisense strand comprise a 3' overhang and/or a 5' overhang of at least 1 nucleotide; for example, one or both of the sense strand and the antisense strand comprise a 3' overhang and/or a 5' overhang of at least 2 nucleotides. In some specific embodiments, the overhang is selected from U, UU, UUU, and AA.

In some embodiments, the antisense strand comprises at least 16 contiguous nucleotides, at least 17 contiguous nucleotides, at least 18 contiguous nucleotides, at least 19 contiguous nucleotides, or at least 20 contiguous nucleotides from a nucleotide sequence as set forth in any one of SEQ ID NOs: 414-826, preferably the antisense strand comprises a nucleotide sequence as set forth in any one of SEQ ID NOs: 414-826.

In some embodiments, the sense strand comprises at least 16 contiguous nucleotides, at least 17 contiguous nucleotides, at least 18 contiguous nucleotides, at least 19 contiguous nucleotides, or at least 20 contiguous nucleotides from a nucleotide sequence as set forth in any one of SEQ ID NOs: 1-413, preferably the antisense strand comprises a nucleotide sequence as set forth in any one of SEQ ID NOs: 1-413.

In some embodiments, the siRNA comprises paired sense and antisense strand sequences as shown in Table 3.

In some preferred embodiments, the antisense strand comprises at least 15 contiguous nucleotides, at least 16 contiguous nucleotides, at least 17 contiguous nucleotides, at least 18 contiguous nucleotides, at least 19 contiguous nucleotides, or at least 20 contiguous nucleosides from a nucleotide sequence set forth in any one of SEQ ID NOs: 473, 612, 690, 757, 761, 816, 817, 818, 819, 820, 814, and 815; preferably, the antisense strand comprises a nucleotide sequence as set forth in any one of SEQ ID NOs: 473, 612, 690, 757, 761, 816, 817, 818, 819, 820, 814 and 815.

In some preferred embodiments, the sense strand comprises at least 15 contiguous nucleotides, at least 16 contiguous nucleotides, at least 17 contiguous nucleotides, at least 18 contiguous nucleotides, at least 19 contiguous nucleotides, or at least 20 contiguous nucleosides from a nucleotide sequence set forth in any one of SEQ ID NOs: 60, 199, 277, 344, 348, 403, 404, 405, 406, 407, 401, and 402, preferably the antisense strand comprises a nucleotide sequence as set forth in any one of SEQ ID NOs: 60, 199, 277, 344, 348, 403, 404, 405, 406, 407, 401, and 402.
In some more preferred embodiments, in the siRNA of the present invention:
(a) the sense strand comprises a nucleotide sequence shown in SEQ ID NO: 60, and the antisense strand comprises a nucleotide sequence shown in SEQ ID NO: 473;
(b) the sense strand comprises a nucleotide sequence shown in SEQ ID NO: 199, and the antisense strand comprises a nucleotide sequence shown in SEQ ID NO: 612
(c) the sense strand comprises a nucleotide sequence shown in SEQ ID NO: 277, and the antisense strand comprises a nucleotide sequence shown in SEQ ID NO: 690;
(d) the sense strand comprises a nucleotide sequence shown in SEQ ID NO: 344, and the antisense strand comprises a nucleotide sequence shown in SEQ ID NO: 757;
(e) the sense strand comprises a nucleotide sequence shown in SEQ ID NO: 348, and the antisense strand comprises a nucleotide sequence shown in SEQ ID NO: 761;
(f)the sense strand comprises a nucleotide sequence shown in SEQ ID NO: 403, and the antisense strand comprises a nucleotide sequence shown in SEQ ID NO: 816;
(g) the sense strand comprises a nucleotide sequence shown in SEQ ID NO: 404, and the antisense strand comprises a nucleotide sequence shown in SEQ ID NO: 817;
(h) the sense strand comprises a nucleotide sequence shown in SEQ ID NO: 405, and the antisense strand comprises a nucleotide sequence shown in SEQ ID NO: 818;
(i)the sense strand comprises a nucleotide sequence shown in SEQ ID NO: 406, and the antisense strand comprises a nucleotide sequence shown in SEQ ID NO: 819;
(j)the sense strand comprises a nucleotide sequence shown in SEQ ID NO: 407, and the antisense strand comprises a nucleotide sequence shown in SEQ ID NO: 820;
(k) the sense strand comprises a nucleotide sequence shown in SEQ ID NO: 401, and the antisense strand comprises a nucleotide sequence shown in SEQ ID NO: 814; or
(l)the sense strand comprises a nucleotide sequence shown in SEQ ID NO: 402, and the antisense strand comprises a nucleotide sequence shown in SEQ ID NO: 815.

In some embodiments, substantially all nucleotides of the sense strand and substantially all nucleotides of the antisense strand are modified nucleotides. In some preferred embodiments, all nucleotides of the sense strand and all nucleotides of the antisense strand are modified nucleotides.

In some specific embodiments, the sense strand and the antisense strand each independently comprise one or more nucleotide modifications selected from the group consisting of: a 2'-O-methyl modified nucleotide, a 2'-fluoro modified nucleotide, a 2'-deoxy modified nucleotide, an inosine ribonucleotide, an abasic nucleotide, an inverted abasic deoxyribonucleotide, a phosphorothioate internucleotide linkage modification, a vinylphosphonate modified nucleotide, a locked nucleotide, a 2'-amino modified nucleotide, a 2'-alkyl modified nucleotide, a morpholino nucleotide, a phosphoramidate, a non-natural base comprising nucleotide, and a terminal nucleotide linked to a cholesteryl derivative or a dodecanoic acid bisdecylamide group, and a deoxyribonucleotide.

In some preferred embodiments, the sense strand and the antisense strand each independently comprise one or more nucleotide modifications selected from the group consisting of: a 2'-O-methyl modified nucleotide, a 2'-fluoro modified nucleotide, a 2'-deoxy modified nucleotide, an inosine ribonucleotide, an abasic nucleotide, an inverted abasic deoxyribonucleotide, and a phosphorothioate internucleotide linkage modification.

In some specific embodiments, the sense strand and/or the antisense strand comprises at least two 2'-fluoro modified nucleotides.

In some specific embodiments, the sense strand and/or the antisense strand comprises at least eight 2'-O-methyl modified nucleotides.

In some specific embodiments, the sense strand and/or the antisense strand comprises 1 to 5 phosphorothioate groups at the 5' end.

In some embodiments, the antisense strand comprises a modified nucleotide sequence set forth in any one of Table 5, and/or the sense strand comprises a modified nucleotide sequence set forth in any one of Table 4.

In some embodiments, the siRNA comprises a paired modified sense strand sequence and modified antisense strand sequence set forth in any one of Table 6.

In some preferred embodiments, wherein:
(1) the sense strand comprises AmsAmAmAmGmGmGmAmCfAfGfUmAmUmUmCmUmCmAmGmAms-STM1(SEQ ID NO: 1655), and the antisense strand comprises (CP1a-U)sCfsUmGfAmGfAmAfUmAfCmUfGmUfCmCfCmUfUmsUfsUm(SEQ ID NO: 1651);
(2) the sense strand comprises STM1s-AmsAmUmUmAmAmAmAmGfGfGfAmCmAmGmUmAmUmUmCmAms-STM1 (SEQ ID NO: 1656), and the antisense strand comprises (CP1a-U)sGfsAmAfUmAfCmUfGmUfCmCfCmUfUmUfUmAfAmsUfsUm;
(3) the sense strand comprises STM1s-AmsAmUmUmAmAmAmAmGfGfGfAmCmAmGmUmAmUmUmCmAms-STM1(SEQ ID NO: 1657), and the antisense strand comprises (VPUm)sGfsAmAfUmAfCmUfGmUfCmCfCmUfUmUfUmAfAmsUfsUm (SEQ ID NO: 1649);
(4) the sense strand comprises IBs-AmsAmUmUmAmAmAmAmGfGfGfAmCmAmGmUmAmUmUmCmAms-IB (SEQ ID NO: 1658), and the antisense strand comprises (CP1a-U)sGfsAmAfUmAfCmUfGmUfCmCfCmUfUmUfUmAfAmsUfsUm(SEQ ID NO: 1650);
(5) the sense strand comprises UmsUmsAmAmAmAmGfGfGfAmCmAmGmUmAmUmUmCmsAm (SEQ ID NO: 1659), and the antisense strand comprises (CP1a-U)sGfsAmAfUmAfCmUfGmUfCmCfCmUfUmUfUmAfAmsUfsUm(SEQ ID NO: 1650);
(6) the sense strand comprises IBs-AmsAmAmAmGmGmGmAmCfAfGfUmAmUmUmCmUmCmAmGmAms-IB (SEQ ID NO: 1660), and the antisense strand comprises (CP1a-U)sCfsUmGfAmGfAmAfUmAfCmUfGmUfCmCfCmUfUmsUfsUm(SEQ ID NO: 1651);
(7) the sense strand comprises AmsAmsGmGmGmAmCfAfGfUmAmUmUmCmUmCmAmGmsAm (SEQ ID NO: 1661), and the antisense strand comprises (CP1a-U)sCfsUmGfAmGfAmAfUmAfCmUfGmUfCmCfCmUfUmsUfsUm(SEQ ID NO: 1651);
(8) the sense strand comprises IBs-AmsAmAmAmGmGmGmAmCfAfGfUmAmUmUmCmUmCmAmGmAms-IBs-GL6(SEQ ID NO: 1662), and the antisense strand comprises (CP1a-U)sCfsUmGfAmGf(PCN-A)AfUmAfCmUfGmUfCmCfCmUfUmsUfsUm(SEQ ID NO: 1652); or
(9) the sense strand comprises AmsAmsGmGmGmAmCfAfGfUmAmUmUmCmUmCmAmGmsAm (SEQ ID NO: 1663), and the antisense strand comprises (CP1a-U)sCfsUmGfAmGf(PCN-A)AfUmAfCmUfGmUfCmCfCmUfUmsUfsUm(SEQ ID NO: 1652);
   (a) the sense strand comprises CmsGmsAmGmGmAmUfGfCfCmUmCmCmCmUmUmCmUmUm, and the antisense strand comprises AmsAfsGmAfAmGfGmGfAmGfGmCfAmUfCmCfUmCfGmsUfsUm;
   (b) the sense strand comprises CmsCmsGmUmUmAmAfGfGfAmCmAmAmGmUmUmCmUmUm, and the antisense strand comprises AmsAfsGmAfAmCfUmUfGmUfCmCfUmUfAmAfCmGfGmsUfsUm;
   (c) the sense strand comprises CmsCmsGmUmUmAmAfGfGfAmCmAmAmGmUmUmCmUmUm, and the antisense strand comprises AmsAfsGmAfAmCfUmUfGmUfCmCfUmUfAmAfCmGfGmsUfsUm;
   (d) the sense strand comprises CmsCmsAmAmGmUmCfCfAfCmCmUmGmCmCmUmAmUmUm, and the antisense strand comprises AmsAfsUmAfGmGfCmAfGmGfUmGfGmAfCmUfUmGfGmsUfsUm;
   (e) the sense strand comprises AmsAmsGmGmGmAmCfAfGfUmAmUmUmCmUmCmAmGmUm , and the antisense strand comprises AmsCfsUmGfAmGfAmAfAfCmUfGmUfCmCfCmUfUmsUfsUm;
   (f)the sense strand comprises UmsUmsAmAmAmAmGfGfGfAmCmAmGmUmAmUmUmCmUm, and the antisense strand comprises AmsGfsAmAfUmAfCmUfGmUfCmCfCmUfUmUfUmAfAmsUfsUm;
   (g) the sense strand comprises AmsAmsGmGmGmAmCfAfGfUmAmUmUmCmUmCmAmGmUm, and the antisense strand comprises AmsCfsUmGfAmGfAmAfUmAfCmUfGmUfCmCfCmUfUmsUfsUm;
   (h) the sense strand comprises CmsCmsAmAmGmUmCfCfAfCmCmUmGmCmCmUmAmUmUm, and the antisense strand comprises AmsAfsUmAfGmGfCmAfGmGfUmGfGmAfCmUfUmGfGmsUfsUm;
   (i)the sense strand comprises CmsCmsGmUmUmAmAfGfGfAmCmAmAmGmUmUmCmUmUm-L96, and the antisense strand comprises AmsAfsGmAfAmCfUmUfGmUfCmCfUmUfAmAfCmGfGmsUfsUm;
   (j) the sense strand comprises CmsGmsAmGmGmAmUfGfCfCmUmCmCmCmUmUmCmUmUm, and the antisense strand comprises AmsAfsGmAfAmGfGmGfAmGfGmCfAmUfCmCfUmCfGmsUfsUm;
   (k) the sense strand comprises IBs-AmCmGmGmGmAmCmAmGfUfAfUmUmCmUmCmAmGmUmimAms-IB, and the antisense strand comprises UmsCfsAmsCfUmGfAmGmAmAmUmAfCmUfGmUfCmCfCmGfsUm; or
   (l)the sense strand comprises AmsAmsGmGmGmAmCfAmGfUfAfUmUmCmUmCmAmGmUmsGmsCm, and the antisense strand comprises GmsCfsAmCmUmGfAmGmAmAmUmAmCmUfGmUfCmCmCmUmUmsUmsUm.

In some embodiments, the siRNA is further conjugated to a ligand moiety comprising N-acetylgalactosamine via a phosphate group or a phosphorothioate group, preferably the sense strand of the siRNA is conjugated to the ligand moiety via a phosphate group or a phosphorthioate group.

In some specific embodiments, the 3' end of the sense strand is conjugated to the ligand moiety via a phosphate group or a phosphorothioate group. In some other specific embodiments, the 5' end of the sense strand is conjugated to the ligand moiety via a phosphate group or a phosphorothioate group.

In some embodiments, the ligand moiety comprises a conjugation group of formula (X'): 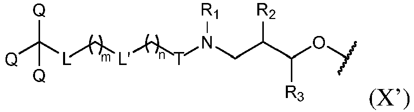 wherein, 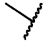 represents the position of attachment to siRNA; Q is independently H, 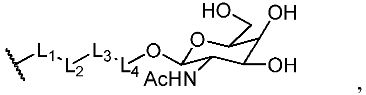 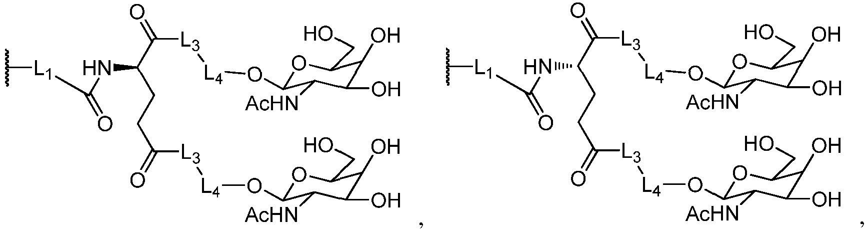 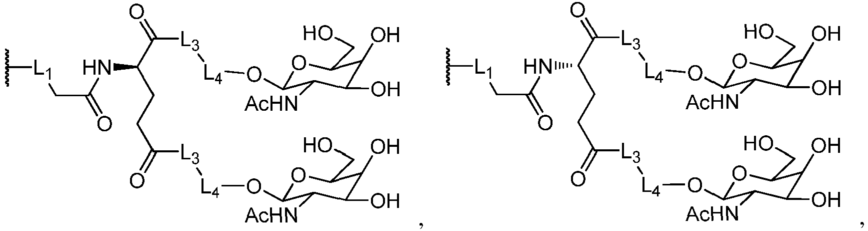 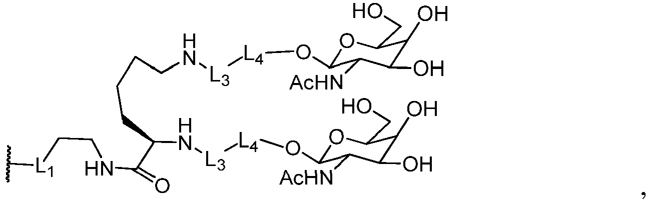 or 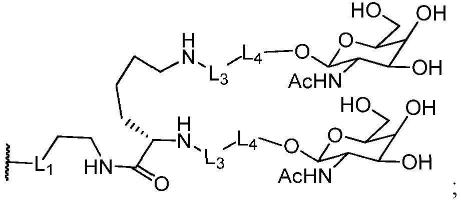
wherein L₁ is a bond, -CH₂-, -CH₂CH₂-, -C(O)-, -CH₂O-, -CH₂O-CH₂CH₂O-, or - NHC(O)-(CH₂NHC(O))ₐ-;
L₂ is a bond or -CH₂CH₂C(O)-;
L₃ is a bond, -(NHCH₂CH₂)_{b}-, -(NHCH₂CH₂CH₂)_{b}-, or -C(O)CH₂-;
L₄ is -(OCH₂CH₂)_{c}-, -(OCH₂CH₂CH₂)_{c}-, -(OCH₂CH₂CH₂CH₂)_{c}-, - (OCH₂CH₂CH₂CH₂CH₂)_{c}-, or -NHC(O)-(CH₂)_{d}-;
wherein a = 0, 1, 2, or 3;
b = 1, 2, 3, 4 or 5;
c = 1, 2, 3, 4 or 5;
d = 1, 2, 3, 4, 5, 6, 7, or 8;
L is a bond, -CH₂O-, or -NHC(O)-;
L' is a bond, -C(O)NH-, -NHC(O)-, or -O(CH₂CH₂O)ₑ-;
wherein e is 1, 2, 3, 4, or 5;
T is a bond, -CH₂-, -C(O)-, -M-, -CH₂-M-, or -C(O)-M-;
wherein M is 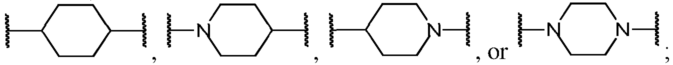
R₁ and R₂ together form -CH₂CH₂O- or -CH₂CH(R)-O-, and R₃ is H;
or R₁ and R₃ together form -C₁₋₂ alkylene-, and R₂ is H;
wherein R is -OR', -CH₂OR', or -CH₂CH₂OR'; wherein R' is H, a hydroxy-protecting group, or a solid support, and the hydroxy-protecting group is preferably - C(O)CH₂CH₂C(O)OH or 4,4'-dimethoxytrityl;
m = 0, 1, 2, 3, 4, 5, 6, 7, 8, 9 or 10;
n = 0, 1, 2, 3, 4, 5, 6, 7, 8, 9 or 10.

In some embodiments, the conjugation ligand targets an asialoglycoprotein receptor (ASGPR).

In some preferred embodiments, the conjugation group is selected from Table 1.

**Table 1. Structure of Conjugation Groups**

| No. | Structure |
|---|---|
| 1 | 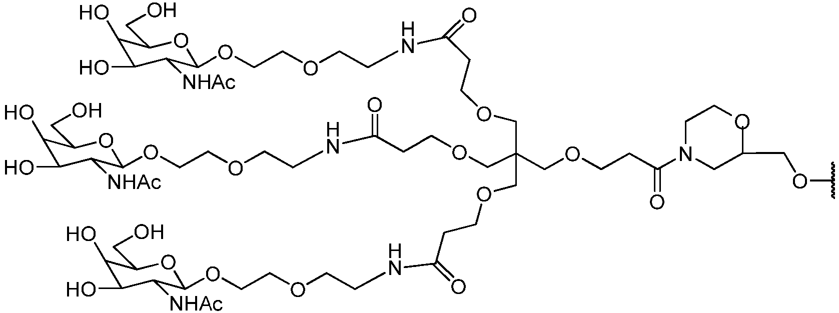 |
| 2 | 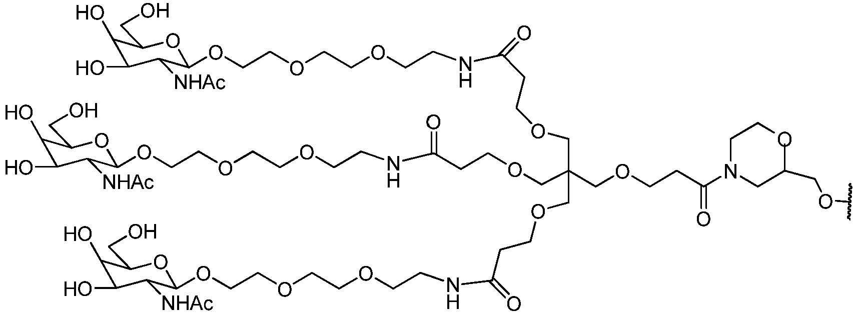 |
| 3 | 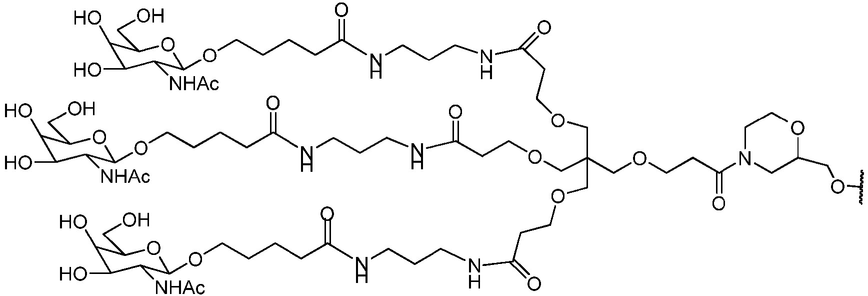 |
| 4 | 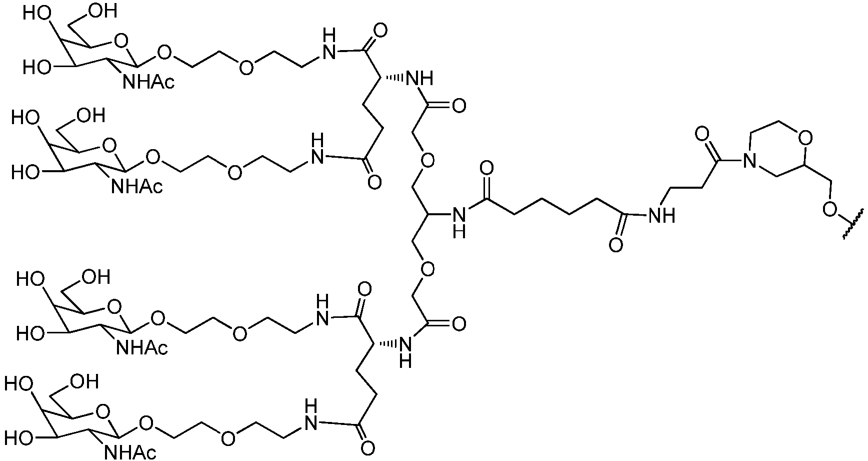 |
| 5 | 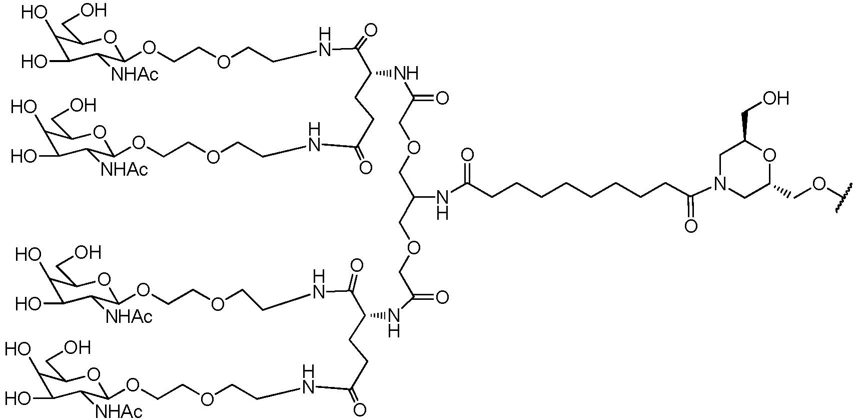 |
| 6 | 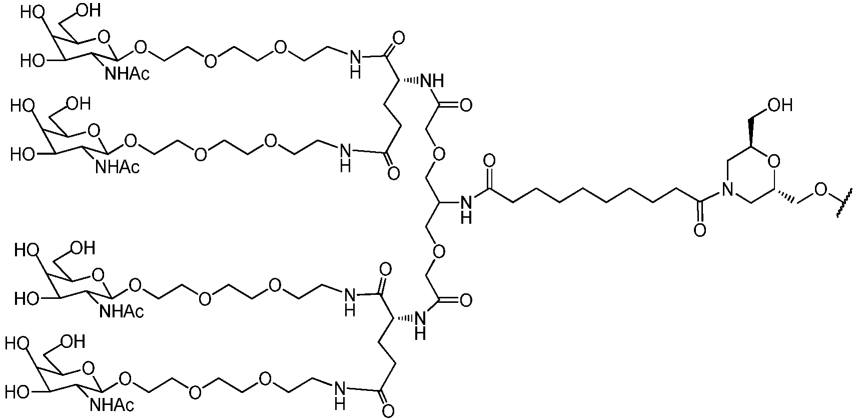 |
| 7 | 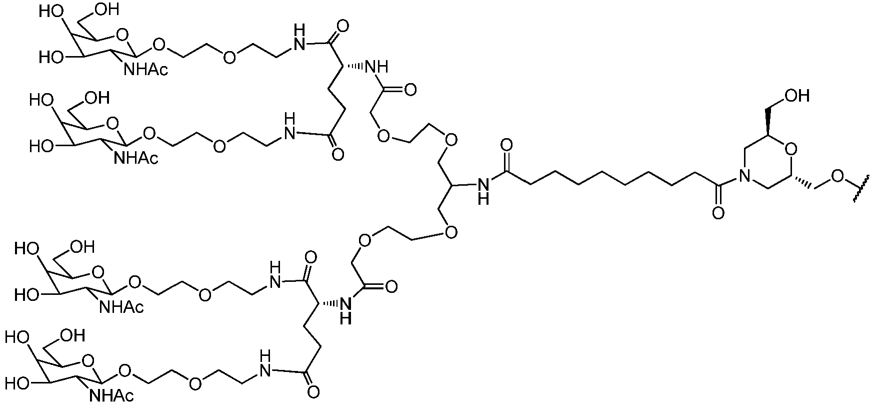 |
| 8 | 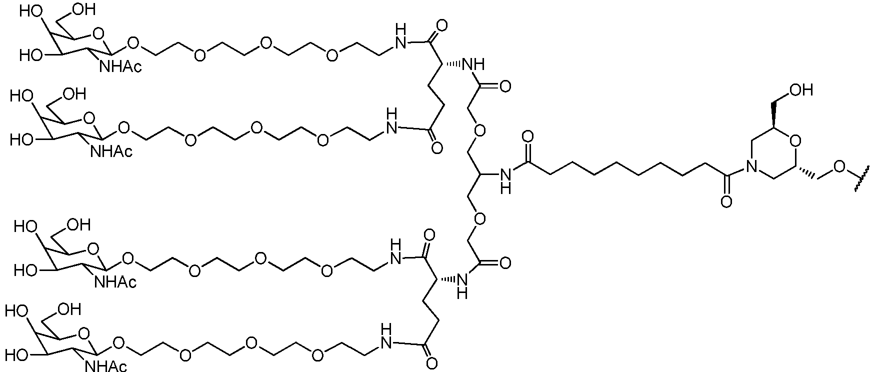 |
| 9 | 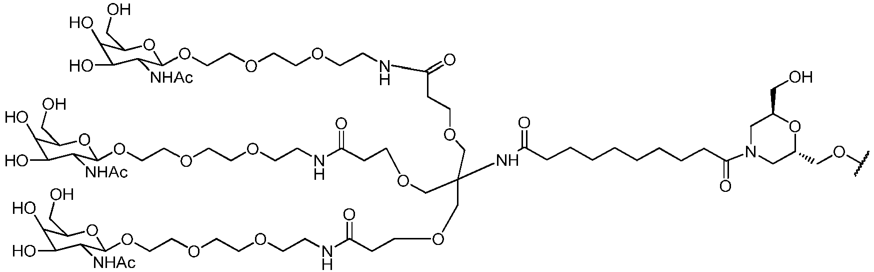 |
| 10 | 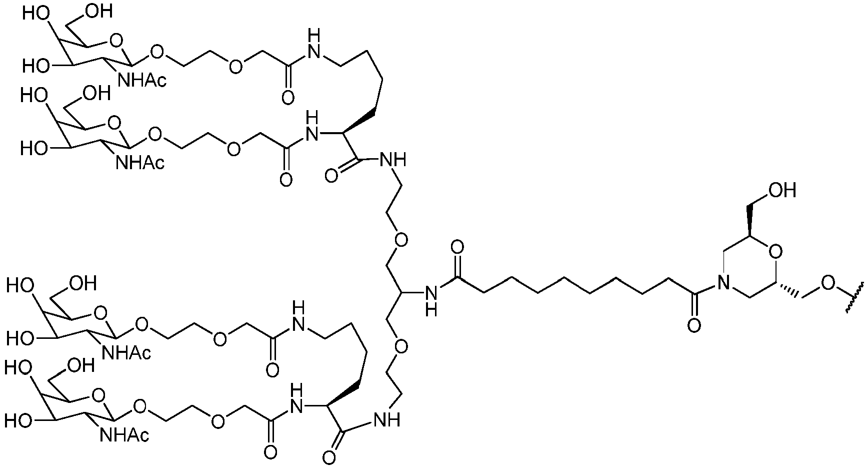 |
| 11 | 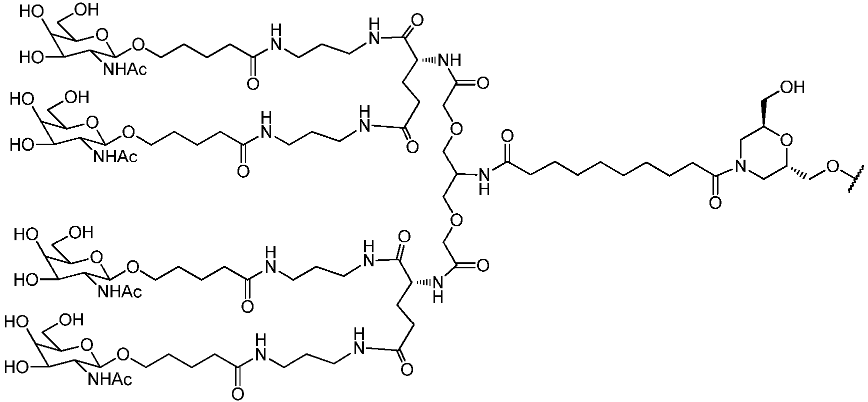 |
| 12 | 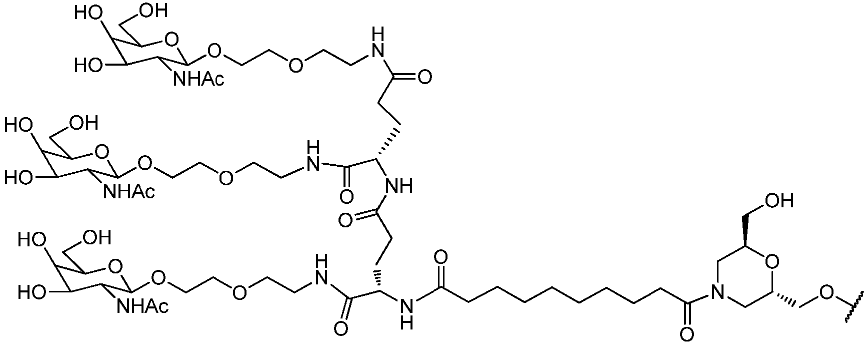 |
| 13 | 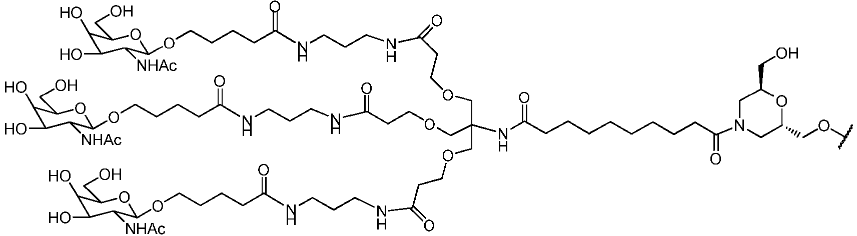 |
| 14 | 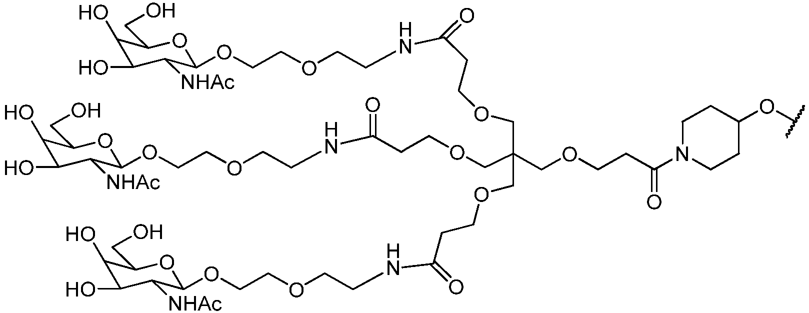 |
| 15 | 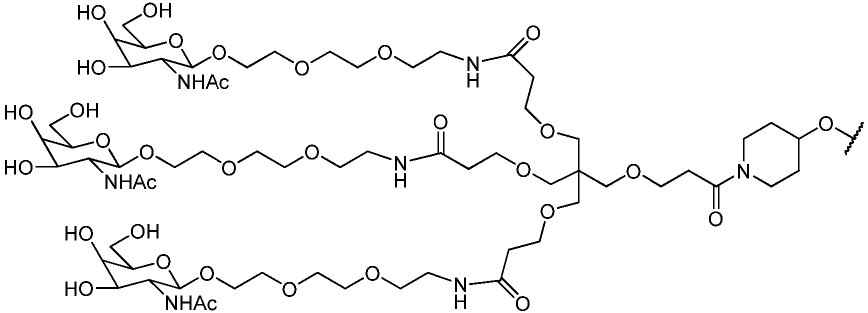 |
| 16 | 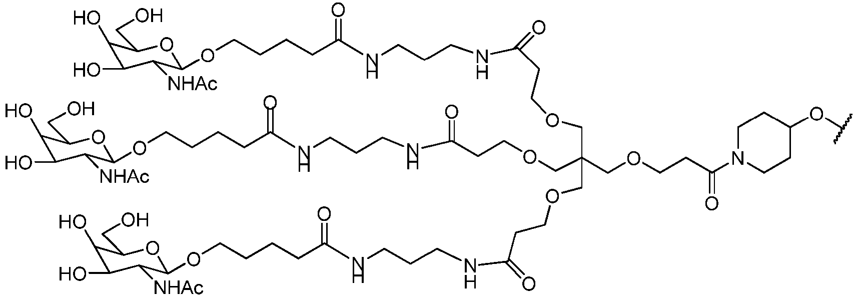 |

In some preferred embodiments, the conjugation group is selected from Table 2.

**Table 2. Structure of Conjugation Groups**

| No. | Structure |
|---|---|
| 17 | 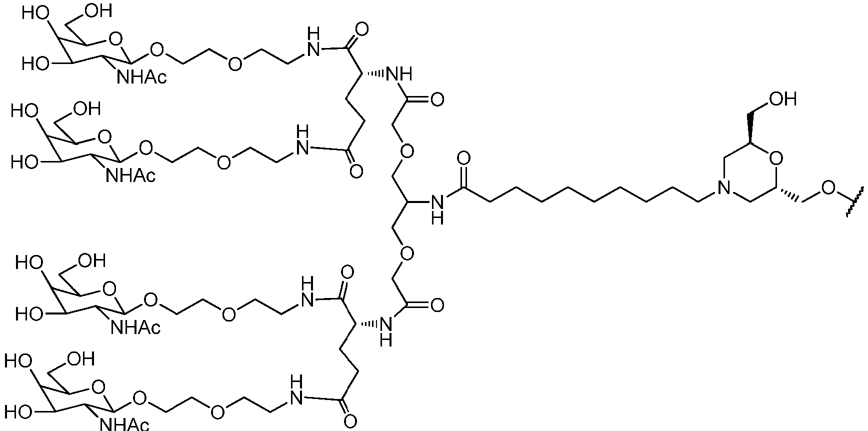 |
| 18 | 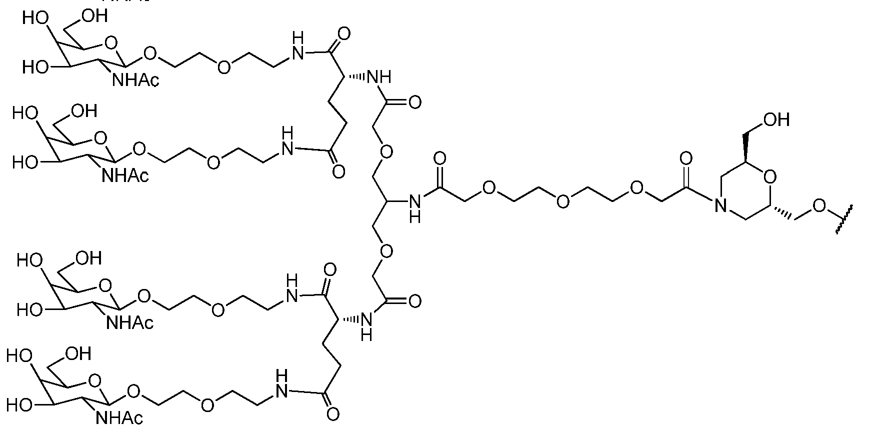 |
| 19 | 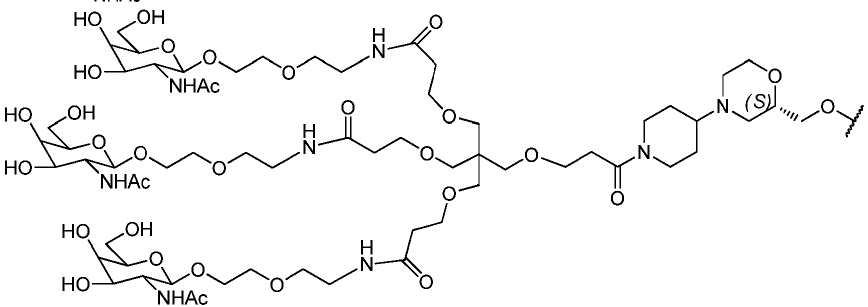 |
| 20 | 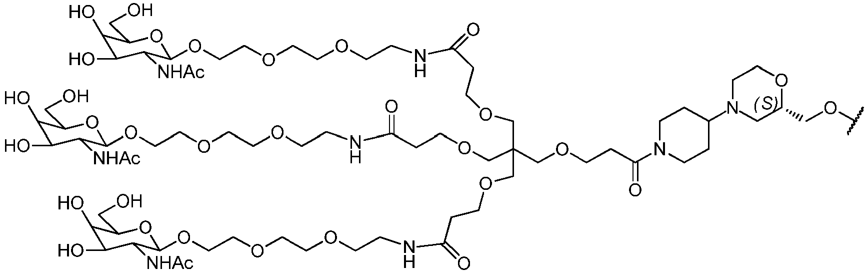 |
| 21 | 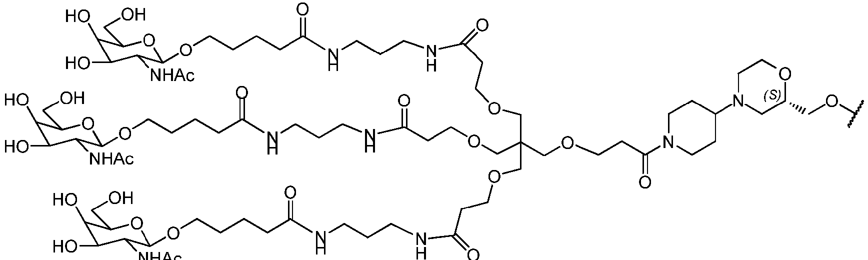 |
| 22 | 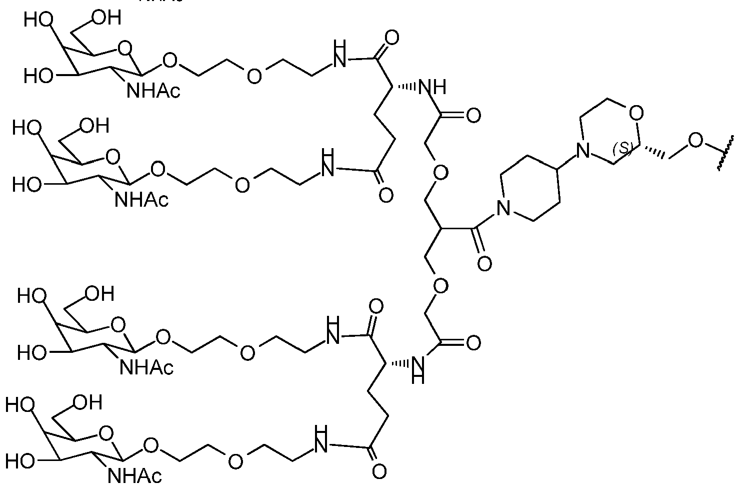 |
| 23-1 | 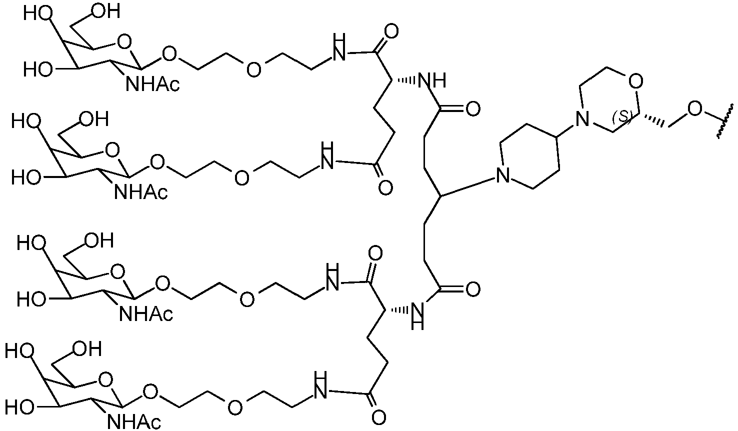 |
| 23-2 | 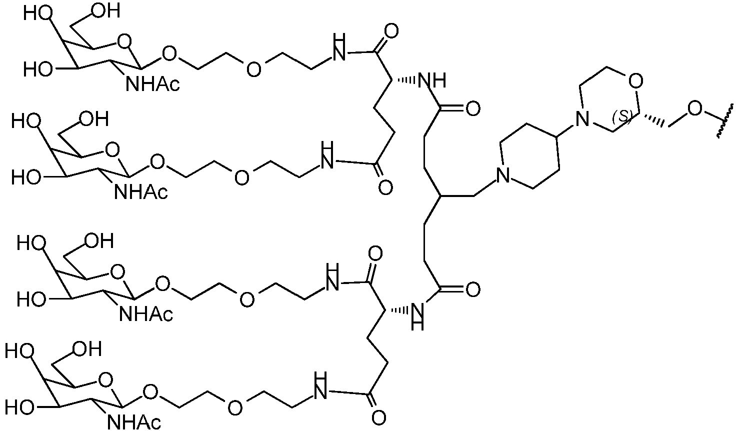 |
| 24 | 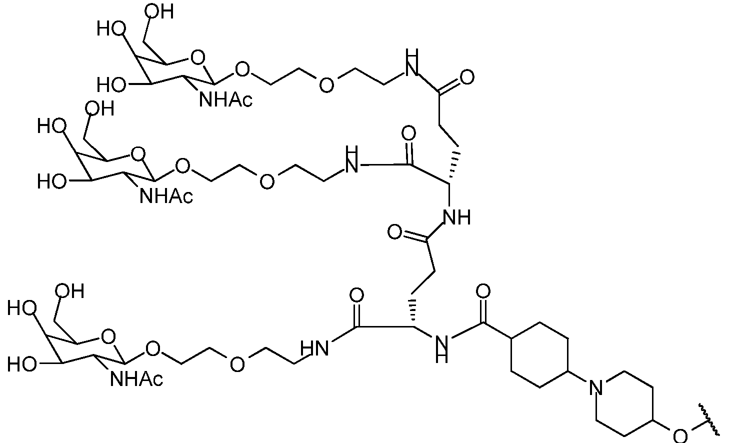 |
| 25 | 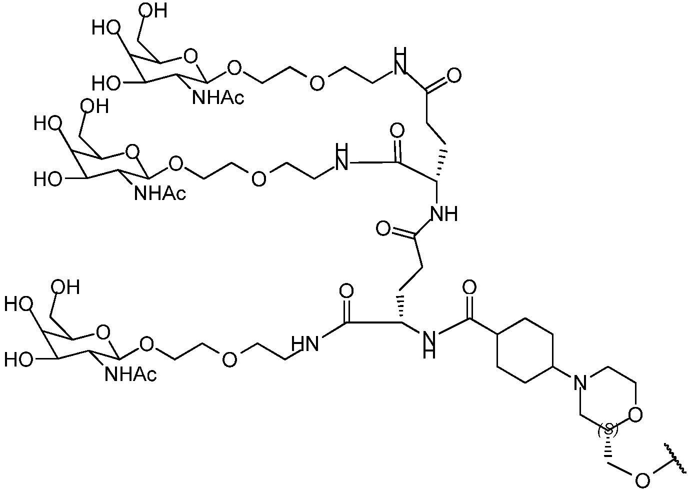 |
| 26 | 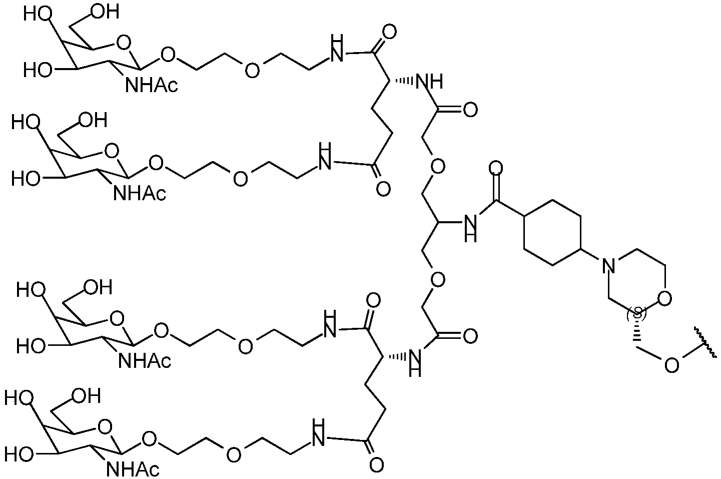 |
| 27 | 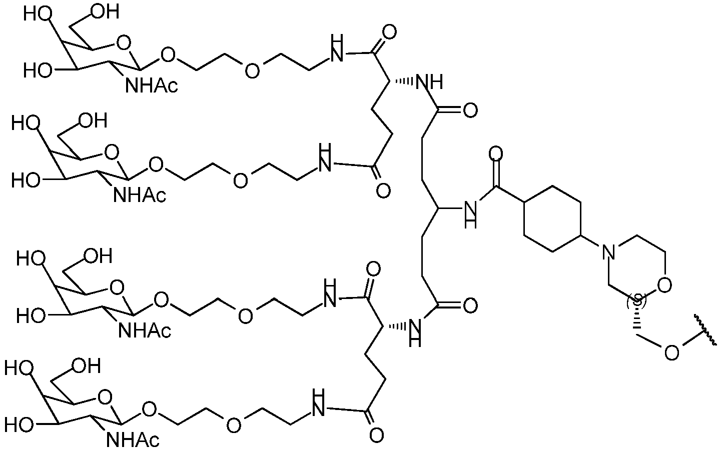 |
| 28 | 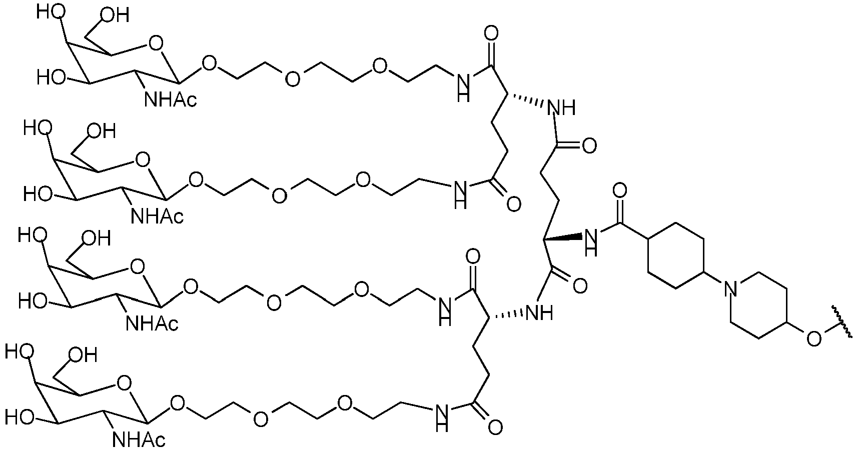 |
| 29 | 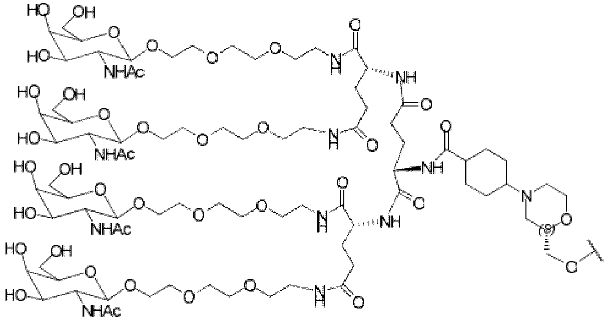 |

In some preferred embodiments, the ligand has the following structure: 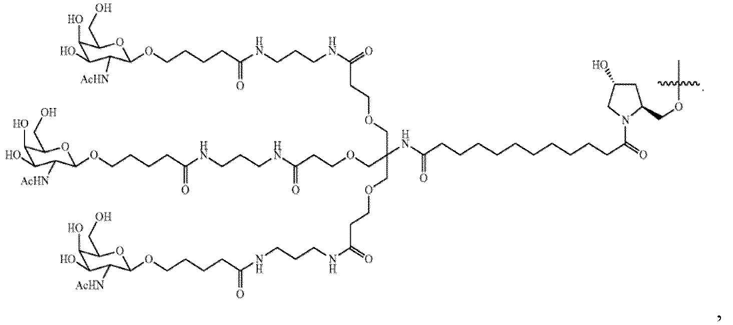 wherein 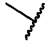 represents the position of attachment to the sense strand of the siRNA via a phosphate group or a phosphorothioate group.

In some preferred embodiments, the ligand has the following structure: 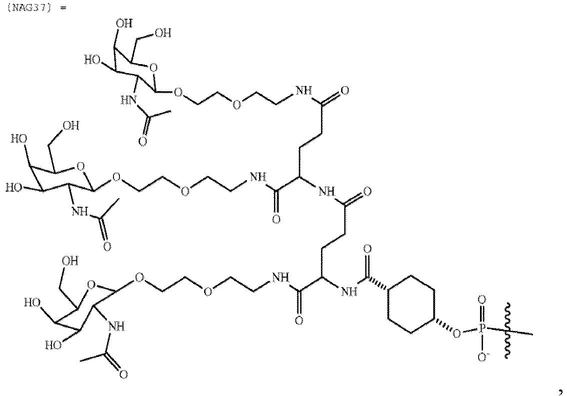 wherein 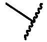 represents the position of attachment to the sense strand of the siRNA via a phosphate group or a phosphorothioate group.

In some preferred embodiments, the ligand has the following structure: 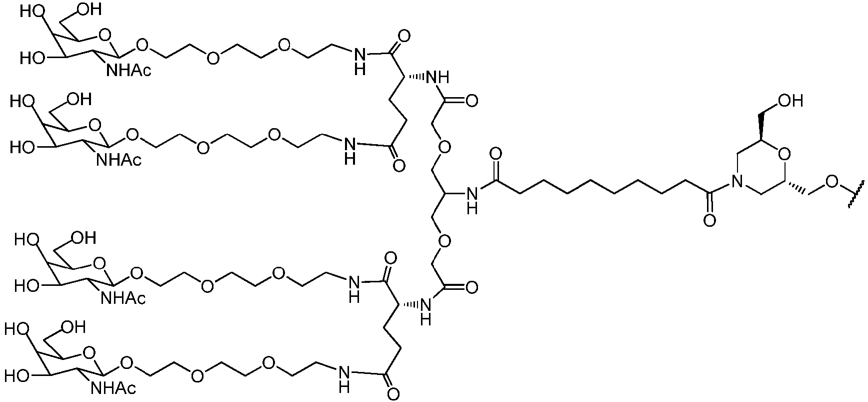 , wherein 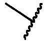 represents the position of attachment to the sense strand of the siRNA via a phosphate group or a phosphorothioate group.

In some specific embodiments, in the siRNA of the present disclosure, wherein:
(1) the sense strand comprises 
STM1s-AmsAmAmAmGmGmGmAmCfAfGfUmAmUmUmCmUmCmAmGmAms-
STM1s-GL6 (SEQ ID NO: 1238),
 and the antisense strand comprises
   (CP1a-U)sCfsUmGfAmGfAmAfUmAfCmUfGmUfCmCfCmUfUmsUfsUm (SEQ ID NO: 1651);
(2) the sense strand comprises 
STM1s-AmsAmUmUmAmAmAmAmGfGfGfAmCmAmGmUmAmUmUmCmAms-
STM1s-GL6 (SEQ ID NO: 1235),
 and the antisense strand comprises
   (CP1a-U)sGfsAmAfUmAfCmUfGmUfCmCfCmUfUmUfUmAfAmsUfsUm (SEQ ID NO: 1650);
(3) the sense strand comprises 
STM1s-AmsAmUmUmAmAmAmAmGfGfGfAmCmAmGmUmAmUmUmCmAms-
STM1s-GL6 (SEQ ID NO: 1235),
 and the antisense strand comprises
   (VPUm)sGfsAmAfUmAfCmUfGmUfCmCfCmUfUmUfUmAfAmsUfsUm (SEQ ID NO: 1649);
(4) the sense strand comprises 
IBs-AmsAmUmUmAmAmAmAmGfGfGfAmCmAmGmUmAmUmUmCmAms-
IBs-GL6 (SEQ ID NO: 1236),
 and the antisense strand comprises
   (CP1a-U)sGfsAmAfUmAfCmUfGmUfCmCfCmUfUmUfUmAfAmsUfsUm(SEQ ID NO: 1650);
(5) the sense strand comprises
   UmsUmsAmAmAmAmGfGfGfAmCmAmGmUmAmUmUmCmsAms-GL6(SEQ ID NO: 1237),
   and the antisense strand comprises
   (CP1a-U)sGfsAmAfUmAfCmUfGmUfCmCfCmUfUmUfUmAfAmsUfsUm(SEQ ID NO: 1650);
(6) the sense strand comprises 
IBs-AmsAmAmAmGmGmGmAmCfAfGfUmAmUmUmCmUmCmAmGmAms-IBs-
GL6 (SEQ ID NO: 1239),
 and the antisense strand comprises
   (CP1a-U)sCfsUmGfAmGfAmAfUmAfCmUfGmUfCmCfCmUfUmsUfsUm(SEQ ID NO: 1651);
(7) the sense strand comprises
   AmsAmsGmGmGmAmCfAfGfUmAmUmUmCmUmCmAmGmsAms-GL6(SEQ ID NO: 1240),
   and the antisense strand comprises
   (CP1a-U)sCfsUmGfAmGfAmAfUmAfCmUfGmUfCmCfCmUfUmsUfsUm(SEQ ID NO: 1651); or
(8) the sense strand comprises 
IBs-AmsAmAmAmGmGmGmAmCfAfGfUmAmUmUmCmUmCmAmGmAms-IBs-
GL6(SEQ ID NO: 1239),
 and the antisense strand comprises 
(CP1a-U)sCfsUmGfAmGf(PCN-
A)AfUmAfCmUfGmUfCmCfCmUfUmsUfsUm(SEQ ID NO: 1652);
 or
(9) the sense strand comprises
   AmsAmsGmGmGmAmCfAfGfUmAmUmUmCmUmCmAmGmsAms-GL6(SEQ ID NO: 1240),
   and the antisense strand comprises
   (CP1a-U)sCfsUmGfAmGf(PCN-A)AfUmAfCmUfGmUfCmCfCmUfUmsUfsUm(SEQ ID NO: 1652).

In a second aspect, the present invention provides a vector comprising a nucleotide sequence encoding the siRNA as disclosed herein.

In a third aspect, the present invention provides a cell comprising a siRNA or vector as disclosed herein.

In a fourth aspect, the present invention provides a pharmaceutical composition comprising a siRNA, vector, or cell disclosed herein, and optionally a pharmaceutically acceptable carrier or excipient.

In a fifth aspect, the present invention provides a kit comprising a siRNA, vector, cell, or pharmaceutical composition as disclosed herein.

In a sixth aspect, the present invention provides a method for treating diseases related to APOC3 in a subject, the method comprising a step of administering to the subject a siRNA, vector, cell, or pharmaceutical composition as disclosed herein.

In a seventh aspect, the present invention provides a method for reducing the risk of developing a disease related to APOC3 in a subject, the method comprising a step of administering to the subject a siRNA, vector, cell, or pharmaceutical composition as disclosed herein.

In some embodiments of the sixth and seventh aspects, the APOC3-related disease is selected from hyperlipidemia and hypertriglyceridemia. In some specific embodiments, the APOC3-related disease is a condition that can be caused by, associated with, or as a consequence of hypertriglyceridemia, such as non-alcoholic fatty liver disease, non-alcoholic steatohepatitis, polycystic ovary syndrome, kidney disease, obesity, type 2 diabetes mellitus, hypertension, atherosclerosis, cardiovascular disease, or pancreatitis.

In some embodiments, the method comprises administering subcutaneously, intravenously, or topically to the subject a siRNA, vector, cell, or pharmaceutical composition as disclosed herein.

In some embodiments, the subject is human.

### Detailed Description

The following describes the embodiments of the present invention through specific examples. Those skilled in the art can easily understand other advantages and effects of the present invention from the disclosure of the present specification. The present invention can also be implemented or applied through other different specific embodiments. Various details in this specification can also be modified or changed in various ways based on different viewpoints and applications without departing from the spirit of the present invention.

It should be understood that the protection scope of the present invention is not limited to the following specific embodiments; it should also be understood that the terms used in the embodiments of the present invention are for describing specific embodiments, not for limiting the protection scope of the present invention.

In the specification and claims of the present invention, the singular forms "a", "an", and "the" include plural referents unless the context clearly dictates otherwise.

When the examples give numerical ranges, it should be understood that, unless otherwise stated in the present invention, both endpoints of each numerical range and any value between the two endpoints can be selected. Unless defined otherwise, all technical and scientific terms used herein have the same meaning as commonly understood by one of ordinary skill in the art. In addition to the specific methods, equipment, and materials used in the embodiments, those skilled in the art can also use methods, equipment, and materials described in the embodiments of the present invention based on their understanding of the prior art and the description of the present invention. Any methods, equipment, and materials that are similar or equivalent to the prior art to implement the present invention shall fall within the protection scope of the present invention. Embodiments of the present invention are described in greater detail below.

### Definitions

The term "siRNA" herein is a class of dsRNA molecules each of which can mediate the silencing of target RNA (e.g., mRNA, e.g., transcript of a gene encoding a protein) complementary thereto. A siRNAs is generally double-stranded, including an antisense strand complementary to the target RNA thereof and a sense strand complementary to this antisense strand. For the sake of convenience, such an mRNA is also referred to herein as mRNA to be silenced, and such a gene is also called target gene.

As used herein, the term "antisense strand" refers to a strand of a siRNA, wherein said strand contains a region that is completely or substantially complementary to the target sequence thereof.

As used herein, the term "complementary region" refers to a region on an antisense strand that is completely or substantially complementary to the target mRNA sequence thereof. In cases where a complementary region is incompletely complementary to the target sequence thereof, a mismatch may be located in an internal or terminal region of the molecule. Typically, a mismatch most tolerant is located in a terminal region, e.g., within 5, 4, 3, 2 or 1 nucleotide at the 5' and/or 3' end. A region in an antisense strand, which is most sensitive to mismatch, is called "seed region". For example, in a siRNA containing a strand of 19 nt, the 19^{th} site (counting from the 5' end to the 3' end) can tolerate some mismatches.

As used herein, the term "complementary" refers to the ability of a first polynucleotide to hybridize with a second polynucleotide under certain conditions, such as stringent conditions. For example, stringent conditions may include 400 mM NaCl, 40 mM PIPES pH 6.4, 1 mM EDTA, and 50°C or 70°C for 12-16 hours.

As used herein, with respect to fulfilling the above required capabilities related to the hybridization ability thereof, said "complementary" sequences may also include or be entirely composed of non-Watson-Crick base pairs and/or base pairs formed from non-natural as well as modified nucleotides. Such non-Watson-Crick base pairs include, but are not limited to, G:U wobble base pairing or Hoogsteen base pairing.

As used herein, a polynucleotide that is "at least partially complementary" or "substantially complementary" to a messenger RNA (mRNA) refers to a polynucleotide that is substantially complementary to a contiguous portion of an mRNA of interest (e.g., an mRNA encoding APOC3). For example, a polynucleotide is at least partially complementary to an mRNA encoding APOC3, when the sequence thereof is substantially complementary to an uninterrupted portion of said APOC3 mRNA.

The terms "complementary," "completely complementary", and "substantially complementary" as used herein may be used with respect to base pairing between the sense strand and the antisense strand of a siRNA, or may be used with respect to base pairing between the antisense strand of a siRNA and a target sequence.

As used herein, the term "sense strand" refers to a strand of a siRNA, wherein the said strand contains a region that is substantially complementary to a region of an antisense strand as defined herein.

"Nucleoside" is a compound comprising two components: one is a purine base or a pyrimidine base, and the other is a ribose or a deoxyribose. "Nucleotide" is a compound comprising three components: one is a purine base or a pyrimidine base, another is a ribose or deoxyribose, and the third is a phosphoric acid. "Oligonucleotide" refers to, for example, a nucleic acid molecule (RNA or DNA) having a length of less than 100, 200, 300, or 400 nucleotides.

The term "base" is a fundamental building block of nucleosides, nucleotides and nucleic acids; as always containing nitrogen, said base is also referred to as "nitrogenous base." Unless otherwise specified, the capital letters herein, i.e., A, U, T, G and C, denote the bases of nucleotides, which is adenine, uracil, thymine, guanine and cytosine, respectively.

As used herein, the term "nucleotide overhang" or "overhang" refers to at least one unpaired nucleotide that protrudes from the duplex structure of the siRNA. Nucleotide overhangs exist, for example, when the 3'-end of one strand of the siRNA extends beyond the 5'-end of the other strand, or vice versa. The siRNA can comprise an overhang having at least one nucleotide; alternatively, the overhang can comprise at least two nucleotides, at least three nucleotides, at least four nucleotides, at least five nucleotides, or more. A nucleotide overhang may comprise or consist of a nucleotide/nucleoside analog (including a deoxynucleotide/nucleoside). One or more overhangs can be on the sense strand, the antisense strand, or any combination thereof. Additionally, one or more nucleotides of the overhang may be present at the 5'-end, 3'-end, or both ends of the antisense or sense strand of the siRNA.

"Blunt end" refers to that there are no unpaired nucleotides at that end of the double-stranded siRNA, i.e., there are no nucleotide overhangs at that end. A "blunt-ended siRNA" is a siRNA that is double-stranded throughout its length, i.e., there are no nucleotide overhangs at either end of the molecule. siRNAs of the present invention include a siRNA having a nucleotide overhang at one end (i.e., a reagent having one overhang and one blunt end) or having a nucleotide overhang at both ends.

Substantially all nucleotides of the iRNA of the present invention are modified. For example, substantially all nucleotides of the sense strand are modified nucleotides, and/or substantially all nucleotides of the antisense strand are modified nucleic acids, and/or substantially all nucleotides of both the sense and antisense strands are modified nucleotides. In other embodiments of the present invention, all nucleotides of the iRNA of the present invention are modified nucleotides. For example, all nucleotides of the sense strand are modified nucleotides, and/or all nucleotides of the antisense strand are modified nucleic acids, and/or all nucleotides of both the sense and antisense strands are modified nucleotides. The term "substantially all nucleotides are modified" refers to that the siRNA of the present invention is mostly but not entirely modified and may include no more than 5, 4, 3, 2, or 1 unmodified nucleotide.

"Modified nucleotides" herein include, but are not limited to: a 2'-O-methyl modified nucleotide, a 2'-fluoro modified nucleotide, a 2'-deoxy modified nucleotide, an inosine ribonucleotide, an abasic nucleotide, an inverted abasic deoxyribonucleotide, a phosphorothioate internucleotide linkage modification, a vinylphosphonate modified nucleotide, a locked nucleotide, a 2'-amino modified nucleotide, a 2'-alkyl modified nucleotide, a morpholino nucleotide, a phosphoramidate, a non-natural base comprising nucleotide, and a terminal nucleotide linked to a cholesteryl derivative or a dodecanoic acid bisdecylamide group, deoxyribonucleotide, or a nucleotide with protection of a conventional protecting group. For example, a 2'-fluoro modified nucleotide refers to a nucleotide formed by substituting the hydroxyl at the 2' position of the ribosyl in a nucleotide with a fluorine atom. Said 2'-deoxy-modified nucleotide refers to a nucleotide formed by substituting the 2'-hydroxyl of the ribosyl with a methoxyl.

As used herein, the term "ligand moiety" refers to a chemical component conjugated with a siRNA, wherein the moiety is capable of changing the distribution, targeting, or lifespan of the siRNA. In a preferred embodiment, such a ligand offers enhanced affinity for selected targets, such as molecules, cells or cell types, and compartments (e.g., cellular or organ compartments, tissues, organs, or regions of the body), compared to for example a siRNA without such a ligand.

As used herein, the term "inhibition" is used interchangeably with "reduction," "silencing", "downregulation", and other similar terms and includes any level of inhibition.

The phrase "inhibiting the expression of APOC3" is intended to refer to inhibiting the expression of any APOC3 gene as well as variants or mutants of the APOC3 gene. Thus, the APOC3 gene may be a wild-type APOC3 gene, a mutant APOC3 gene, or in the case of a genetically manipulated cell, cell population, or organism, a transgenic APOC3 gene.

"Inhibition of APOC3 gene expression" includes any level of inhibition of the APOC3 gene, such as at least partial inhibition of APOC3 gene expression. APOC3 gene expression can be assessed based on the level or change in the level of any variable associated with APOC3 gene expression, such as APOC3 mRNA levels, APOC3 protein levels, or lipid levels. This level can be assessed in an individual cell or population of cells (including, for example, samples derived from a subject).

Inhibition can be assessed by a reduction in absolute or relative levels of one or more variables associated with APOC3 expression compared to control levels. The control level can be any type of control level utilized in the art, such as a pre-dose baseline level or a level determined from a similar untreated or control (e.g., a buffer-only control or an inert control)-treated subject, cell, or sample.

"Hydroxy-protecting group" refers to a group that can prevent a hydroxyl from undergoing chemical reactions and can be removed under specific conditions to restore the hydroxyl. The main hydroxy-protecting groups include silane-type, acyl-type, or ether-type protecting groups, preferably the following: trimethylsilyl (TMS), triethylsilyl (TES), dimethylisopropylsilyl (DMIPS), diethylisopropylsilyl (DEIPS), tert-butyldimethylsilyl (TBDMS), tert-butyldiphenylsilyl (TBDPS), triisopropylsilyl (TIPS), acetyl (Ac), chloroacetyl, dichloroacetyl, trichloroacetyl, trifluoroacetyl (TFA), benzoyl, p-methoxybenzoyl, 9-fluorenylmethoxycarbonyl (Fmoc), allyloxycarbonyl (Alloc), 2,2,2-trichloroethoxycarbonyl (Troc), benzyloxycarbonyl (Cbz), tert-butoxycarbonyl (Boc), benzyl (Bn), p-methoxybenzyl (PMB), allyl, triphenylmethyl (Tr), di-p-methoxytrityl (DMTr), methoxymethyl (MOM), benzyloxymethyl (BOM), 2,2,2-trichloroethoxymethyl, 2-methoxyethoxymethyl (MEM), methylthiomethyl (MTM), p-methoxybenzyloxymethyl (PMBM).

"Halo-" or "halogen" refers to (substitution by) fluorine (F), chlorine (Cl), bromine (Br), and iodine (I).

"C₁₋₆ haloalkyl" refers to the aforementioned "C₁₋₆ alkyl", with one or more halogen groups. In some embodiments, a C₁₋₄ haloalkyl is particularly preferred, and a C₁₋₂ haloalkyl is even more preferred. Exemplary haloalkyls include, but are not limited to: -CF₃, -CH₂F, -CHF₂, -CHFCH₂F, -CH₂CHF₂, -CF₂CF₃, -CCl₃, -CH₂Cl, -CHCl₂, and 2,2,2-trifluoro-1,1-dimethyl-ethyl. The haloalkyls may be substituted at any substitutable connection site, for example, 1 to 5 substituents, 1 to 3 substituents, or 1 substituent.

"C₁₋₆ alkylene" refers to a divalent group formed by removing another hydrogen of C₁₋₆ alkyl and can be substituted or unsubstituted. In some embodiments, C₁₋₄ alkylene, C₂₋₄ alkylene, and C₁₋₂ alkylene are preferred. The unsubstituted alkylenes include, but are not limited to: methylene group (-CH₂-), ethylene group (-CH₂CH₂-), propylene group (-CH₂CH₂CH₂-), butylene group (-CH₂CH₂CH₂CH₂-), pentylene group (-CH₂CH₂CH₂CH₂CH₂-), and hexylene group (-CH₂CH₂CH₂CH₂CH₂CH₂-). Examples of said substituted alkylenes, such as a alkylene substituted by one or more alkyl (methyl) groups, include but are not limited to: substituted methylene (-CH(CH₃)-, and -C(CH₃)₂-), substituted ethylene (-CH(CH₃)CH₂-, -CH₂CH(CH₃)-, -C(CH₃)₂CH₂-, and -CH₂C(CH₃)₂-), substituted propylene (-CH(CH₃)CH₂CH₂-, -CH₂CH(CH₃)CH₂-, - CH₂CH₂CH(CH₃)-, -C(CH₃)₂CH₂CH₂-, -CH₂C(CH₃)₂CH₂-, and -CH₂CH₂C(CH₃)₂-).

As used herein, the term "vector" refers to a nucleic acid molecule capable of amplifying or expressing another nucleic acid to which it is linked.

### I. siRNA

The present invention provides a small interfering RNA (siRNA) for inhibiting the expression of apolipoprotein C3 (APOC3) in cells, the siRNA comprising a sense strand and an antisense strand forming a double-stranded region, wherein the length of the sense strand and the antisense strand is each independently 15-30 nucleotides, and the antisense strand comprises at least 15 contiguous nucleotides from a nucleotide sequence as set forth in any one of SEQ ID NOs: 414-826.

In some embodiments, the double-stranded region formed by the sense strand and the antisense strand is completely complementary. In other embodiments, the double-stranded region formed by the sense strand and the antisense strand is substantially complementary and may contain 1, 2, 3, 4, or 5 non-complementary sites.

In some specific embodiments, the sense strand comprises at least 15 contiguous nucleotides from a nucleotide sequence as set forth in any one of SEQ ID NOs: 1-413.

In some embodiments, the length of the sense strand and the antisense strand is each independently 17-27 nucleotides, preferably 19-25 nucleotides, more preferably 21-23 nucleotides.

In some embodiments, the double-stranded region is 15-25 base pairs in length, preferably 17-21 base pairs, more preferably 19 base pairs.

One or both of the sense strand and the antisense strand comprise a 3' overhang and/or a 5' overhang of at least 1 nucleotide; for example, one or both of the sense strand and the antisense strand comprise a 3' overhang and/or a 5' overhang of at least 2 nucleotides. In some preferred embodiments, the antisense strand has a 3' overhang and/or a 5' overhang of at least 1 nucleotide. For example, the antisense strand includes a 3' overhang and/or a 5' overhang having 1, 2, or 3 nucleotides. In some preferred embodiments, the sense strand has a 3' overhang and/or a 5' overhang of at least 1 nucleotide. For example, the sense strand includes a 3' overhang and/or a 5' overhang having 1, 2, or 3 nucleotides.

In some embodiments, the sense strand and the antisense strand are the same in length. In some embodiments, the full length of the sense strand is complementary to the full length of the antisense strand to form a double strand with blunt ends. In some other embodiments, the sense strand and the antisense strand are the same in length, and a portion of the sense strand is complementary to a portion of the antisense strand, that is, both the sense strand and the antisense strand have a 5' overhang.

In some embodiments, the sense strand and the antisense strand are different in length. In a preferred embodiment, the 5' end of the antisense strand has an overhang of at least 1 nucleotide, more preferably 2 or 3 nucleotides.

In some embodiments, the antisense strand comprises at least 16 contiguous nucleotides, at least 17 contiguous nucleotides, at least 18 contiguous nucleotides, at least 19 contiguous nucleotides, or at least 20 contiguous nucleotides from a nucleotide sequence as set forth in any one of SEQ ID NOs: 414-826, preferably the antisense strand comprises a nucleotide sequence as set forth in any one of SEQ ID NOs: 414-826.

In some embodiments, the sense strand comprises at least 16 contiguous nucleotides, at least 17 contiguous nucleotides, at least 18 contiguous nucleotides, at least 19 contiguous nucleotides, or at least 20 contiguous nucleotides from a nucleotide sequence as set forth in any one of SEQ ID NOs: 1-413, preferably the antisense strand comprises a nucleotide sequence as set forth in any one of SEQ ID NOs: 1-413.

In some embodiments, the siRNA comprises paired sense and antisense strand sequences as shown in Table 3.

In some embodiments, the antisense strand comprises at least 15 contiguous nucleotides, at least 16 contiguous nucleotides, at least 17 contiguous nucleotides, at least 18 contiguous nucleotides, at least 19 contiguous nucleotides, or at least 20 contiguous nucleosides from a nucleotide sequence set forth in any one of SEQ ID NOs: 473, 612, 690, 757, 761, 816, 817, 818, 819, 820, 814, and 815; preferably, the antisense strand comprises a nucleotide sequence as set forth in any one of SEQ ID NOs: 473, 612, 690, 757, 761, 816, 817, 818, 819, 820, 814 and 815.

In some embodiments, the sense strand comprises at least 15 contiguous nucleotides, at least 16 contiguous nucleotides, at least 17 contiguous nucleotides, at least 18 contiguous nucleotides, at least 19 contiguous nucleotides, or at least 20 contiguous nucleosides from a nucleotide sequence set forth in any one of SEQ ID NOs: 60, 199, 277, 344, 348, 403, 404, 405, 406, 407, 401, and 402, preferably the antisense strand comprises a nucleotide sequence as set forth in any one of SEQ ID NOs: 60, 199, 277, 344, 348, 403, 404, 405, 406, 407, 401, and 402.

In some embodiments,
(a) the sense strand comprises a nucleotide sequence shown in SEQ ID NO: 60, and the antisense strand comprises a nucleotide sequence shown in SEQ ID NO: 473;
(b) the sense strand comprises a nucleotide sequence shown in SEQ ID NO: 199, and the antisense strand comprises a nucleotide sequence shown in SEQ ID NO: 612
(c) the sense strand comprises a nucleotide sequence shown in SEQ ID NO: 277, and the antisense strand comprises a nucleotide sequence shown in SEQ ID NO: 690;
(d) the sense strand comprises a nucleotide sequence shown in SEQ ID NO: 344, and the antisense strand comprises a nucleotide sequence shown in SEQ ID NO: 757;
(e) the sense strand comprises a nucleotide sequence shown in SEQ ID NO: 348, and the antisense strand comprises a nucleotide sequence shown in SEQ ID NO: 761;
(f)the sense strand comprises a nucleotide sequence shown in SEQ ID NO: 403, and the antisense strand comprises a nucleotide sequence shown in SEQ ID NO: 816;
(g) the sense strand comprises a nucleotide sequence shown in SEQ ID NO: 404, and the antisense strand comprises a nucleotide sequence shown in SEQ ID NO: 817;
(h) the sense strand comprises a nucleotide sequence shown in SEQ ID NO: 405, and the antisense strand comprises a nucleotide sequence shown in SEQ ID NO: 818;
(i) the sense strand comprises a nucleotide sequence shown in SEQ ID NO: 406, and the antisense strand comprises a nucleotide sequence shown in SEQ ID NO: 819;
(j) the sense strand comprises a nucleotide sequence shown in SEQ ID NO: 407, and the antisense strand comprises a nucleotide sequence shown in SEQ ID NO: 820;
(k) the sense strand comprises a nucleotide sequence shown in SEQ ID NO: 401, and the antisense strand comprises a nucleotide sequence shown in SEQ ID NO: 814; or
(l) the sense strand comprises a nucleotide sequence shown in SEQ ID NO: 402, and the antisense strand comprises a nucleotide sequence shown in SEQ ID NO: 815.

### II. Nucleotide Modification

In some embodiments, substantially all nucleotides of the sense strand and substantially all nucleotides of the antisense strand are modified nucleotides. In some embodiments, at least 80% of the nucleotides of the sense strand are modified nucleotides, and/or at least 80% of the nucleotides of the antisense strand are modified nucleotides.

In some embodiments, all nucleotides of the sense strand and/or all nucleotides of the antisense strand are modified nucleotides.

The modification of a nucleotide described in the present invention may be a modification at the phosphate group, ribose group, and/or base group of the nucleotide.

In some specific embodiments, the sense strand and the antisense strand each independently comprise one or more nucleotide modifications selected from the group consisting of: a 2'-O-methyl modified nucleotide, a 2'-fluoro modified nucleotide, a 2'-deoxy modified nucleotide, an inosine ribonucleotide, an abasic nucleotide, an inverted abasic deoxyribonucleotide, a phosphorothioate internucleotide linkage modification, a vinylphosphonate modified nucleotide, a locked nucleotide, a 2'-amino modified nucleotide, a 2'-alkyl modified nucleotide, a morpholino nucleotide, a phosphoramidate, a non-natural base comprising nucleotide, and a terminal nucleotide linked to a cholesteryl derivative or a dodecanoic acid bisdecylamide group, and a deoxyribonucleotide.

In some preferred embodiments, the sense strand and the antisense strand each independently comprise one or more nucleotide modifications selected from the group consisting of: a 2'-O-methyl modified nucleotide, a 2'-fluoro modified nucleotide, a 2'-deoxy modified nucleotide, an inosine ribonucleotide, an abasic nucleotide, an inverted abasic deoxyribonucleotide, and a phosphorothioate internucleotide linkage modification.

In some preferred embodiments, the sense strand and/or the antisense strand comprises at least two 2'-fluoro modified nucleotides.

In some preferred embodiments, the sense strand and/or the antisense strand comprises at least eight 2'-O-methyl modified nucleotides.

In some preferred embodiments, the 3' end and/or 5' end of the sense strand and/or the antisense strand comprises 1 to 5 phosphorothioate groups, preferably 2 to 3 phosphorthioate groups. In some more preferred embodiments, the sense strand and/or the antisense strand comprises 1 to 5 phosphorothioate groups at the 5' end.

In some preferred embodiments, the antisense strand comprises a modified nucleotide sequence set forth in any one of Table 5, and/or the sense strand comprises a modified nucleotide sequence set forth in any one of Table 4. In some preferred embodiments, the siRNA comprises a paired modified sense strand sequence and modified antisense strand sequence set forth in any one of Table 6.

In some embodiments, in the siRNA that inhibits APOC3 expression in a cell of the present invention:
(1) the sense strand comprises AmsAmAmAmGmGmGmAmCfAfGfUmAmUmUmCmUmCmAmGmAms-STM1(SEQ ID NO: 1655), and the antisense strand comprises (CP1a-U)sCfsUmGfAmGfAmAfUmAfCmUfGmUfCmCfCmUfUmsUfsUm(SEQ ID NO: 1651);
(2) the sense strand comprises STM1s-AmsAmUmUmAmAmAmAmGfGfGfAmCmAmGmUmAmUmUmCmAms-STM1 (SEQ ID NO: 1656), and the antisense strand comprises (CP1a-U)sGfsAmAfUmAfCmUfGmUfCmCfCmUfUmUfUmAfAmsUfsUm;
(3) the sense strand comprises STM1s-AmsAmUmUmAmAmAmAmGfGfGfAmCmAmGmUmAmUmUmCmAms-STM1(SEQ ID NO: 1657), and the antisense strand comprises (VPUm)sGfsAmAfUmAfCmUfGmUfCmCfCmUfUmUfUmAfAmsUfsUm (SEQ ID NO: 1649);
(4) the sense strand comprises IBs-AmsAmUmUmAmAmAmAmGfGfGfAmCmAmGmUmAmUmUmCmAms-IB (SEQ ID NO: 1658), and the antisense strand comprises (CP1a-U)sGfsAmAfUmAfCmUfGmUfCmCfCmUfUmUfUmAfAmsUfsUm(SEQ ID NO: 1650);
(5) the sense strand comprises UmsUmsAmAmAmAmGfGfGfAmCmAmGmUmAmUmUmCmsAm (SEQ ID NO: 1659), and the antisense strand comprises (CP1a-U)sGfsAmAfUmAfCmUfGmUfCmCfCmUfUmUfUmAfAmsUfsUm(SEQ ID NO: 1650);
(6) the sense strand comprises IBs-AmsAmAmAmGmGmGmAmCfAfGfUmAmUmUmCmUmCmAmGmAms-IB (SEQ ID NO: 1660), and the antisense strand comprises (CP1a-U)sCfsUmGfAmGfAmAfUmAfCmUfGmUfCmCfCmUfUmsUfsUm(SEQ ID NO: 1651);
(7) the sense strand comprises AmsAmsGmGmGmAmCfAfGfUmAmUmUmCmUmCmAmGmsAm (SEQ ID NO: 1661), and the antisense strand comprises (CP1a-U)sCfsUmGfAmGfAmAfUmAfCmUfGmUfCmCfCmUfUmsUfsUm(SEQ ID NO: 1651);
(8) the sense strand comprises IBs-AmsAmAmAmGmGmGmAmCfAfGfUmAmUmUmCmUmCmAmGmAms-IBs-GL6(SEQ ID NO: 1662), and the antisense strand comprises (CP1a-U)sCfsUmGfAmGf(PCN-A)AfUmAfCmUfGmUfCmCfCmUfUmsUfsUm(SEQ ID NO: 1652); or
(9) the sense strand comprises AmsAmsGmGmGmAmCfAfGfUmAmUmUmCmUmCmAmGmsAm (SEQ ID NO: 1663), and the antisense strand comprises (CP1a-U)sCfsUmGfAmGf(PCN-A)AfUmAfCmUfGmUfCmCfCmUfUmsUfsUm(SEQ ID NO: 1652);
   (a) the sense strand comprises CmsGmsAmGmGmAmUfGfCfCmUmCm CmCmUmUmCmUmUm, and the antisense strand comprises AmsAfsGmAfAmGfG mGfAmGfGmCfAmUfCmCfUmCfGmsUfsUm;
   (b) the sense strand comprises CmsCmsGmUmUmAmAfGfGfAmCmAm AmGmUmUmCmUmUm, and the antisense strand comprises AmsAfsGmAfAmCfU mUfGmUfCmCfUmUfAmAfCmGfGmsUfsUm;
   (c) the sense strand comprises CmsCmsGmUmUmAmAfGfGfAmCmAm AmGmUmUmCmUmUm, and the antisense strand comprises AmsAfsGmAfAmCfU mUfGmUfCmCfUmUfAmAfCmGfGmsUfsUm;
   (d) the sense strand comprises CmsCmsAmAmGmUmCfCfAfCmCmUmG mCmCmUmAmUmUm, and the antisense strand comprises AmsAfsUmAfGmGfCm AfGmGfUmGfGmAfCmUfUmGfGmsUfsUm;
   (e) the sense strand comprises AmsAmsGmGmGmAmCfAfGfUmAmUm UmCmUmCmAmGmUm, and the antisense strand comprises AmsCfsUmGfAmGfA mAfUmAfCmUfGmUfCmCfCmUfUmsUfsUm;
   (f) the sense strand comprises UmsUmsAmAmAmAmGfGfGfAmCmAm GmUmAmUmUmCmUm, and the antisense strand comprises AmsGfsAmAfUmAfC mUfGmUfCmCfCmUfUmUfUmAfAmsUfsUm;
   (g) the sense strand comprises AmsAmsGmGmGmAmCfAfGfUmAmUm UmCmUmCmAmGmUm, and the antisense strand comprises AmsCfsUmGfAmGfA mAfUmAfCmUfGmUfCmCfCmUfUmsUfsUm;
   (h) the sense strand comprises CmsCmsAmAmGmUmCfCfAfCmCmUmG mCmCmUmAmUmUm, and the antisense strand comprises AmsAfsUmAfGmGfCm AfGmGfUmGfGmAfCmUfUmGfGmsUfsUm;
   (i) the sense strand comprises CmsCmsGmUmUmAmAfGfGfAmCmAm AmGmUmUmCmUmUm, and the antisense strand comprises AmsAfsGmAfAmCfU mUfGmUfCmCfUmUfAmAfCmGfGmsUfsUm;
   (j) the sense strand comprises CmsGmsAmGmGmAmUfGfCfCmUmCm CmCmUmUmCmUmUm, and the antisense strand comprises AmsAfsGmAfAmGfG mGfAmGfGmCfAmUfCmCfUmCfGmsUfsUm;
   (k) the sense strand comprises IBs-AmCmGmGmGmAmCmAmGfUfAfU mUmCmUmCmAmGmUmimAms-IB, and the antisense strand comprises UmsCfsA msCfUmGfAmGmAmAmUmAfCmUfGmUfCmCfCmGfsUm; or
   (1) the sense strand comprises AmsAmsGmGmGmAmCfAmGfUfAfUmU mCmUmCmAmGmUmsGmsCm, and the antisense strand comprises GmsCfsAmCm UmGfAmGmAmAmUmAmCmUfGmUfCmCmCmUmUmsUmsUm.

### III. Ligand

The siRNA of the present invention is further conjugated to a ligand moiety comprising N-acetylgalactosamine via a phosphate group or a phosphorothioate group. In a preferred embodiment, the sense strand of the siRNA is conjugated to the ligand moiety via a phosphate group or a phosphorothioate group. In some preferred embodiments, the 3' end of the sense strand is conjugated to the ligand moiety via a phosphate group or a phosphorothioate group. In some other preferred embodiments, the 5' end of the sense strand is conjugated to the ligand moiety via a phosphate group or a phosphorothioate group.

In some embodiments, the ligand moiety comprises a conjugation group of formula (X'): 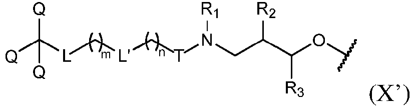 wherein, 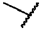 represents the position of attachment to siRNA;
Q is independently H, 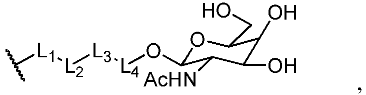 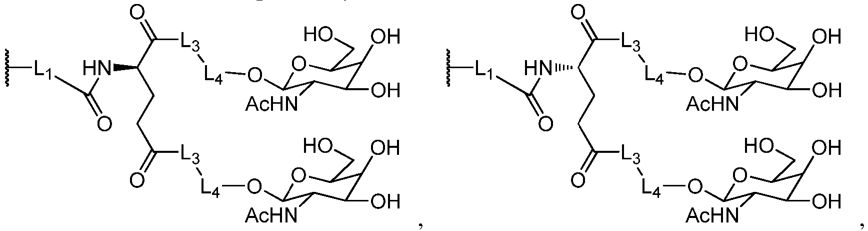 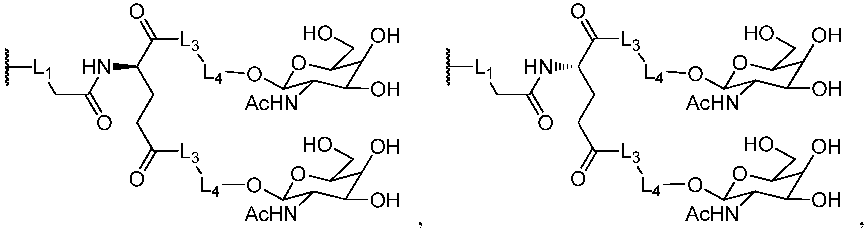 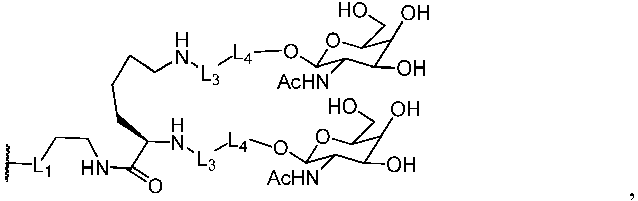 or 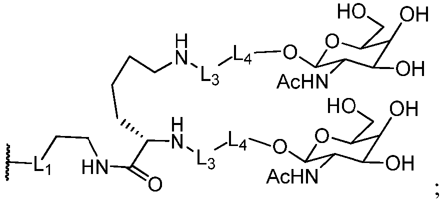
   wherein L₁ is a bond, -CH₂-, -CH₂CH₂-, -C(O)-, -CH₂O-, -CH₂O-CH₂CH₂O-, or -NHC(O)-(CH₂NHC(O))ₐ-;
   L₂ is a bond or -CH₂CH₂C(O)-;
   L₃ is a bond, -(NHCH₂CH₂)_{b}-, -(NHCH₂CH₂CH₂)_{b}-, or -C(O)CH₂-;
   L₄ is -(OCH₂CH₂)_{c}-, -(OCH₂CH₂CH₂)_{c}-, -(OCH₂CH₂CH₂CH₂)_{c}-, - (OCH₂CH₂CH₂CH₂CH₂)_{c}-, or -NHC(O)-(CH₂)_{d}-;
      wherein a = 0, 1, 2, or 3;
      b = 1, 2, 3, 4 or 5;
      c = 1, 2, 3, 4 or 5;
      d = 1, 2, 3, 4, 5, 6, 7, or 8; L is a bond, -CH₂O-, or -NHC(O)-; L' is a bond, -C(O)NH-, -NHC(O)-, or -O(CH₂CH₂O)ₑ-;
   wherein e is 1, 2, 3, 4, or 5;
T is a bond, -CH₂-, -C(O)-, -M-, -CH₂-M-, or -C(O)-M-;
   wherein M is 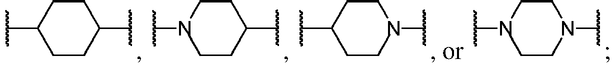
R₁ and R₂ together form -CH₂CH₂O- or -CH₂CH(R)-O-, and R₃ is H;
or R₁ and R₃ together form -C₁₋₂ alkylene-, and R₂ is H;
   wherein R is -OR', -CH₂OR', or -CH₂CH₂OR'; wherein R' is H, a hydroxy-protecting group, or a solid support, and the hydroxy-protecting group is preferably -C(O)CH₂CH₂C(O)OH or 4,4'-dimethoxytrityl;
m = 0, 1, 2, 3, 4, 5, 6, 7, 8, 9 or 10;
n = 0, 1, 2, 3, 4, 5, 6, 7, 8, 9 or 10.

In some embodiments, the conjugation group is as shown in formula (I'): 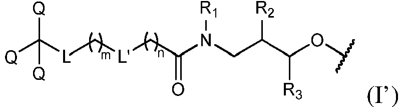 wherein, 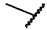 represents the position of attachment to siRNA;
Q is independently H, 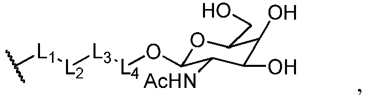 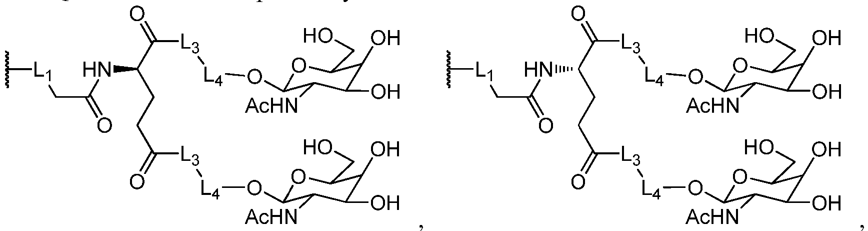 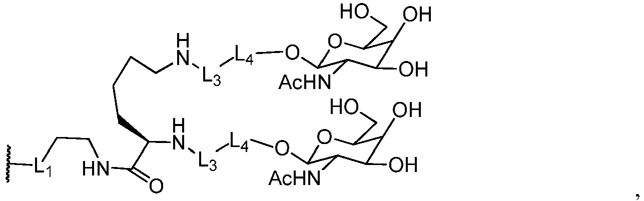 or 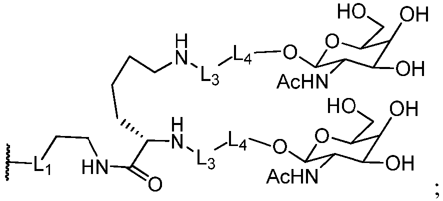
   wherein L₁ is a bond, -CH₂-, -CH₂CH₂-, -C(O)-, -CH₂O-, -CH₂O-CH₂CH₂O-, or -NHC(O)-(CH₂NHC(O))ₐ-;
   L₂ is a bond or -CH₂CH₂C(O)-;
   L₃ is a bond, -(NHCH₂CH₂)_{b}-, -(NHCH₂CH₂CH₂)_{b}-, or -C(O)CH₂-;
   L₄ is -(OCH₂CH₂)_{c}-, -(OCH₂CH₂CH₂)_{c}-, -(OCH₂CH₂CH₂CH₂)_{c}-, - (OCH₂CH₂CH₂CH₂CH₂)_{c}-, or -NHC(O)-(CH₂)_{d}-;
      wherein a = 0, 1, 2, or 3;
      b = 1, 2, 3, 4 or 5;
      c = 1, 2, 3, 4 or 5;
      d = 1, 2, 3, 4, 5, 6, 7, or 8;
L is -CH₂O- or -NHC(O)-;
L' is a bond, -C(O)NH- or -NHC(O)-;
R₁ and R₂ together form -CH₂CH₂O- or -CH₂CH(R)-O-, and R₃ is H;
or R₁ and R₃ together form -C₁₋₂ alkylene-, and R₂ is H;
   wherein R is -OR', -CH₂OR', or -CH₂CH₂OR'; wherein R' is H, a hydroxy-protecting group, or a solid support, and the hydroxy-protecting group is preferably -C(O)CH₂CH₂C(O)OH or 4,4'-dimethoxytrityl;
m = 0, 1, 2, 3, 4, 5, 6, 7, 8, 9 or 10;
n = 0, 1, 2, 3, 4, 5, 6, 7, 8, 9 or 10.

In some specific embodiments,
Wherein,
Q is independently H or 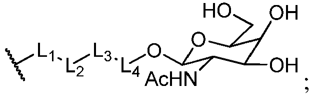
   wherein L₁ is -CH₂O- or -NHC(O)-(CH₂NHC(O))ₐ-;
   L₂ is -CH₂CH₂C(O)-;
   L₃ is -(NHCH₂CH₂)_{b}- or -(NHCH₂CH₂CH₂)_{b}-;
   L₄ is -(OCH₂CH₂)_{c}- or -NHC(O)-(CH₂)_{d}-;
      wherein a = 0, 1, 2, or 3;
      b = 1, 2, 3, 4 or 5;
      c = 1, 2, 3, 4 or 5;
      d = 1, 2, 3, 4, 5, 6, 7, or 8;
L is -CH₂O-;
L' is a bond;
R₁ and R₂ together form -CH₂CH₂O- or -CH₂CH(R)-O-, and R₃ is H;
or R₁ and R₃ together form -C₁₋₂ alkylene-, and R₂ is H;
   wherein R is -OR', -CH₂OR', or -CH₂CH₂OR'; wherein R' is H, a hydroxy-protecting group, or a solid support, and the hydroxy-protecting group is preferably -C(O)CH₂CH₂C(O)OH or 4,4'-dimethoxytrityl;
m = 0, 1, 2, 3, 4, 5, 6, 7, 8, 9 or 10;
n = 0, 1, 2, 3, 4, 5, 6, 7, 8, 9 or 10.

In some embodiments, the conjugation group is as shown in formula (I'-1), formula (I'-2), or formula (I'-3): 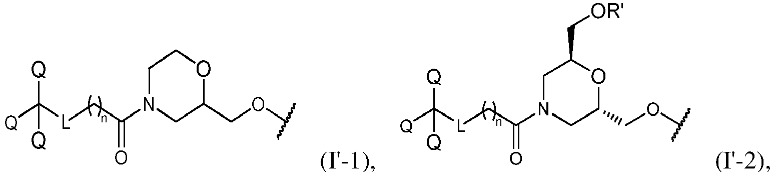 or 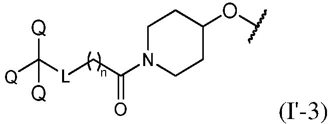 wherein, 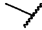 represents the position of attachment to siRNA;
Q is 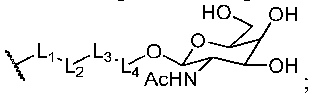
   wherein L₁ is -CH₂O- or -NHC(O)-;
   L₂ is -CH₂CH₂C(O)-;
   L₃ is -(NHCH₂CH₂)_{b}- or -(NHCH₂CH₂CH₂)_{b}-;
   L₄ is -(OCH₂CH₂)_{c}- or -NHC(O)-(CH₂)_{d}-;
      wherein b=1, 2, 3, 4, or 5;
      c = 1, 2, 3, 4 or 5;
      d = 1, 2, 3, 4, 5, 6, 7, or 8;
L is -CH₂O-;
R' is H, a hydroxy-protecting group, or a solid support, wherein the hydroxy-protecting group is preferably -C(O)CH₂CH₂C(O)OH or 4,4'-dimethoxytrityl;
n = 0, 1, 2, 3, 4, 5, 6, 7, 8, 9 or 10.

In some specific embodiments,
Wherein,
Q is independently H, 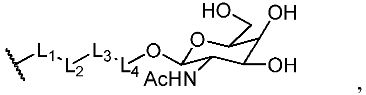 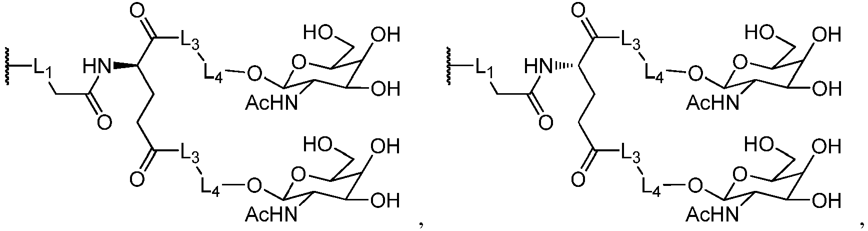 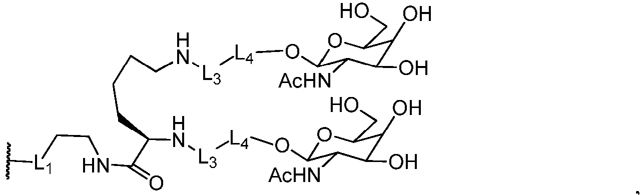 or 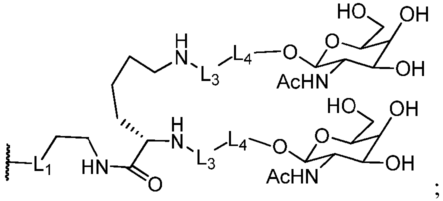
   wherein L₁ is -CH₂O-, -CH₂O-CH₂CH₂O-, or -NHC(O)-(CH₂NHC(O))ₐ-;
   L₂ is -CH₂CH₂C(O)-;
   L₃ is -(NHCH₂CH₂)_{b}-, -(NHCH₂CH₂CH₂)_{b}-, or -C(O)CH₂-;
   L₄ is -(OCH₂CH₂)_{c}- or -NHC(O)-(CH₂)_{d}-;
      wherein a = 0, 1, 2, or 3;
      b = 1, 2, 3, 4 or 5;
      c = 1, 2, 3, 4 or 5;
      d = 1, 2, 3, 4, 5, 6, 7, or 8;
L is -CH₂O- or -NHC(O)-;
L' is a bond or -C(O)NH-;
R₁ and R₂ together form -CH₂CH₂O- or -CH₂CH(R)-O-, and R₃ is H;
or R₁ and R₃ together form -C₁₋₂ alkylene-, and R₂ is H;
   wherein R is -OR', -CH₂OR', or -CH₂CH₂OR'; wherein R' is H, a hydroxy-protecting group, or a solid support, and the hydroxy-protecting group is preferably -C(O)CH₂CH₂C(O)OH or 4,4'-dimethoxytrityl;
m = 0, 1, 2, 3, 4, 5, 6, 7, 8, 9 or 10;
n = 0, 1, 2, 3, 4, 5, 6, 7, 8, 9 or 10.

In some embodiments, the conjugation group is as shown in formula (II'-1) or formula (II'-2): 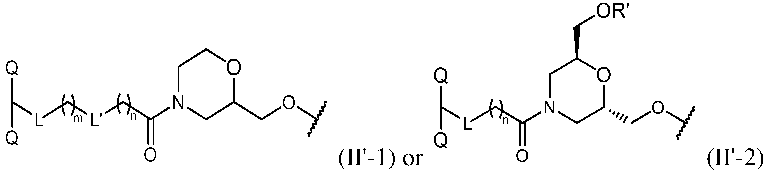 wherein, 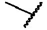 represents the position of attachment to siRNA;
Q is independently 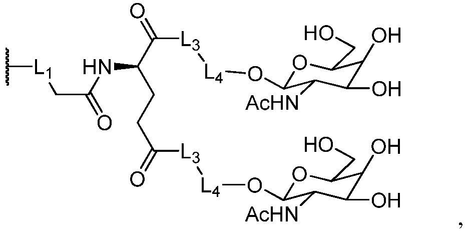 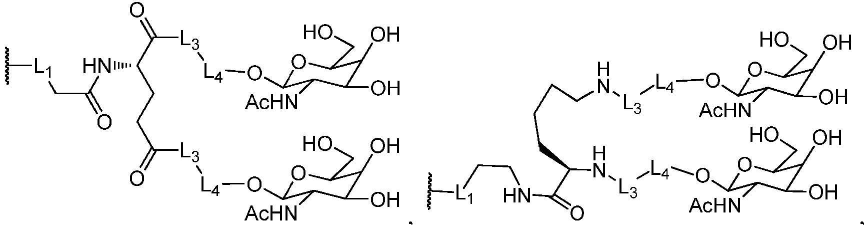 or 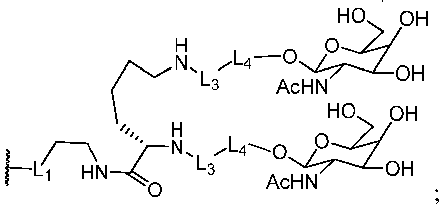
   wherein L₁ is -CH₂O- or -CH₂O-CH₂CH₂O-;
   L₃ is -(NHCH₂CH₂)_{b}-, -(NHCH₂CH₂CH₂)_{b}-, or -C(O)CH₂-;
   L₄ is -(OCH₂CH₂)_{c}- or -NHC(O)-(CH₂)_{d}-;
      wherein b=1, 2, 3, 4, or 5;
      c = 1, 2, 3, 4 or 5;
      d = 1, 2, 3, 4, 5, 6, 7, or 8;
L is -NHC(O)-;
L' is a bond or -C(O)NH-;
R' is H, a hydroxy-protecting group, or a solid support, wherein the hydroxy-protecting group is preferably -C(O)CH₂CH₂C(O)OH or 4,4'-dimethoxytrityl;
m 0, 1, 2, 3, 4, 5, 6, 7, 8, 9 or 10;
n = 0, 1, 2, 3, 4, 5, 6, 7, 8, 9 or 10.

In some specific embodiments, wherein:
Q is independently H, 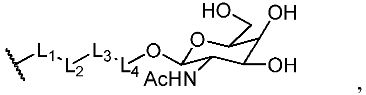 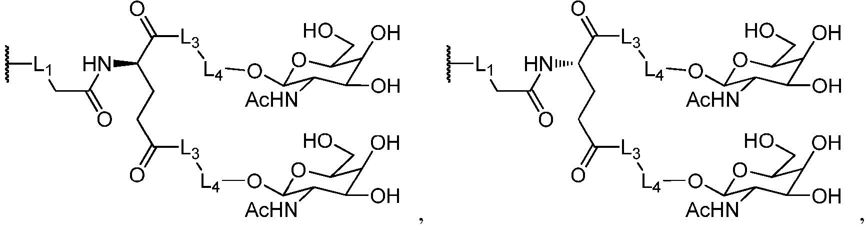 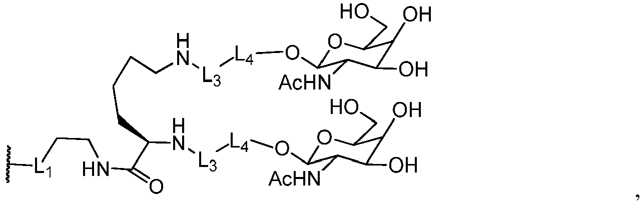 or 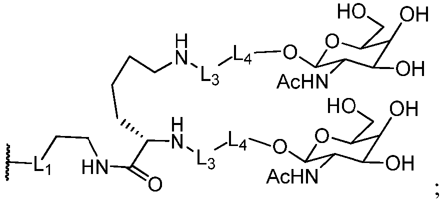
   wherein L₁ is -CH₂-, -C(O)-, -CH₂O-, -CH₂O-CH₂CH₂O-, or -NHC(O)-(CH₂NHC(O))ₐ-;
   L₂ is a bond;
   L₃ is -(NHCH₂CH₂)_{b}-, -(NHCH₂CH₂CH₂)_{b}-, or -C(O)CH₂-;
   L₄ is -(OCH₂CH₂)_{c}- or -NHC(O)-(CH₂)_{d}-;
      wherein a = 0, 1, 2, or 3;
      b = 1, 2, 3, 4 or 5;
      c = 1, 2, 3, 4 or 5;
      d = 1, 2, 3, 4, 5, 6, 7, or 8;
L is -CH₂O- or -NHC(O)-;
L' is a bond or -C(O)NH-;
R₁ and R₂ together form -CH₂CH₂O- or -CH₂CH(R)-O-, and R₃ is H;
or R₁ and R₃ together form -C₁₋₂ alkylene-, and R₂ is H;
   wherein R is -OR', -CH₂OR', or -CH₂CH₂OR'; wherein R' is H, a hydroxy-protecting group, or a solid support, and the hydroxy-protecting group is preferably -C(O)CH₂CH₂C(O)OH or 4,4'-dimethoxytrityl;
m = 0, 1, 2, 3, 4, 5, 6, 7, 8, 9 or 10;
n = 0, 1, 2, 3, 4, 5, 6, 7, 8, 9 or 10.

In some embodiments, the conjugation group is as shown in formula (II'-2): 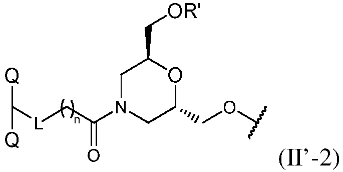 wherein, 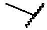 represents the position of attachment to siRNA;
Q is independently 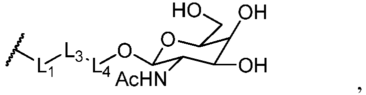 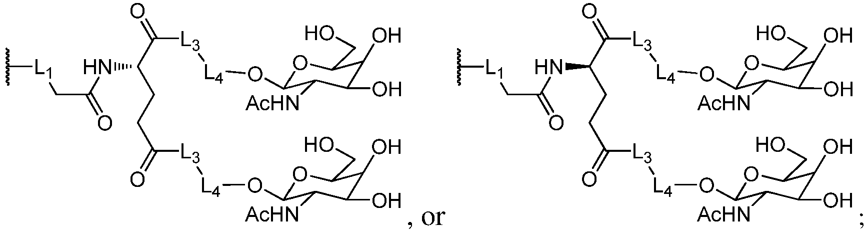
   wherein L₁ is -CH₂- or -C(O)-;
   L₃ is -(NHCH₂CH₂)_{b}-;
   L₄ is -(OCH₂CH₂)_{c}-;
      wherein b=1, 2, 3, 4, or 5;
      c = 1, 2, 3, 4 or 5;
L is -CH₂O- or -NHC(O)-;
R' is H, a hydroxy-protecting group, or a solid support, wherein the hydroxy-protecting group is preferably -C(O)CH₂CH₂C(O)OH or 4,4'-dimethoxytrityl;
n = 0, 1, 2, 3, 4, 5, 6, 7, 8, 9 or 10.

In some specific embodiments, wherein:
Q is independently H, 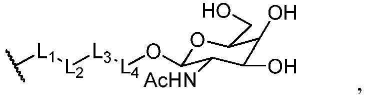 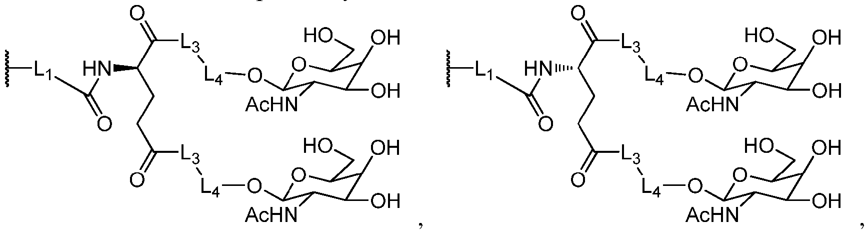 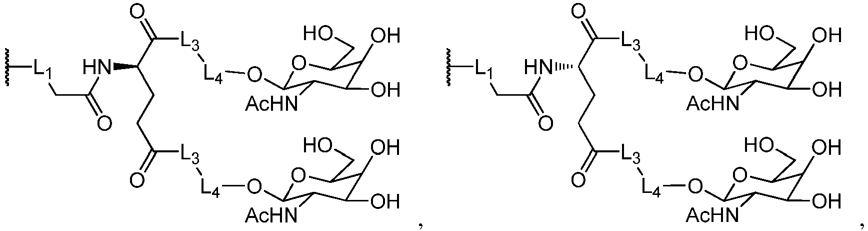 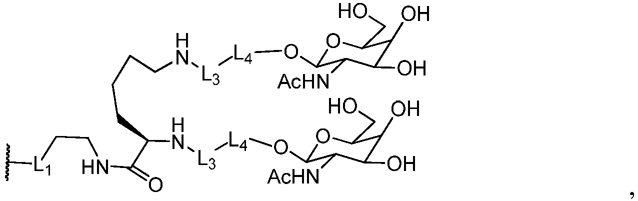 or 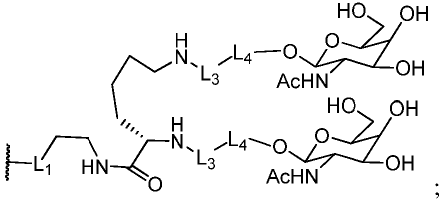
   wherein L₁ is a bond, -CH₂-, -CH₂CH₂-, -C(O)-, -CH₂O-, -CH₂O-CH₂CH₂O-, or -NHC(O)-(CH₂NHC(O))ₐ-;
   L₂ is a bond or -CH₂CH₂C(O)-;
   L₃ is a bond, -(NHCH₂CH₂)_{b}-, -(NHCH₂CH₂CH₂)_{b}-, or -C(O)CH₂-;
   L₄ is -(OCH₂CH₂)_{c}-, -(OCH₂CH₂CH₂)_{c}-, -(OCH₂CH₂CH₂CH₂)_{c}-, - (OCH₂CH₂CH₂CH₂CH₂)_{c}-, or -NHC(O)-(CH₂)_{d}-;
      wherein a = 0, 1, 2, or 3;
      b = 1, 2, 3, 4 or 5;
      c = 1, 2, 3, 4 or 5;
      d = 1, 2, 3, 4, 5, 6, 7, or 8;
L is a bond, -CH₂O-, or -NHC(O)-;
L' is a bond, -C(O)NH-, -NHC(O)-, or -O(CH₂CH₂O)ₑ-;
   wherein e is 1, 2, 3, 4, or 5;
T is a bond, -CH₂-, -M-, -CH₂-M-, or -C(O)-M-;
   wherein M is 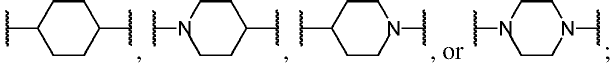
R₁ and R₂ together form -CH₂CH₂O- or -CH₂CH(R)-O-, and R₃ is H;
or R₁ and R₃ together form -C₁₋₂ alkylene-, and R₂ is H;
   wherein R is -OR', -CH₂OR', or -CH₂CH₂OR'; wherein R' is H, a hydroxy-protecting group, or a solid support, and the hydroxy-protecting group is preferably -C(O)CH₂CH₂C(O)OH or 4,4'-dimethoxytrityl;
m = 0, 1, 2, 3, 4, 5, 6, 7, 8, 9 or 10;
n = 0, 1, 2, 3, 4, 5, 6, 7, 8, 9 or 10.

In some specific embodiments, wherein:
T is -M-, -CH₂-M-, or -C(O)-M-, wherein M is

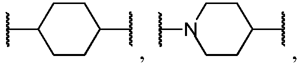

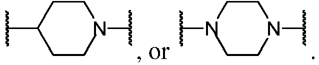

In some specific embodiments, wherein:
Q is independently H or 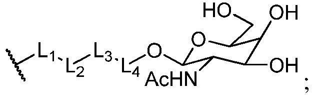
   wherein L₁ is -CH₂O- or -NHC(O)-(CH₂NHC(O))ₐ-;
   L₂ is -CH₂CH₂C(O)-;
   L₃ is -(NHCH₂CH₂)_{b}- or -(NHCH₂CH₂CH₂)_{b}-;
   L₄ is -(OCH₂CH₂)_{c}- or -NHC(O)-(CH₂)_{d}-;
      wherein a = 0, 1, 2, or 3;
      b = 1, 2, 3, 4 or 5;
      c = 1, 2, 3, 4 or 5;
      d = 1, 2, 3, 4, 5, 6, 7, or 8;
L is a bond or -CH₂O-;
L' is a bond or -O(CH₂CH₂O)ₑ-;
   wherein e is 1, 2, 3, 4, or 5;
R₁ and R₂ together form -CH₂CH₂O- or -CH₂CH(R)-O-, and R₃ is H;
or R₁ and R₃ together form -C₁₋₂ alkylene-, and R₂ is H;
   wherein R is -OR', -CH₂OR', or -CH₂CH₂OR'; wherein R' is H, a hydroxy-protecting group, or a solid support, and the hydroxy-protecting group is preferably -C(O)CH₂CH₂C(O)OH or 4,4'-dimethoxytrityl;
m = 0, 1, 2, 3, 4, 5, 6, 7, 8, 9 or 10;
n = 0, 1, 2, 3, 4, 5, 6, 7, 8, 9 or 10;
wherein T is as defined above.

In some embodiments, the conjugation group is as shown in formula (III'-1), formula (III'-2), or formula (III'-3): 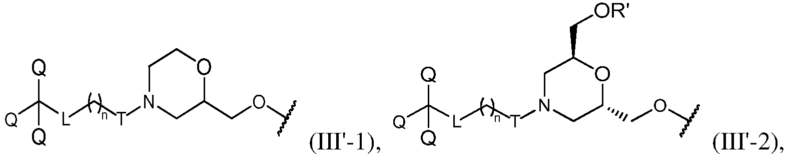 or 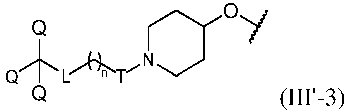 wherein,
Q is 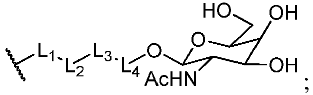
   wherein L₁ is -CH₂O- or -NHC(O)-;
   L₂ is -CH₂CH₂C(O)-;
   L₃ is -(NHCH₂CH₂)_{b}- or -(NHCH₂CH₂CH₂)_{b}-;
   L₄ is -(OCH₂CH₂)_{c}- or -NHC(O)-(CH₂)_{d}-;
      wherein b=1, 2, 3, 4, or 5;
      c = 1, 2, 3, 4 or 5;
      d = 1, 2, 3, 4, 5, 6, 7, or 8;
L is a bond or -CH₂O-;
wherein R' is H, a hydroxy-protecting group, or a solid support, wherein the hydroxy-protecting group is preferably -C(O)CH₂CH₂C(O)OH or 4,4'-dimethoxytrityl;
n = 0, 1, 2, 3, 4, 5, 6, 7, 8, 9 or 10;
wherein T is as defined above.

In some specific embodiments, wherein:
Q is independently H, 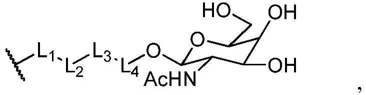 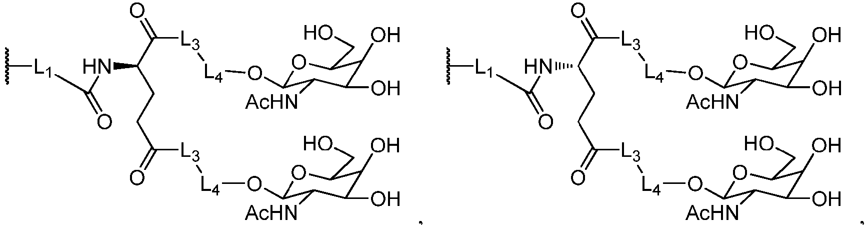 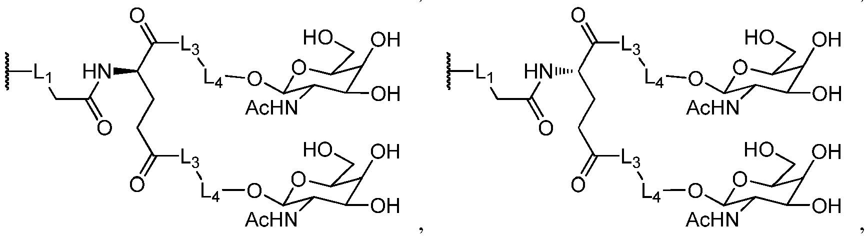 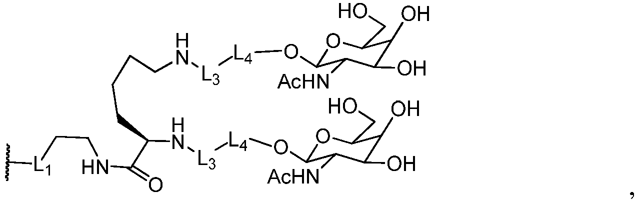 or 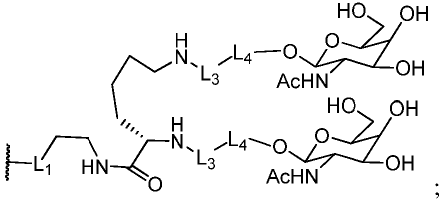
   wherein L₁ is -CH₂-, -CH₂O-, or -C(O)-;
   L₂ is a bond;
   L₃ is -(NHCH₂CH₂)_{b}-, -(NHCH₂CH₂CH₂)_{b}-, or -C(O)CH₂-;
   L₄ is -(OCH₂CH₂)_{c}- or -NHC(O)-(CH₂)_{d}-;
      wherein b=1, 2, 3, 4, or 5;
      c = 1, 2, 3, 4 or 5;
      d = 1, 2, 3, 4, 5, 6, 7, or 8;
L is a bond or -NHC(O)-;
L' is a bond;
R₁ and R₂ together form -CH₂CH₂O- or -CH₂CH(R)-O-, and R₃ is H;
or R₁ and R₃ together form -C₁₋₂ alkylene-, and R₂ is H;
   wherein R is -OR', -CH₂OR', or -CH₂CH₂OR'; wherein R' is H, a hydroxy-protecting group, or a solid support, and the hydroxy-protecting group is preferably -C(O)CH₂CH₂C(O)OH or 4,4'-dimethoxytrityl;
m = 0, 1, 2, 3, 4, 5, 6, 7, 8, 9 or 10;
n = 0, 1, 2, 3, 4, 5, 6, 7, 8, 9 or 10;
wherein T is as defined above.

In some embodiments, the conjugation group is as shown in formula (IV-1) or formula (IV-2): 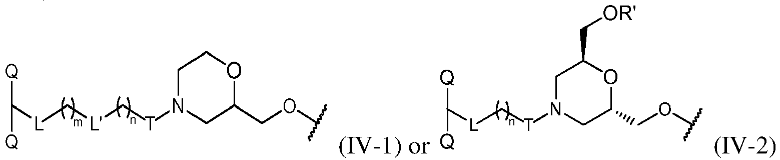 wherein,
Q is independently 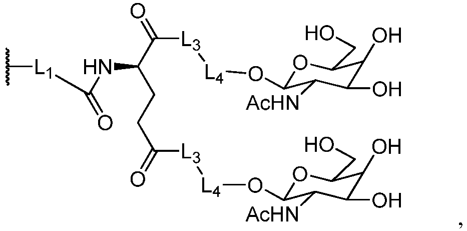 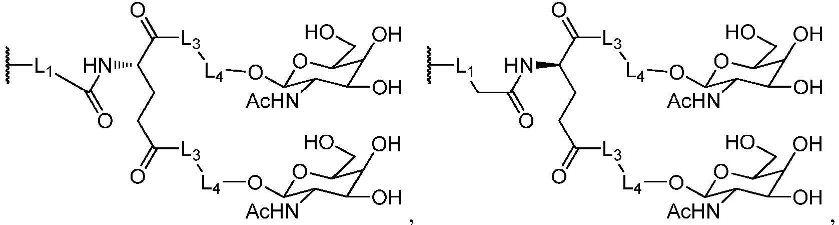 or 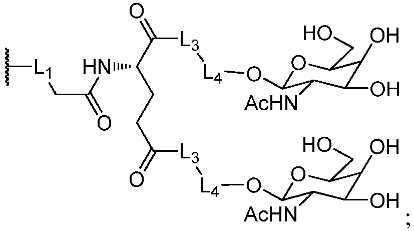
   wherein L₁ is -CH₂-, -CH₂O-, or -C(O)-;
   L₃ is -(NHCH₂CH₂)_{b}-, -(NHCH₂CH₂CH₂)_{b}-, or -C(O)CH₂-;
   L₄ is -(OCH₂CH₂)_{c}- or -NHC(O)-(CH₂)_{d}-;
      wherein b=1, 2, 3, 4, or 5;
      c = 1, 2, 3, 4 or 5;
      d = 1, 2, 3, 4, 5, 6, 7, or 8;
L is a bond or -NHC(O)-;
L' is a bond;
wherein R' is H, a hydroxy-protecting group, or a solid support, wherein the hydroxy-protecting group is preferably -C(O)CH₂CH₂C(O)OH or 4,4'-dimethoxytrityl;
m = 0, 1, 2, 3, 4, 5, 6, 7, 8, 9 or 10;
n = 0, 1, 2, 3, 4, 5, 6, 7, 8, 9 or 10;
wherein T is as defined above.

In some specific embodiments, wherein:
Q is independently H, 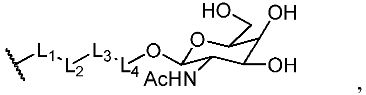 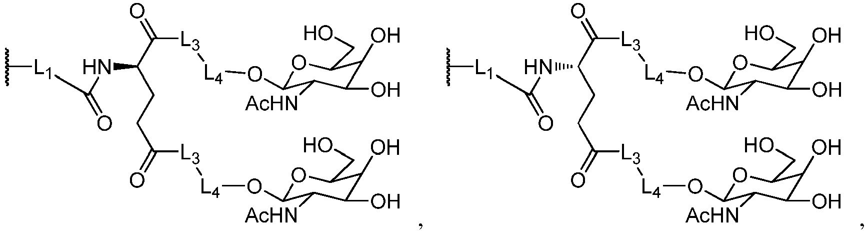 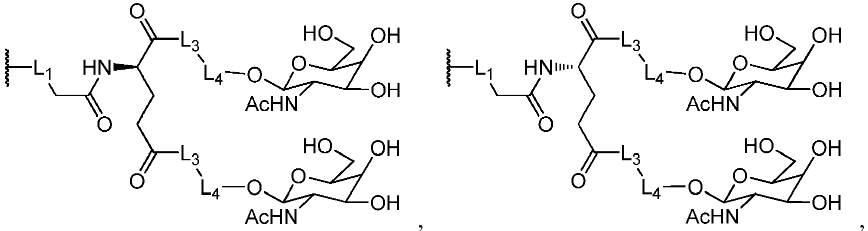 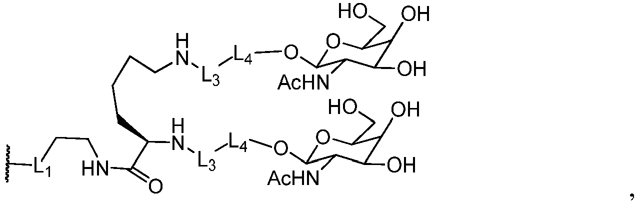 or 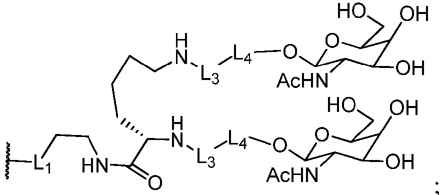
   wherein L₁ is a bond, -CH₂-, -CH₂CH₂-, -C(O)-, -CH₂O-, -CH₂O-CH₂CH₂O-, or -NHC(O)-(CH₂NHC(O))ₐ-;
   L₂ is a bond or -CH₂CH₂C(O)-;
   L₃ is a bond, -(NHCH₂CH₂)_{b}-, -(NHCH₂CH₂CH₂)_{b}-, or -C(O)CH₂-;
   L₄ is -(OCH₂CH₂)_{c}-, -(OCH₂CH₂CH₂)_{c}-, -(OCH₂CH₂CH₂CH₂)_{c}-, - (OCH₂CH₂CH₂CH₂CH₂)_{c}-, or -NHC(O)-(CH₂)_{d}-;
      wherein a = 0, 1, 2, or 3;
      b = 1, 2, 3, 4 or 5;
      c = 1, 2, 3, 4 or 5;
      d = 1, 2, 3, 4, 5, 6, 7, or 8;
L is a bond, -CH₂O-, or -NHC(O)-;
L' is -O(CH₂CH₂O)ₑ-;
   wherein e is 1, 2, 3, 4, or 5;
T is a bond, -CH₂-, -C(O)-, -M-, -CH₂-M-, or -C(O)-M-;
   wherein M is 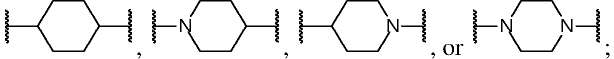
R₁ and R₂ together form -CH₂CH₂O- or -CH₂CH(R)-O-, and R₃ is H;
or R₁ and R₃ together form -C₁₋₂ alkylene-, and R₂ is H;
   wherein R is -OR', -CH₂OR', or -CH₂CH₂OR'; wherein R' is H, a hydroxy-protecting group, or a solid support, and the hydroxy-protecting group is preferably -C(O)CH₂CH₂C(O)OH or 4,4'-dimethoxytrityl;
m = 0, 1, 2, 3, 4, 5, 6, 7, 8, 9 or 10;
n = 0, 1, 2, 3, 4, 5, 6, 7, 8, 9 or 10.

In some preferred embodiments, the conjugation group is selected from Tables 1 and 2.

In some embodiments, the ligand targets an asialoglycoprotein receptor (ASGPR).

In a preferred embodiment, the ligand has the following structure: 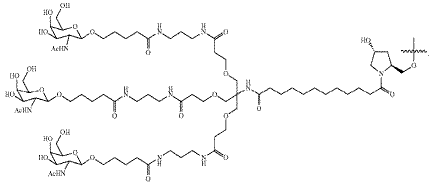 wherein 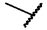 , represents the position of attachment to the sense strand of the siRNA via a phosphate group or a phosphorothioate group.

In a preferred embodiment, the ligand has the following structure: 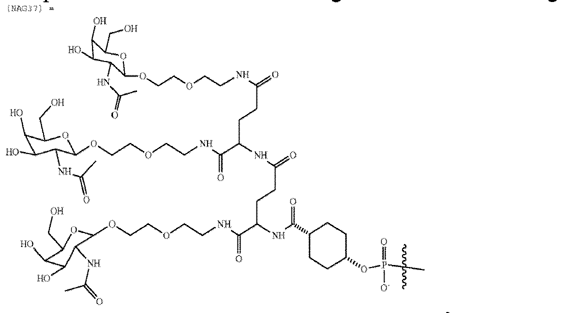 wherein 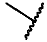 represents the position of attachment to the sense strand of the siRNA via a phosphate group or a phosphorothioate group.

In a preferred embodiment, the ligand has the following structure: 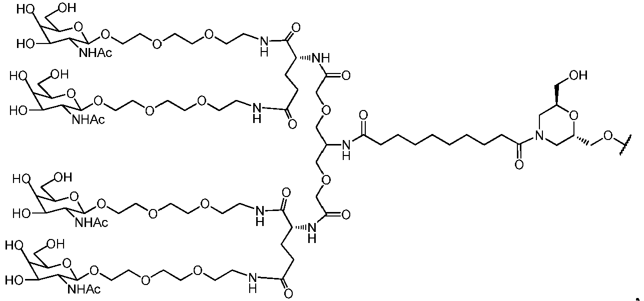 wherein 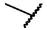 represents the position of attachment to the sense strand of the siRNA via a phosphate group or a phosphorothioate group.

In some specific embodiments, in the siRNA of the present disclosure, wherein:
(1) the sense strand comprises 
STM1s-AmsAmAmAmGmGmGmAmCfAfGfUmAmUmUmCmUmCmAmGmAms-
STM1s-GL6 (SEQ ID NO: 1238),
 and the antisense strand comprises
   (CP1a-U)sCfsUmGfAmGfAmAfUmAfCmUfGmUfCmCfCmUfUmsUfsUm (SEQ ID NO: 1651);
(2) the sense strand comprises 
STM1s-AmsAmUmUmAmAmAmAmGfGfGfAmCmAmGmUmAmUmUmCmAms-
STM1s-GL6 (SEQ ID NO: 1235),
 and the antisense strand comprises
   (CP1a-U)sGfsAmAfUmAfCmUfGmUfCmCfCmUfUmUfUmAfAmsUfsUm (SEQ ID NO: 1650);
(3) the sense strand comprises 
STM1s-AmsAmUmUmAmAmAmAmGfGfGfAmCmAmGmUmAmUmUmCmAms-
STM1s-GL6 (SEQ ID NO: 1235),
 and the antisense strand comprises
   (VPUm)sGfsAmAfUmAfCmUfGmUfCmCfCmUfUmUfUmAfAmsUfsUm (SEQ ID NO: 1649);
(4) the sense strand comprises 
IBs-AmsAmUmUmAmAmAmAmGfGfGfAmCmAmGmUmAmUmUmCmAms-
IBs-GL6 (SEQ ID NO: 1236),
 and the antisense strand comprises
   (CP1a-U)sGfsAmAfUmAfCmUfGmUfCmCfCmUfUmUfUmAfAmsUfsUm(SEQ ID NO: 1650);
(5) the sense strand comprises
   UmsUmsAmAmAmAmGfGfGfAmCmAmGmUmAmUmUmCmsAms-GL6(SEQ ID NO: 1237),
   and the antisense strand comprises
   (CP1a-U)sGfsAmAfUmAfCmUfGmUfCmCfCmUfUmUfUmAfAmsUfsUm(SEQ ID NO: 1650);
(6) the sense strand comprises 
IBs-AmsAmAmAmGmGmGmAmCfAfGfUmAmUmUmCmUmCmAmGmAms-IBs-
GL6 (SEQ ID NO: 1239),
 and the antisense strand comprises
   (CP1a-U)sCfsUmGfAmGfAmAfUmAfCmUfGmUfCmCfCmUfUmsUfsUm(SEQ ID NO: 1651);
(7) the sense strand comprises
   AmsAmsGmGmGmAmCfAfGfUmAmUmUmCmUmCmAmGmsAms-GL6(SEQ ID NO: 1240),
   and the antisense strand comprises
   (CP1a-U)sCfsUmGfAmGfAmAfUmAfCmUfGmUfCmCfCmUfUmsUfsUm(SEQ ID NO:1651); or
(8) the sense strand comprises 
IBs-AmsAmAmAmGmGmGmAmCfAfGfUmAmUmUmCmUmCmAmGmAms-IBs-
GL6(SEQ ID NO: 1239),
 and the antisense strand comprises 
(CP1a-U)sCfsUmGfAmGf(PCN-
A)AfUmAfCmUfGmUfCmCfCmUfUmsUfsUm(SEQ ID NO:1652);
 or
(9) the sense strand comprises
   AmsAmsGmGmGmAmCfAfGfUmAmUmUmCmUmCmAmGmsAms-GL6(SEQ ID NO: 1240),
   and the antisense strand comprises
   (CP1a-U)sCfsUmGfAmGf(PCN-A)AfUmAfCmUfGmUfCmCfCmUfUmsUfsUm(SEQ ID NO: 1652).

### IV. Inhibition of APOC3 Gene Expression

The siRNA of the present invention is capable of inhibiting APOC3 gene expression by at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99%.

Inhibition of APOC3 gene expression can be manifested by a reduction in the levels of mRNA expressed by a first cell or population of cells (such cells may be present, for example, in a sample derived from a subject), wherein the APOC3 gene is transcribed and the cell or cells have been treated (e.g., by contacting the cell or cells with the siRNA of the present invention, or by administering the siRNA of the present invention to a subject in which the cells are present or previously present, such that the APOC3 gene expression is inhibited compared to a second cell or population of cells (one or more control cells) that is substantially identical to the first cell or population of cells but has not been so treated.

In a preferred embodiment, the inhibition is assessed by expressing the level of mRNA in treated cells as a percentage of the level of mRNA in control cells using the following formula. In some specific embodiments, the 2^{-△△Ct} value is calculated and converted into a percentage to obtain the residual inhibition rate, where △△Ct = [(Ct_{target gene of experimental group} - Ct_{internal reference of experimental group}) - (Ct_{target gene of control group} - Ct_{internal reference of control group})].

Inhibition of the expression of APOC3 protein can be manifested by a reduction in the levels of APOC3 protein expressed by a cell or population of cells (e.g., levels of the protein expressed in a sample derived from a subject). As explained above with respect to the assessment of mRNA repression, inhibition of protein expression levels in a treated cell or population of cells can similarly be expressed as a percentage of the levels of the protein in a control cell or population of cells.

A control cell or population of cells that can be used to assess inhibition of APOC3 gene expression includes a cell or population of cells that has not been exposed to the siRNA of the present invention. For example, the control cell or population of cells may be derived from an individual subject (e.g., a human or animal subject) prior to treatment of the subject with siRNA.

### V. Vector

The present invention provides a vector comprising a nucleotide sequence encoding the siRNA of the invention. The vector of the present invention can amplify or express a nucleotide encoding the siRNA of the invention linked thereto.

A siRNA targeting the APOC3 gene can be expressed from a transcription unit inserted into a DNA or RNA vector. Expression can be transient (within hours to weeks) or sustained (weeks to months or longer), depending on the particular construct used and the target tissue or cell type. A nucleotide encoding the siRNA that targets the APOC3 gene can be introduced into a linear construct, a circular plasmid, or a viral vector. A nucleotide encoding the siRNA that targets the PCSK gene can be stably expressed by integration into the cell genome, or can be stably inherited and expressed extrachromosomally. Generally speaking, a vector expressing the siRNA is usually a DNA plasmid or a viral vector.

Viral vector systems comprising a sequence encoding the siRNA that targets the APOC3 gene include, but are not limited to: (a) adenoviral vectors; (b) retroviral vectors; (c) adeno-associated viral vectors; (d) herpes simplex virus vectors; (e) SV40 vectors; (f) polyomavirus vectors; (g) papillomavirus vectors; (h) picornavirus vectors; (i) poxvirus vectors; and (j) helper virus-dependent or gutless adenoviral vectors.

### VI. Cell

The present invention provides a cell comprising the siRNA or vector of the invention, wherein the siRNA or vector of the invention can be transcribed in the cell.

### VII. Pharmaceutical Composition

The present invention provides a pharmaceutical composition comprising a siRNA, vector, or cell disclosed herein, and optionally a pharmaceutically acceptable carrier or excipient.

As used herein, "pharmaceutically acceptable" refers to those compounds, materials, compositions, and/or dosage forms which, within the scope of sound medical judgment, are suitable for contact with tissues of human subjects and animal subjects without undue toxicity, irritation, allergic reactions, or other problems or complications, commensurate with a reasonable benefit/risk ratio.

As used herein, a pharmaceutically acceptable carrier refers to a pharmaceutical carrier that facilitates the administration of siRNA or a vector or cell comprising a coding sequence thereof to the human body and/or facilitates absorption or action thereof. For example: diluents, excipients such as water, and fillers such as starch and sucrose; binders such as cellulose derivatives, alginates, gelatin, and polyvinylpyrrolidone; wetting agents such as glycerin; disintegrants such as agar, calcium carbonate, and sodium bicarbonate; absorption accelerators such as quaternary ammonium compounds; surfactants such as cetyl alcohol; adsorption carriers such as kaolin and bentonite; lubricants such as talc, calcium/magnesium stearate, and polyethylene glycol. Additional adjuvants such as flavoring agents and sweeteners can also be added to the composition.

For example, a pharmaceutical composition comprising the siRNA, vector, or cell of the present invention may comprise a pharmaceutically acceptable diluent or a sustained-release matrix into which the siRNA or vector of the present invention is embedded.

### VIII. Kit

The present invention provides a kit comprising the siRNA, vector, or cell of the present invention.

The present invention also provides a kit for using the siRNA of the present invention and/or for performing the method of the present invention. Such a kit includes one or more siRNAs, vectors, or cells of the present invention, and may further include instructions for use. Instructions for inhibiting the expression of APOC3 in a cell by contacting the cell with the siRNA or vector of the present invention in an amount effective to inhibit APOC3 expression may be recorded in the instructions for use.

In the case where the siRNA or vector of the present invention is contacted with a cell *in vitro,* optionally, the kit of the present invention may further comprise a means for contacting the cell with the siRNA or vector of the present invention (e.g., an injection device) or a means for measuring inhibition of APOC3 (e.g., a device for measuring inhibition of APOC3 mRNA or protein). Such a means for measuring inhibition of APOC3 may comprise a device for obtaining a sample (e.g., a plasma sample) from a subject.

In the case where the siRNA, vector, or cell into which the siRNA or vector of the present invention has been introduced *in vitro* is administered into the body, the kit of the present invention may optionally further comprise a means for administering the siRNA, vector, or cell of the present invention to a subject or a means for determining a therapeutically effective amount or a prophylactically effective amount.

### IX. Treatment Methods and Pharmaceutical Uses

The present invention provides a method capable of inhibiting APOC3 expression in a cell, the method comprising: (a) contacting the cell with an siRNA, vector, cell, or pharmaceutical composition of the present invention; and (b) culturing the cell.

The present invention provides a method for treating diseases or symptoms related to APOC3 expression in a subject, the method comprising the step of administering to the subject the siRNA, vector, cell, or pharmaceutical composition of the present invention.

The siRNA provided by the present invention is capable of inhibiting the expression of APOC3, thereby reducing the levels of triglycerides, in particular the levels of serum triglycerides, and is used for treating APOC3-related diseases and conditions. The APOC3-related diseases and conditions are hypertriglyceridemia and diseases that may be caused by, associated with, or as a consequence of hypertriglyceridemia. Conditions that can be caused by, associated with, or as a consequence of hypertriglyceridemia include, but are not limited to, pancreatitis, metabolic syndrome, type 2 diabetes mellitus, familial chylomicronemia syndrome (PCS), chylomicronemia, multifactorial chylomicronemia, lipodystrophy syndrome (e.g., familial partial lipodystrophy), obesity, dyslipidemia, non-alcoholic fatty liver disease, non-alcoholic steatohepatitis, hyperlipidemia, hypertriglyceridemia, abnormal lipid and/or cholesterol metabolism, atherosclerosis, cardiovascular disease, coronary artery disease, polycystic ovarian syndrome, kidney disease, and other dyslipidemia and metabolism-related disorders and diseases. The hypertriglyceridemia includes, but is not limited to, non-familial hypertriglyceridemia, familial hypertriglyceridemia, heterozygous familial hypertriglyceridemia, and homozygous familial hypertriglyceridemia.

In some embodiments, the method of the present invention for treating a disease or symptom related to APOC3 expression in a subject comprises administering the siRNA or pharmaceutical composition to the subject subcutaneously, intravenously, or topically. In some embodiments, the subject is a human.

The present invention also relates to the siRNA, vector, cell, or pharmaceutical composition of the present invention for use in treating a disease or symptom related to APOC3 expression in a subject.

The present invention also relates to the use of the siRNA, vector, cell, or pharmaceutical composition of the present invention in the manufacture of a medicament for treating a disease or symptom related to APOC3 expression in a subject. The medicament of the present invention can be prepared into emulsions, microemulsions, and microparticles.

The present invention also provides a method for reducing the risk of developing APOC3-related diseases and conditions in a subject, the method comprising the step of administering to the subject the siRNA, vector, cell, or pharmaceutical composition of the present invention.

The siRNA of the present invention can reduce the risk of developing APOC3-related diseases and conditions by significantly reducing triglyceride levels. The APOC3-related diseases and conditions are hypertriglyceridemia and diseases that may be caused by, associated with, or as a consequence of hypertriglyceridemia. Conditions that can be caused by, associated with, or as a consequence of hypertriglyceridemia include, but are not limited to, pancreatitis, metabolic syndrome, type 2 diabetes mellitus, familial chylomicronemia syndrome (FCS), chylomicronemia, multifactorial chylomicronemia, lipodystrophy syndrome (e.g., familial partial lipodystrophy), obesity, dyslipidemia, non-alcoholic fatty liver disease, non-alcoholic steatohepatitis, hyperlipidemia, hypertriglyceridemia, abnormal lipid and/or cholesterol metabolism, atherosclerosis, cardiovascular disease, coronary artery disease, polycystic ovarian syndrome, kidney disease, and other dyslipidemia and metabolism-related disorders and diseases. The hypertriglyceridemia includes, but is not limited to, non-familial hypertriglyceridemia, familial hypertriglyceridemia, heterozygous familial hypertriglyceridemia, and homozygous familial hypertriglyceridemia.

In some embodiments, the method of the present invention for reducing the risk of developing APOC3-related diseases and conditions in a subject comprises administering the siRNA or pharmaceutical composition to the subject subcutaneously, intravenously, or topically. In some embodiments, the subject is a human.

The present invention also relates to the siRNA, vector, cell, or pharmaceutical composition of the present invention for use in reducing the risk of developing APOC3-related diseases and conditions in a subject.

The present invention also relates to the use of the siRNA, vector, cell, or pharmaceutical composition of the present invention in the manufacture of a medicament for reducing the risk of developing APOC3-related diseases and conditions in a subject. The medicament of the present invention can be prepared into emulsions, microemulsions, and microparticles.

### Sequence

The RNA sequence provided by the present invention targets the APOC3 gene (or target gene, target mRNA sequence, or target sequence).

**Table 3. Nucleotide Sequences of Sense and Antisense Strands Targeting APOC3 Gene Used in the Present Invention**

| SEQ ID NO: | Sense Strand Sequence (5'→3') | SEQ ID NO: | Antisense Strand Sequence (5'→3') |
|---|---|---|---|
| 1 | AGGCAGCUGCUCCAGGAAU | 414 | AUUCCUGGAGCAGCUGCCUUU |
| 2 | GGCAGCUGCUCCAGGAACA | 415 | UGUUCCUGGAGCAGCUGCCUU |
| 3 | GCAGCUGCUCCAGGAACAA | 416 | UUGUUCCUGGAGCAGCUGCUU |
| 4 | CAGCUGCUCCAGGAACAGA | 417 | UCUGUUCCUGGAGCAGCUGUU |
| 5 | AGCUGCUCCAGGAACAGAA | 418 | UUCUGUUCCUGGAGCAGCUUU |
| 6 | GCUGCUCCAGGAACAGAGA | 419 | UCUCUGUUCCUGGAGCAGCUU |
| 7 | UGCUCCAGGAACAGAGGUA | 420 | UACCUCUGUUCCUGGAGCAUU |
| 8 | CAGGAACAGAGGUGCCAUA | 421 | UAUGGCACCUCUGUUCCUGUU |
| 9 | AGGAACAGAGGUGCCAUGU | 422 | ACAUGGCACCUCUGUUCCUUU |
| 10 | GGAACAGAGGUGCCAUGCA | 423 | UGCAUGGCACCUCUGUUCCUU |
| 11 | GAACAGAGGUGCCAUGCAA | 424 | UUGCAUGGCACCUCUGUUCUU |
| 12 | AACAGAGGUGCCAUGCAGU | 425 | ACUGCAUGGCACCUCUGUUUU |
| 13 | CAUGCAGCCCCGGGUACUU | 426 | AAGUACCCGGGGCUGCAUGUU |
| 14 | AUGCAGCCCCGGGUACUCU | 427 | AGAGUACCCGGGGCUGCAUUU |
| 15 | UGCAGCCCCGGGUACUCCU | 428 | AGGAGUACCCGGGGCUGCAUU |
| 16 | GCAGCCCCGGGUACUCCUU | 429 | AAGGAGUACCCGGGGCUGCUU |
| 17 | CAGCCCCGGGUACUCCUUA | 430 | UAAGGAGUACCCGGGGCUGUU |
| 18 | AGCCCCGGGUACUCCUUGU | 431 | ACAAGGAGUACCCGGGGCUUU |
| 19 | GCCCCGGGUACUCCUUGUU | 432 | AACAAGGAGUACCCGGGGCUU |
| 20 | CCCCGGGUACUCCUUGUUA | 433 | UAACAAGGAGUACCCGGGGUU |
| 21 | CCCGGGUACUCCUUGUUGU | 434 | ACAACAAGGAGUACCCGGGUU |
| 22 | CCGGGUACUCCUUGUUGUU | 435 | AACAACAAGGAGUACCCGGUU |
| 23 | CGGGUACUCCUUGUUGUUA | 436 | UAACAACAAGGAGUACCCGUU |
| 24 | GGGUACUCCUUGUUGUUGU | 437 | ACAACAACAAGGAGUACCCUU |
| 25 | GGUACUCCUUGUUGUUGCU | 438 | AGCAACAACAAGGAGUACCUU |
| 26 | GUACUCCUUGUUGUUGCCU | 439 | AGGCAACAACAAGGAGUACUU |
| 27 | ACUCCUUGUUGUUGCCCUU | 440 | AAGGGCAACAACAAGGAGUUU |
| 28 | CUCCUUGUUGUUGCCCUCU | 441 | AGAGGGCAACAACAAGGAGUU |
| 29 | UCCUUGUUGUUGCCCUCCU | 442 | AGGAGGGCAACAACAAGGAUU |
| 30 | CCUUGUUGUUGCCCUCCUA | 443 | UAGGAGGGCAACAACAAGGUU |
| 31 | CUUGUUGUUGCCCUCCUGA | 444 | UCAGGAGGGCAACAACAAGUU |
| 32 | UUGUUGUUGCCCUCCUGGU | 445 | ACCAGGAGGGCAACAACAAUU |
| 33 | UGUUGUUGCCCUCCUGGCA | 446 | UGCCAGGAGGGCAACAACAUU |
| 34 | GCCCUCCUGGCGCUCCUGA | 447 | UCAGGAGCGCCAGGAGGGCUU |
| 35 | UCCUGGCGCUCCUGGCCUU | 448 | AAGGCCAGGAGCGCCAGGAUU |
| 36 | CUGGCGCUCCUGGCCUCUA | 449 | UAGAGGCCAGGAGCGCCAGUU |
| 37 | UGGCGCUCCUGGCCUCUGU | 450 | ACAGAGGCCAGGAGCGCCAUU |
| 38 | GGCGCUCCUGGCCUCUGCU | 451 | AGCAGAGGCCAGGAGCGCCUU |
| 39 | GCGCUCCUGGCCUCUGCCU | 452 | AGGCAGAGGCCAGGAGCGCUU |
| 40 | CUGGCCUCUGCCCGAGCUU | 453 | AAGCUCGGGCAGAGGCCAGUU |
| 41 | UGGCCUCUGCCCGAGCUUU | 454 | AAAGCUCGGGCAGAGGCCAUU |
| 42 | GGCCUCUGCCCGAGCUUCA | 455 | UGAAGCUCGGGCAGAGGCCUU |
| 43 | GCCUCUGCCCGAGCUUCAA | 456 | UUGAAGCUCGGGCAGAGGCUU |
| 44 | CCUCUGCCCGAGCUUCAGA | 457 | UCUGAAGCUCGGGCAGAGGUU |
| 45 | CUCUGCCCGAGCUUCAGAA | 458 | UUCUGAAGCUCGGGCAGAGUU |
| 46 | UCUGCCCGAGCUUCAGAGA | 459 | UCUCUGAAGCUCGGGCAGAUU |
| 47 | CUGCCCGAGCUUCAGAGGU | 460 | ACCUCUGAAGCUCGGGCAGUU |
| 48 | UGCCCGAGCUUCAGAGGCU | 461 | AGCCUCUGAAGCUCGGGCAUU |
| 49 | AGCUUCAGAGGCCGAGGAU | 462 | AUCCUCGGCCUCUGAAGCUUU |
| 50 | GCUUCAGAGGCCGAGGAUA | 463 | UAUCCUCGGCCUCUGAAGCUU |
| 51 | CUUCAGAGGCCGAGGAUGU | 464 | ACAUCCUCGGCCUCUGAAGUU |
| 52 | UUCAGAGGCCGAGGAUGCU | 465 | AGCAUCCUCGGCCUCUGAAUU |
| 53 | UCAGAGGCCGAGGAUGCCU | 466 | AGGCAUCCUCGGCCUCUGAUU |
| 54 | CAGAGGCCGAGGAUGCCUU | 467 | AAGGCAUCCUCGGCCUCUGUU |
| 55 | AGAGGCCGAGGAUGCCUCU | 468 | AGAGGCAUCCUCGGCCUCUUU |
| 56 | GAGGCCGAGGAUGCCUCCU | 469 | AGGAGGCAUCCUCGGCCUCUU |
| 57 | GGCCGAGGAUGCCUCCCUU | 470 | AAGGGAGGCAUCCUCGGCCUU |
| 58 | GCCGAGGAUGCCUCCCUUU | 471 | AAAGGGAGGCAUCCUCGGCUU |
| 59 | CCGAGGAUGCCUCCCUUCU | 472 | AGAAGGGAGGCAUCCUCGGUU |
| 60 | CGAGGAUGCCUCCCUUCUU | 473 | AAGAAGGGAGGCAUCCUCGUU |
| 61 | GAGGAUGCCUCCCUUCUCA | 474 | UGAGAAGGGAGGCAUCCUCUU |
| 62 | AGGAUGCCUCCCUUCUCAA | 475 | UUGAGAAGGGAGGCAUCCUUU |
| 63 | GGAUGCCUCCCUUCUCAGU | 476 | ACUGAGAAGGGAGGCAUCCUU |
| 64 | GAUGCCUCCCUUCUCAGCU | 477 | AGCUGAGAAGGGAGGCAUCUU |
| 65 | AUGCCUCCCUUCUCAGCUU | 478 | AAGCUGAGAAGGGAGGCAUUU |
| 66 | UGCCUCCCUUCUCAGCUUU | 479 | AAAGCUGAGAAGGGAGGCAUU |
| 67 | GCCUCCCUUCUCAGCUUCA | 480 | UGAAGCUGAGAAGGGAGGCUU |
| 68 | CCUCCCUUCUCAGCUUCAU | 481 | AUGAAGCUGAGAAGGGAGGUU |
| 69 | CUCCCUUCUCAGCUUCAUA | 482 | UAUGAAGCUGAGAAGGGAGUU |
| 70 | UCCCUUCUCAGCUUCAUGU | 483 | ACAUGAAGCUGAGAAGGGAUU |
| 71 | CCCUUCUCAGCUUCAUGCA | 484 | UGCAUGAAGCUGAGAAGGGUU |
| 72 | CCUUCUCAGCUUCAUGCAA | 485 | UUGCAUGAAGCUGAGAAGGUU |
| 73 | CUUCUCAGCUUCAUGCAGA | 486 | UCUGCAUGAAGCUGAGAAGUU |
| 74 | UUCUCAGCUUCAUGCAGGA | 487 | UCCUGCAUGAAGCUGAGAAUU |
| 75 | UCUCAGCUUCAUGCAGGGU | 488 | ACCCUGCAUGAAGCUGAGAUU |
| 76 | CUCAGCUUCAUGCAGGGUU | 489 | AACCCUGCAUGAAGCUGAGUU |
| 77 | UCAGCUUCAUGCAGGGUUA | 490 | UAACCCUGCAUGAAGCUGAUU |
| 78 | CAGCUUCAUGCAGGGUUAU | 491 | AUAACCCUGCAUGAAGCUGUU |
| 79 | AGCUUCAUGCAGGGUUACA | 492 | UGUAACCCUGCAUGAAGCUUU |
| 80 | GCUUCAUGCAGGGUUACAU | 493 | AUGUAACCCUGCAUGAAGCUU |
| 81 | CUUCAUGCAGGGUUACAUA | 494 | UAUGUAACCCUGCAUGAAGUU |
| 82 | UUCAUGCAGGGUUACAUGA | 495 | UCAUGUAACCCUGCAUGAAUU |
| 83 | UCAUGCAGGGUUACAUGAA | 496 | UUCAUGUAACCCUGCAUGAUU |
| 84 | CAUGCAGGGUUACAUGAAA | 497 | UUUCAUGUAACCCUGCAUGUU |
| 85 | AUGCAGGGUUACAUGAAGU | 498 | ACUUCAUGUAACCCUGCAUUU |
| 86 | UGCAGGGUUACAUGAAGCA | 499 | UGCUUCAUGUAACCCUGCAUU |
| 87 | GCAGGGUUACAUGAAGCAU | 500 | AUGCUUCAUGUAACCCUGCUU |
| 88 | CAGGGUUACAUGAAGCACA | 501 | UGUGCUUCAUGUAACCCUGUU |
| 89 | AGGGUUACAUGAAGCACGU | 502 | ACGUGCUUCAUGUAACCCUUU |
| 90 | GGGUUACAUGAAGCACGCU | 503 | AGCGUGCUUCAUGUAACCCUU |
| 91 | GUUACAUGAAGCACGCCAU | 504 | AUGGCGUGCUUCAUGUAACUU |
| 92 | ACAUGAAGCACGCCACCAA | 505 | UUGGUGGCGUGCUUCAUGUUU |
| 93 | CAUGAAGCACGCCACCAAA | 506 | UUUGGUGGCGUGCUUCAUGUU |
| 94 | AUGAAGCACGCCACCAAGA | 507 | UCUUGGUGGCGUGCUUCAUUU |
| 95 | UGAAGCACGCCACCAAGAU | 508 | AUCUUGGUGGCGUGCUUCAUU |
| 96 | GAAGCACGCCACCAAGACU | 509 | AGUCUUGGUGGCGUGCUUCUU |
| 97 | AAGCACGCCACCAAGACCA | 510 | UGGUCUUGGUGGCGUGCUUUU |
| 98 | AGCACGCCACCAAGACCGU | 511 | ACGGUCUUGGUGGCGUGCUUU |
| 99 | GCACGCCACCAAGACCGCU | 512 | AGCGGUCUUGGUGGCGUGCUU |
| 100 | CGCCACCAAGACCGCCAAA | 513 | UUUGGCGGUCUUGGUGGCGUU |
| 101 | GCCACCAAGACCGCCAAGA | 514 | UCUUGGCGGUCUUGGUGGCUU |
| 102 | CCACCAAGACCGCCAAGGA | 515 | UCCUUGGCGGUCUUGGUGGUU |
| 103 | CACCAAGACCGCCAAGGAU | 516 | AUCCUUGGCGGUCUUGGUGUU |
| 104 | ACCAAGACCGCCAAGGAUA | 517 | UAUCCUUGGCGGUCUUGGUUU |
| 105 | CCAAGACCGCCAAGGAUGU | 518 | ACAUCCUUGGCGGUCUUGGUU |
| 106 | CAAGACCGCCAAGGAUGCA | 519 | UGCAUCCUUGGCGGUCUUGUU |
| 107 | AAGACCGCCAAGGAUGCAU | 520 | AUGCAUCCUUGGCGGUCUUUU |
| 108 | AGACCGCCAAGGAUGCACU | 521 | AGUGCAUCCUUGGCGGUCUUU |
| 109 | GACCGCCAAGGAUGCACUA | 522 | UAGUGCAUCCUUGGCGGUCUU |
| 110 | ACCGCCAAGGAUGCACUGA | 523 | UCAGUGCAUCCUUGGCGGUUU |
| 111 | CCGCCAAGGAUGCACUGAA | 524 | UUCAGUGCAUCCUUGGCGGUU |
| 112 | CGCCAAGGAUGCACUGAGU | 525 | ACUCAGUGCAUCCUUGGCGUU |
| 113 | GCCAAGGAUGCACUGAGCA | 526 | UGCUCAGUGCAUCCUUGGCUU |
| 114 | CCAAGGAUGCACUGAGCAA | 527 | UUGCUCAGUGCAUCCUUGGUU |
| 115 | CAAGGAUGCACUGAGCAGU | 528 | ACUGCUCAGUGCAUCCUUGUU |
| 116 | AAGGAUGCACUGAGCAGCA | 529 | UGCUGCUCAGUGCAUCCUUUU |
| 117 | AGGAUGCACUGAGCAGCGU | 530 | ACGCUGCUCAGUGCAUCCUUU |
| 118 | GGAUGCACUGAGCAGCGUA | 531 | UACGCUGCUCAGUGCAUCCUU |
| 119 | GAUGCACUGAGCAGCGUGU | 532 | ACACGCUGCUCAGUGCAUCUU |
| 120 | AUGCACUGAGCAGCGUGCA | 533 | UGCACGCUGCUCAGUGCAUUU |
| 121 | UGCACUGAGCAGCGUGCAA | 534 | UUGCACGCUGCUCAGUGCAUU |
| 122 | GCACUGAGCAGCGUGCAGA | 535 | UCUGCACGCUGCUCAGUGCUU |
| 123 | CACUGAGCAGCGUGCAGGA | 536 | UCCUGCACGCUGCUCAGUGUU |
| 124 | ACUGAGCAGCGUGCAGGAA | 537 | UUCCUGCACGCUGCUCAGUUU |
| 125 | CUGAGCAGCGUGCAGGAGU | 538 | ACUCCUGCACGCUGCUCAGUU |
| 126 | UGAGCAGCGUGCAGGAGUU | 539 | AACUCCUGCACGCUGCUCAUU |
| 127 | GAGCAGCGUGCAGGAGUCU | 540 | AGACUCCUGCACGCUGCUCUU |
| 128 | AGCAGCGUGCAGGAGUCCU | 541 | AGGACUCCUGCACGCUGCUUU |
| 129 | GCAGCGUGCAGGAGUCCCA | 542 | UGGGACUCCUGCACGCUGCUU |
| 130 | CAGCGUGCAGGAGUCCCAA | 543 | UUGGGACUCCUGCACGCUGUU |
| 131 | AGCGUGCAGGAGUCCCAGA | 544 | UCUGGGACUCCUGCACGCUUU |
| 132 | GCGUGCAGGAGUCCCAGGU | 545 | ACCUGGGACUCCUGCACGCUU |
| 133 | CGUGCAGGAGUCCCAGGUA | 546 | UACCUGGGACUCCUGCACGUU |
| 134 | GUGCAGGAGUCCCAGGUGA | 547 | UCACCUGGGACUCCUGCACUU |
| 135 | UGCAGGAGUCCCAGGUGGU | 548 | ACCACCUGGGACUCCUGCAUU |
| 136 | GUCCCAGGUGGCCCAGCAA | 549 | UUGCUGGGCCACCUGGGACUU |
| 137 | UCCCAGGUGGCCCAGCAGA | 550 | UCUGCUGGGCCACCUGGGAUU |
| 138 | CCCAGGUGGCCCAGCAGGU | 551 | ACCUGCUGGGCCACCUGGGUU |
| 139 | CCAGGUGGCCCAGCAGGCU | 552 | AGCCUGCUGGGCCACCUGGUU |
| 140 | CAGGUGGCCCAGCAGGCCA | 553 | UGGCCUGCUGGGCCACCUGUU |
| 141 | AGGUGGCCCAGCAGGCCAA | 554 | UUGGCCUGCUGGGCCACCUUU |
| 142 | GGUGGCCCAGCAGGCCAGA | 555 | UCUGGCCUGCUGGGCCACCUU |
| 143 | GUGGCCCAGCAGGCCAGGA | 556 | UCCUGGCCUGCUGGGCCACUU |
| 144 | GCAGGCCAGGGGCUGGGUA | 557 | UACCCAGCCCCUGGCCUGCUU |
| 145 | CAGGCCAGGGGCUGGGUGA | 558 | UCACCCAGCCCCUGGCCUGUU |
| 146 | AGGCCAGGGGCUGGGUGAU | 559 | AUCACCCAGCCCCUGGCCUUU |
| 147 | GGCCAGGGGCUGGGUGACU | 560 | AGUCACCCAGCCCCUGGCCUU |
| 148 | GCCAGGGGCUGGGUGACCA | 561 | UGGUCACCCAGCCCCUGGCUU |
| 149 | CCAGGGGCUGGGUGACCGA | 562 | UCGGUCACCCAGCCCCUGGUU |
| 150 | CAGGGGCUGGGUGACCGAU | 563 | AUCGGUCACCCAGCCCCUGUU |
| 151 | AGGGGCUGGGUGACCGAUA | 564 | UAUCGGUCACCCAGCCCCUUU |
| 152 | GGGGCUGGGUGACCGAUGA | 565 | UCAUCGGUCACCCAGCCCCUU |
| 153 | GGGCUGGGUGACCGAUGGU | 566 | ACCAUCGGUCACCCAGCCCUU |
| 154 | GGCUGGGUGACCGAUGGCU | 567 | AGCCAUCGGUCACCCAGCCUU |
| 155 | GCUGGGUGACCGAUGGCUU | 568 | AAGCCAUCGGUCACCCAGCUU |
| 156 | CUGGGUGACCGAUGGCUUU | 569 | AAAGCCAUCGGUCACCCAGUU |
| 157 | UGGGUGACCGAUGGCUUCA | 570 | UGAAGCCAUCGGUCACCCAUU |
| 158 | GGGUGACCGAUGGCUUCAA | 571 | UUGAAGCCAUCGGUCACCCUU |
| 159 | GGUGACCGAUGGCUUCAGU | 572 | ACUGAAGCCAUCGGUCACCUU |
| 160 | GUGACCGAUGGCUUCAGUU | 573 | AACUGAAGCCAUCGGUCACUU |
| 161 | UGACCGAUGGCUUCAGUUU | 574 | AAACUGAAGCCAUCGGUCAUU |
| 162 | GACCGAUGGCUUCAGUUCU | 575 | AGAACUGAAGCCAUCGGUCUU |
| 163 | ACCGAUGGCUUCAGUUCCU | 576 | AGGAACUGAAGCCAUCGGUUU |
| 164 | CCGAUGGCUUCAGUUCCCU | 577 | AGGGAACUGAAGCCAUCGGUU |
| 165 | CGAUGGCUUCAGUUCCCUA | 578 | UAGGGAACUGAAGCCAUCGUU |
| 166 | GAUGGCUUCAGUUCCCUGA | 579 | UCAGGGAACUGAAGCCAUCUU |
| 167 | AUGGCUUCAGUUCCCUGAA | 580 | UUCAGGGAACUGAAGCCAUUU |
| 168 | UGGCUUCAGUUCCCUGAAA | 581 | UUUCAGGGAACUGAAGCCAUU |
| 169 | GGCUUCAGUUCCCUGAAAA | 582 | UUUUCAGGGAACUGAAGCCUU |
| 170 | GCUUCAGUUCCCUGAAAGA | 583 | UCUUUCAGGGAACUGAAGCUU |
| 171 | CUUCAGUUCCCUGAAAGAU | 584 | AUCUUUCAGGGAACUGAAGUU |
| 172 | UUCAGUUCCCUGAAAGACU | 585 | AGUCUUUCAGGGAACUGAAUU |
| 173 | UCAGUUCCCUGAAAGACUA | 586 | UAGUCUUUCAGGGAACUGAUU |
| 174 | CAGUUCCCUGAAAGACUAU | 587 | AUAGUCUUUCAGGGAACUGUU |
| 175 | AGUUCCCUGAAAGACUACU | 588 | AGUAGUCUUUCAGGGAACUUU |
| 176 | GUUCCCUGAAAGACUACUA | 589 | UAGUAGUCUUUCAGGGAACUU |
| 177 | UUCCCUGAAAGACUACUGA | 590 | UCAGUAGUCUUUCAGGGAAUU |
| 178 | UCCCUGAAAGACUACUGGA | 591 | UCCAGUAGUCUUUCAGGGAUU |
| 179 | CCCUGAAAGACUACUGGAA | 592 | UUCCAGUAGUCUUUCAGGGUU |
| 180 | CCUGAAAGACUACUGGAGU | 593 | ACUCCAGUAGUCUUUCAGGUU |
| 181 | CUGAAAGACUACUGGAGCA | 594 | UGCUCCAGUAGUCUUUCAGUU |
| 182 | UGAAAGACUACUGGAGCAU | 595 | AUGCUCCAGUAGUCUUUCAUU |
| 183 | GAAAGACUACUGGAGCACU | 596 | AGUGCUCCAGUAGUCUUUCUU |
| 184 | AAAGACUACUGGAGCACCA | 597 | UGGUGCUCCAGUAGUCUUUUU |
| 185 | AGACUACUGGAGCACCGUU | 598 | AACGGUGCUCCAGUAGUCUUU |
| 186 | GACUACUGGAGCACCGUUA | 599 | UAACGGUGCUCCAGUAGUCUU |
| 187 | ACUACUGGAGCACCGUUAA | 600 | UUAACGGUGCUCCAGUAGUUU |
| 188 | CUACUGGAGCACCGUUAAA | 601 | UUUAACGGUGCUCCAGUAGUU |
| 189 | UACUGGAGCACCGUUAAGA | 602 | UCUUAACGGUGCUCCAGUAUU |
| 190 | ACUGGAGCACCGUUAAGGA | 603 | UCCUUAACGGUGCUCCAGUUU |
| 191 | CUGGAGCACCGUUAAGGAU | 604 | AUCCUUAACGGUGCUCCAGUU |
| 192 | UGGAGCACCGUUAAGGACA | 605 | UGUCCUUAACGGUGCUCCAUU |
| 193 | GGAGCACCGUUAAGGACAA | 606 | UUGUCCUUAACGGUGCUCCUU |
| 194 | GAGCACCGUUAAGGACAAA | 607 | UUUGUCCUUAACGGUGCUCUU |
| 195 | AGCACCGUUAAGGACAAGU | 608 | ACUUGUCCUUAACGGUGCUUU |
| 196 | GCACCGUUAAGGACAAGUU | 609 | AACUUGUCCUUAACGGUGCUU |
| 197 | CACCGUUAAGGACAAGUUU | 610 | AAACUUGUCCUUAACGGUGUU |
| 198 | ACCGUUAAGGACAAGUUCU | 611 | AGAACUUGUCCUUAACGGUUU |
| 199 | CCGUUAAGGACAAGUUCUU | 612 | AAGAACUUGUCCUUAACGGUU |
| 200 | CGUUAAGGACAAGUUCUCU | 613 | AGAGAACUUGUCCUUAACGUU |
| 201 | GUUAAGGACAAGUUCUCUA | 614 | UAGAGAACUUGUCCUUAACUU |
| 202 | UUAAGGACAAGUUCUCUGA | 615 | UCAGAGAACUUGUCCUUAAUU |
| 203 | UAAGGACAAGUUCUCUGAA | 616 | UUCAGAGAACUUGUCCUUAUU |
| 204 | AAGGACAAGUUCUCUGAGU | 617 | ACUCAGAGAACUUGUCCUUUU |
| 205 | AGGACAAGUUCUCUGAGUU | 618 | AACUCAGAGAACUUGUCCUUU |
| 206 | GGACAAGUUCUCUGAGUUU | 619 | AAACUCAGAGAACUUGUCCUU |
| 207 | GACAAGUUCUCUGAGUUCU | 620 | AGAACUCAGAGAACUUGUCUU |
| 208 | ACAAGUUCUCUGAGUUCUA | 621 | UAGAACUCAGAGAACUUGUUU |
| 209 | CAAGUUCUCUGAGUUCUGA | 622 | UCAGAACUCAGAGAACUUGUU |
| 210 | AAGUUCUCUGAGUUCUGGA | 623 | UCCAGAACUCAGAGAACUUUU |
| 211 | AGUUCUCUGAGUUCUGGGA | 624 | UCCCAGAACUCAGAGAACUUU |
| 212 | GUUCUCUGAGUUCUGGGAU | 625 | AUCCCAGAACUCAGAGAACUU |
| 213 | UUCUCUGAGUUCUGGGAUU | 626 | AAUCCCAGAACUCAGAGAAUU |
| 214 | UCUCUGAGUUCUGGGAUUU | 627 | AAAUCCCAGAACUCAGAGAUU |
| 215 | CUCUGAGUUCUGGGAUUUA | 628 | UAAAUCCCAGAACUCAGAGUU |
| 216 | UCUGAGUUCUGGGAUUUGA | 629 | UCAAAUCCCAGAACUCAGAUU |
| 217 | CUGAGUUCUGGGAUUUGGA | 630 | UCCAAAUCCCAGAACUCAGUU |
| 218 | UGAGUUCUGGGAUUUGGAU | 631 | AUCCAAAUCCCAGAACUCAUU |
| 219 | GAGUUCUGGGAUUUGGACU | 632 | AGUCCAAAUCCCAGAACUCUU |
| 220 | AGUUCUGGGAUUUGGACCU | 633 | AGGUCCAAAUCCCAGAACUUU |
| 221 | UUCUGGGAUUUGGACCCUA | 634 | UAGGGUCCAAAUCCCAGAAUU |
| 222 | UCUGGGAUUUGGACCCUGA | 635 | UCAGGGUCCAAAUCCCAGAUU |
| 223 | CUGGGAUUUGGACCCUGAA | 636 | UUCAGGGUCCAAAUCCCAGUU |
| 224 | UGGGAUUUGGACCCUGAGA | 637 | UCUCAGGGUCCAAAUCCCAUU |
| 225 | GGGAUUUGGACCCUGAGGU | 638 | ACCUCAGGGUCCAAAUCCCUU |
| 226 | GGAUUUGGACCCUGAGGUU | 639 | AACCUCAGGGUCCAAAUCCUU |
| 227 | GAUUUGGACCCUGAGGUCA | 640 | UGACCUCAGGGUCCAAAUCUU |
| 228 | AUUUGGACCCUGAGGUCAA | 641 | UUGACCUCAGGGUCCAAAUUU |
| 229 | UUUGGACCCUGAGGUCAGA | 642 | UCUGACCUCAGGGUCCAAAUU |
| 230 | UUGGACCCUGAGGUCAGAU | 643 | AUCUGACCUCAGGGUCCAAUU |
| 231 | UGGACCCUGAGGUCAGACU | 644 | AGUCUGACCUCAGGGUCCAUU |
| 232 | GGACCCUGAGGUCAGACCA | 645 | UGGUCUGACCUCAGGGUCCUU |
| 233 | GACCCUGAGGUCAGACCAA | 646 | UUGGUCUGACCUCAGGGUCUU |
| 234 | ACCCUGAGGUCAGACCAAU | 647 | AUUGGUCUGACCUCAGGGUUU |
| 235 | CCCUGAGGUCAGACCAACU | 648 | AGUUGGUCUGACCUCAGGGUU |
| 236 | CCUGAGGUCAGACCAACUU | 649 | AAGUUGGUCUGACCUCAGGUU |
| 237 | CUGAGGUCAGACCAACUUU | 650 | AAAGUUGGUCUGACCUCAGUU |
| 238 | UGAGGUCAGACCAACUUCA | 651 | UGAAGUUGGUCUGACCUCAUU |
| 239 | GAGGUCAGACCAACUUCAA | 652 | UUGAAGUUGGUCUGACCUCUU |
| 240 | AGGUCAGACCAACUUCAGU | 653 | ACUGAAGUUGGUCUGACCUUU |
| 241 | GGUCAGACCAACUUCAGCU | 654 | AGCUGAAGUUGGUCUGACCUU |
| 242 | GUCAGACCAACUUCAGCCA | 655 | UGGCUGAAGUUGGUCUGACUU |
| 243 | CCAACUUCAGCCGUGGCUA | 656 | UAGCCACGGCUGAAGUUGGUU |
| 244 | CAACUUCAGCCGUGGCUGU | 657 | ACAGCCACGGCUGAAGUUGUU |
| 245 | AACUUCAGCCGUGGCUGCU | 658 | AGCAGCCACGGCUGAAGUUUU |
| 246 | ACUUCAGCCGUGGCUGCCU | 659 | AGGCAGCCACGGCUGAAGUUU |
| 247 | CUUCAGCCGUGGCUGCCUA | 660 | UAGGCAGCCACGGCUGAAGUU |
| 248 | UUCAGCCGUGGCUGCCUGA | 661 | UCAGGCAGCCACGGCUGAAUU |
| 249 | UCAGCCGUGGCUGCCUGAA | 662 | UUCAGGCAGCCACGGCUGAUU |
| 250 | CAGCCGUGGCUGCCUGAGA | 663 | UCUCAGGCAGCCACGGCUGUU |
| 251 | AGCCGUGGCUGCCUGAGAU | 664 | AUCUCAGGCAGCCACGGCUUU |
| 252 | GCCGUGGCUGCCUGAGACU | 665 | AGUCUCAGGCAGCCACGGCUU |
| 253 | CCGUGGCUGCCUGAGACCU | 666 | AGGUCUCAGGCAGCCACGGUU |
| 254 | CGUGGCUGCCUGAGACCUU | 667 | AAGGUCUCAGGCAGCCACGUU |
| 255 | GUGGCUGCCUGAGACCUCA | 668 | UGAGGUCUCAGGCAGCCACUU |
| 256 | UGGCUGCCUGAGACCUCAA | 669 | UUGAGGUCUCAGGCAGCCAUU |
| 257 | GGCUGCCUGAGACCUCAAU | 670 | AUUGAGGUCUCAGGCAGCCUU |
| 258 | GCUGCCUGAGACCUCAAUA | 671 | UAUUGAGGUCUCAGGCAGCUU |
| 259 | CUGCCUGAGACCUCAAUAU | 672 | AUAUUGAGGUCUCAGGCAGUU |
| 260 | UGCCUGAGACCUCAAUACU | 673 | AGUAUUGAGGUCUCAGGCAUU |
| 261 | GCCUGAGACCUCAAUACCU | 674 | AGGUAUUGAGGUCUCAGGCUU |
| 262 | CCUGAGACCUCAAUACCCU | 675 | AGGGUAUUGAGGUCUCAGGUU |
| 263 | UGAGACCUCAAUACCCCAA | 676 | UUGGGGUAUUGAGGUCUCAUU |
| 264 | GAGACCUCAAUACCCCAAA | 677 | UUUGGGGUAUUGAGGUCUCUU |
| 265 | AGACCUCAAUACCCCAAGU | 678 | ACUUGGGGUAUUGAGGUCUUU |
| 266 | GACCUCAAUACCCCAAGUU | 679 | AACUUGGGGUAUUGAGGUCUU |
| 267 | ACCUCAAUACCCCAAGUCU | 680 | AGACUUGGGGUAUUGAGGUUU |
| 268 | CCUCAAUACCCCAAGUCCA | 681 | UGGACUUGGGGUAUUGAGGUU |
| 269 | CUCAAUACCCCAAGUCCAU | 682 | AUGGACUUGGGGUAUUGAGUU |
| 270 | UCAAUACCCCAAGUCCACU | 683 | AGUGGACUUGGGGUAUUGAUU |
| 271 | CAAUACCCCAAGUCCACCU | 684 | AGGUGGACUUGGGGUAUUGUU |
| 272 | AAUACCCCAAGUCCACCUA | 685 | UAGGUGGACUUGGGGUAUUUU |
| 273 | AUACCCCAAGUCCACCUGU | 686 | ACAGGUGGACUUGGGGUAUUU |
| 274 | UACCCCAAGUCCACCUGCU | 687 | AGCAGGUGGACUUGGGGUAUU |
| 275 | CCCCAAGUCCACCUGCCUA | 688 | UAGGCAGGUGGACUUGGGGUU |
| 276 | CCCAAGUCCACCUGCCUAU | 689 | AUAGGCAGGUGGACUUGGGUU |
| 277 | CCAAGUCCACCUGCCUAUU | 690 | AAUAGGCAGGUGGACUUGGUU |
| 278 | CAAGUCCACCUGCCUAUCU | 691 | AGAUAGGCAGGUGGACUUGUU |
| 279 | AAGUCCACCUGCCUAUCCA | 692 | UGGAUAGGCAGGUGGACUUUU |
| 280 | AGUCCACCUGCCUAUCCAU | 693 | AUGGAUAGGCAGGUGGACUUU |
| 281 | GUCCACCUGCCUAUCCAUU | 694 | AAUGGAUAGGCAGGUGGACUU |
| 282 | UCCACCUGCCUAUCCAUCU | 695 | AGAUGGAUAGGCAGGUGGAUU |
| 283 | CCACCUGCCUAUCCAUCCU | 696 | AGGAUGGAUAGGCAGGUGGUU |
| 284 | CACCUGCCUAUCCAUCCUA | 697 | UAGGAUGGAUAGGCAGGUGUU |
| 285 | ACCUGCCUAUCCAUCCUGU | 698 | ACAGGAUGGAUAGGCAGGUUU |
| 286 | CCUGCCUAUCCAUCCUGCA | 699 | UGCAGGAUGGAUAGGCAGGUU |
| 287 | CUGCCUAUCCAUCCUGCGA | 700 | UCGCAGGAUGGAUAGGCAGUU |
| 288 | UGCCUAUCCAUCCUGCGAA | 701 | UUCGCAGGAUGGAUAGGCAUU |
| 289 | GCCUAUCCAUCCUGCGAGU | 702 | ACUCGCAGGAUGGAUAGGCUU |
| 290 | CCUAUCCAUCCUGCGAGCU | 703 | AGCUCGCAGGAUGGAUAGGUU |
| 291 | CUAUCCAUCCUGCGAGCUU | 704 | AAGCUCGCAGGAUGGAUAGUU |
| 292 | UAUCCAUCCUGCGAGCUCU | 705 | AGAGCUCGCAGGAUGGAUAUU |
| 293 | AUCCAUCCUGCGAGCUCCU | 706 | AGGAGCUCGCAGGAUGGAUUU |
| 294 | UCCAUCCUGCGAGCUCCUU | 707 | AAGGAGCUCGCAGGAUGGAUU |
| 295 | CCAUCCUGCGAGCUCCUUA | 708 | UAAGGAGCUCGCAGGAUGGUU |
| 296 | CAUCCUGCGAGCUCCUUGA | 709 | UCAAGGAGCUCGCAGGAUGUU |
| 297 | AUCCUGCGAGCUCCUUGGA | 710 | UCCAAGGAGCUCGCAGGAUUU |
| 298 | UCCUGCGAGCUCCUUGGGU | 711 | ACCCAAGGAGCUCGCAGGAUU |
| 299 | CCUGCGAGCUCCUUGGGUU | 712 | AACCCAAGGAGCUCGCAGGUU |
| 300 | CUGCGAGCUCCUUGGGUCU | 713 | AGACCCAAGGAGCUCGCAGUU |
| 301 | UGCGAGCUCCUUGGGUCCU | 714 | AGGACCCAAGGAGCUCGCAUU |
| 302 | GCGAGCUCCUUGGGUCCUA | 715 | UAGGACCCAAGGAGCUCGCUU |
| 303 | CGAGCUCCUUGGGUCCUGU | 716 | ACAGGACCCAAGGAGCUCGUU |
| 304 | GAGCUCCUUGGGUCCUGCA | 717 | UGCAGGACCCAAGGAGCUCUU |
| 305 | AGCUCCUUGGGUCCUGCAA | 718 | UUGCAGGACCCAAGGAGCUUU |
| 306 | GCUCCUUGGGUCCUGCAAU | 719 | AUUGCAGGACCCAAGGAGCUU |
| 307 | CUCCUUGGGUCCUGCAAUU | 720 | AAUUGCAGGACCCAAGGAGUU |
| 308 | UCCUUGGGUCCUGCAAUCU | 721 | AGAUUGCAGGACCCAAGGAUU |
| 309 | CCUUGGGUCCUGCAAUCUU | 722 | AAGAUUGCAGGACCCAAGGUU |
| 310 | CUUGGGUCCUGCAAUCUCU | 723 | AGAGAUUGCAGGACCCAAGUU |
| 311 | UUGGGUCCUGCAAUCUCCA | 724 | UGGAGAUUGCAGGACCCAAUU |
| 312 | UGGGUCCUGCAAUCUCCAA | 725 | UUGGAGAUUGCAGGACCCAUU |
| 313 | GGGUCCUGCAAUCUCCAGA | 726 | UCUGGAGAUUGCAGGACCCUU |
| 314 | GGUCCUGCAAUCUCCAGGA | 727 | UCCUGGAGAUUGCAGGACCUU |
| 315 | GUCCUGCAAUCUCCAGGGU | 728 | ACCCUGGAGAUUGCAGGACUU |
| 316 | CCUGCAAUCUCCAGGGCUA | 729 | UAGCCCUGGAGAUUGCAGGUU |
| 317 | CUGCAAUCUCCAGGGCUGU | 730 | ACAGCCCUGGAGAUUGCAGUU |
| 318 | UGCAAUCUCCAGGGCUGCU | 731 | AGCAGCCCUGGAGAUUGCAUU |
| 319 | CUCCAGGGCUGCCCCUGUA | 732 | UACAGGGGCAGCCCUGGAGUU |
| 320 | UCCAGGGCUGCCCCUGUAA | 733 | UUACAGGGGCAGCCCUGGAUU |
| 321 | CCAGGGCUGCCCCUGUAGA | 734 | UCUACAGGGGCAGCCCUGGUU |
| 322 | CAGGGCUGCCCCUGUAGGU | 735 | ACCUACAGGGGCAGCCCUGUU |
| 323 | AGGGCUGCCCCUGUAGGUU | 736 | AACCUACAGGGGCAGCCCUUU |
| 324 | GGGCUGCCCCUGUAGGUUA | 737 | UAACCUACAGGGGCAGCCCUU |
| 325 | GGCUGCCCCUGUAGGUUGU | 738 | ACAACCUACAGGGGCAGCCUU |
| 326 | GCUGCCCCUGUAGGUUGCU | 739 | AGCAACCUACAGGGGCAGCUU |
| 327 | CUGCCCCUGUAGGUUGCUU | 740 | AAGCAACCUACAGGGGCAGUU |
| 328 | UGCCCCUGUAGGUUGCUUA | 741 | UAAGCAACCUACAGGGGCAUU |
| 329 | GCCCCUGUAGGUUGCUUAA | 742 | UUAAGCAACCUACAGGGGCUU |
| 330 | CCCCUGUAGGUUGCUUAAA | 743 | UUUAAGCAACCUACAGGGGUU |
| 331 | CCCUGUAGGUUGCUUAAAA | 744 | UUUUAAGCAACCUACAGGGUU |
| 332 | CCUGUAGGUUGCUUAAAAA | 745 | UUUUUAAGCAACCUACAGGUU |
| 333 | CUGUAGGUUGCUUAAAAGA | 746 | UCUUUUAAGCAACCUACAGUU |
| 334 | UGUAGGUUGCUUAAAAGGA | 747 | UCCUUUUAAGCAACCUACAUU |
| 335 | GUAGGUUGCUUAAAAGGGA | 748 | UCCCUUUUAAGCAACCUACUU |
| 336 | UAGGUUGCUUAAAAGGGAU | 749 | AUCCCUUUUAAGCAACCUAUU |
| 337 | AGGUUGCUUAAAAGGGACA | 750 | UGUCCCUUUUAAGCAACCUUU |
| 338 | GGUUGCUUAAAAGGGACAA | 751 | UUGUCCCUUUUAAGCAACCUU |
| 339 | GUUGCUUAAAAGGGACAGU | 752 | ACUGUCCCUUUUAAGCAACUU |
| 340 | UUGCUUAAAAGGGACAGUA | 753 | UACUGUCCCUUUUAAGCAAUU |
| 341 | UGCUUAAAAGGGACAGUAU | 754 | AUACUGUCCCUUUUAAGCAUU |
| 342 | GCUUAAAAGGGACAGUAUU | 755 | AAUACUGUCCCUUUUAAGCUU |
| 343 | CUUAAAAGGGACAGUAUUU | 756 | AAAUACUGUCCCUUUUAAGUU |
| 344 | UUAAAAGGGACAGUAUUCU | 757 | AGAAUACUGUCCCUUUUAAUU |
| 345 | UAAAAGGGACAGUAUUCUU | 758 | AAGAAUACUGUCCCUUUUAUU |
| 346 | AAAAGGGACAGUAUUCUCA | 759 | UGAGAAUACUGUCCCUUUUUU |
| 347 | AAAGGGACAGUAUUCUCAA | 760 | UUGAGAAUACUGUCCCUUUUU |
| 348 | AAGGGACAGUAUUCUCAGU | 761 | ACUGAGAAUACUGUCCCUUUU |
| 349 | AGGGACAGUAUUCUCAGUA | 762 | UACUGAGAAUACUGUCCCUUU |
| 350 | GGGACAGUAUUCUCAGUGU | 763 | ACACUGAGAAUACUGUCCCUU |
| 351 | GGACAGUAUUCUCAGUGCU | 764 | AGCACUGAGAAUACUGUCCUU |
| 352 | GACAGUAUUCUCAGUGCUU | 765 | AAGCACUGAGAAUACUGUCUU |
| 353 | ACAGUAUUCUCAGUGCUCU | 766 | AGAGCACUGAGAAUACUGUUU |
| 354 | CAGUAUUCUCAGUGCUCUU | 767 | AAGAGCACUGAGAAUACUGUU |
| 355 | AGUAUUCUCAGUGCUCUCU | 768 | AGAGAGCACUGAGAAUACUUU |
| 356 | GUAUUCUCAGUGCUCUCCU | 769 | AGGAGAGCACUGAGAAUACUU |
| 357 | UAUUCUCAGUGCUCUCCUA | 770 | UAGGAGAGCACUGAGAAUAUU |
| 358 | AUUCUCAGUGCUCUCCUAU | 771 | AUAGGAGAGCACUGAGAAUUU |
| 359 | UUCUCAGUGCUCUCCUACU | 772 | AGUAGGAGAGCACUGAGAAUU |
| 360 | UCUCAGUGCUCUCCUACCU | 773 | AGGUAGGAGAGCACUGAGAUU |
| 361 | CUCAGUGCUCUCCUACCCU | 774 | AGGGUAGGAGAGCACUGAGUU |
| 362 | GCUCUCCUACCCCACCUCA | 775 | UGAGGUGGGGUAGGAGAGCUU |
| 363 | CUCUCCUACCCCACCUCAU | 776 | AUGAGGUGGGGUAGGAGAGUU |
| 364 | UCUCCUACCCCACCUCAUA | 777 | UAUGAGGUGGGGUAGGAGAUU |
| 365 | CUCCUACCCCACCUCAUGU | 778 | ACAUGAGGUGGGGUAGGAGUU |
| 366 | UCCUACCCCACCUCAUGCU | 779 | AGCAUGAGGUGGGGUAGGAUU |
| 367 | CCUACCCCACCUCAUGCCU | 780 | AGGCAUGAGGUGGGGUAGGUU |
| 368 | CUACCCCACCUCAUGCCUA | 781 | UAGGCAUGAGGUGGGGUAGUU |
| 369 | UACCCCACCUCAUGCCUGA | 782 | UCAGGCAUGAGGUGGGGUAUU |
| 370 | CCCCUCCAGGCAUGCUGGU | 783 | ACCAGCAUGCCUGGAGGGGUU |
| 371 | CCCUCCAGGCAUGCUGGCU | 784 | AGCCAGCAUGCCUGGAGGGUU |
| 372 | CCUCCAGGCAUGCUGGCCU | 785 | AGGCCAGCAUGCCUGGAGGUU |
| 373 | CUCCAGGCAUGCUGGCCUU | 786 | AAGGCCAGCAUGCCUGGAGUU |
| 374 | AGGCAUGCUGGCCUCCCAA | 787 | UUGGGAGGCCAGCAUGCCUUU |
| 375 | GGCAUGCUGGCCUCCCAAU | 788 | AUUGGGAGGCCAGCAUGCCUU |
| 376 | GCAUGCUGGCCUCCCAAUA | 789 | UAUUGGGAGGCCAGCAUGCUU |
| 377 | CAUGCUGGCCUCCCAAUAA | 790 | UUAUUGGGAGGCCAGCAUGUU |
| 378 | AUGCUGGCCUCCCAAUAAA | 791 | UUUAUUGGGAGGCCAGCAUUU |
| 379 | UGCUGGCCUCCCAAUAAAA | 792 | UUUUAUUGGGAGGCCAGCAUU |
| 380 | GCUGGCCUCCCAAUAAAGU | 793 | ACUUUAUUGGGAGGCCAGCUU |
| 381 | CUGGCCUCCCAAUAAAGCU | 794 | AGCUUUAUUGGGAGGCCAGUU |
| 382 | UGGCCUCCCAAUAAAGCUA | 795 | UAGCUUUAUUGGGAGGCCAUU |
| 383 | GGCCUCCCAAUAAAGCUGA | 796 | UCAGCUUUAUUGGGAGGCCUU |
| 384 | GCCUCCCAAUAAAGCUGGA | 797 | UCCAGCUUUAUUGGGAGGCUU |
| 385 | CCUCCCAAUAAAGCUGGAU | 798 | AUCCAGCUUUAUUGGGAGGUU |
| 386 | CUCCCAAUAAAGCUGGACA | 799 | UGUCCAGCUUUAUUGGGAGUU |
| 387 | UCCCAAUAAAGCUGGACAA | 800 | UUGUCCAGCUUUAUUGGGAUU |
| 388 | CCCAAUAAAGCUGGACAAA | 801 | UUUGUCCAGCUUUAUUGGGUU |
| 389 | CCAAUAAAGCUGGACAAGA | 802 | UCUUGUCCAGCUUUAUUGGUU |
| 390 | CAAUAAAGCUGGACAAGAA | 803 | UUCUUGUCCAGCUUUAUUGUU |
| 391 | AAUAAAGCUGGACAAGAAA | 804 | UUUCUUGUCCAGCUUUAUUUU |
| 392 | AUAAAGCUGGACAAGAAGU | 805 | ACUUCUUGUCCAGCUUUAUUU |
| 393 | UAAAGCUGGACAAGAAGCU | 806 | AGCUUCUUGUCCAGCUUUAUU |
| 394 | AAAGCUGGACAAGAAGCUA | 807 | UAGCUUCUUGUCCAGCUUUUU |
| 395 | AAGCUGGACAAGAAGCUGU | 808 | ACAGCUUCUUGUCCAGCUUUU |
| 396 | AGCUGGACAAGAAGCUGCU | 809 | AGCAGCUUCUUGUCCAGCUUU |
| 397 | GCUGGACAAGAAGCUGCUA | 810 | UAGCAGCUUCUUGUCCAGCUU |
| 398 | CUGGACAAGAAGCUGCUAU | 811 | AUAGCAGCUUCUUGUCCAGUU |
| 399 | UGGACAAGAAGCUGCUAUA | 812 | UAUAGCAGCUUCUUGUCCAUU |
| 400 | GGACAAGAAGCUGCUAUGA | 813 | UCAUAGCAGCUUCUUGUCCUU |
| 401 | ACGGGACAGUAUUCUCAGUG A | 814 | UCACUGAGAAUACUGUCCCGU |
| 402 | AAGGGACAGUAUUCUCAGUG C | 815 | GCACUGAGAAUACUGUCCCUU UU |
| 403 | UUAAAAGGGACAGUAUUCU | 816 | AGAAUACUGUCCCUUUUAAUU |
| 404 | AAGGGACAGUAUUCUCAGU | 817 | ACUGAGAAUACUGUCCCUUUU |
| 405 | CCAAGUCCACCUGCCUAUU | 818 | AAUAGGCAGGUGGACUUGGUU |
| 406 | CCGUUAAGGACAAGUUCUU | 819 | AAGAACUUGUCCUUAACGGUU |
| 407 | CGAGGAUGCCUCCCUUCUU | 820 | AAGAAGGGAGGCAUCCUCGUU |
| 408 | AAGGGACAGUAUUCUCAGA | 821 | UCUGAGAAUACUGUCCCUUUU |
| 409 | AAAAGGGACAGUAUUCUCAG U | 822 | ACUGAGAAUACUGUCCCUUUU |
| 410 | AAAAGGGACAGUAUUCUCAG U | 823 | ACUGAGAAUACUGUCCCUUUU |
| 411 | AAAAGGGACAGUAUUCUCAG U | 824 | ACUGAGAAUACUGUCCCUUUU AA |
| 412 | AAGGGACAGUAUUCUCAGA | 825 | UCUGAGAAUACUGUCCCUUUU |
| 413 | AAGGGACAGUAUUCUCAGA | 826 | UCUGAGAAUACUGUCCCUUUU |

Table 4 below shows the modified RNA sequences used in the present invention.

The abbreviations used herein have the meaning as follows:
A. U, G, and C represent a natural adenine ribonucleotide, an uracil ribonucleotide, a guanine ribonucleotide, and a cytosine ribonucleotide, respectively.
i or I represents an inosine ribonucleotide.
m represents that the left nucleotide is a 2'-OCH₃ modified nucleotide. For example, Am, Um, Gm, and Cm represent 2'-OCH₃ modified A, U, G, and C, respectively.
f represents that the left nucleotide is a 2'-F modified nucleotide. For example, Af, Uf, Gf, and Cf represent 2'-F modified A, U, G, and C, respectively.
"s" or "s-" represents that the two flanking nucleotides and/or the delivery moiety are linked by a phosphorothioate group.
VP represents that the right nucleotide is a vinylphosphonate modified nucleotide. See, for example, PCT Publication Nos. WO2011139702, WO2013033230, and WO2019105419.
IB represents an inverted abasic deoxyribonucleotide, which can be of the following three structures depending on its position/linking method in siRNA (for the 5' end, interstrand, and 3' end of the nucleic acid strand, respectively):

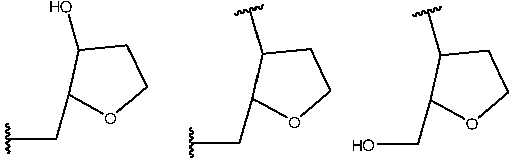

IB is well known in the art. See, for example, F. Czauderna, Nucleic Acids Res., 2003, 31(11), 2705-16 and PCT Publication Nos. WO2016011123 and WO2019051402. L96 represents a GalNAc delivery moiety of the following structure well known in the art, wherein 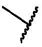 represents a position linked to siRNA by a phosphate group or a phosphorothioate group. See, for example, PCT Publication Nos. WO2009073809 and WO2009082607.

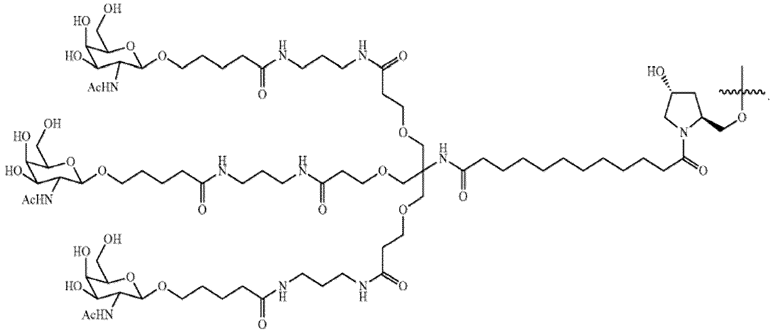

NAG37 represents a GalNAc delivery moiety of the following structure well known in the art, wherein 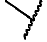 represents a position linked to siRNA. See, for example, PCT Publication No. WO2018044350.

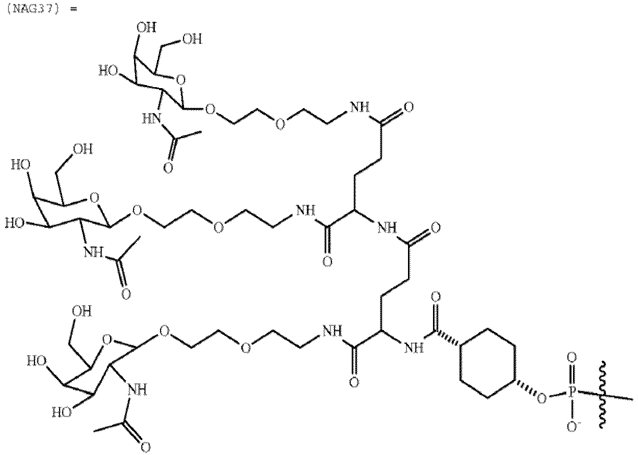

GL6 represents a GalNAc delivery moiety of the following structure, wherein 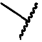 represents a position linked to siRNA by a phosphate group or a phosphorothioate group:

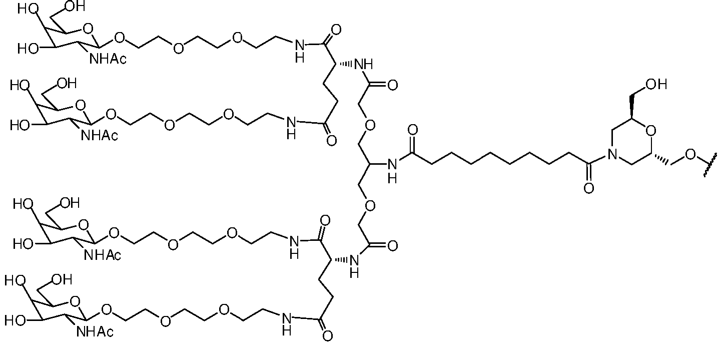

GL12 represents a GalNAc delivery moiety of the following structure, wherein 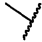 represents a position linked to siRNA by a phosphate group or a phosphorothioate group. The related intermediate synthesis methods are described in the examples below.

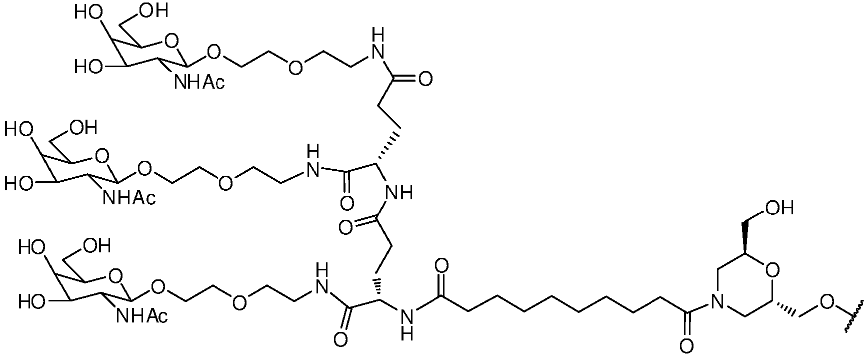

STM1 represents a nucleotide substitute of the following structure. The related intermediate synthesis methods are described in Example 15 below.

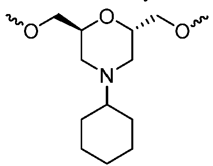

PCN represents a nucleotide substitute of the following structure, wherein Base can be any base. For example, PCN-A represents that Base is adenine. The related intermediate synthesis methods are described in Example 17 below.

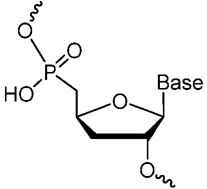

CPla represents a nucleotide substitute of the following structure, wherein Base can be any base. For example, CP1a-U represents that Base is uracil. The related intermediate synthesis methods are described in Example 16 below.

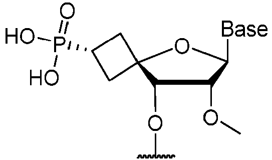

**Table 4 Modified RNA Sequences of the Sense Strand of siRNA Targeting APOC3 Gene Used in the Present Invention**

| Sense Strand No. | Sequence (5'→3') | SEQ ID NO: |
|---|---|---|
| SR000001S | AmsGmsGmCmAmGmCfUfGfCmUmCmCmAmGmGmAmAmUm | 827 |
| SR000002S | GmsGmsCmAmGmCmUfGfCfUmCmCmAmGmGmAmAmCmAm | 828 |
| SR000003S | GmsCmsAmGmCmUmGfCfUfCmCmAmGmGmAmAmCmAmAm | 829 |
| SR000004S | CmsAmsGmCmUmGmCfUfCfCmAmGmGmAmAmCmAmGmAm | 830 |
| SR000005S | AmsGmsCmUmGmCmUfCfCfAmGmGmAmAmCmAmGmAmAm | 831 |
| SR000006S | GmsCmsUmGmCmUmCfCfAfGmGmAmAmCmAmGmAmGmAm | 832 |
| SR000007S | UmsGmsCmUmCmCmAfGfGfAmAmCmAmGmAmGmGmUmAm | 833 |
| SR000008S | CmsAmsGmGmAmAmCfAfGfAmGmGmUmGmCmCmAmUmAm | 834 |
| SR000009S | AmsGmsGmAmAmCmAfGfAfGmGmUmGmCmCmAmUmGmUm | 835 |
| SR000010S | GmsGmsAmAmCmAmGfAfGfGmUmGmCmCmAmUmGmCmAm | 836 |
| SR000011S | GmsAmsAmCmAmGmAfGfGfUmGmCmCmAmUmGmCmAmAm | 837 |
| SR000012S | AmsAmsCmAmGmAmGfGfUfGmCmCmAmUmGmCmAmGmUm | 838 |
| SR000013S | CmsAmsUmGmCmAmGfCfCfCmCmGmGmGmUmAmCmUmUm | 839 |
| SR000014S | AmsUmsGmCmAmGmCfCfCfCmGmGmGmUmAmCmUmCmUm | 840 |
| SR000015S | UmsGmsCmAmGmCmCfCfCfGmGmGmUmAmCmUmCmCmUm | 841 |
| SR000016S | GmsCmsAmGmCmCmCfCfGfGmGmUmAmCmUmCmCmUmUm | 842 |
| SR000017S | CmsAmsGmCmCmCmCfGfGfGmUmAmCmUmCmCmUmUmAm | 843 |
| SR000018S | AmsGmsCmCmCmCmGfGfGfUmAmCmUmCmCmUmUmGmUm | 844 |
| SR000019S | GmsCmsCmCmCmGmGfGfUfAmCmUmCmCmUmUmGmUmUm | 845 |
| SR000020S | CmsCmsCmCmGmGmGfUfAfCmUmCmCmUmUmGmUmUmAm | 846 |
| SR000021S | CmsCmsCmGmGmGmUfAfCfUmCmCmUmUmGmUmUmGmUm | 847 |
| SR000022S | CmsCmsGmGmGmUmAfCfUfCmCmUmUmGmUmUmGmUmUm | 848 |
| SR000023S | CmsGmsGmGmUmAmCfUfCfCmUmUmGmUmUmGmUmUmAm | 849 |
| SR000024S | GmsGmsGmUmAmCmUfCfCfUmUmGmUmUmGmUmUmGmUm | 850 |
| SR000025S | GmsGmsUmAmCmUmCfCfUfUmGmUmUmGmUmUmGmCmUm | 851 |
| SR000026S | GmsUmsAmCmUmCmCfUfUfGmUmUmGmUmUmGmCmCmUm | 852 |
| SR000027S | AmsCmsUmCmCmUmUfGfUfUmGmUmUmGmCmCmCmUmUm | 853 |
| SR000028S | CmsUmsCmCmUmUmGfUfUfGmUmUmGmCmCmCmUmCmUm | 854 |
| SR000029S | UmsCmsCmUmUmGmUfUfGfUmUmGmCmCmCmUmCmCmUm | 855 |
| SR000030S | CmsCmsUmUmGmUmUfGfUfUmGmCmCmCmUmCmCmUmAm | 856 |
| SR000031S | CmsUmsUmGmUmUmGfUfUfGmCmCmCmUmCmCmUmGmAm | 857 |
| SR000032S | UmsUmsGmUmUmGmUfUfGfCmCmCmUmCmCmUmGmGmUm | 858 |
| SR000033S | UmsGmsUmUmGmUmUfGfCfCmCmUmCmCmUmGmGmCmAm | 859 |
| SR000034S | GmsCmsCmCmUmCmCfUfGfGmCmGmCmUmCmCmUmGmAm | 860 |
| SR000035S | UmsCmsCmUmGmGmCfGfCfUmCmCmUmGmGmCmCmUmUm | 861 |
| SR000036S | CmsUmsGmGmCmGmCfUfCfCmUmGmGmCmCmUmCmUmAm | 862 |
| SR000037S | UmsGmsGmCmGmCmUfCfCfUmGmGmCmCmUmCmUmGmUm | 863 |
| SR000038S | GmsGmsCmGmCmUmCfCfUfGmGmCmCmUmCmUmGmCmUm | 864 |
| SR000039S | GmsCmsGmCmUmCmCfUfGfGmCmCmUmCmUmGmCmCmUm | 865 |
| SR000040S | CmsUmsGmGmCmCmUfCfUfGmCmCmCmGmAmGmCmUmUm | 866 |
| SR000041S | UmsGmsGmCmCmUmCfUfGfCmCmCmGmAmGmCmUmUmUm | 867 |
| SR000042S | GmsGmsCmCmUmCmUfGfCfCmCmGmAmGmCmUmUmCmAm | 868 |
| SR000043S | GmsCmsCmUmCmUmGfCfCfCmGmAmGmCmUmUmCmAmAm | 869 |
| SR000044S | CmsCmsUmCmUmGmCfCfCfGmAmGmCmUmUmCmAmGmAm | 870 |
| SR000045S | CmsUmsCmUmGmCmCfCfGfAmGmCmUmUmCmAmGmAmAm | 871 |
| SR000046S | UmsCmsUmGmCmCmCfGfAfGmCmUmUmCmAmGmAmGmAm | 872 |
| SR000047S | CmsUmsGmCmCmCmGfAfGfCmUmUmCmAmGmAmGmGmUm | 873 |
| SR000048S | UmsGmsCmCmCmGmAfGfCfUmUmCmAmGmAmGmGmCmUm | 874 |
| SR000049S | AmsGmsCmUmUmCmAfGfAfGmGmCmCmGmAmGmGmAmUm | 875 |
| SR000050S | GmsCmsUmUmCmAmGfAfGfGmCmCmGmAmGmGmAmUmAm | 876 |
| SR000051S | CmsUmsUmCmAmGmAfGfGfCmCmGmAmGmGmAmUmGmUm | 877 |
| SR000052S | UmsUmsCmAmGmAmGfGfCfCmGmAmGmGmAmUmGmCmUm | 878 |
| SR000053S | UmsCmsAmGmAmGmGfCfCfGmAmGmGmAmUmGmCmCmUm | 879 |
| SR000054S | CmsAmsGmAmGmGmCfCfGfAmGmGmAmUmGmCmCmUmUm | 880 |
| SR000055S | AmsGmsAmGmGmCmCfGfAfGmGmAmUmGmCmCmUmCmUm | 881 |
| SR000056S | GmsAmsGmGmCmCmGfAfGfGmAmUmGmCmCmUmCmCmUm | 882 |
| SR000057S | GmsGmsCmCmGmAmGfGfAfUmGmCmCmUmCmCmCmUmUm | 883 |
| SR000058S | GmsCmsCmGmAmGmGfAfUfGmCmCmUmCmCmCmUmUmUm | 884 |
| SR000059S | CmsCmsGmAmGmGmAfUfGfCmCmUmCmCmCmUmUmCmUm | 885 |
| SR000060S | CmsGmsAmGmGmAmUfGfCfCmUmCmCmCmUmUmCmUmUm | 886 |
| SR000061S | GmsAmsGmGmAmUmGfCfCfUmCmCmCmUmUmCmUmCmAm | 887 |
| SR000062S | AmsGmsGmAmUmGmCfCfUfCmCmCmUmUmCmUmCmAmAm | 888 |
| SR000063S | GmsGmsAmUmGmCmCfUfCfCmCmUmUmCmUmCmAmGmUm | 889 |
| SR000064S | GmsAmsUmGmCmCmUfCfCfCmUmUmCmUmCmAmGmCmUm | 890 |
| SR000065S | AmsUmsGmCmCmUmCfCfCfUmUmCmUmCmAmGmCmUmUm | 891 |
| SR000066S | UmsGmsCmCmUmCmCfCfUfUmCmUmCmAmGmCmUmUmUm | 892 |
| SR000067S | GmsCmsCmUmCmCmCfUfUfCmUmCmAmGmCmUmUmCmAm | 893 |
| SR000068S | CmsCmsUmCmCmCmUfUfCfUmCmAmGmCmUmUmCmAmUm | 894 |
| SR000069S | CmsUmsCmCmCmUmUfCfUfCmAmGmCmUmUmCmAmUmAm | 895 |
| SR000070S | UmsCmsCmCmUmUmCfUfCfAmGmCmUmUmCmAmUmGmUm | 896 |
| SR000071S | CmsCmsCmUmUmCmUfCfAfGmCmUmUmCmAmUmGmCmAm | 897 |
| SR000072S | CmsCmsUmUmCmUmCfAfGfCmUmUmCmAmUmGmCmAmAm | 898 |
| SR000073S | CmsUmsUmCmUmCmAfGfCfUmUmCmAmUmGmCmAmGmAm | 899 |
| SR000074S | UmsUmsCmUmCmAmGfCfUfUmCmAmUmGmCmAmGmGmAm | 900 |
| SR000075S | UmsCmsUmCmAmGmCfUfUfCmAmUmGmCmAmGmGmGmUm | 901 |
| SR000076S | CmsUmsCmAmGmCmUfUfCfAmUmGmCmAmGmGmGmUmUm | 902 |
| SR000077S | UmsCmsAmGmCmUmUfCfAfUmGmCmAmGmGmGmUmUmAm | 903 |
| SR000078S | CmsAmsGmCmUmUmCfAfUfGmCmAmGmGmGmUmUmAmUm | 904 |
| SR000079S | AmsGmsCmUmUmCmAfUfGfCmAmGmGmGmUmUmAmCmAm | 905 |
| SR000080S | GmsCmsUmUmCmAmUfGfCfAmGmGmGmUmUmAmCmAmUm | 906 |
| SR000081S | CmsUmsUmCmAmUmGfCfAfGmGmGmUmUmAmCmAmUmAm | 907 |
| SR000082S | UmsUmsCmAmUmGmCfAfGfGmGmUmUmAmCmAmUmGmAm | 908 |
| SR000083S | UmsCmsAmUmGmCmAfGfGfGmUmUmAmCmAmUmGmAmAm | 909 |
| SR000084S | CmsAmsUmGmCmAmGfGfGfUmUmAmCmAmUmGmAmAmAm | 910 |
| SR000085S | AmsUmsGmCmAmGmGfGfUfUmAmCmAmUmGmAmAmGmUm | 911 |
| SR000086S | UmsGmsCmAmGmGmGfUfUfAmCmAmUmGmAmAmGmCmAm | 912 |
| SR000087S | GmsCmsAmGmGmGmUfUfAfCmAmUmGmAmAmGmCmAmUm | 913 |
| SR000088S | CmsAmsGmGmGmUmUfAfCfAmUmGmAmAmGmCmAmCmAm | 914 |
| SR000089S | AmsGmsGmGmUmUmAfCfAfUmGmAmAmGmCmAmCmGmUm | 915 |
| SR000090S | GmsGmsGmUmUmAmCfAfUfGmAmAmGmCmAmCmGmCmUm | 916 |
| SR000091S | GmsUmsUmAmCmAmUfGfAfAmGmCmAmCmGmCmCmAmUm | 917 |
| SR000092S | AmsCmsAmUmGmAmAfGfCfAmCmGmCmCmAmCmCmAmAm | 918 |
| SR000093S | CmsAmsUmGmAmAmGfCfAfCmGmCmCmAmCmCmAmAmAm | 919 |
| SR000094S | AmsUmsGmAmAmGmCfAfCfGmCmCmAmCmCmAmAmGmAm | 920 |
| SR000095S | UmsGmsAmAmGmCmAfCfGfCmCmAmCmCmAmAmGmAmUm | 921 |
| SR000096S | GmsAmsAmGmCmAmCfGfCfCmAmCmCmAmAmGmAmCmUm | 922 |
| SR000097S | AmsAmsGmCmAmCmGfCfCfAmCmCmAmAmGmAmCmCmAm | 923 |
| SR000098S | AmsGmsCmAmCmGmCfCfAfCmCmAmAmGmAmCmCmGmUm | 924 |
| SR000099S | GmsCmsAmCmGmCmCfAfCfCmAmAmGmAmCmCmGmCmUm | 925 |
| SR000100S | CmsGmsCmCmAmCmCfAfAfGmAmCmCmGmCmCmAmAmAm | 926 |
| SR000101S | GmsCmsCmAmCmCmAfAfGfAmCmCmGmCmCmAmAmGmAm | 927 |
| SR000102S | CmsCmsAmCmCmAmAfGfAfCmCmGmCmCmAmAmGmGmAm | 928 |
| SR000103S | CmsAmsCmCmAmAmGfAfCfCmGmCmCmAmAmGmGmAmUm | 929 |
| SR000104S | AmsCmsCmAmAmGmAfCfCfGmCmCmAmAmGmGmAmUmAm | 930 |
| SR000105S | CmsCmsAmAmGmAmCfCfGfCmCmAmAmGmGmAmUmGmUm | 931 |
| SR000106S | CmsAmsAmGmAmCmCfGfCfCmAmAmGmGmAmUmGmCmAm | 932 |
| SR000107S | AmsAmsGmAmCmCmGfCfCfAmAmGmGmAmUmGmCmAmUm | 933 |
| SR000108S | AmsGmsAmCmCmGmCfCfAfAmGmGmAmUmGmCmAmCmUm | 934 |
| SR000109S | GmsAmsCmCmGmCmCfAfAfGmGmAmUmGmCmAmCmUmAm | 935 |
| SR000110S | AmsCmsCmGmCmCmAfAfGfGmAmUmGmCmAmCmUmGmAm | 936 |
| SR000111S | CmsCmsGmCmCmAmAfGfGfAmUmGmCmAmCmUmGmAmAm | 937 |
| SR000112S | CmsGmsCmCmAmAmGfGfAfUmGmCmAmCmUmGmAmGmUm | 938 |
| SR000113S | GmsCmsCmAmAmGmGfAfUfGmCmAmCmUmGmAmGmCmAm | 939 |
| SR000114S | CmsCmsAmAmGmGmAfUfGfCmAmCmUmGmAmGmCmAmAm | 940 |
| SR000115S | CmsAmsAmGmGmAmUfGfCfAmCmUmGmAmGmCmAmGmUm | 941 |
| SR000116S | AmsAmsGmGmAmUmGfCfAfCmUmGmAmGmCmAmGmCmAm | 942 |
| SR000117S | AmsGmsGmAmUmGmCfAfCfUmGmAmGmCmAmGmCmGmUm | 943 |
| SR000118S | GmsGmsAmUmGmCmAfCfUfGmAmGmCmAmGmCmGmUmAm | 944 |
| SR000119S | GmsAmsUmGmCmAmCfUfGfAmGmCmAmGmCmGmUmGmUm | 945 |
| SR000120S | AmsUmsGmCmAmCmUfGfAfGmCmAmGmCmGmUmGmCmAm | 946 |
| SR000121S | UmsGmsCmAmCmUmGfAfGfCmAmGmCmGmUmGmCmAmAm | 947 |
| SR000122S | GmsCmsAmCmUmGmAfGfCfAmGmCmGmUmGmCmAmGmAm | 948 |
| SR000123S | CmsAmsCmUmGmAmGfCfAfGmCmGmUmGmCmAmGmGmAm | 949 |
| SR000124S | AmsCmsUmGmAmGmCfAfGfCmGmUmGmCmAmGmGmAmAm | 950 |
| SR000125S | CmsUmsGmAmGmCmAfGfCfGmUmGmCmAmGmGmAmGmUm | 951 |
| SR000126S | UmsGmsAmGmCmAmGfCfGfUmGmCmAmGmGmAmGmUmUm | 952 |
| SR000127S | GmsAmsGmCmAmGmCfGfUfGmCmAmGmGmAmGmUmCmUm | 953 |
| SR000128S | AmsGmsCmAmGmCmGfUfGfCmAmGmGmAmGmUmCmCmUm | 954 |
| SR000129S | GmsCmsAmGmCmGmUfGfCfAmGmGmAmGmUmCmCmCmAm | 955 |
| SR000130S | CmsAmsGmCmGmUmGfCfAfGmGmAmGmUmCmCmCmAmAm | 956 |
| SR000131S | AmsGmsCmGmUmGmCfAfGfGmAmGmUmCmCmCmAmGmAm | 957 |
| SR000132S | GmsCmsGmUmGmCmAfGfGfAmGmUmCmCmCmAmGmGmUm | 958 |
| SR000133S | CmsGmsUmGmCmAmGfGfAfGmUmCmCmCmAmGmGmUmAm | 959 |
| SR000134S | GmsUmsGmCmAmGmGfAfGfUmCmCmCmAmGmGmUmGmAm | 960 |
| SR000135S | UmsGmsCmAmGmGmAfGfUfCmCmCmAmGmGmUmGmGmUm | 961 |
| SR000136S | GmsUmsCmCmCmAmGfGfUfGmGmCmCmCmAmGmCmAmAm | 962 |
| SR000137S | UmsCmsCmCmAmGmGfUfGfGmCmCmCmAmGmCmAmGmAm | 963 |
| SR000138S | CmsCmsCmAmGmGmUfGfGfCmCmCmAmGmCmAmGmGmUm | 964 |
| SR000139S | CmsCmsAmGmGmUmGfGfCfCmCmAmGmCmAmGmGmCmUm | 965 |
| SR000140S | CmsAmsGmGmUmGmGfCfCfCmAmGmCmAmGmGmCmCmAm | 966 |
| SR000141S | AmsGmsGmUmGmGmCfCfCfAmGmCmAmGmGmCmCmAmAm | 967 |
| SR000142S | GmsGmsUmGmGmCmCfCfAfGmCmAmGmGmCmCmAmGmAm | 968 |
| SR000143S | GmsUmsGmGmCmCmCfAfGfCmAmGmGmCmCmAmGmGmAm | 969 |
| SR000144S | GmsCmsAmGmGmCmCfAfGfGmGmGmCmUmGmGmGmUmAm | 970 |
| SR000145S | CmsAmsGmGmCmCmAfGfGfGmGmCmUmGmGmGmUmGmAm | 971 |
| SR000146S | AmsGmsGmCmCmAmGfGfGfGmCmUmGmGmGmUmGmAmUm | 972 |
| SR000147S | GmsGmsCmCmAmGmGfGfGfCmUmGmGmGmUmGmAmCmUm | 973 |
| SR000148S | GmsCmsCmAmGmGmGfGfCfUmGmGmGmUmGmAmCmCmAm | 974 |
| SR000149S | CmsCmsAmGmGmGmGfCfUfGmGmGmUmGmAmCmCmGmAm | 975 |
| SR000150S | CmsAmsGmGmGmGmCfUfGfGmGmUmGmAmCmCmGmAmUm | 976 |
| SR000151S | AmsGmsGmGmGmCmUfGfGfGmUmGmAmCmCmGmAmUmAm | 977 |
| SR000152S | GmsGmsGmGmCmUmGfGfGfUmGmAmCmCmGmAmUmGmAm | 978 |
| SR000153S | GmsGmsGmCmUmGmGfGfUfGmAmCmCmGmAmUmGmGmUm | 979 |
| SR000154S | GmsGmsCmUmGmGmGfUfGfAmCmCmGmAmUmGmGmCmUm | 980 |
| SR000155S | GmsCmsUmGmGmGmUfGfAfCmCmGmAmUmGmGmCmUmUm | 981 |
| SR000156S | CmsUmsGmGmGmUmGfAfCfCmGmAmUmGmGmCmUmUmUm | 982 |
| SR000157S | UmsGmsGmGmUmGmAfCfCfGmAmUmGmGmCmUmUmCmAm | 983 |
| SR000158S | GmsGmsGmUmGmAmCfCfGfAmUmGmGmCmUmUmCmAmAm | 984 |
| SR000159S | GmsGmsUmGmAmCmCfGfAfUmGmGmCmUmUmCmAmGmUm | 985 |
| SR000160S | GmsUmsGmAmCmCmGfAfUfGmGmCmUmUmCmAmGmUmUm | 986 |
| SR000161S | UmsGmsAmCmCmGmAfUfGfGmCmUmUmCmAmGmUmUmUm | 987 |
| SR000162S | GmsAmsCmCmGmAmUfGfGfCmUmUmCmAmGmUmUmCmUm | 988 |
| SR000163S | AmsCmsCmGmAmUmGfGfCfUmUmCmAmGmUmUmCmCmUm | 989 |
| SR000164S | CmsCmsGmAmUmGmGfCfUfUmCmAmGmUmUmCmCmCmUm | 990 |
| SR000165S | CmsGmsAmUmGmGmCfUfUfCmAmGmUmUmCmCmCmUmAm | 991 |
| SR000166S | GmsAmsUmGmGmCmUfUfCfAmGmUmUmCmCmCmUmGmAm | 992 |
| SR000167S | AmsUmsGmGmCmUmUfCfAfGmUmUmCmCmCmUmGmAmAm | 993 |
| SR000168S | UmsGmsGmCmUmUmCfAfGfUmUmCmCmCmUmGmAmAmAm | 994 |
| SR000169S | GmsGmsCmUmUmCmAfGfUfUmCmCmCmUmGmAmAmAmAm | 995 |
| SR000170S | GmsCmsUmUmCmAmGfUfUfCmCmCmUmGmAmAmAmGmAm | 996 |
| SR000171S | CmsUmsUmCmAmGmUfUfCfCmCmUmGmAmAmAmGmAmUm | 997 |
| SR000172S | UmsUmsCmAmGmUmUfCfCfCmUmGmAmAmAmGmAmCmUm | 998 |
| SR000173S | UmsCmsAmGmUmUmCfCfCfUmGmAmAmAmGmAmCmUmAm | 999 |
| SR000174S | CmsAmsGmUmUmCmCfCfUfGmAmAmAmGmAmCmUmAmUm | 1000 |
| SR000175S | AmsGmsUmUmCmCmCfUfGfAmAmAmGmAmCmUmAmCmUm | 1001 |
| SR000176S | GmsUmsUmCmCmCmUfGfAfAmAmGmAmCmUmAmCmUmAm | 1002 |
| SR000177S | UmsUmsCmCmCmUmGfAfAfAmGmAmCmUmAmCmUmGmAm | 1003 |
| SR000178S | UmsCmsCmCmUmGmAfAfAfGmAmCmUmAmCmUmGmGmAm | 1004 |
| SR000179S | CmsCmsCmUmGmAmAfAfGfAmCmUmAmCmUmGmGmAmAm | 1005 |
| SR000180S | CmsCmsUmGmAmAmAfGfAfCmUmAmCmUmGmGmAmGmUm | 1006 |
| SR000181S | CmsUmsGmAmAmAmGfAfCfUmAmCmUmGmGmAmGmCmAm | 1007 |
| SR000182S | UmsGmsAmAmAmGmAfCfUfAmCmUmGmGmAmGmCmAmUm | 1008 |
| SR000183S | GmsAmsAmAmGmAmCfUfAfCmUmGmGmAmGmCmAmCmUm | 1009 |
| SR000184S | AmsAmsAmGmAmCmUfAfCfUmGmGmAmGmCmAmCmCmAm | 1010 |
| SR000185S | AmsGmsAmCmUmAmCfUfGfGmAmGmCmAmCmCmGmUmUm | 1011 |
| SR000186S | GmsAmsCmUmAmCmUfGfGfAmGmCmAmCmCmGmUmUmAm | 1012 |
| SR000187S | AmsCmsUmAmCmUmGfGfAfGmCmAmCmCmGmUmUmAmAm | 1013 |
| SR000188S | CmsUmsAmCmUmGmGfAfGfCmAmCmCmGmUmUmAmAmAm | 1014 |
| SR000189S | UmsAmsCmUmGmGmAfGfCfAmCmCmGmUmUmAmAmGmAm | 1015 |
| SR000190S | AmsCmsUmGmGmAmGfCfAfCmCmGmUmUmAmAmGmGmAm | 1016 |
| SR000191S | CmsUmsGmGmAmGmCfAfCfCmGmUmUmAmAmGmGmAmUm | 1017 |
| SR000192S | UmsGmsGmAmGmCmAfCfCfGmUmUmAmAmGmGmAmCmAm | 1018 |
| SR000193S | GmsGmsAmGmCmAmCfCfGfUmUmAmAmGmGmAmCmAmAm | 1019 |
| SR000194S | GmsAmsGmCmAmCmCfGfUfUmAmAmGmGmAmCmAmAmAm | 1020 |
| SR000195S | AmsGmsCmAmCmCmGfUfUfAmAmGmGmAmCmAmAmGmUm | 1021 |
| SR000196S | GmsCmsAmCmCmGmUfUfAfAmGmGmAmCmAmAmGmUmUm | 1022 |
| SR000197S | CmsAmsCmCmGmUmUfAfAfGmGmAmCmAmAmGmUmUmUm | 1023 |
| SR000198S | AmsCmsCmGmUmUmAfAfGfGmAmCmAmAmGmUmUmCmUm | 1024 |
| SR000199S | CmsCmsGmUmUmAmAfGfGfAmCmAmAmGmUmUmCmUmUm | 1025 |
| SR000200S | CmsGmsUmUmAmAmGfGfAfCmAmAmGmUmUmCmUmCmUm | 1026 |
| SR000201S | GmsUmsUmAmAmGmGfAfCfAmAmGmUmUmCmUmCmUmAm | 1027 |
| SR000202S | UmsUmsAmAmGmGmAfCfAfAmGmUmUmCmUmCmUmGmAm | 1028 |
| SR000203S | UmsAmsAmGmGmAmCfAfAfGmUmUmCmUmCmUmGmAmAm | 1029 |
| SR000204S | AmsAmsGmGmAmCmAfAfGfUmUmCmUmCmUmGmAmGmUm | 1030 |
| SR000205S | AmsGmsGmAmCmAmAfGfUfUmCmUmCmUmGmAmGmUmUm | 1031 |
| SR000206S | GmsGmsAmCmAmAmGfUfUfCmUmCmUmGmAmGmUmUmUm | 1032 |
| SR000207S | GmsAmsCmAmAmGmUfUfCfUmCmUmGmAmGmUmUmCmUm | 1033 |
| SR000208S | AmsCmsAmAmGmUmUfCfUfCmUmGmAmGmUmUmCmUmAm | 1034 |
| SR000209S | CmsAmsAmGmUmUmCfUfCfUmGmAmGmUmUmCmUmGmAm | 1035 |
| SR000210S | AmsAmsGmUmUmCmUfCfUfGmAmGmUmUmCmUmGmGmAm | 1036 |
| SR0002115 | AmsGmsUmUmCmUmCfUfGfAmGmUmUmCmUmGmGmGmAm | 1037 |
| SR000212S | GmsUmsUmCmUmCmUfGfAfGmUmUmCmUmGmGmGmAmUm | 1038 |
| SR000213S | UmsUmsCmUmCmUmGfAfGfUmUmCmUmGmGmGmAmUmUm | 1039 |
| SR000214S | UmsCmsUmCmUmGmAfGfUfUmCmUmGmGmGmAmUmUmUm | 1040 |
| SR000215S | CmsUmsCmUmGmAmGfUfUfCmUmGmGmGmAmUmUmUmAm | 1041 |
| SR000216S | UmsCmsUmGmAmGmUfUfCfUmGmGmGmAmUmUmUmGmAm | 1042 |
| SR000217S | CmsUmsGmAmGmUmUfCfUfGmGmGmAmUmUmUmGmGmAm | 1043 |
| SR000218S | UmsGmsAmGmUmUmCfUfGfGmGmAmUmUmUmGmGmAmUm | 1044 |
| SR000219S | GmsAmsGmUmUmCmUfGfGfGmAmUmUmUmGmGmAmCmUm | 1045 |
| SR000220S | AmsGmsUmUmCmUmGfGfGfAmUmUmUmGmGmAmCmCmUm | 1046 |
| SR000221S | UmsUmsCmUmGmGmGfAfUfUmUmGmGmAmCmCmCmUmAm | 1047 |
| SR000222S | UmsCmsUmGmGmGmAfUfUfUmGmGmAmCmCmCmUmGmAm | 1048 |
| SR000223S | CmsUmsGmGmGmAmUfUfUfGmGmAmCmCmCmUmGmAmAm | 1049 |
| SR000224S | UmsGmsGmGmAmUmUfUfGfGmAmCmCmCmUmGmAmGmAm | 1050 |
| SR000225S | GmsGmsGmAmUmUmUfGfGfAmCmCmCmUmGmAmGmGmUm | 1051 |
| SR000226S | GmsGmsAmUmUmUmGfGfAfCmCmCmUmGmAmGmGmUmUm | 1052 |
| SR000227S | GmsAmsUmUmUmGmGfAfCfCmCmUmGmAmGmGmUmCmAm | 1053 |
| SR000228S | AmsUmsUmUmGmGmAfCfCfCmUmGmAmGmGmUmCmAmAm | 1054 |
| SR000229S | UmsUmsUmGmGmAmCfCfCfUmGmAmGmGmUmCmAmGmAm | 1055 |
| SR000230S | UmsUmsGmGmAmCmCfCfUfGmAmGmGmUmCmAmGmAmUm | 1056 |
| SR000231S | UmsGmsGmAmCmCmCfUfGfAmGmGmUmCmAmGmAmCmUm | 1057 |
| SR000232S | GmsGmsAmCmCmCmUfGfAfGmGmUmCmAmGmAmCmCmAm | 1058 |
| SR000233S | GmsAmsCmCmCmUmGfAfGfGmUmCmAmGmAmCmCmAmAm | 1059 |
| SR000234S | AmsCmsCmCmUmGmAfGfGfUmCmAmGmAmCmCmAmAmUm | 1060 |
| SR000235S | CmsCmsCmUmGmAmGfGfUfCmAmGmAmCmCmAmAmCmUm | 1061 |
| SR000236S | CmsCmsUmGmAmGmGfUfCfAmGmAmCmCmAmAmCmUmUm | 1062 |
| SR000237S | CmsUmsGmAmGmGmUfCfAfGmAmCmCmAmAmCmUmUmUm | 1063 |
| SR000238S | UmsGmsAmGmGmUmCfAfGfAmCmCmAmAmCmUmUmCmAm | 1064 |
| SR000239S | GmsAmsGmGmUmCmAfGfAfCmCmAmAmCmUmUmCmAmAm | 1065 |
| SR000240S | AmsGmsGmUmCmAmGfAfCfCmAmAmCmUmUmCmAmGmUm | 1066 |
| SR000241S | GmsGmsUmCmAmGmAfCfCfAmAmCmUmUmCmAmGmCmUm | 1067 |
| SR000242S | GmsUmsCmAmGmAmCfCfAfAmCmUmUmCmAmGmCmCmAm | 1068 |
| SR000243S | CmsCmsAmAmCmUmUfCfAfGmCmCmGmUmGmGmCmUmAm | 1069 |
| SR000244S | CmsAmsAmCmUmUmCfAfGfCmCmGmUmGmGmCmUmGmUm | 1070 |
| SR000245S | AmsAmsCmUmUmCmAfGfCfCmGmUmGmGmCmUmGmCmUm | 1071 |
| SR000246S | AmsCmsUmUmCmAmGfCfCfGmUmGmGmCmUmGmCmCmUm | 1072 |
| SR000247S | CmsUmsUmCmAmGmCfCfGfUmGmGmCmUmGmCmCmUmAm | 1073 |
| SR000248S | UmsUmsCmAmGmCmCfGfUfGmGmCmUmGmCmCmUmGmAm | 1074 |
| SR000249S | UmsCmsAmGmCmCmGfUfGfGmCmUmGmCmCmUmGmAmAm | 1075 |
| SR000250S | CmsAmsGmCmCmGmUfGfGfCmUmGmCmCmUmGmAmGmAm | 1076 |
| SR000251S | AmsGmsCmCmGmUmGfGfCfUmGmCmCmUmGmAmGmAmUm | 1077 |
| SR000252S | GmsCmsCmGmUmGmGfCfUfGmCmCmUmGmAmGmAmCmUm | 1078 |
| SR000253S | CmsCmsGmUmGmGmCfUfGfCmCmUmGmAmGmAmCmCmUm | 1079 |
| SR000254S | CmsGmsUmGmGmCmUfGfCfCmUmGmAmGmAmCmCmUmUm | 1080 |
| SR000255S | GmsUmsGmGmCmUmGfCfCfUmGmAmGmAmCmCmUmCmAm | 1081 |
| SR000256S | UmsGmsGmCmUmGmCfCfUfGmAmGmAmCmCmUmCmAmAm | 1082 |
| SR000257S | GmsGmsCmUmGmCmCfUfGfAmGmAmCmCmUmCmAmAmUm | 1083 |
| SR000258S | GmsCmsUmGmCmCmUfGfAfGmAmCmCmUmCmAmAmUmAm | 1084 |
| SR000259S | CmsUmsGmCmCmUmGfAfGfAmCmCmUmCmAmAmUmAmUm | 1085 |
| SR000260S | UmsGmsCmCmUmGmAfGfAfCmCmUmCmAmAmUmAmCmUm | 1086 |
| SR000261S | GmsCmsCmUmGmAmGfAfCfCmUmCmAmAmUmAmCmCmUm | 1087 |
| SR000262S | CmsCmsUmGmAmGmAfCfCfUmCmAmAmUmAmCmCmCmUm | 1088 |
| SR000263S | UmsGmsAmGmAmCmCfUfCfAmAmUmAmCmCmCmCmAmAm | 1089 |
| SR000264S | GmsAmsGmAmCmCmUfCfAfAmUmAmCmCmCmCmAmAmAm | 1090 |
| SR000265S | AmsGmsAmCmCmUmCfAfAfUmAmCmCmCmCmAmAmGmUm | 1091 |
| SR000266S | GmsAmsCmCmUmCmAfAfUfAmCmCmCmCmAmAmGmUmUm | 1092 |
| SR000267S | AmsCmsCmUmCmAmAfUfAfCmCmCmCmAmAmGmUmCmUm | 1093 |
| SR000268S | CmsCmsUmCmAmAmUfAfCfCmCmCmAmAmGmUmCmCmAm | 1094 |
| SR000269S | CmsUmsCmAmAmUmAfCfCfCmCmAmAmGmUmCmCmAmUm | 1095 |
| SR000270S | UmsCmsAmAmUmAmCfCfCfCmAmAmGmUmCmCmAmCmUm | 1096 |
| SR000271S | CmsAmsAmUmAmCmCfCfCfAmAmGmUmCmCmAmCmCmUm | 1097 |
| SR000272S | AmsAmsUmAmCmCmCfCfAfAmGmUmCmCmAmCmCmUmAm | 1098 |
| SR000273S | AmsUmsAmCmCmCmCfAfAfGmUmCmCmAmCmCmUmGmUm | 1099 |
| SR000274S | UmsAmsCmCmCmCmAfAfGfUmCmCmAmCmCmUmGmCmUm | 1100 |
| SR000275S | CmsCmsCmCmAmAmGfUfCfCmAmCmCmUmGmCmCmUmAm | 1101 |
| SR000276S | CmsCmsCmAmAmGmUfCfCfAmCmCmUmGmCmCmUmAmUm | 1102 |
| SR000277S | CmsCmsAmAmGmUmCfCfAfCmCmUmGmCmCmUmAmUmUm | 1103 |
| SR000278S | CmsAmsAmGmUmCmCfAfCfCmUmGmCmCmUmAmUmCmUm | 1104 |
| SR000279S | AmsAmsGmUmCmCmAfCfCfUmGmCmCmUmAmUmCmCmAm | 1105 |
| SR000280S | AmsGmsUmCmCmAmCfCfUfGmCmCmUmAmUmCmCmAmUm | 1106 |
| SR000281S | GmsUmsCmCmAmCmCfUfGfCmCmUmAmUmCmCmAmUmUm | 1107 |
| SR000282S | UmsCmsCmAmCmCmUfGfCfCmUmAmUmCmCmAmUmCmUm | 1108 |
| SR000283S | CmsCmsAmCmCmUmGfCfCfUmAmUmCmCmAmUmCmCmUm | 1109 |
| SR000284S | CmsAmsCmCmUmGmCfCfUfAmUmCmCmAmUmCmCmUmAm | 1110 |
| SR000285S | AmsCmsCmUmGmCmCfUfAfUmCmCmAmUmCmCmUmGmUm | 1111 |
| SR000286S | CmsCmsUmGmCmCmUfAfUfCmCmAmUmCmCmUmGmCmAm | 1112 |
| SR000287S | CmsUmsGmCmCmUmAfUfCfCmAmUmCmCmUmGmCmGmAm | 1113 |
| SR000288S | UmsGmsCmCmUmAmUfCfCfAmUmCmCmUmGmCmGmAmAm | 1114 |
| SR000289S | GmsCmsCmUmAmUmCfCfAfUmCmCmUmGmCmGmAmGmUm | 1115 |
| SR000290S | CmsCmsUmAmUmCmCfAfUfCmCmUmGmCmGmAmGmCmUm | 1116 |
| SR000291S | CmsUmsAmUmCmCmAfUfCfCmUmGmCmGmAmGmCmUmUm | 1117 |
| SR000292S | UmsAmsUmCmCmAmUfCfCfUmGmCmGmAmGmCmUmCmUm | 1118 |
| SR000293S | AmsUmsCmCmAmUmCfCfUfGmCmGmAmGmCmUmCmCmUm | 1119 |
| SR000294S | UmsCmsCmAmUmCmCfUfGfCmGmAmGmCmUmCmCmUmUm | 1120 |
| SR000295S | CmsCmsAmUmCmCmUfGfCfGmAmGmCmUmCmCmUmUmAm | 1121 |
| SR000296S | CmsAmsUmCmCmUmGfCfGfAmGmCmUmCmCmUmUmGmAm | 1122 |
| SR000297S | AmsUmsCmCmUmGmCfGfAfGmCmUmCmCmUmUmGmGmAm | 1123 |
| SR000298S | UmsCmsCmUmGmCmGfAfGfCmUmCmCmUmUmGmGmGmUm | 1124 |
| SR000299S | CmsCmsUmGmCmGmAfGfCfUmCmCmUmUmGmGmGmUmUm | 1125 |
| SR000300S | CmsUmsGmCmGmAmGfCfUfCmCmUmUmGmGmGmUmCmUm | 1126 |
| SR000301S | UmsGmsCmGmAmGmCfUfCfCmUmUmGmGmGmUmCmCmUm | 1127 |
| SR000302S | GmsCmsGmAmGmCmUfCfCfUmUmGmGmGmUmCmCmUmAm | 1128 |
| SR000303S | CmsGmsAmGmCmUmCfCfUfUmGmGmGmUmCmCmUmGmUm | 1129 |
| SR000304S | GmsAmsGmCmUmCmCfUfUfGmGmGmUmCmCmUmGmCmAm | 1130 |
| SR000305S | AmsGmsCmUmCmCmUfUfGfGmGmUmCmCmUmGmCmAmAm | 1131 |
| SR000306S | GmsCmsUmCmCmUmUfGfGfGmUmCmCmUmGmCmAmAmUm | 1132 |
| SR000307S | CmsUmsCmCmUmUmGfGfGfUmCmCmUmGmCmAmAmUmUm | 1133 |
| SR000308S | UmsCmsCmUmUmGmGfGfUfCmCmUmGmCmAmAmUmCmUm | 1134 |
| SR000309S | CmsCmsUmUmGmGmGfUfCfCmUmGmCmAmAmUmCmUmUm | 1135 |
| SR000310S | CmsUmsUmGmGmGmUfCfCfUmGmCmAmAmUmCmUmCmUm | 1136 |
| SR000311S | UmsUmsGmGmGmUmCfCfUfGmCmAmAmUmCmUmCmCmAm | 1137 |
| SR000312S | UmsGmsGmGmUmCmCfUfGfCmAmAmUmCmUmCmCmAmAm | 1138 |
| SR000313S | GmsGmsGmUmCmCmUfGfCfAmAmUmCmUmCmCmAmGmAm | 1139 |
| SR000314S | GmsGmsUmCmCmUmGfCfAfAmUmCmUmCmCmAmGmGmAm | 1140 |
| SR000315S | GmsUmsCmCmUmGmCfAfAfUmCmUmCmCmAmGmGmGmUm | 1141 |
| SR000316S | CmsCmsUmGmCmAmAfUfCfUmCmCmAmGmGmGmCmUmAm | 1142 |
| SR000317S | CmsUmsGmCmAmAmUfCfUfCmCmAmGmGmGmCmUmGmUm | 1143 |
| SR000318S | UmsGmsCmAmAmUmCfUfCfCmAmGmGmGmCmUmGmCmUm | 1144 |
| SR000319S | CmsUmsCmCmAmGmGfGfCfUmGmCmCmCmCmUmGmUmAm | 1145 |
| SR000320S | UmsCmsCmAmGmGmGfCfUfGmCmCmCmCmUmGmUmAmAm | 1146 |
| SR000321S | CmsCmsAmGmGmGmCfUfGfCmCmCmCmUmGmUmAmGmAm | 1147 |
| SR000322S | CmsAmsGmGmGmCmUfGfCfCmCmCmUmGmUmAmGmGmUm | 1148 |
| SR000323S | AmsGmsGmGmCmUmGfCfCfCmCmUmGmUmAmGmGmUmUm | 1149 |
| SR000324S | GmsGmsGmCmUmGmCfCfCfCmUmGmUmAmGmGmUmUmAm | 1150 |
| SR000325S | GmsGmsCmUmGmCmCfCfCfUmGmUmAmGmGmUmUmGmUm | 1151 |
| SR000326S | GmsCmsUmGmCmCmCfCfUfGmUmAmGmGmUmUmGmCmUm | 1152 |
| SR000327S | CmsUmsGmCmCmCmCfUfGfUmAmGmGmUmUmGmCmUmUm | 1153 |
| SR000328S | UmsGmsCmCmCmCmUfGfUfAmGmGmUmUmGmCmUmUmAm | 1154 |
| SR000329S | GmsCmsCmCmCmUmGfUfAfGmGmUmUmGmCmUmUmAmAm | 1155 |
| SR000330S | CmsCmsCmCmUmGmUfAfGfGmUmUmGmCmUmUmAmAmAm | 1156 |
| SR000331S | CmsCmsCmUmGmUmAfGfGfUmUmGmCmUmUmAmAmAmAm | 1157 |
| SR000332S | CmsCmsUmGmUmAmGfGfUfUmGmCmUmUmAmAmAmAmAm | 1158 |
| SR000333S | CmsUmsGmUmAmGmGfUfUfGmCmUmUmAmAmAmAmGmAm | 1159 |
| SR000334S | UmsGmsUmAmGmGmUfUfGfCmUmUmAmAmAmAmGmGmAm | 1160 |
| SR000335S | GmsUmsAmGmGmUmUfGfCfUmUmAmAmAmAmGmGmGmAm | 1161 |
| SR000336S | UmsAmsGmGmUmUmGfCfUfUmAmAmAmAmGmGmGmAmUm | 1162 |
| SR000337S | AmsGmsGmUmUmGmCfUfUfAmAmAmAmGmGmGmAmCmAm | 1163 |
| SR000338S | GmsGmsUmUmGmCmUfUfAfAmAmAmGmGmGmAmCmAmAm | 1164 |
| SR000339S | GmsUmsUmGmCmUmUfAfAfAmAmGmGmGmAmCmAmGmUm | 1165 |
| SR000340S | UmsUmsGmCmUmUmAfAfAfAmGmGmGmAmCmAmGmUmAm | 1166 |
| SR000341S | UmsGmsCmUmUmAmAfAfAfGmGmGmAmCmAmGmUmAmUm | 1167 |
| SR000342S | GmsCmsUmUmAmAmAfAfGfGmGmAmCmAmGmUmAmUmUm | 1168 |
| SR000343S | CmsUmsUmAmAmAmAfGfGfGmAmCmAmGmUmAmUmUmUm | 1169 |
| SR000344S | UmsUmsAmAmAmAmGfGfGfAmCmAmGmUmAmUmUmCmUm | 1170 |
| SR000345S | UmsAmsAmAmAmGmGfGfAfCmAmGmUmAmUmUmCmUmUm | 1171 |
| SR000346S | AmsAmsAmAmGmGmGfAfCfAmGmUmAmUmUmCmUmCmAm | 1172 |
| SR000347S | AmsAmsAmGmGmGmAfCfAfGmUmAmUmUmCmUmCmAmAm | 1173 |
| SR000348S | AmsAmsGmGmGmAmCfAfGfUmAmUmUmCmUmCmAmGmUm | 1174 |
| SR000349S | AmsGmsGmGmAmCmAfGfUfAmUmUmCmUmCmAmGmUmAm | 1175 |
| SR000350S | GmsGmsGmAmCmAmGfUfAfUmUmCmUmCmAmGmUmGmUm | 1176 |
| SR000351S | GmsGmsAmCmAmGmUfAfUfUmCmUmCmAmGmUmGmCmUm | 1177 |
| SR000352S | GmsAmsCmAmGmUmAfUfUfCmUmCmAmGmUmGmCmUmUm | 1178 |
| SR000353S | AmsCmsAmGmUmAmUfUfCfUmCmAmGmUmGmCmUmCmUm | 1179 |
| SR000354S | CmsAmsGmUmAmUmUfCfUfCmAmGmUmGmCmUmCmUmUm | 1180 |
| SR000355S | AmsGmsUmAmUmUmCfUfCfAmGmUmGmCmUmCmUmCmUm | 1181 |
| SR000356S | GmsUmsAmUmUmCmUfCfAfGmUmGmCmUmCmUmCmCmUm | 1182 |
| SR000357S | UmsAmsUmUmCmUmCfAfGfUmGmCmUmCmUmCmCmUmAm | 1183 |
| SR000358S | AmsUmsUmCmUmCmAfGfUfGmCmUmCmUmCmCmUmAmUm | 1184 |
| SR000359S | UmsUmsCmUmCmAmGfUfGfCmUmCmUmCmCmUmAmCmUm | 1185 |
| SR000360S | UmsCmsUmCmAmGmUfGfCfUmCmUmCmCmUmAmCmCmUm | 1186 |
| SR000361S | CmsUmsCmAmGmUmGfCfUfCmUmCmCmUmAmCmCmCmUm | 1187 |
| SR000362S | GmsCmsUmCmUmCmCfUfAfCmCmCmCmAmCmCmUmCmAm | 1188 |
| SR000363S | CmsUmsCmUmCmCmUfAfCfCmCmCmAmCmCmUmCmAmUm | 1189 |
| SR000364S | UmsCmsUmCmCmUmAfCfCfCmCmAmCmCmUmCmAmUmAm | 1190 |
| SR000365S | CmsUmsCmCmUmAmCfCfCfCmAmCmCmUmCmAmUmGmUm | 1191 |
| SR000366S | UmsCmsCmUmAmCmCfCfCfAmCmCmUmCmAmUmGmCmUm | 1192 |
| SR000367S | CmsCmsUmAmCmCmCfCfAfCmCmUmCmAmUmGmCmCmUm | 1193 |
| SR000368S | CmsUmsAmCmCmCmCfAfCfCmUmCmAmUmGmCmCmUmAm | 1194 |
| SR000369S | UmsAmsCmCmCmCmAfCfCfUmCmAmUmGmCmCmUmGmAm | 1195 |
| SR000370S | CmsCmsCmCmUmCmCfAfGfGmCmAmUmGmCmUmGmGmUm | 1196 |
| SR000371S | CmsCmsCmUmCmCmAfGfGfCmAmUmGmCmUmGmGmCmUm | 1197 |
| SR000372S | CmsCmsUmCmCmAmGfGfCfAmUmGmCmUmGmGmCmCmUm | 1198 |
| SR000373S | CmsUmsCmCmAmGmGfCfAfUmGmCmUmGmGmCmCmUmUm | 1199 |
| SR000374S | AmsGmsGmCmAmUmGfCfUfGmGmCmCmUmCmCmCmAmAm | 1200 |
| SR000375S | GmsGmsCmAmUmGmCfUfGfGmCmCmUmCmCmCmAmAmUm | 1201 |
| SR000376S | GmsCmsAmUmGmCmUfGfGfCmCmUmCmCmCmAmAmUmAm | 1202 |
| SR000377S | CmsAmsUmGmCmUmGfGfCfCmUmCmCmCmAmAmUmAmAm | 1203 |
| SR000378S | AmsUmsGmCmUmGmGfCfCfUmCmCmCmAmAmUmAmAmAm | 1204 |
| SR000379S | UmsGmsCmUmGmGmCfCfUfCmCmCmAmAmUmAmAmAmAm | 1205 |
| SR000380S | GmsCmsUmGmGmCmCfUfCfCmCmAmAmUmAmAmAmGmUm | 1206 |
| SR000381S | CmsUmsGmGmCmCmUfCfCfCmAmAmUmAmAmAmGmCmUm | 1207 |
| SR000382S | UmsGmsGmCmCmUmCfCfCfAmAmUmAmAmAmGmCmUmAm | 1208 |
| SR000383S | GmsGmsCmCmUmCmCfCfAfAmUmAmAmAmGmCmUmGmAm | 1209 |
| SR000384S | GmsCmsCmUmCmCmCfAfAfUmAmAmAmGmCmUmGmGmAm | 1210 |
| SR000385S | CmsCmsUmCmCmCmAfAfUfAmAmAmGmCmUmGmGmAmUm | 1211 |
| SR000386S | CmsUmsCmCmCmAmAfUfAfAmAmGmCmUmGmGmAmCmAm | 1212 |
| SR000387S | UmsCmsCmCmAmAmUfAfAfAmGmCmUmGmGmAmCmAmAm | 1213 |
| SR000388S | CmsCmsCmAmAmUmAfAfAfGmCmUmGmGmAmCmAmAmAm | 1214 |
| SR000389S | CmsCmsAmAmUmAmAfAfGfCmUmGmGmAmCmAmAmGmAm | 1215 |
| SR000390S | CmsAmsAmUmAmAmAfGfCfUmGmGmAmCmAmAmGmAmAm | 1216 |
| SR000391S | AmsAmsUmAmAmAmGfCfUfGmGmAmCmAmAmGmAmAmAm | 1217 |
| SR000392S | AmsUmsAmAmAmGmCfUfGfGmAmCmAmAmGmAmAmGmUm | 1218 |
| SR000393S | UmsAmsAmAmGmCmUfGfGfAmCmAmAmGmAmAmGmCmUm | 1219 |
| SR000394S | AmsAmsAmGmCmUmGfGfAfCmAmAmGmAmAmGmCmUmAm | 1220 |
| SR000395S | AmsAmsGmCmUmGmGfAfCfAmAmGmAmAmGmCmUmGmUm | 1221 |
| SR000396S | AmsGmsCmUmGmGmAfCfAfAmGmAmAmGmCmUmGmCmUm | 1222 |
| SR000397S | GmsCmsUmGmGmAmCfAfAfGmAmAmGmCmUmGmCmUmAm | 1223 |
| SR000398S | CmsUmsGmGmAmCmAfAfGfAmAmGmCmUmGmCmUmAmUm | 1224 |
| SR000399S | UmsGmsGmAmCmAmAfGfAfAmGmCmUmGmCmUmAmUmAm | 1225 |
| SR000400S | GmsGmsAmCmAmAmGfAfAfGmCmUmGmCmUmAmUmGmAm | 1226 |
| SR002955S | NAG37s-IBs- AmCmGmGmGmAmCmAmGfUfAfUmUmCmUmCmAmGmUmimAms-IB | 1227 |
| SR004479S | AmsAmsGmGmGmAmCfAmGfUfAfUmUmCmUmCmAmGmUmsGmsCm- L96 | 1228 |
| SR004443S | UmsUmsAmAmAmAmGfGfGfAmCmAmGmUmAmUmUmCmUm-L96 | 1229 |
| SR004444S | AmsAmsGmGmGmAmCfAfGfUmAmUmUmCmUmCmAmGmUm-L96 | 1230 |
| SR004445S | CmsCmsAmAmGmUmCfCfAfCmCmUmGmCmCmUmAmUmUm-L96 | 1231 |
| SR004446S | CmsCmsGmUmUmAmAfGfGfAmCmAmAmGmUmUmCmUmUm-L96 | 1232 |
| SR004447S | CmsGmsAmGmGmAmUfGfCfCmUmCmCmCmUmUmCmUmUm-L96 | 1233 |
| SR002959S | IBs-AmCmGmGmGmAmCmAmGfUfAfUmUmCmUmCmAmGmUmImAms- IB | 1234 |
| SR011326S | STM1s- AmsAmUmUmAmAmAmAmGfGfGfAmCmAmGmUmAmUmUmCmAms- STM1s-GL6 | 1235 |
| SR015173S | IBs-AmsAmUmUmAmAmAmAmGfGfGfAmCmAmGmUmAmUmUmCmAms- IBs-GL6 | 1236 |
| SR015114S | UmsUmsAmAmAmAmGfGfGfAmCmAmGmUmAmUmUmCmsAms-GL6 | 1237 |
| SR013765S | STM1s- AmsAmAmAmGmGmGmAmCfAfGfUmAmUmUmCmUmCmAmGmAms- STM1s-GL6 | 1238 |
| SR015172S | IBs-AmsAmAmAmGmGmGmAmCfAfGfUmAmUmUmCmUmCmAmGmAms- IBs-GL6 | 1239 |
| SR015113S | AmsAmsGmGmGmAmCfAfGfUmAmUmUmCmUmCmAmGmsAms-GL6 | 1240 |

**Table 5 Modified RNA Sequences of the Antisense Strand of siRNA Targeting APOC3 Gene Used in the Present Invention**

| Antisense Strand No. | Sequence (5'→3') | SEQ ID NO: |
|---|---|---|
| SR000401A | AmsUfsUmCfCmUfGmGfAmGfCmAfGmCfUmGfCmCfUmsUfsUm | 1241 |
| SR000402A | UmsGfsUmUfCmCfUmGfGmAfGmCfAmGfCmUfGmCfCmsUfsUm | 1242 |
| SR000403A | UmsUfsGmUfUmCfCmUfGmGfAmGfCmAfGmCfUmGfCmsUfsUm | 1243 |
| SR000404A | UmsCfsUmGfUmUfCmCfUmGfGmAfGmCfAmGfCmUfGmsUfsUm | 1244 |
| SR000405A | UmsUfsCmUfGmUfUmCfCmUfGmGfAmGfCmAfGmCfUmsUfsUm | 1245 |
| SR000406A | UmsCfsUmCfUmGfUmUfCmCfUmGfGmAfGmCfAmGfCmsUfsUm | 1246 |
| SR000407A | UmsAfsCmCfUmCfUmGfUmUfCmCfUmGfGmAfGmCfAmsUfsUm | 1247 |
| SR000408A | UmsAfsUmGfGmCfAmCfCmUfCmUfGmUfUmCfCmUfGmsUfsUm | 1248 |
| SR000409A | AmsCfsAmUfGmGfCmAfCmCfUmCfUmGfUmUfCmCfUmsUfsUm | 1249 |
| SR000410A | UmsGfsCmAfUmGfGmCfAmCfCmUfCmUfGmUfUmCfCmsUfsUm | 1250 |
| SR000411A | UmsUfsGmCfAmUfGmGfCmAfCmCfUmCfUmGfUmUfCmsUfsUm | 1251 |
| SR000412A | AmsCfsUmGfCmAfUmGfGmCfAmCfCmUfCmUfGmUfUmsUfsUm | 1252 |
| SR000413A | AmsAfsGmUfAmCfCmCfGmGfGmGfCmUfGmCfAmUfGmsUfsUm | 1253 |
| SR000414A | AmsGfsAmGfUmAfCmCfCmGfGmGfGmCfUmGfCmAfUmsUfsUm | 1254 |
| SR000415A | AmsGfsGmAfGmUfAmCfCmCfGmGfGmGfCmUfGmCfAmsUfsUm | 1255 |
| SR000416A | AmsAfsGmGfAmGfUmAfCmCfCmGfGmGfGmCfUmGfCmsUfsUm | 1256 |
| SR000417A | UmsAfsAmGfGmAfGmUfAmCfCmCfGmGfGmGfCmUfGmsUfsUm | 1257 |
| SR000418A | AmsCfsAmAfGmGfAmGfUmAfCmCfCmGfGmGfGmCfUmsUfsUm | 1258 |
| SR000419A | AmsAfsCmAfAmGfGmAfGmUfAmCfCmCfGmGfGmGfCmsUfsUm | 1259 |
| SR000420A | UmsAfsAmCfAmAfGmGfAmGfUmAfCmCfCmGfGmGfGmsUfsUm | 1260 |
| SR000421A | AmsCfsAmAfCmAfAmGfGmAfGmUfAmCfCmCfGmGfGmsUfsUm | 1261 |
| SR000422A | AmsAfsCmAfAmCfAmAfGmGfAmGfUmAfCmCfCmGfGmsUfsUm | 1262 |
| SR000423A | UmsAfsAmCfAmAfCmAfAmGfGmAfGmUfAmCfCmCfGmsUfsUm | 1263 |
| SR000424A | AmsCfsAmAfCmAfAmCfAmAfGmGfAmGfUmAfCmCfCmsUfsUm | 1264 |
| SR000425A | AmsGfsCmAfAmCfAmAfCmAfAmGfGmAfGmUfAmCfCmsUfsUm | 1265 |
| SR000426A | AmsGfsGmCfAmAfCmAfAmCfAmAfGmGfAmGfUmAfCmsUfsUm | 1266 |
| SR000427A | AmsAfsGmGfGmCfAmAfCmAfAmCfAmAfGmGfAmGfUmsUfsUm | 1267 |
| SR000428A | AmsGfsAmGfGmGfCmAfAmCfAmAfCmAfAmGfGmAfGmsUfsUm | 1268 |
| SR000429A | AmsGfsGmAfGmGfGmCfAmAfCmAfAmCfAmAfGmGfAmsUfsUm | 1269 |
| SR000430A | UmsAfsGmGfAmGfGmGfCmAfAmCfAmAfCmAfAmGfGmsUfsUm | 1270 |
| SR000431A | UmsCfsAmGfGmAfGmGfGmCfAmAfCmAfAmCfAmAfGmsUfsUm | 1271 |
| SR000432A | AmsCfsCmAfGmGfAmGfGmGfCmAfAmCfAmAfCmAfAmsUfsUm | 1272 |
| SR000433A | UmsGfsCmCfAmGfGmAfGmGfGmCfAmAfCmAfAmCfAmsUfsUm | 1273 |
| SR000434A | UmsCfsAmGfGmAfGmCfGmCfCmAfGmGfAmGfGmGfCmsUfsUm | 1274 |
| SR000435A | AmsAfsGmGfCmCfAmGfGmAfGmCfGmCfCmAfGmGfAmsUfsUm | 1275 |
| SR000436A | UmsAfsGmAfGmGfCmCfAmGfGmAfGmCfGmCfCmAfGmsUfsUm | 1276 |
| SR000437A | AmsCfsAmGfAmGfGmCfCmAfGmGfAmGfCmGfCmCfAmsUfsUm | 1277 |
| SR000438A | AmsGfsCmAfGmAfGmGfCmCfAmGfGmAfGmCfGmCfCmsUfsUm | 1278 |
| SR000439A | AmsGfsGmCfAmGfAmGfGmCfCmAfGmGfAmGfCmGfCmsUfsUm | 1279 |
| SR000440A | AmsAfsGmCfUmCfGmGfGmCfAmGfAmGfGmCfCmAfGmsUfsUm | 1280 |
| SR000441A | AmsAfsAmGfCmUfCmGfGmGfCmAfGmAfGmGfCmCfAmsUfsUm | 1281 |
| SR000442A | UmsGfsAmAfGmCfUmCfGmGfGmCfAmGfAmGfGmCfCmsUfsUm | 1282 |
| SR000443A | UmsUfsGmAfAmGfCmUfCmGfGmGfCmAfGmAfGmGfCmsUfsUm | 1283 |
| SR000444A | UmsCfsUmGfAmAfGmCfUmCfGmGfGmCfAmGfAmGfGmsUfsUm | 1284 |
| SR000445A | UmsUfsCmUfGmAfAmGfCmUfCmGfGmGfCmAfGmAfGmsUfsUm | 1285 |
| SR000446A | UmsCfsUmCfUmGfAmAfGmCfUmCfGmGfGmCfAmGfAmsUfsUm | 1286 |
| SR000447A | AmsCfsCmUfCmUfGmAfAmGfCmUfCmGfGmGfCmAfGmsUfsUm | 1287 |
| SR000448A | AmsGfsCmCfUmCfUmGfAmAfGmCfUmCfGmGfGmCfAmsUfsUm | 1288 |
| SR000449A | AmsUfsCmCfUmCfGmGfCmCfUmCfUmGfAmAfGmCfUmsUfsUm | 1289 |
| SR000450A | UmsAfsUmCfCmUfCmGfGmCfCmUfCmUfGmAfAmGfCmsUfsUm | 1290 |
| SR000451A | AmsCfsAmUfCmCfUmCfGmGfCmCfUmCfUmGfAmAfGmsUfsUm | 1291 |
| SR000452A | AmsGfsCmAfUmCfCmUfCmGfGmCfCmUfCmUfGmAfAmsUfsUm | 1292 |
| SR000453A | AmsGfsGmCfAmUfCmCfUmCfGmGfCmCfUmCfUmGfAmsUfsUm | 1293 |
| SR000454A | AmsAfsGmGfCmAfUmCfCmUfCmGfGmCfCmUfCmUfGmsUfsUm | 1294 |
| SR000455A | AmsGfsAmGfGmCfAmUfCmCfUmCfGmGfCmCfUmCfUmsUfsUm | 1295 |
| SR000456A | AmsGfsGmAfGmGfCmAfUmCfCmUfCmGfGmCfCmUfCmsUfsUm | 1296 |
| SR000457A | AmsAfsGmGfGmAfGmGfCmAfUmCfCmUfCmGfGmCfCmsUfsUm | 1297 |
| SR000458A | AmsAfsAmGfGmGfAmGfGmCfAmUfCmCfUmCfGmGfCmsUfsUm | 1298 |
| SR000459A | AmsGfsAmAfGmGfGmAfGmGfCmAfUmCfCmUfCmGfGmsUfsUm | 1299 |
| SR000460A | AmsAfsGmAfAmGfGmGfAmGfGmCfAmUfCmCfUmCfGmsUfsUm | 1300 |
| SR000461A | UmsGfsAmGfAmAfGmGfGmAfGmGfCmAfUmCfCmUfCmsUfsUm | 1301 |
| SR000462A | UmsUfsGmAfGmAfAmGfGmGfAmGfGmCfAmUfCmCftJmsUfsUm | 1302 |
| SR000463A | AmsCfsUmGfAmGfAmAfGmGfGmAfGmGfCmAfUmCfCmsUfsUm | 1303 |
| SR000464A | AmsGfsCmUfGmAfGmAfAmGfGmGfAmGfGmCfAmUfCmsUfsUm | 1304 |
| SR000465A | AmsAfsGmCfUmGfAmGfAmAfGmGfGmAfGmGfCmAfUmsUfsUm | 1305 |
| SR000466A | AmsAfsAmGfCmUfGmAfGmAfAmGfGmGfAmGfGmCfAmsUfsUm | 1306 |
| SR000467A | UmsGfsAmAfGmCfUmGfAmGfAmAfGmGfGmAfGmGfCmsUfsUm | 1307 |
| SR000468A | AmsUfsGmAfAmGfCmUfGmAfGmAfAmGfGmGfAmGfGmsUfsUm | 1308 |
| SR000469A | UmsAfsUmGfAmAfGmCfUmGfAmGfAmAfGmGfGmAfGmsUfsUm | 1309 |
| SR000470A | AmsCfsAmUfGmAfAmGfCmUfGmAfGmAfAmGfGmGfAmsUfsUm | 1310 |
| SR000471A | UmsGfsCmAfUmGfAmAfGmCfUmGfAmGfAmAfGmGfGmsUfsUm | 1311 |
| SR000472A | UmsUfsGmCfAmUfGmAfAmGfCmUfGmAfGmAfAmGfGmsUfsUm | 1312 |
| SR000473A | UmsCfsUmGfCmAfUmGfAmAfGmCfUmGfAmGfAmAfGmsUfsUm | 1313 |
| SR000474A | UmsCfsCmUfGmCfAmUfGmAfAmGfCmUfGmAfGmAfAmsUfsUm | 1314 |
| SR000475A | AmsCfsCmCfUmGfCmAfUmGfAmAfGmCfUmGfAmGfAmsUfsUm | 1315 |
| SR000476A | AmsAfsCmCfCmUfGmCfAmUfGmAfAmGfCmUfGmAfGmsUfsUm | 1316 |
| SR000477A | UmsAfsAmCfCmCfUmGfCmAfUmGfAmAfGmCfUmGfAmsUfsUm | 1317 |
| SR000478A | AmsUfsAmAfCmCfCmUfGmCfAmUfGmAfAmGfCmUfGmsUfsUm | 1318 |
| SR000479A | UmsGfsUmAfAmCfCmCfUmGfCmAfUmGfAmAfGmCfUmsUfsUm | 1319 |
| SR000480A | AmsUfsGmUfAmAfCmCfCmUfGmCfAmUfGmAfAmGfCmsUfsUm | 1320 |
| SR000481A | UmsAfsUmGfUmAfAmCfCmCfUmGfCmAfUmGfAmAfGmsUfsUm | 1321 |
| SR000482A | UmsCfsAmUfGmUfAmAfCmCfCmUfGmCfAmUfGmAfAmsUfsUm | 1322 |
| SR000483A | UmsUfsCmAfUmGfUmAfAmCfCmCfUmGfCmAfUmGfAmsUfsUm | 1323 |
| SR000484A | UmsUfsUmCfAmUfGmUfAmAfCmCfCmUfGmCfAmUfGmsUfsUm | 1324 |
| SR000485A | AmsCfsUmUfCmAfUmGfUmAfAmCfCmCfUmGfCmAfUmsUfsUm | 1325 |
| SR000486A | UmsGfsCmUfUmCfAmUfGmUfAmAfCmCfCmUfGmCfAmsUfsUm | 1326 |
| SR000487A | AmsUfsGmCfUmUfCmAfUmGfUmAfAmCfCmCfUmGfCmsUfsUm | 1327 |
| SR000488A | UmsGfsUmGfCmUfUmCfAmUfGmUfAmAfCmCfCmUfGmsUfsUm | 1328 |
| SR000489A | AmsCfsGmUfGmCfUmUfCmAfUmGfUmAfAmCfCmCfUmsUfsUm | 1329 |
| SR000490A | AmsGfsCmGfUmGfCmUfUmCfAmUfGmUfAmAfCmCfCmsUfsUm | 1330 |
| SR000491A | AmsUfsGmGfCmGfUmGfCmUfUmCfAmUfGmUfAmAfCmsUfsUm | 1331 |
| SR000492A | UmsUfsGmGfUmGfGmCfGmUfGmCfUmUfCmAfUmGfUmsUfsUm | 1332 |
| SR000493A | UmsUfsUmGfGmUfGmGfCmGfUmGfCmUfUmCfAmUfGmsUfsUm | 1333 |
| SR000494A | UmsCfsUmUfGmGfUmGfGmCfGmUfGmCfUmUfCmAfUmsUfsUm | 1334 |
| SR000495A | AmsUfsCmUfUmGfGmUfGmGfCmGfUmGfCmUfUmCfAmsUfsUm | 1335 |
| SR000496A | AmsGfsUmCfUmUfGmGfUmGfGmCfGmUfGmCfUmUfCmsUfsUm | 1336 |
| SR000497A | UmsGfsGmUfCmUfUmGfGmUfGmGfCmGfUmGfCmUfUmsUfsUm | 1337 |
| SR000498A | AmsCfsGmGfUmCfUmUfGmGfUmGfGmCfGmUfGmCfUmsUfsUm | 1338 |
| SR000499A | AmsGfsCmGfGmUfCmUfUmGfGmUfGmGfCmGfUmGfCmsUfsUm | 1339 |
| SR000500A | UmsUfsUmGfGmCfGmGfUmCfUmUfGmGfUmGfGmCfGmsUfsUm | 1340 |
| SR000501A | UmsCfsUmUfGmGfCmGfGmUfCmUfUmGfGmUfGmGfCmsUfsUm | 1341 |
| SR000502A | UmsCfsCmUfUmGfGmCfGmGfUmCfUmUfGmGfUmGfGmsUfsUm | 1342 |
| SR000503A | AmsUfsCmCfUmUfGmGfCmGfGmUfCmUfUmGfGmUfGmsUfsUm | 1343 |
| SR000504A | UmsAfsUmCfCmUfUmGfGmCfGmGfUmCfUmUfGmGfUmsUfsUm | 1344 |
| SR000505A | AmsCfsAmUfCmCfUmUfGmGfCmGfGmUfCmUfUmGfGmsUfsUm | 1345 |
| SR000506A | UmsGfsCmAfUmCfCmUfUmGfGmCfGmGfUmCfUmUfGmsUfsUm | 1346 |
| SR000507A | AmsUfsGmCfAmUfCmCfUmUfGmGfCmGfGmUfCmUfUmsUfsUm | 1347 |
| SR000508A | AmsGfsUmGfCmAfUmCfCmUfUmGfGmCfGmGfUmCfUmsUfsUm | 1348 |
| SR000509A | UmsAfsGmUfGmCfAmUfCmCfUmUfGmGfCmGfGmUfCmsUfsUm | 1349 |
| SR000510A | UmsCfsAmGfUmGfCmAfUmCfCmUfUmGfGmCfGmGfUmsUfsUm | 1350 |
| SR000511A | UmsUfsCmAfGmUfGmCfAmUfCmCfUmUfGmGfCmGfGmsUfsUm | 1351 |
| SR000512A | AmsCfsUmCfAmGfUmGfCmAfUmCfCmUfUmGfGmCfGmsUfsUm | 1352 |
| SR000513A | UmsGfsCmUfCmAfGmUfGmCfAmUfCmCfUmUfGmGfCmsUfsUm | 1353 |
| SR000514A | UmsUfsGmCfUmCfAmGfUmGfCmAfUmCfCmUfUmGfGmsUfsUm | 1354 |
| SR000515A | AmsCfsUmGfCmUfCmAfGmUfGmCfAmUfCmCfUmUfGmsUfsUm | 1355 |
| SR000516A | UmsGfsCmUfGmCfUmCfAmGfUmGfCmAfUmCfCmUfUmsUfsUm | 1356 |
| SR000517A | AmsCfsGmCfUmGfCmUfCmAfGmUfGmCfAmUfCmCfUmsUfsUm | 1357 |
| SR000518A | UmsAfsCmGfCmUfGmCfUmCfAmGfUmGfCmAfUmCfCmsUfsUm | 1358 |
| SR000519A | AmsCfsAmCfGmCfUmGfCmUfCmAfGmUfGmCfAmUfCmsUfsUm | 1359 |
| SR000520A | UmsGfsCmAfCmGfCmUfGmCfUmCfAmGfUmGfCmAfUmsUfsUm | 1360 |
| SR000521A | UmsUfsGmCfAmCfGmCfUmGfCmUfCmAfGmUfGmCfAmsUfsUm | 1361 |
| SR000522A | UmsCfsUmGfCmAfCmGfCmUfGmCfUmCfAmGfUmGfCmsUfsUm | 1362 |
| SR000523A | UmsCfsCmUfGmCfAmCfGmCfUmGfCmUfCmAfGmUfGmsUfsUm | 1363 |
| SR000524A | UmsUfsCmCfUmGfCmAfCmGfCmUfGmCfUmCfAmGfUmsUfsUm | 1364 |
| SR000525A | AmsCfsUmCfCmUfGmCfAmCfGmCfUmGfCmUfCmAfGmsUfsUm | 1365 |
| SR000526A | AmsAfsCmUfCmCfUmGfCmAfCmGfCmUfGmCfUmCfAmsUfsUm | 1366 |
| SR000527A | AmsGfsAmCfUmCfCmUfGmCfAmCfGmCfUmGfCmUfCmsUfsUm | 1367 |
| SR000528A | AmsGfsGmAfCmUfCmCfUmGfCmAfCmGfCmUfGmCfUmsUfsUm | 1368 |
| SR000529A | UmsGfsGmGfAmCfUmCfCmUfGmCfAmCfGmCfUmGfCmsUfsUm | 1369 |
| SR000530A | UmsUfsGmGfGmAfCmUfCmCfUmGfCmAfCmGfCmUfGmsUfsUm | 1370 |
| SR000531A | UmsCfsUmGfGmGfAmCfUmCfCmUfGmCfAmCfGmCfUmsUfsUm | 1371 |
| SR000532A | AmsCfsCmUfGmGfGmAfCmUfCmCfUmGfCmAfCmGfCmsUfsUm | 1372 |
| SR000533A | UmsAfsCmCfUmGfGmGfAmCfUmCfCmUfGmCfAmCfGmsUfsUm | 1373 |
| SR000534A | UmsCfsAmCfCmUfGmGfGmAfCmUfCmCfUmGfCmAfCmsUfsUm | 1374 |
| SR000535A | AmsCfsCmAfCmCfUmGfGmGfAmCfUmCfCmUfGmCfAmsUfsUm | 1375 |
| SR000536A | UmsUfsGmCfUmGfGmGfCmCfAmCfCmUfGmGfGmAfCmsUfsUm | 1376 |
| SR000537A | UmsCfsUmGfCmUfGmGfGmCfCmAfCmCfUmGfGmGfAmsUfsUm | 1377 |
| SR000538A | AmsCfsCmUfGmCfUmGfGmGfCmCfAmCfCmUfGmGfGmsUfsUm | 1378 |
| SR000539A | AmsGfsCmCfUmGfCmUfGmGfGmCfCmAfCmCfUmGfGmsUfsUm | 1379 |
| SR000540A | UmsGfsGmCfCmUfGmCfUmGfGmGfCmCfAmCfCmUfGmsUfsUm | 1380 |
| SR000541A | UmsUfsGmGfCmCfUmGfCmUfGmGfGmCfCmAfCmCfUmsUfsUm | 1381 |
| SR000542A | UmsCfsUmGfGmCfCmUfGmCfUmGfGmGfCmCfAmCfCmsUfsUm | 1382 |
| SR000543A | UmsCfsCmUfGmGfCmCfUmGfCmUfGmGfGmCfCmAfCmsUfsUm | 1383 |
| SR000544A | UmsAfsCmCfCmAfGmCfCmCfCmUfGmGfCmCfUmGfCmsUfsUm | 1384 |
| SR000545A | UmsCfsAmCfCmCfAmGfCmCfCmCfUmGfGmCfCmUfGmsUfsUm | 1385 |
| SR000546A | AmsUfsCmAfCmCfCmAfGmCfCmCfCmUfGmGfCmCfUmsUfsUm | 1386 |
| SR000547A | AmsGfsUmCfAmCfCmCfAmGfCmCfCmCfUmGfGmCfCmsUfsUm | 1387 |
| SR000548A | UmsGfsGmUfCmAfCmCfCmAfGmCfCmCfCmUfGmGfCmsUfsUm | 1388 |
| SR000549A | UmsCfsGmGfUmCfAmCfCmCfAmGfCmCfCmCfUmGfGmsUfsUm | 1389 |
| SR000550A | AmsUfsCmGfGmUfCmAfCmCfCmAfGmCfCmCfCmUfGmsUfsUm | 1390 |
| SR000551A | UmsAfsUmCfGmGfUmCfAmCfCmCfAmGfCmCfCmCfUmsUfsUm | 1391 |
| SR000552A | UmsCfsAmUfCmGfGmUfCmAfCmCfCmAfGmCfCmCfCmsUfsUm | 1392 |
| SR000553A | AmsCfsCmAfUmCfGmGfUmCfAmCfCmCfAmGfCmCfCmsUfsUm | 1393 |
| SR000554A | AmsGfsCmCfAmUfCmGfGmUfCmAfCmCfCmAfGmCfCmsUfsUm | 1394 |
| SR000555A | AmsAfsGmCfCmAfUmCfGmGfUmCfAmCfCmCfAmGfCmsUfsUm | 1395 |
| SR000556A | AmsAfsAmGfCmCfAmUfCmGfGmUfCmAfCmCfCmAfGmsUfsUm | 1396 |
| SR000557A | UmsGfsAmAfGmCfCmAfUmCfGmGfUmCfAmCfCmCfAmsUfsUm | 1397 |
| SR000558A | UmsUfsGmAfAmGfCmCfAmUfCmGfGmUfCmAfCmCfCmsUfsUm | 1398 |
| SR000559A | AmsCfsUmGfAmAfGmCfCmAfUmCfGmGfUmCfAmCfCmsUfsUm | 1399 |
| SR000560A | AmsAfsCmUfGmAfAmGfCmCfAmUfCmGfGmUfCmAfCmsUfsUm | 1400 |
| SR000561A | AmsAfsAmCfUmGfAmAfGmCfCmAfUmCfGmGfUmCfAmsUfsUm | 1401 |
| SR000562A | AmsGfsAmAfCmUfGmAfAmGfCmCfAmUfCmGfGmUfCmsUfsUm | 1402 |
| SR000563A | AmsGfsGmAfAmCfUmGfAmAfGmCfCmAfUmCfGmGfUmsUfsUm | 1403 |
| SR000564A | AmsGfsGmGfAmAfCmUfGmAfAmGfCmCfAmUfCmGfGmsUfsUm | 1404 |
| SR000565A | UmsAfsGmGfGmAfAmCfUmGfAmAfGmCfCmAfUmCfGmsUfsUm | 1405 |
| SR000566A | UmsCfsAmGfGmGfAmAfCmUfGmAfAmGfCmCfAmUfCmsUfsUm | 1406 |
| SR000567A | UmsUfsCmAfGmGfGmAfAmCfUmGfAmAfGmCfCmAfUmsUfsUm | 1407 |
| SR000568A | UmsUfsUmCfAmGfGmGfAmAfCmUfGmAfAmGfCmCfAmsUfsUm | 1408 |
| SR000569A | UmsUfsUmUfCmAfGmGfGmAfAmCfUmGfAmAfGmCfCmsUfsUm | 1409 |
| SR000570A | UmsCfsUmUfUmCfAmGfGmGfAmAfCmUfGmAfAmGfCmsUfsUm | 1410 |
| SR000571A | AmsUfsCmUfUmUfCmAfGmGfGmAfAmCfUmGfAmAfGmsUfsUm | 1411 |
| SR000572A | AmsGfsUmCfUmUfUmCfAmGfGmGfAmAfCmUfGmAfAmsUfsUm | 1412 |
| SR000573A | UmsAfsGmUfCmUfUmUfCmAfGmGfGmAfAmCfUmGfAmsUfsUm | 1413 |
| SR000574A | AmsUfsAmGfUmCfUmUfUmCfAmGfGmGfAmAfCmUfGmsUfsUm | 1414 |
| SR000575A | AmsGfsUmAfGmUfCmUfUmUfCmAfGmGfGmAfAmCfUmsUfsUm | 1415 |
| SR000576A | UmsAfsGmUfAmGfUmCfUmUfUmCfAmGfGmGfAmAfCmsUfsUm | 1416 |
| SR000577A | UmsCfsAmGfUmAfGmUfCmUfUmUfCmAfGmGfGmAfAmsUfsUm | 1417 |
| SR000578A | UmsCfsCmAfGmUfAmGfUmCfUmUfUmCfAmGfGmGfAmsUfsUm | 1418 |
| SR000579A | UmsUfsCmCfAmGfUmAfGmUfCmUfUmUfCmAfGmGfGmsUfsUm | 1419 |
| SR000580A | AmsCfsUmCfCmAfGmUfAmGfUmCfUmUfUmCfAmGfGmsUfsUm | 1420 |
| SR000581A | UmsGfsCmUfCmCfAmGfUmAfGmUfCmUfUmUfCmAfGmsUfsUm | 1421 |
| SR000582A | AmsUfsGmCfUmCfCmAfGmUfAmGfUmCfUmUfUmCfAmsUfsUm | 1422 |
| SR000583A | AmsGfsUmGfCmUfCmCfAmGfUmAfGmUfCmUfUmUfCmsUfsUm | 1423 |
| SR000584A | UmsGfsGmUfGmCfUmCfCmAfGmUfAmGfUmCfUmUfUmsUfsUm | 1424 |
| SR000585A | AmsAfsCmGfGmUfGmCfUmCfCmAfGmUfAmGfUmCfUmsUfsUm | 1425 |
| SR000586A | UmsAfsAmCfGmGfUmGfCmUfCmCfAmGfUmAfGmUfCmsUfsUm | 1426 |
| SR000587A | UmsUfsAmAfCmGfGmUfGmCfUmCfCmAfGmUfAmGfUmsUfsUm | 1427 |
| SR000588A | UmsUfsUmAfAmCfGmGfUmGfCmUfCmCfAmGfUmAfGmsUfsUm | 1428 |
| SR000589A | UmsCfsUmUfAmAfCmGfGmUfGmCfUmCfCmAfGmUfAmsUfsUm | 1429 |
| SR000590A | UmsCfsCmUfUmAfAmCfGmGfUmGfCmUfCmCfAmGfUmsUfsUm | 1430 |
| SR000591A | AmsUfsCmCfUmUfAmAfCmGfGmUfGmCfUmCfCmAfGmsUfsUm | 1431 |
| SR000592A | UmsGfsUmCfCmUfUmAfAmCfGmGfUmGfCmUfCmCfAmsUfsUm | 1432 |
| SR000593A | UmsUfsGmUfCmCfUmUfAmAfCmGfGmUfGmCfUmCfCmsUfsUm | 1433 |
| SR000594A | UmsUfsUmGfUmCfCmUfUmAfAmCfGmGfUmGfCmUfCmsUfsUm | 1434 |
| SR000595A | AmsCfsUmUfGmUfCmCfUmUfAmAfCmGfGmUfGmCfUmsUfsUm | 1435 |
| SR000596A | AmsAfsCmUfUmGfUmCfCmUfUmAfAmCfGmGfUmGfCmsUfsUm | 1436 |
| SR000597A | AmsAfsAmCfUmUfGmUfCmCfUmUfAmAfCmGfGmUfGmsUfsUm | 1437 |
| SR000598A | AmsGfsAmAfCmUfUmGfUmCfCmUfUmAfAmCfGmGfUmsUfsUm | 1438 |
| SR000599A | AmsAfsGmAfAmCfUmUfGmUfCmCfUmUfAmAfCmGfGmsUfsUm | 1439 |
| SR000600A | AmsGfsAmGfAmAfCmUfUmGfUmCfCmUfUmAfAmCfGmsUfsUm | 1440 |
| SR000601A | UmsAfsGmAfGmAfAmCfUmUfGmUfCmCfUmUfAmAfCmsUfsUm | 1441 |
| SR000602A | UmsCfsAmGfAmGfAmAfCmUfUmGfUmCfCmUfUmAfAmsUfsUm | 1442 |
| SR000603A | UmsUfsCmAfGmAfGmAfAmCfUmUfGmUfCmCfUmUfAmsUfsUm | 1443 |
| SR000604A | AmsCfsUmCfAmGfAmGfAmAfCmUfUmGfUmCfCmUfUmsUfsUm | 1444 |
| SR000605A | AmsAfsCmUfCmAfGmAfGmAfAmCfUmUfGmUfCmCfUmsUfsUm | 1445 |
| SR000606A | AmsAfsAmCfUmCfAmGfAmGfAmAfCmUfUmGfUmCfCmsUfsUm | 1446 |
| SR000607A | AmsGfsAmAfCmUfCmAfGmAfGmAfAmCfUmUfGmUfCmsUfsUm | 1447 |
| SR000608A | UmsAfsGmAfAmCfUmCfAmGfAmGfAmAfCmUfUmGfUmsUfsUm | 1448 |
| SR000609A | UmsCfsAmGfAmAfCmUfCmAfGmAfGmAfAmCfUmUfGmsUfsUm | 1449 |
| SR000610A | UmsCfsCmAfGmAfAmCfUmCfAmGfAmGfAmAfCmUfUmsUfsUm | 1450 |
| SR000611A | UmsCfsCmCfAmGfAmAfCmUfCmAfGmAfGmAfAmCfUmsUfsUm | 1451 |
| SR000612A | AmsUfsCmCfCmAfGmAfAmCfUmCfAmGfAmGfAmAfCmsUfsUm | 1452 |
| SR000613A | AmsAfsUmCfCmCfAmGfAmAfCmUfCmAfGmAfGmAfAmsUfsUm | 1453 |
| SR000614A | AmsAfsAmUfCmCfCmAfGmAfAmCfUmCfAmGfAmGfAmsUfsUm | 1454 |
| SR000615A | UmsAfsAmAfUmCfCmCfAmGfAmAfCmUfCmAfGmAfGmsUfsUm | 1455 |
| SR000616A | UmsCfsAmAfAmUfCmCfCmAfGmAfAmCfUmCfAmGfAmsUfsUm | 1456 |
| SR000617A | UmsCfsCmAfAmAfUmCfCmCfAmGfAmAfCmUfCmAfGmsUfsUm | 1457 |
| SR000618A | AmsUfsCmCfAmAfAmUfCmCfCmAfGmAfAmCfUmCfAmsUfsUm | 1458 |
| SR000619A | AmsGfsUmCfCmAfAmAfUmCfCmCfAmGfAmAfCmUfCmsUfsUm | 1459 |
| SR000620A | AmsGfsGmUfCmCfAmAfAmUfCmCfCmAfGmAfAmCfUmsUfsUm | 1460 |
| SR000621A | UmsAfsGmGfGmUfCmCfAmAfAmUfCmCfCmAfGmAfAmsUfsUm | 1461 |
| SR000622A | UmsCfsAmGfGmGfUmCfCmAfAmAfUmCfCmCfAmGfAmsUfsUm | 1462 |
| SR000623A | UmsUfsCmAfGmGfGmUfCmCfAmAfAmUfCmCfCmAfGmsUfsUm | 1463 |
| SR000624A | UmsCfsUmCfAmGfGmGfUmCfCmAfAmAfUmCfCmCfAmsUfsUm | 1464 |
| SR000625A | AmsCfsCmUfCmAfGmGfGmUfCmCfAmAfAmUfCmCfCmsUfsUm | 1465 |
| SR000626A | AmsAfsCmCfUmCfAmGfGmGfUmCfCmAfAmAfUmCfCmsUfsUm | 1466 |
| SR000627A | UmsGfsAmCfCmUfCmAfGmGfGmUfCmCfAmAfAmUfCmsUfsUm | 1467 |
| SR000628A | UmsUfsGmAfCmCfUmCfAmGfGmGfUmCfCmAfAmAfUmsUfsUm | 1468 |
| SR000629A | UmsCfsUmGfAmCfCmUfCmAfGmGfGmUfCmCfAmAfAmsUfsUm | 1469 |
| SR000630A | AmsUfsCmUfGmAfCmCfUmCfAmGfGmGfUmCfCmAfAmsUfsUm | 1470 |
| SR000631A | AmsGfsUmCfUmGfAmCfCmUfCmAfGmGfGmUfCmCfAmsUfsUm | 1471 |
| SR000632A | UmsGfsGmUfCmUfGmAfCmCfUmCfAmGfGmGfUmCfCmsUfsUm | 1472 |
| SR000633A | UmsUfsGmGfUmCfUmGfAmCfCmUfCmAfGmGfGmUfCmsUfsUm | 1473 |
| SR000634A | AmsUfsUmGfGmUfCmUfGmAfCmCfUmCfAmGfGmGfUmsUfsUm | 1474 |
| SR000635A | AmsGfsUmUfGmGfUmCfUmGfAmCfCmUfCmAfGmGfGmsUfsUm | 1475 |
| SR000636A | AmsAfsGmUfUmGfGmUfCmUfGmAfCmCfUmCfAmGfGmsUfsUm | 1476 |
| SR000637A | AmsAfsAmGfUmUfGmGfUmCfUmGfAmCfCmUfCmAfGmsUfsUm | 1477 |
| SR000638A | UmsGfsAmAfGmUfUmGfGmUfCmUfGmAfCmCfUmCfAmsUfsUm | 1478 |
| SR000639A | UmsUfsGmAfAmGfUmUfGmGfUmCfUmGfAmCfCmUfCmsUfsUm | 1479 |
| SR000640A | AmsCfsUmGfAmAfGmUfUmGfGmUfCmUfGmAfCmCfUmsUfsUm | 1480 |
| SR000641A | AmsGfsCmUfGmAfAmGfUmUfGmGfUmCfUmGfAmCfCmsUfsUm | 1481 |
| SR000642A | UmsGfsGmCfUmGfAmAfGmUfUmGfGmUfCmUfGmAfCmsUfsUm | 1482 |
| SR000643A | UmsAfsGmCfCmAfCmGfGmCfUmGfAmAfGmUfUmGfGmsUfsUm | 1483 |
| SR000644A | AmsCfsAmGfCmCfAmCfGmGfCmUfGmAfAmGfUmUfGmsUfsUm | 1484 |
| SR000645A | AmsGfsCmAfGmCfCmAfCmGfGmCfUmGfAmAfGmUfUmsUfsUm | 1485 |
| SR000646A | AmsGfsGmCfAmGfCmCfAmCfGmGfCmUfGmAfAmGfUmsUfsUm | 1486 |
| SR000647A | UmsAfsGmGfCmAfGmCfCmAfCmGfGmCfUmGfAmAfGmsUfsUm | 1487 |
| SR000648A | UmsCfsAmGfGmCfAmGfCmCfAmCfGmGfCmUfGmAfAmsUfsUm | 1488 |
| SR000649A | UmsUfsCmAfGmGfCmAfGmCfCmAfCmGfGmCfUmGfAmsUfsUm | 1489 |
| SR000650A | UmsCfsUmCfAmGfGmCfAmGfCmCfAmCfGmGfCmUfGmsUfsUm | 1490 |
| SR000651A | AmsUfsCmUfCmAfGmGfCmAfGmCfCmAfCmGfGmCfUmsUfsUm | 1491 |
| SR000652A | AmsGfsUmCfUmCfAmGfGmCfAmGfCmCfAmCfGmGfCmsUfsUm | 1492 |
| SR000653A | AmsGfsGmUfCmUfCmAfGmGfCmAfGmCfCmAfCmGfGmsUfsUm | 1493 |
| SR000654A | AmsAfsGmGfUmCfUmCfAmGfGmCfAmGfCmCfAmCfGmsUfsUm | 1494 |
| SR000655A | UmsGfsAmGfGmUfCmUfCmAfGmGfCmAfGmCfCmAfCmsUfsUm | 1495 |
| SR000656A | UmsUfsGmAfGmGfUmCfUmCfAmGfGmCfAmGfCmCfAmsUfsUm | 1496 |
| SR000657A | AmsUfsUmGfAmGfGmUfCmUfCmAfGmGfCmAfGmCfCmsUfsUm | 1497 |
| SR000658A | UmsAfsUmUfGmAfGmGfUmCfUmCfAmGfGmCfAmGfCmsUfsUm | 1498 |
| SR000659A | AmsUfsAmUfUmGfAmGfGmUfCmUfCmAfGmGfCmAfGmsUfsUm | 1499 |
| SR000660A | AmsGfsUmAfUmUfGmAfGmGfUmCfUmCfAmGfGmCfAmsUfsUm | 1500 |
| SR000661A | AmsGfsGmUfAmUfUmGfAmGfGmUfCmUfCmAfGmGfCmsUfsUm | 1501 |
| SR000662A | AmsGfsGmGfUmAfUmUfGmAfGmGfUmCfUmCfAmGfGmsUfsUm | 1502 |
| SR000663A | UmsUfsGmGfGmGfUmAfUmUfGmAfGmGfUmCfUmCfAmsUfsUm | 1503 |
| SR000664A | UmsUfsUmGfGmGfGmUfAmUfUmGfAmGfGmUfCmUfCmsUfsUm | 1504 |
| SR000665A | AmsCfsUmUfGmGfGmGfUmAfUmUfGmAfGmGfUmCfUmsUfsUm | 1505 |
| SR000666A | AmsAfsCmUfUmGfGmGfGmUfAmUfUmGfAmGfGmUfCmsUfsUm | 1506 |
| SR000667A | AmsGfsAmCfUmUfGmGfGmGfUmAfUmUfGmAfGmGfUmsUfsUm | 1507 |
| SR000668A | UmsGfsGmAfCmUfUmGfGmGfGmUfAmUfUmGfAmGfGmsUfsUm | 1508 |
| SR000669A | AmsUfsGmGfAmCfUmUfGmGfGmGfUmAfUmUfGmAfGmsUfsUm | 1509 |
| SR000670A | AmsGfsUmGfGmAfCmUfUmGfGmGfGmUfAmUfUmGfAmsUfsUm | 1510 |
| SR000671A | AmsGfsGmUfGmGfAmCfUmUfGmGfGmGfUmAfUmUfGmsUfsUm | 1511 |
| SR000672A | UmsAfsGmGfUmGfGmAfCmUfUmGfGmGfGmUfAmUfUmsUfsUm | 1512 |
| SR000673A | AmsCfsAmGfGmUfGmGfAmCfUmUfGmGfGmGfUmAfUmsUfsUm | 1513 |
| SR000674A | AmsGfsCmAfGmGfUmGfGmAfCmUfUmGfGmGfGmUfAmsUfsUm | 1514 |
| SR000675A | UmsAfsGmGfCmAfGmGfUmGfGmAfCmUfUmGfGmGfGmsUfsUm | 1515 |
| SR000676A | AmsUfsAmGfGmCfAmGfGmUfGmGfAmCfUmUfGmGfGmsUfsUm | 1516 |
| SR000677A | AmsAfsUmAfGmGfCmAfGmGfUmGfGmAfCmUfUmGfGmsUfsUm | 1517 |
| SR000678A | AmsGfsAmUfAmGfGmCfAmGfGmUfGmGfAmCfUmUfGmsUfsUm | 1518 |
| SR000679A | UmsGfsGmAfUmAfGmGfCmAfGmGfUmGfGmAfCmUfUmsUfsUm | 1519 |
| SR000680A | AmsUfsGmGfAmUfAmGfGmCfAmGfGmUfGmGfAmCfUmsUfsUm | 1520 |
| SR000681A | AmsAfsUmGfGmAfUmAfGmGfCmAfGmGfUmGfGmAfCmsUfsUm | 1521 |
| SR000682A | AmsGfsAmUfGmGfAmUfAmGfGmCfAmGfGmUfGmGfAmsUfsUm | 1522 |
| SR000683A | AmsGfsGmAfUmGfGmAfUmAfGmGfCmAfGmGfUmGfGmsUfsUm | 1523 |
| SR000684A | UmsAfsGmGfAmUfGmGfAmUfAmGfGmCfAmGfGmUfGmsUfsUm | 1524 |
| SR000685A | AmsCfsAmGfGmAfUmGfGmAfUmAfGmGfCmAfGmGfUmsUfsUm | 1525 |
| SR000686A | UmsGfsCmAfGmGfAmUfGmGfAmUfAmGfGmCfAmGfGmsUfsUm | 1526 |
| SR000687A | UmsCfsGmCfAmGfGmAfUmGfGmAfUmAfGmGfCmAfGmsUfsUm | 1527 |
| SR000688A | UmsUfsCmGfCmAfGmGfAmUfGmGfAmUfAmGfGmCfAmsUfsUm | 1528 |
| SR000689A | AmsCfsUmCfGmCfAmGfGmAfUmGfGmAfUmAfGmGfCmsUfsUm | 1529 |
| SR000690A | AmsGfsCmUfCmGfCmAfGmGfAmUfGmGfAmUfAmGfGmsUfsUm | 1530 |
| SR000691A | AmsAfsGmCfUmCfGmCfAmGfGmAfUmGfGmAfUmAfGmsUfsUm | 1531 |
| SR000692A | AmsGfsAmGfCmUfCmGfCmAfGmGfAmUfGmGfAmUfAmsUfsUm | 1532 |
| SR000693A | AmsGfsGmAfGmCfUmCfGmCfAmGfGmAfUmGfGmAfUmsUfsUm | 1533 |
| SR000694A | AmsAfsGmGfAmGfCmUfCmGfCmAfGmGfAmUfGmGfAmsUfsUm | 1534 |
| SR000695A | UmsAfsAmGfGmAfGmCfUmCfGmCfAmGfGmAfUmGfGmsUfsUm | 1535 |
| SR000696A | UmsCfsAmAfGmGfAmGfCmUfCmGfCmAfGmGfAmUfGmsUfsUm | 1536 |
| SR000697A | UmsCfsCmAfAmGfGmAfGmCfUmCfGmCfAmGfGmAfUmsUfsUm | 1537 |
| SR000698A | AmsCfsCmCfAmAfGmGfAmGfCmUfCmGfCmAfGmGfAmsUfsUm | 1538 |
| SR000699A | AmsAfsCmCfCmAfAmGfGmAfGmCfUmCfGmCfAmGfGmsUfsUm | 1539 |
| SR000700A | AmsGfsAmCfCmCfAmAfGmGfAmGfCmUfCmGfCmAfGmsUfsUm | 1540 |
| SR000701A | AmsGfsGmAfCmCfCmAfAmGfGmAfGmCfUmCfGmCfAmsUfsUm | 1541 |
| SR000702A | UmsAfsGmGfAmCfCmCfAmAfGmGfAmGfCmUfCmGfCmsUfsUm | 1542 |
| SR000703A | AmsCfsAmGfGmAfCmCfCmAfAmGfGmAfGmCfUmCfGmsUfsUm | 1543 |
| SR000704A | UmsGfsCmAfGmGfAmCfCmCfAmAfGmGfAmGfCmUfCmsUfsUm | 1544 |
| SR000705A | UmsUfsGmCfAmGfGmAfCmCfCmAfAmGfGmAfGmCfUmsUfsUm | 1545 |
| SR000706A | AmsUfsUmGfCmAfGmGfAmCfCmCfAmAfGmGfAmGfCmsUfsUm | 1546 |
| SR000707A | AmsAfsUmUfGmCfAmGfGmAfCmCfCmAfAmGfGmAfGmsUfsUm | 1547 |
| SR000708A | AmsGfsAmUfUmGfCmAfGmGfAmCfCmCfAmAfGmGfAmsUfsUm | 1548 |
| SR000709A | AmsAfsGmAfUmUfGmCfAmGfGmAfCmCfCmAfAmGfGmsUfsUm | 1549 |
| SR000710A | AmsGfsAmGfAmUfUmGfCmAfGmGfAmCfCmCfAmAfGmsUfsUm | 1550 |
| SR000711A | UmsGfsGmAfGmAfUmUfGmCfAmGfGmAfCmCfCmAfAmsUfsUm | 1551 |
| SR000712A | UmsUfsGmGfAmGfAmUfUmGfCmAfGmGfAmCfCmCfAmsUfsUm | 1552 |
| SR000713A | UmsCfsUmGfGmAfGmAfUmUfGmCfAmGfGmAfCmCfCmsUfsUm | 1553 |
| SR000714A | UmsCfsCmUfGmGfAmGfAmUfUmGfCmAfGmGfAmCfCmsUfsUm | 1554 |
| SR000715A | AmsCfsCmCfUmGfGmAfGmAfUmUfGmCfAmGfGmAfCmsUfsUm | 1555 |
| SR000716A | UmsAfsGmCfCmCfUmGfGmAfGmAfUmUfGmCfAmGfGmsUfsUm | 1556 |
| SR000717A | AmsCfsAmGfCmCfCmUfGmGfAmGfAmUfUmGfCmAfGmsUfsUm | 1557 |
| SR000718A | AmsGfsCmAfGmCfCmCfUmGfGmAfGmAfUmUfGmCfAmsUfsUm | 1558 |
| SR000719A | UmsAfsCmAfGmGfGmGfCmAfGmCfCmCfUmGfGmAfGmsUfsUm | 1559 |
| SR000720A | UmsUfsAmCfAmGfGmGfGmCfAmGfCmCfCmUfGmGfAmsUfsUm | 1560 |
| SR000721A | UmsCfsUmAfCmAfGmGfGmGfCmAfGmCfCmCfUmGfGmsUfsUm | 1561 |
| SR000722A | AmsCfsCmUfAmCfAmGfGmGfGmCfAmGfCmCfCmUfGmsUfsUm | 1562 |
| SR000723A | AmsAfsCmCfUmAfCmAfGmGfGmGfCmAfGmCfCmCfUmsUfsUm | 1563 |
| SR000724A | UmsAfsAmCfCmUfAmCfAmGfGmGfGmCfAmGfCmCfCmsUfsUm | 1564 |
| SR000725A | AmsCfsAmAfCmCfUmAfCmAfGmGfGmGfCmAfGmCfCmsUfsUm | 1565 |
| SR000726A | AmsGfsCmAfAmCfCmUfAmCfAmGfGmGfGmCfAmGfCmsUfsUm | 1566 |
| SR000727A | AmsAfsGmCfAmAfCmCfUmAfCmAfGmGfGmGfCmAfGmsUfsUm | 1567 |
| SR000728A | UmsAfsAmGfCmAfAmCfCmUfAmCfAmGfGmGfGmCfAmsUfsUm | 1568 |
| SR000729A | UmsUfsAmAfGmCfAmAfCmCfUmAfCmAfGmGfGmGfCmsUfsUm | 1569 |
| SR000730A | UmsUfsUmAfAmGfCmAfAmCfCmUfAmCfAmGfGmGfGmsUfsUm | 1570 |
| SR000731A | UmsUfsUmUfAmAfGmCfAmAfCmCfUmAfCmAfGmGfGmsUfsUm | 1571 |
| SR000732A | UmsUfsUmUfUmAfAmGfCmAfAmCfCmUfAmCfAmGfGmsUfsUm | 1572 |
| SR000733A | UmsCfsUmUfUmUfAmAfGmCfAmAfCmCfUmAfCmAfGmsUfsUm | 1573 |
| SR000734A | UmsCfsCmUfUmUfUmAfAmGfCmAfAmCfCmUfAmCfAmsUfsUm | 1574 |
| SR000735A | UmsCfsCmCfUmUfUmUfAmAfGmCfAmAfCmCfUmAfCmsUfsUm | 1575 |
| SR000736A | AmsUfsCmCfCmUfUmUfUmAfAmGfCmAfAmCfCmUfAmsUfsUm | 1576 |
| SR000737A | UmsGfsUmCfCmCfUmUfUmUfAmAfGmCfAmAfCmCfUmsUfsUm | 1577 |
| SR000738A | UmsUfsGmUfCmCfCmUfUmUfUmAfAmGfCmAfAmCfCmsUfsUm | 1578 |
| SR000739A | AmsCfsUmGfUmCfCmCfUmUfUmUfAmAfGmCfAmAfCmsUfsUm | 1579 |
| SR000740A | UmsAfsCmUfGmUfCmCfCmUfUmUfUmAfAmGfCmAfAmsUfsUm | 1580 |
| SR000741A | AmsUfsAmCfUmGfUmCfCmCfUmUfUmUfAmAfGmCfAmsUfsUm | 1581 |
| SR000742A | AmsAfsUmAfCmUfGmUfCmCfCmUfUmUfUmAfAmGfCmsUfsUm | 1582 |
| SR000743A | AmsAfsAmUfAmCfUmGfUmCfCmCfUmUfUmUfAmAfGmsUfsUm | 1583 |
| SR000744A | AmsGfsAmAfUmAfCmUfGmUfCmCfCmUfUmUfUmAfAmsUfsUm | 1584 |
| SR000745A | AmsAfsGmAfAmUfAmCfUmGfUmCfCmCfUmUfUmUfAmsUfsUm | 1585 |
| SR000746A | UmsGfsAmGfAmAfUmAfCmUfGmUfCmCfCmUfUmUfUmsUfsUm | 1586 |
| SR000747A | UmsUfsGmAfGmAfAmUfAmCfUmGfUmCfCmCfUmUfUmsUfsUm | 1587 |
| SR000748A | AmsCfsUmGfAmGfAmAfUmAfCmUfGmUfCmCfCmUfUmsUfsUm | 1588 |
| SR000749A | UmsAfsCmUfGmAfGmAfAmUfAmCfUmGfUmCfCmCfUmsUfsUm | 1589 |
| SR000750A | AmsCfsAmCfUmGfAmGfAmAfUmAfCmUfGmUfCmCfCmsUfsUm | 1590 |
| SR000751A | AmsGfsCmAfCmUfGmAfGmAfAmUfAmCfUmGfUmCfCmsUfsUm | 1591 |
| SR000752A | AmsAfsGmCfAmCfUmGfAmGfAmAfUmAfCmUfGmUfCmsUfsUm | 1592 |
| SR000753A | AmsGfsAmGfCmAfCmUfGmAfGmAfAmUfAmCfUmGfUmsUfsUm | 1593 |
| SR000754A | AmsAfsGmAfGmCfAmCfUmGfAmGfAmAfUmAfCmUfGmsUfsUm | 1594 |
| SR000755A | AmsGfsAmGfAmGfCmAfCmUfGmAfGmAfAmUfAmCfUmsUfsUm | 1595 |
| SR000756A | AmsGfsGmAfGmAfGmCfAmCfUmGfAmGfAmAfUmAfCmsUfsUm | 1596 |
| SR000757A | UmsAfsGmGfAmGfAmGfCmAfCmUfGmAfGmAfAmUfAmsUfsUm | 1597 |
| SR000758A | AmsUfsAmGfGmAfGmAfGmCfAmCfUmGfAmGfAmAfUmsUfsUm | 1598 |
| SR000759A | AmsGfsUmAfGmGfAmGfAmGfCmAfCmUfGmAfGmAfAmsUfsUm | 1599 |
| SR000760A | AmsGfsGmUfAmGfGmAfGmAfGmCfAmCfUmGfAmGfAmsUfsUm | 1600 |
| SR000761A | AmsGfsGmGfUmAfGmGfAmGfAmGfCmAfCmUfGmAfGmsUfsUm | 1601 |
| SR000762A | UmsGfsAmGfGmUfGmGfGmGfUmAfGmGfAmGfAmGfCmsUfsUm | 1602 |
| SR000763A | AmsUfsGmAfGmGfUmGfGmGfGmUfAmGfGmAfGmAfGmsUfsUm | 1603 |
| SR000764A | UmsAfsUmGfAmGfGmUfGmGfGmGfUmAfGmGfAmGfAmsUfsUm | 1604 |
| SR000765A | AmsCfsAmUfGmAfGmGfUmGfGmGfGmUfAmGfGmAfGmsUfsUm | 1605 |
| SR000766A | AmsGfsCmAfUmGfAmGfGmUfGmGfGmGfUmAfGmGfAmsUfsUm | 1606 |
| SR000767A | AmsGfsGmCfAmUfGmAfGmGfUmGfGmGfGmUfAmGfGmsUfsUm | 1607 |
| SR000768A | UmsAfsGmGfCmAfUmGfAmGfGmUfGmGfGmGfUmAfGmsUfsUm | 1608 |
| SR000769A | UmsCfsAmGfGmCfAmUfGmAfGmGfUmGfGmGfGmUfAmsUfsUm | 1609 |
| SR000770A | AmsCfsCmAfGmCfAmUfGmCfCmUfGmGfAmGfGmGfGmsUfsUm | 1610 |
| SR000771A | AmsGfsCmCfAmGfCmAfUmGfCmCfUmGfGmAfGmGfGmsUfsUm | 1611 |
| SR000772A | AmsGfsGmCfCmAfGmCfAmUfGmCfCmUfGmGfAmGfGmsUfsUm | 1612 |
| SR000773A | AmsAfsGmGfCmCfAmGfCmAfUmGfCmCfUmGfGmAfGmsUfsUm | 1613 |
| SR000774A | UmsUfsGmGfGmAfGmGfCmCfAmGfCmAfUmGfCmCfUmsUfsUm | 1614 |
| SR000775A | AmsUfsUmGfGmGfAmGfGmCfCmAfGmCfAmUfGmCfCmsUfsUm | 1615 |
| SR000776A | UmsAfsUmUfGmGfGmAfGmGfCmCfAmGfCmAfUmGfCmsUfsUm | 1616 |
| SR000777A | UmsUfsAmUfUmGfGmGfAmGfGmCfCmAfGmCfAmUfGmsUfsUm | 1617 |
| SR000778A | UmsUfsUmAfUmUfGmGfGmAfGmGfCmCfAmGfCmAfUmsUfsUm | 1618 |
| SR000779A | UmsUfsUmUfAmUfUmGfGmGfAmGfGmCfCmAfGmCfAmsUfsUm | 1619 |
| SR000780A | AmsCfsUmUfUmAfUmUfGmGfGmAfGmGfCmCfAmGfCmsUfsUm | 1620 |
| SR000781A | AmsGfsCmUfUmUfAmUfUmGfGmGfAmGfGmCfCmAfGmsUfsUm | 1621 |
| SR000782A | UmsAfsGmCfUmUfUmAfUmUfGmGfGmAfGmGfCmCfAmsUfsUm | 1622 |
| SR000783A | UmsCfsAmGfCmUfUmUfAmUfUmGfGmGfAmGfGmCfCmsUfsUm | 1623 |
| SR000784A | UmsCfsCmAfGmCfUmUfUmAfUmUfGmGfGmAfGmGfCmsUfsUm | 1624 |
| SR000785A | AmsUfsCmCfAmGfCmUfUmUfAmUfUmGfGmGfAmGfGmsUfsUm | 1625 |
| SR000786A | UmsGfsUmCfCmAfGmCIfUmUfUmAfUmUfGmGfGmAfGmsUfsUm | 1626 |
| SR000787A | UmsUfsGmUfCmCfAmGfCmUfUmUfAmUfUmGfGmGfAmsUfsUm | 1627 |
| SR000788A | UmsUfsUmGfUmCfCmAfGmCfUmUfUmAfUmUfGmGfGmsUfsUm | 1628 |
| SR000789A | UmsCfsUmUfGmUfCmCfAmGfCmUfUmUfAmUfUmGfGmsUfsUm | 1629 |
| SR000790A | UmsUfsCmUfUmGfUmCfCmAfGmCfUmUfUmAfUmUfGmsUfsUm | 1630 |
| SR000791A | UmsUfsUmCfUmUfGmUfCmCfAmGfCmUfUmUfAmUfUmsUfsUm | 1631 |
| SR000792A | AmsCfsUmUfCmUfUmGfUmCfCmAfGmCfUmUfUmAfUmsUfsUm | 1632 |
| SR000793A | AmsGfsCmUfUmCfUmUfGmUfCmCfAmGfCmUfUmUfAmsUfsUm | 1633 |
| SR000794A | UmsAfsGmCfUmUfCmUfUmGfUmCfCmAfGmCfUmUfUmsUfsUm | 1634 |
| SR000795A | AmsCfsAmGfCmUfUmCfUmUfGmUfCmCfAmGfCmUfUmsUfsUm | 1635 |
| SR000796A | AmsGfsCmAfGmCfUmUfCmUfUmGfUmCfCmAfGmCfUmsUfsUm | 1636 |
| SR000797A | UmsAfsGmCfAmGfCmUfUmCfUmUfGmUfCmCfAmGfCmsUfsUm | 1637 |
| SR000798A | AmsUfsAmGfCmAfGmCfUmUfCmUfUmGfUmCfCmAfGmsUfsUm | 1638 |
| SR000799A | UmsAfsUmAfGmCfAmGfCmUfUmCfUmUfGmUfCmCfAmsUfsUm | 1639 |
| SR000800A | UmsCfsAmUfAmGfCmAfGmCfUmUfCmUfUmGfUmCfCmsUfsUm | 1640 |
| SR002963A | UmsCfsAmsCfUmGfAmGmAmAmUmAfCmUfGmUfCmCfCmGfsUm | 1641 |
| SR004480A | GmsCfsAmCmUmGfAmGmAmAmUmAmCmUfGmUfCmCmCmUmU msUmsUm | 1642 |
| SR004448A | AmsGfsAmAfUmAfCmUfGmUfCmCfCmUfUmUfUmAfAmsUfsUm | 1643 |
| SR004449A | AmsCfsUmGfAmGfAmAfUmAfCmUfGmUfCmCfCmUfUmsUfsUm | 1644 |
| SR004450A | AmsAfsUmAfGmGfCmAfGmGfUmGfGmAfCmUfUmGfGmsUfsUm | 1645 |
| SR004451A | AmsAfsGmAfAmCfUmUfGmUfCmCfUmUfAmAfCmGfGmsUfsUm | 1646 |
| SR004452A | AmsAfsGmAfAmGfGmGfAmGfGmCfAmUfCmCfUmCfGmsUfsUm | 1647 |
| SR002967A | UmsCfsAmsCfUmGfAmGmAmAmUmAfCmUfGmUfCmCfCmGfsUm | 1648 |
| SR011332A | (VPUm)sGfsAmAfUmAfCmUfGmUfCmCfCmUfUmUfUmAfAmsUfs Um | 1649 |
| SR013861A | (CP1a- U)sGfsAmAfUmAfCmUfGmUfCmCfCmUfUmUfUmAfAmsUfsUm | 1650 |
| SR013801A | (CP1a- U)sCfsUmGfAmGfAmAfUmAfCmUfGmUfCmCfCmUfUmsUfsUm | 1651 |
| SR015110A | (CP1a-U)sCfsUmGfAmGf(PCN- A)AfUmAfCmUfGmUfCmCfCmUfUmsUfsUm | 1652 |
| SR0152050A | UmsdGsAmAmdTAmdCUmGmUmCmdCCmUfUmUmUmAmAmsUm sUm | 1653 |
| SR011331A | UmsGfsAmAfUmAfCmUfGmUfCmCfCmUfUmUfUmAfAmsUfsUm | 1654 |

**Table 6 Paired Modified Sense Strand and Modified Antisense Strand of siRNA Targeting APOC3 Gene Used in the Present Invention**

| Double Strand No. | Sense Strand No. | Antisense Strand No. |
|---|---|---|
| DR000001 | SR000001S | SR000401A |
| DR000002 | SR000002S | SR000402A |
| DR000003 | SR000003S | SR000403A |
| DR000004 | SR000004S | SR000404A |
| DR000005 | SR000005S | SR000405A |
| DR000006 | SR000006S | SR000406A |
| DR000007 | SR000007S | SR000407A |
| DR000008 | SR000008S | SR000408A |
| DR000009 | SR000009S | SR000409A |
| DR000010 | SR000010S | SR000410A |
| DR000011 | SR000011S | SR000411A |
| DR000012 | SR000012S | SR000412A |
| DR000013 | SR000013S | SR000413A |
| DR000014 | SR000014S | SR000414A |
| DR000015 | SR000015S | SR000415A |
| DR000016 | SR000016S | SR000416A |
| DR000017 | SR000017S | SR000417A |
| DR000018 | SR000018S | SR000418A |
| DR000019 | SR000019S | SR000419A |
| DR000020 | SR000020S | SR000420A |
| DR000021 | SR000021S | SR000421A |
| DR000022 | SR000022S | SR000422A |
| DR000023 | SR000023S | SR000423A |
| DR000024 | SR000024S | SR000424A |
| DR000025 | SR000025S | SR000425A |
| DR000026 | SR000026S | SR000426A |
| DR000027 | SR000027S | SR000427A |
| DR000028 | SR000028S | SR000428A |
| DR000029 | SR000029S | SR000429A |
| DR000030 | SR000030S | SR000430A |
| DR000031 | SR000031S | SR000431A |
| DR000032 | SR000032S | SR000432A |
| DR000033 | SR000033S | SR000433A |
| DR000034 | SR000034S | SR000434A |
| DR000035 | SR000035S | SR000435A |
| DR000036 | SR000036S | SR000436A |
| DR000037 | SR000037S | SR000437A |
| DR000038 | SR000038S | SR000438A |
| DR000039 | SR000039S | SR000439A |
| DR000040 | SR000040S | SR000440A |
| DR000041 | SR000041S | SR000441A |
| DR000042 | SR000042S | SR000442A |
| DR000043 | SR000043S | SR000443A |
| DR000044 | SR000044S | SR000444A |
| DR000045 | SR000045S | SR000445A |
| DR000046 | SR000046S | SR000446A |
| DR000047 | SR000047S | SR000447A |
| DR000048 | SR000048S | SR000448A |
| DR000049 | SR000049S | SR000449A |
| DR000050 | SR000050S | SR000450A |
| DR000051 | SR000051S | SR000451A |
| DR000052 | SR000052S | SR000452A |
| DR000053 | SR000053S | SR000453A |
| DR000054 | SR000054S | SR000454A |
| DR000055 | SR000055S | SR000455A |
| DR000056 | SR000056S | SR000456A |
| DR000057 | SR000057S | SR000457A |
| DR000058 | SR000058S | SR000458A |
| DR000059 | SR000059S | SR000459A |
| DR000060 | SR000060S | SR000460A |
| DR000061 | SR000061S | SR000461A |
| DR000062 | SR000062S | SR000462A |
| DR000063 | SR000063S | SR000463A |
| DR000064 | SR000064S | SR000464A |
| DR000065 | SR000065S | SR000465A |
| DR000066 | SR000066S | SR000466A |
| DR000067 | SR000067S | SR000467A |
| DR000068 | SR000068S | SR000468A |
| DR000069 | SR000069S | SR000469A |
| DR000070 | SR000070S | SR000470A |
| DR000071 | SR000071S | SR000471A |
| DR000072 | SR000072S | SR000472A |
| DR000073 | SR000073S | SR000473A |
| DR000074 | SR000074S | SR000474A |
| DR000075 | SR000075S | SR000475A |
| DR000076 | SR000076S | SR000476A |
| DR000077 | SR000077S | SR000477A |
| DR000078 | SR000078S | SR000478A |
| DR000079 | SR000079S | SR000479A |
| DR000080 | SR000080S | SR000480A |
| DR000081 | SR000081S | SR000481A |
| DR000082 | SR000082S | SR000482A |
| DR000083 | SR000083S | SR000483A |
| DR000084 | SR000084S | SR000484A |
| DR000085 | SR000085S | SR000485A |
| DR000086 | SR000086S | SR000486A |
| DR000087 | SR000087S | SR000487A |
| DR000088 | SR000088S | SR000488A |
| DR000089 | SR000089S | SR000489A |
| DR000090 | SR000090S | SR000490A |
| DR000091 | SR000091S | SR000491A |
| DR000092 | SR000092S | SR000492A |
| DR000093 | SR000093S | SR000493A |
| DR000094 | SR000094S | SR000494A |
| DR000095 | SR000095S | SR000495A |
| DR000096 | SR000096S | SR000496A |
| DR000097 | SR000097S | SR000497A |
| DR000098 | SR000098S | SR000498A |
| DR000099 | SR000099S | SR000499A |
| DR000100 | SR000100S | SR000500A |
| DR000101 | SR000101S | SR000501A |
| DR000102 | SR000102S | SR000502A |
| DR000103 | SR000103S | SR000503A |
| DR000104 | SR000104S | SR000504A |
| DR000105 | SR000105S | SR000505A |
| DR000106 | SR000106S | SR000506A |
| DR000107 | SR000107S | SR000507A |
| DR000108 | SR000108S | SR000508A |
| DR000109 | SR000109S | SR000509A |
| DR000110 | SR000110S | SR000510A |
| DR000111 | SR000111S | SR000511A |
| DR000112 | SR000112S | SR000512A |
| DR000113 | SR000113S | SR000513A |
| DR000114 | SR000114S | SR000514A |
| DR000115 | SR000115S | SR000515A |
| DR000116 | SR000116S | SR000516A |
| DR000117 | SR000117S | SR000517A |
| DR000118 | SR000118S | SR000518A |
| DR000119 | SR000119S | SR000519A |
| DR000120 | SR000120S | SR000520A |
| DR000121 | SR000121S | SR000521A |
| DR000122 | SR000122S | SR000522A |
| DR000123 | SR000123S | SR000523A |
| DR000124 | SR000124S | SR000524A |
| DR000125 | SR000125S | SR000525A |
| DR000126 | SR000126S | SR000526A |
| DR000127 | SR000127S | SR000527A |
| DR000128 | SR000128S | SR000528A |
| DR000129 | SR000129S | SR000529A |
| DR000130 | SR000130S | SR000530A |
| DR000131 | SR000131S | SR000531A |
| DR000132 | SR000132S | SR000532A |
| DR000133 | SR000133S | SR000533A |
| DR000134 | SR000134S | SR000534A |
| DR000135 | SR000135S | SR000535A |
| DR000136 | SR000136S | SR000536A |
| DR000137 | SR000137S | SR000537A |
| DR000138 | SR000138S | SR000538A |
| DR000139 | SR000139S | SR000539A |
| DR000140 | SR000140S | SR000540A |
| DR000141 | SR000141S | SR000541A |
| DR000142 | SR000142S | SR000542A |
| DR000143 | SR000143S | SR000543A |
| DR000144 | SR000144S | SR000544A |
| DR000145 | SR000145S | SR000545A |
| DR000146 | SR000146S | SR000546A |
| DR000147 | SR000147S | SR000547A |
| DR000148 | SR000148S | SR000548A |
| DR000149 | SR000149S | SR000549A |
| DR000150 | SR000150S | SR000550A |
| DR000151 | SR000151S | SR000551A |
| DR000152 | SR000152S | SR000552A |
| DR000153 | SR000153S | SR000553A |
| DR000154 | SR000154S | SR000554A |
| DR000155 | SR000155S | SR000555A |
| DR000156 | SR000156S | SR000556A |
| DR000157 | SR000157S | SR000557A |
| DR000158 | SR000158S | SR000558A |
| DR000159 | SR000159S | SR000559A |
| DR000160 | SR000160S | SR000560A |
| DR000161 | SR000161S | SR000561A |
| DR000162 | SR000162S | SR000562A |
| DR000163 | SR000163S | SR000563A |
| DR000164 | SR000164S | SR000564A |
| DR000165 | SR000165S | SR000565A |
| DR000166 | SR000166S | SR000566A |
| DR000167 | SR000167S | SR000567A |
| DR000168 | SR000168S | SR000568A |
| DR000169 | SR000169S | SR000569A |
| DR000170 | SR000170S | SR000570A |
| DR000171 | SR000171S | SR000571A |
| DR000172 | SR000172S | SR000572A |
| DR000173 | SR000173S | SR000573A |
| DR000174 | SR000174S | SR000574A |
| DR000175 | SR000175S | SR000575A |
| DR000176 | SR000176S | SR000576A |
| DR000177 | SR000177S | SR000577A |
| DR000178 | SR000178S | SR000578A |
| DR000179 | SR000179S | SR000579A |
| DR000180 | SR000180S | SR000580A |
| DR000181 | SR000181S | SR000581A |
| DR000182 | SR000182S | SR000582A |
| DR000183 | SR000183S | SR000583A |
| DR000184 | SR000184S | SR000584A |
| DR000185 | SR000185S | SR000585A |
| DR000186 | SR000186S | SR000586A |
| DR000187 | SR000187S | SR000587A |
| DR000188 | SR000188S | SR000588A |
| DR000189 | SR000189S | SR000589A |
| DR000190 | SR000190S | SR000590A |
| DR000191 | SR000191S | SR000591A |
| DR000192 | SR000192S | SR000592A |
| DR000193 | SR000193S | SR000593A |
| DR000194 | SR000194S | SR000594A |
| DR000195 | SR000195S | SR000595A |
| DR000196 | SR000196S | SR000596A |
| DR000197 | SR000197S | SR000597A |
| DR000198 | SR000198S | SR000598A |
| DR000199 | SR000199S | SR000599A |
| DR000200 | SR000200S | SR000600A |
| DR000201 | SR000201S | SR000601A |
| DR000202 | SR000202S | SR000602A |
| DR000203 | SR000203S | SR000603A |
| DR000204 | SR000204S | SR000604A |
| DR000205 | SR000205S | SR000605A |
| DR000206 | SR000206S | SR000606A |
| DR000207 | SR000207S | SR000607A |
| DR000208 | SR000208S | SR000608A |
| DR000209 | SR000209S | SR000609A |
| DR000210 | SR000210S | SR000610A |
| DR000211 | SR000211S | SR000611A |
| DR000212 | SR000212S | SR000612A |
| DR000213 | SR000213S | SR000613A |
| DR000214 | SR000214S | SR000614A |
| DR000215 | SR000215S | SR000615A |
| DR000216 | SR000216S | SR000616A |
| DR000217 | SR000217S | SR000617A |
| DR000218 | SR000218S | SR000618A |
| DR000219 | SR000219S | SR000619A |
| DR000220 | SR000220S | SR000620A |
| DR000221 | SR000221S | SR000621A |
| DR000222 | SR000222S | SR000622A |
| DR000223 | SR000223S | SR000623A |
| DR000224 | SR000224S | SR000624A |
| DR000225 | SR000225S | SR000625A |
| DR000226 | SR000226S | SR000626A |
| DR000227 | SR000227S | SR000627A |
| DR000228 | SR000228S | SR000628A |
| DR000229 | SR000229S | SR000629A |
| DR000230 | SR000230S | SR000630A |
| DR000231 | SR000231S | SR000631A |
| DR000232 | SR000232S | SR000632A |
| DR000233 | SR000233S | SR000633A |
| DR000234 | SR000234S | SR000634A |
| DR000235 | SR000235S | SR000635A |
| DR000236 | SR000236S | SR000636A |
| DR000237 | SR000237S | SR000637A |
| DR000238 | SR000238S | SR000638A |
| DR000239 | SR000239S | SR000639A |
| DR000240 | SR000240S | SR000640A |
| DR000241 | SR000241S | SR000641A |
| DR000242 | SR000242S | SR000642A |
| DR000243 | SR000243S | SR000643A |
| DR000244 | SR000244S | SR000644A |
| DR000245 | SR000245S | SR000645A |
| DR000246 | SR000246S | SR000646A |
| DR000247 | SR000247S | SR000647A |
| DR000248 | SR000248S | SR000648A |
| DR000249 | SR000249S | SR000649A |
| DR000250 | SR000250S | SR000650A |
| DR000251 | SR000251S | SR000651A |
| DR000252 | SR000252S | SR000652A |
| DR000253 | SR000253S | SR000653A |
| DR000254 | SR000254S | SR000654A |
| DR000255 | SR000255S | SR000655A |
| DR000256 | SR000256S | SR000656A |
| DR000257 | SR000257S | SR000657A |
| DR000258 | SR000258S | SR000658A |
| DR000259 | SR000259S | SR000659A |
| DR000260 | SR000260S | SR000660A |
| DR000261 | SR000261S | SR000661A |
| DR000262 | SR000262S | SR000662A |
| DR000263 | SR000263S | SR000663A |
| DR000264 | SR000264S | SR000664A |
| DR000265 | SR000265S | SR000665A |
| DR000266 | SR000266S | SR000666A |
| DR000267 | SR000267S | SR000667A |
| DR000268 | SR000268S | SR000668A |
| DR000269 | SR000269S | SR000669A |
| DR000270 | SR000270S | SR000670A |
| DR000271 | SR000271S | SR000671A |
| DR000272 | SR000272S | SR000672A |
| DR000273 | SR000273S | SR000673A |
| DR000274 | SR000274S | SR000674A |
| DR000275 | SR000275S | SR000675A |
| DR000276 | SR000276S | SR000676A |
| DR000277 | SR000277S | SR000677A |
| DR000278 | SR000278S | SR000678A |
| DR000279 | SR000279S | SR000679A |
| DR000280 | SR000280S | SR000680A |
| DR000281 | SR000281S | SR000681A |
| DR000282 | SR000282S | SR000682A |
| DR000283 | SR000283S | SR000683A |
| DR000284 | SR000284S | SR000684A |
| DR000285 | SR000285S | SR000685A |
| DR000286 | SR000286S | SR000686A |
| DR000287 | SR000287S | SR000687A |
| DR000288 | SR000288S | SR000688A |
| DR000289 | SR000289S | SR000689A |
| DR000290 | SR000290S | SR000690A |
| DR000291 | SR000291S | SR000691A |
| DR000292 | SR000292S | SR000692A |
| DR000293 | SR000293S | SR000693A |
| DR000294 | SR000294S | SR000694A |
| DR000295 | SR000295S | SR000695A |
| DR000296 | SR000296S | SR000696A |
| DR000297 | SR000297S | SR000697A |
| DR000298 | SR000298S | SR000698A |
| DR000299 | SR000299S | SR000699A |
| DR000300 | SR000300S | SR000700A |
| DR000301 | SR000301S | SR000701A |
| DR000302 | SR000302S | SR000702A |
| DR000303 | SR000303S | SR000703A |
| DR000304 | SR000304S | SR000704A |
| DR000305 | SR000305S | SR000705A |
| DR000306 | SR000306S | SR000706A |
| DR000307 | SR000307S | SR000707A |
| DR000308 | SR000308S | SR000708A |
| DR000309 | SR000309S | SR000709A |
| DR000310 | SR000310S | SR000710A |
| DR000311 | SR000311S | SR000711A |
| DR000312 | SR000312S | SR000712A |
| DR000313 | SR000313S | SR000713A |
| DR000314 | SR000314S | SR000714A |
| DR000315 | SR000315S | SR000715A |
| DR000316 | SR000316S | SR000716A |
| DR000317 | SR000317S | SR000717A |
| DR000318 | SR000318S | SR000718A |
| DR000319 | SR000319S | SR000719A |
| DR000320 | SR000320S | SR000720A |
| DR000321 | SR000321S | SR000721A |
| DR000322 | SR000322S | SR000722A |
| DR000323 | SR000323S | SR000723A |
| DR000324 | SR000324S | SR000724A |
| DR000325 | SR000325S | SR000725A |
| DR000326 | SR000326S | SR000726A |
| DR000327 | SR000327S | SR000727A |
| DR000328 | SR000328S | SR000728A |
| DR000329 | SR000329S | SR000729A |
| DR000330 | SR000330S | SR000730A |
| DR000331 | SR000331S | SR000731A |
| DR000332 | SR000332S | SR000732A |
| DR000333 | SR000333S | SR000733A |
| DR000334 | SR000334S | SR000734A |
| DR000335 | SR000335S | SR000735A |
| DR000336 | SR000336S | SR000736A |
| DR000337 | SR000337S | SR000737A |
| DR000338 | SR000338S | SR000738A |
| DR000339 | SR000339S | SR000739A |
| DR000340 | SR000340S | SR000740A |
| DR000341 | SR000341S | SR000741A |
| DR000342 | SR000342S | SR000742A |
| DR000343 | SR000343S | SR000743A |
| DR000344 | SR000344S | SR000744A |
| DR000345 | SR000345S | SR000745A |
| DR000346 | SR000346S | SR000746A |
| DR000347 | SR000347S | SR000747A |
| DR000348 | SR000348S | SR000748A |
| DR000349 | SR000349S | SR000749A |
| DR000350 | SR000350S | SR000750A |
| DR000351 | SR000351S | SR000751A |
| DR000352 | SR000352S | SR000752A |
| DR000353 | SR000353S | SR000753A |
| DR000354 | SR000354S | SR000754A |
| DR000355 | SR000355S | SR000755A |
| DR000356 | SR000356S | SR000756A |
| DR000357 | SR000357S | SR000757A |
| DR000358 | SR000358S | SR000758A |
| DR000359 | SR000359S | SR000759A |
| DR000360 | SR000360S | SR000760A |
| DR000361 | SR000361S | SR000761A |
| DR000362 | SR000362S | SR000762A |
| DR000363 | SR000363S | SR000763A |
| DR000364 | SR000364S | SR000764A |
| DR000365 | SR000365S | SR000765A |
| DR000366 | SR000366S | SR000766A |
| DR000367 | SR000367S | SR000767A |
| DR000368 | SR000368S | SR000768A |
| DR000369 | SR000369S | SR000769A |
| DR000370 | SR000370S | SR000770A |
| DR000371 | SR000371S | SR000771A |
| DR000372 | SR000372S | SR000772A |
| DR000373 | SR000373S | SR000773A |
| DR000374 | SR000374S | SR000774A |
| DR000375 | SR000375S | SR000775A |
| DR000376 | SR000376S | SR000776A |
| DR000377 | SR000377S | SR000777A |
| DR000378 | SR000378S | SR000778A |
| DR000379 | SR000379S | SR000779A |
| DR000380 | SR000380S | SR000780A |
| DR000381 | SR000381S | SR000781A |
| DR000382 | SR000382S | SR000782A |
| DR000383 | SR000383S | SR000783A |
| DR000384 | SR000384S | SR000784A |
| DR000385 | SR000385S | SR000785A |
| DR000386 | SR000386S | SR000786A |
| DR000387 | SR000387S | SR000787A |
| DR000388 | SR000388S | SR000788A |
| DR000389 | SR000389S | SR000789A |
| DR000390 | SR000390S | SR000790A |
| DR000391 | SR000391S | SR000791A |
| DR000392 | SR000392S | SR000792A |
| DR000393 | SR000393S | SR000793A |
| DR000394 | SR000394S | SR000794A |
| DR000395 | SR000395S | SR000795A |
| DR000396 | SR000396S | SR000796A |
| DR000397 | SR000397S | SR000797A |
| DR000398 | SR000398S | SR000798A |
| DR000399 | SR000399S | SR000799A |
| DR000400 | SR000400S | SR000800A |
| DR001478 | SR002955S | SR002963A |
| DR002252 | SR004479S | SR004480A |
| DR002222 | SR004443S | SR004448A |
| DR002223 | SR004444S | SR004449A |
| DR002224 | SR004445S | SR004450A |
| DR002225 | SR004446S | SR004451A |
| DR002226 | SR004447S | SR004452A |
| DR001482 | SR002959S | SR002967A |
| DR005722 | SR011326S | SR011332A |
| DR007153 | SR011326S | SR013861A |
| DR007849 | SR015173S | SR013861A |
| DR007816 | SR015114S | SR013861A |
| DR007093 | SR013765S | SR013801A |
| DR007850 | SR015172S | SR013801A |
| DR007848 | SR015172S | SR015110A |
| DR007815 | SR015113S | SR013801A |
| DR007846 | SR015113S | SR015110A |
| DR007919 | SR015114S | SR0152050A |
| DR005720 | SR011326S | SR011331A |

### EXAMPLES

The sources of materials used in the examples are as follows:
Huh7 cell line, Nanjing Cobioer Biotechnology Co., Ltd., Cat# CBP60202;
Hep3B cell line, Nanjing Cobioer Biotechnology Co., Ltd., Cat# CBP60197;
PHHs, Shanghai Xuanyi Biotechnology Co., Ltd., Cat# QYLF-HPMC;
HEK293A cell line, Nanjing Cobioer Biotechnology Co., Ltd., Cat# CBP60436;
Balb/c mice, Zhejiang Vital River Laboratory Animal Technology Co., Ltd., Cat# Balb/c.

### Example 1 Preparation of Compound E7

### 1. Preparation of Intermediate 3-4

### 1.1 Preparation of Compound 2

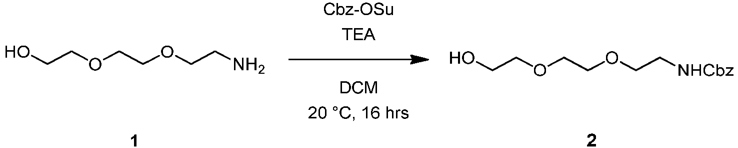

To compound 1 (300 g, 2.01 mol) in DCM (1.80 L), benzyl(2,5-dioxopyrrolidin-1-yl) carbonate (600 g, 2.40 mol) was added slowly, and TEA (203 g, 2.01 mol, 280 mL) was added dropwise at 15°C. The mixture was stirred at 25°C for 16 h until TLC (dichloromethane: methanol = 10:1) showed that reactant 1 (R_{f} = 0.32) was retained and a significant new spot (R_{f} = 0.52) was detected. The reaction mixture was washed with saturated sodium bicarbonate solution (1.00 L×2). The organic phase was washed with brine (1.00 L), dried over anhydrous Na₂SO₄, and concentrated under reduced pressure. Compound 2 (about 385 g) was obtained as a yellow oil without further purification.

### 1.2 Preparation of Compound 2A

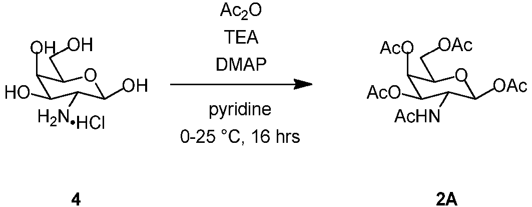

To a solution of compound 4 (350 g, 1.62 mol, HCl) and Ac₂O (994 g, 9.74 mol, 912 mL) in pyridine (1.75 L), DMAP (19.8 g, 162 mmol) was added in one portion and TEA (164 g, 1.62 mol, 226 mL) was added dropwise at 0-15°C. The mixture was stirred at 25°C for 16 h until LCMS (product: RT = 0.687 min) showed complete consumption of the starting reactant. EtOAc (1.40 L) was added to the mixture at 25°C and stirred for 30 min. The resulting mixture was then filtered and the filter cake was washed with EtOAc (300 mL). The filter cake was ground with water (1.45 L) at 25°C for 30 min. The mixture was filtered and the filter cake was washed with water (175 mL × 3). The filter cake was collected to obtain compound 2A (about 580 g) as a white solid.

### 1.3 Preparation of Compound 2B

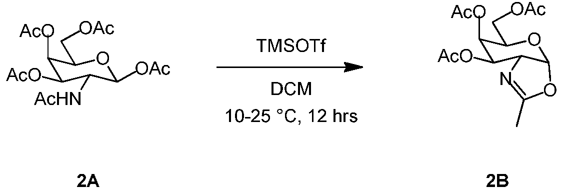

Three reactions were performed in parallel.

To a solution of compound 2A (200 g, 514 mmol) in DCM (800 mL), TMSOTf (137 g, 616 mmol, 111 mL) was added dropwise over 0.5 h at 10-15°C. The mixture was stirred at 25°C for 3 h until TLC (dichloromethane:methanol = 20:1) showed complete consumption of compound 2A (R_{f} = 0.54) and formation of a new spot (R_{f} = 0.24). The three reactions were combined. The mixture was cooled to 0-15°C and slowly poured into NaHCO₃ (300 g dissolved in 3.00 L of water) at 0-5°C. The organic phase was separated and the aqueous phase was extracted with DCM (1.00 L × 3). The organic phases were combined, dried over Na₂SO₄, filtered, and concentrated under reduced pressure. Compound 2B (about 507 g) was obtained as a yellow oil for use in the next step without further purification.

### 1.4 Preparation of Compound 3

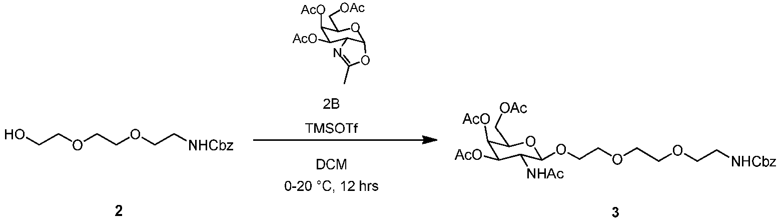

To a mixture of compound 2B (250 g, 759 mmol) and compound 2 (151 g, 531 mmol) in DCM (1.00 L), TMSOTf (84.4 g, 380 mmol, 69.0 mL) was added dropwise at 0-10°C. The mixture was stirred at 20°C for 12 h until TLC (dichloromethane:methanol = 20:1) showed complete consumption of compound 2 (R_{f} = 0.33) and formation of a new spot (R_{f} = 0.03). The combined reaction mixture was cooled to 0-5°C and then poured into NaHCO₃ (100 g in 1 L of water) and stirred at 5-10°C for 10 min to separate the phases. The aqueous phase was extracted with DCM (500 mL × 2). The combined organic phases were dried over Na₂SO₄, filtered, and concentrated under reduced pressure. Compound 3 (about 360 g) was obtained as a yellow oil without further purification.

¹H NMR : (400 MHz, DMSO).

δ=7.79-7.37 (m, 1H), 7.35-7.26 (m, 5H), 5.21-5.20 (m, 1H), 5.00-4.95 (m, 3H), 4.55-4.53 (m, 1H), 4.03-3.86 (m, 3H), 3.61-3.59 (m, 1H), 3.59-3.57 (m, 1H), 3.48-3.40 (m, 6H), 3.39-3.31 (m, 2H), 3.14-3.13 (m, 2H), 2.09 (s, 3H), 1.99 (s, 3H), 1.88 (s, 3H), 1.76-1.74 (m, 3H).

### 1.5 Preparation of Intermediate 3-4 (TFA salt)

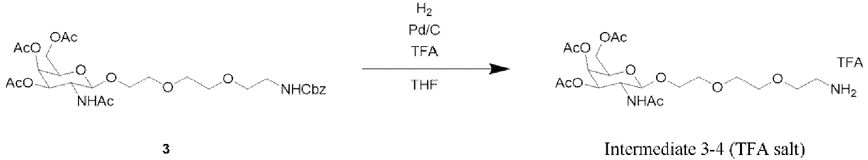

Three reactions were performed in parallel.

To a mixture of Pd/C (18.0 g, 16.3 mmol, 10% content) in THF (1.80 L), compound 3 (180 g, 293 mmol) and TFA (33.5 g, 293 mmol, 21.8 mL) were added under an argon atmosphere. The suspension was degassed and purged three times with hydrogen. The mixture was stirred at 30°C for 2 h under an H₂ (50 Psi) atmosphere until LCMS (product: RT = 0.697 min) showed that compound 3 was consumed and a product peak was detected. The three reactions were combined. The mixture was filtered through celite and the filtrate was concentrated under reduced pressure to remove the solvent. Intermediate 3-4 (TFA salt) (393 g, 660 mmol, yield 74.8%, purity 99.6%, TFA) was obtained as a yellow solid without further purification.

¹H NMR : (400 MHz, DMSO-d6)

δ = 7.92 (d, *J =* 9.1 Hz, 4H), 5.27-5.17 (m, 1H), 5.03-4.91 (m, 1H), 4.60-4.50 (m, 1H), 4.09-3.97 (m, 4H), 3.85 (s, 2H), 3.65-3.46 (m, 10H), 3.04-2.92 (m, 2H), 2.10 (s, 3H), 2.00 (s, 3H), 1.94-1.86 (m, 3H), 1.82-1.71 (m, 4H).

### 2. Preparation of Intermediate 3-5

### 2.1 Preparation of Compound 5

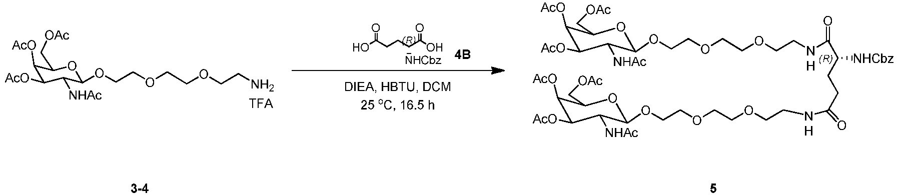

To a solution of compound 4B (10.0 g, 35.5 mmol, 1.00 eq) and compound 3-4 (46.3 g, 78.2 mmol, 2.20 eq, TFA) prepared above in DCM (1.00 L), DIEA (30.3 g, 234 mmol, 40.8 mL, 6.60 eq) was added in one portion at 25°C. The mixture was stirred at 25°C for half an hour. HBTU (30.3 g, 234 mmol, 40.8 mL, 6.60 eq) was added to the mixture The mixture was stirred at 25°C for 16 h until LCMS (product: RT = 0.681 min) showed that the reaction was complete. The mixture was concentrated under reduced pressure. To it, 0.50 N HCl (200 mL × 2) was added at 20°C, and the mixture was then extracted with DCM (3 × 500 mL). The organic phases were combined, washed with saturated NaHCO₃ (3 × 800 mL) until the pH reached 8, then washed with brine (3 × 500 mL), dried over Na₂SO₄, and concentrated under reduced pressure. The residue was purified by column chromatography (SiO₂, DCM:MeOH = 50:1-15:1) The residue was concentrated under reduced pressure at 40°C and purified by preparative MPLC (column: 800 g Agela C18; mobile phase: [water-ACN]; 15-45%, 25 min; 45%, 10 min). Compound 5 (about 180 g + 75.0 g + 87.0 g + 40.0 g + 38.0 g) was obtained as a yellow solid after vacuum drying.

After 9 runs, 417.0 g of compound 3-4 was converted to compound 5.

### 2.2 Preparation of Intermediate 3-3

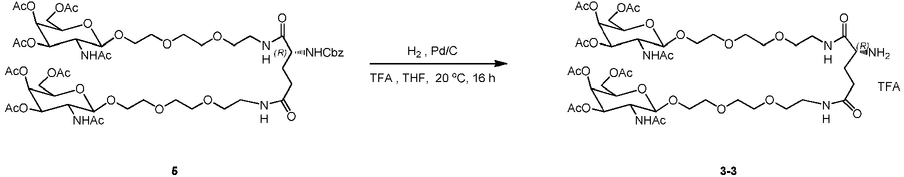

To Pd/C (3.00 g, 10% content) in THF (300 mL), compound 5 (73.0 g, 61.7 mmol, 1.00 eq) and TFA (7.04 g, 61.7 mmol, 4.57 mL, 1.00 eq) were added under an argon atmosphere. The suspension was degassed and purged three times with hydrogen. The mixture was stirred at 20°C for 16 h under an H₂ (20 Psi) atmosphere until TLC (dichloromethane:methanol = 8:1, R_{f} = 0.0) showed that the reaction was complete. The mixture was filtered through celite and the filtrate was concentrated under pressure to remove the solvent to obtain compound 3-3 (about 33.4 g + 129 g + 75.0 g) as a white solid.
¹H NMR: (400 MHz, DMSO)
*δ*=8.53 (t, *J =* 5.2 Hz, 1H), 8.18 (d, *J =* 2.4 Hz, 3H), 8.03 (t, *J* = 5.2 Hz, 1H), 7.84 (dd, *J* = 3.6 Hz, 2H), 5.22 (d, *J* = 3.2 Hz, 2H), 4.96 (dd, *J* = 3.2 Hz, 2H), 4.55 (d, J=8.4 Hz, 2H), 4.02 (t, J=8.8 Hz, 6H), 3.77-3.59 (m, 5H), 3.58-3.45 (m, 21H), 3.40-3.20 (m, 4H), 2.18 (t, *J =* 7.6 Hz, 2H), 2.17 (d, *J* =8.0 Hz, 6H), 2.10 (s, 6H), 1.99 (s, 6H), 1.90-1.80 (m, 8H), 1.77 (s, 6H).

### 3. Preparation of Compound E7

### 3.1 Preparation of Compound 3

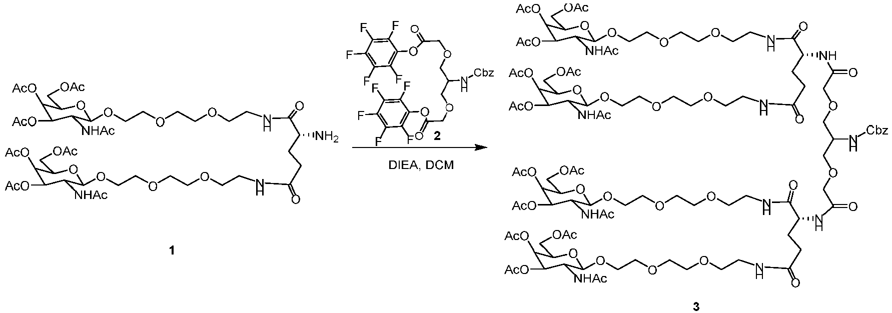

Compound 1 (2.00 g, 1.87 mmol, prepared as described above for intermediate 3-3) was dissolved in DCM (20.0 mL) at room temperature. DIEA (0.135 mL, 0.814 mmol) and compound 2 (0.550 g, 0.814 mmol) were added in sequence, and the mixture was replaced with nitrogen three times. The reaction mixture was stirred at 25°C for 16 h until LC-MS/MS detected the MS response of the product and TLC (dichloromethane/methanol = 5/1) showed that the starting material disappeared and new spots were formed. The reaction mixture was concentrated under reduced pressure. The obtained crude product was purified by column chromatography (dichloromethane/methanol = 5/1) to obtain compound 3 (about 780 mg) as a white solid.
¹H NMR (400 MHz, CD₃OD)
*δ*=7.28-7.42 (m, 5H), 5.30-5.34 (m, 4H), 5.04-5.14 (m, 6H), 4.63-4.67 (m, 4H), 4.36-4.44 (m, 2H), 4.00-4.20 (m, 23H), 3.91-3.95 (m, 4H), 3.69-3.77 (m, 9H), 3.52-3.67 (m, 32H), 3.34-3.43 (m, 9H), 2.29-2.31 (m, 4H), 2.14 (s, 12H), 2.03 (s, 12H), 1.92-1.96 (m, 24H). LCMS: m/z = 1221.6 (M/2+H)⁺.

### 3.2 Preparation of Compound 4

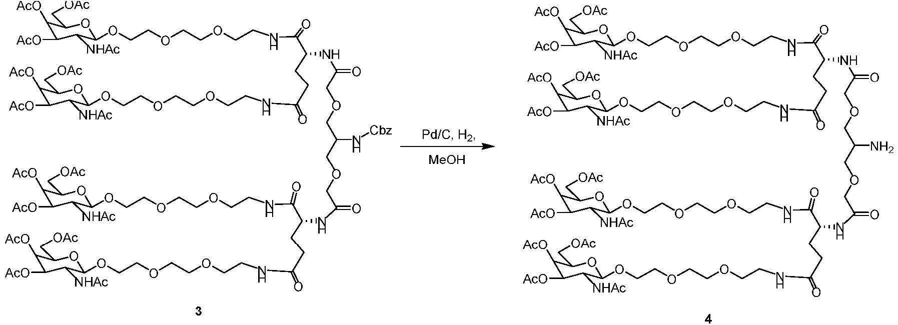

Compound 3 (1.10 g, 0.451 mmol) was dissolved in MeOH (10.0 mL) at room temperature, and wet Pd/C (0.050 g, 0.451 mmol) with a mass fraction of 10% was added to the solution. The reaction mixture was replaced with hydrogen three times and then stirred at 25°C for 18 h under a hydrogen atmosphere (14.696 psi) until LC-MS/MS detected the MS response of the product and TLC (dichloromethane/methanol = 10/1, color development: phosphomolybdic acid) showed that the starting material was completely consumed and new spots were formed. The reaction mixture was filtered and the filtrate was concentrated under reduced pressure to obtain compound 4 (about 840 mg) as a white solid.
¹H NMR (400 MHz, CD₃OD)
*δ*=5.32-5.34 (m 4H), 5.06-5.10 (m, 4H), 4.63-4.65 (m, 4H), 4.38-4.40 (m, 2H), 3.99-4.20 (m, 20H), 3.90-3.97 (m, 4H), 3.69-3.76 (m, 6H), 3.50-3.68 (m, 36H), 3.35-3.44 (m, 11H), 2.28-2.38 (m, 4H), 2.15 (s, 12H), 2.03 (s, 12H), 1.90-1.94 (m, 24H). LCMS: m/z = 1154.7 (M/2+H)⁺.

### 3.3 Preparation of Compound 6

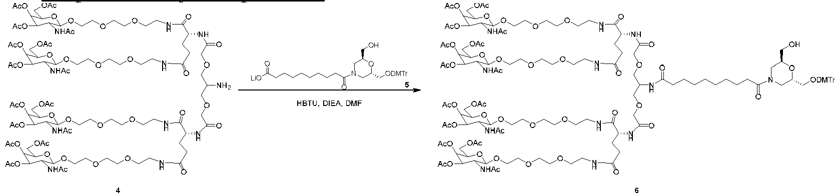

Compound 5 (232 mg, 0.364 mmol) was dissolved in DCM (10.0 mL) at room temperature, and HBTU (207 mg, 0.546 mmol), DIEA (0.181 mL, 1.09 mmol), and compound 4 (840 mg, 0.364 mmol) were added in sequence to the solution. The reaction mixture was replaced with nitrogen three times The reaction mixture was stirred at 25°C for 1 h until LC-MS/MS detected that the starting material disappeared and TLC (dichloromethane/methanol = 5/1) showed that the starting material disappeared and new spots were formed. The reaction mixture was concentrated under reduced pressure. The obtained crude product was purified by column chromatography (dichloromethane/methanol = 8/1-5/1) to obtain compound 6 (about 620 mg) as a white solid.
¹H NMR (400 MHz, CD₃OD)
*δ*=7.41-7.43 (m, 2H), 7.23-7.34 (m, 7H), 6.83-6.90 (m, 4H), 5.31-5.35 (m, 4H), 5.01-5.12 (m, 4H), 4.63-4.65 (m, 4H), 4.41-4.45 (m, 2H), 4.31-4.33 (m, 1H), 3.99-4.22 (m, 22H), 3.87-3.97 (m, 6H), 3.58-3.81 (m, 45H), 3.34-3.43 (m, 10H), 2.19-2.40 (m, 10H), 2.14 (s, 12H), 2.02 (s, 12H), 1.92-1.96 (mz, 24H), 1.48-1.63 (m, 4H), 1.28-1.38 (m, 8H). LCMS: m/z = 1460.0 (M/2+H)⁺.

### 4. Preparation of Compound E7

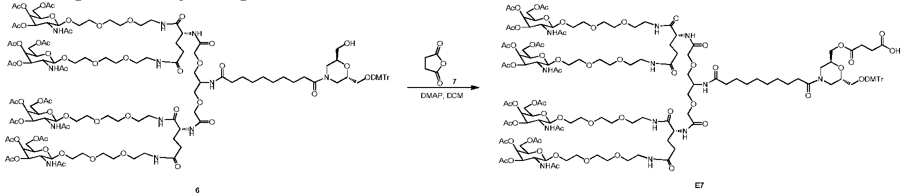

Compound 6 (300 mg, 0.103 mmol) was dissolved in DCM (10.0 mL) at room temperature, and DIEA (0.102 mL, 0.618 mmol), compound 7 (10.3 mg, 0.103 mmol), and DMAP (12.6 mg, 0.103 mmol) were added in sequence to the solution. The reaction mixture was replaced with nitrogen three times. The reaction mixture was stirred at 25°C for 2 h until LC-MS/MS detected that the starting material disappeared. The reaction mixture was concentrated under reduced pressure. The obtained crude product was separated by preparative MPLC (column: Waters Xbridge BEH C18 100*30 mm*10 µm; mobile phase: water-ACN; B%: 17%-57%, 5 min) to obtain compound E7 (53.0 mg, yield 17.08%, purity 78.94%) as a white solid.
¹H NMR (400 MHz, CD₃OD)
*δ*=7.41-7.45 (m, 2H), 7.17-7.34 (m, 7H), 6.85-6.89 (m, 4H), 5.32-5.36 (m, 4H), 5.03-5.13 (m, 4H), 4.63-4.67 (m, 4H), 4.38-4.47 (m, 2H), 4.32-4.34 (m, 1H), 4.01-4.26 (m, 22H), 3.88-4.00 (m, 6H), 3.77-3.81 (m, 7H), 3.49-3.76 (m, 45H), 3.33-3.47 (m, 10H), 2.56-2.62 (m, 2H), 2.45-2.55 (m, 3H), 2.21-2.38 (m, 7H), 2.14 (s, 12H), 2.05-2.11 (m, 2H), 2.02 (s, 12H), 1.92-1.96 (m, 24H), 1.47-1.68 (m, 4H), 1.28-1.34 (m, 8H)
MS: m/z = 3022.36 (M+H)⁺.

### Example 2 Preparation of siRNA

The siRNA of the present invention was prepared using the solid-phase phosphoramidite method well known in the art. The specific methods can be referred to, for example, PCT Publication Nos. WO2016081444 and WO2019105419, and are briefly described below.

### 1. Preparation of siRNA With No Ligand Conjugated

### 1.1 Synthesis of Sense Strand (SS)

Using the solid-phase phosphoramidite method, a blank CPG solid support was used as the starting cycle, and nucleoside monomers were linked one by one according to the arrangement of sense strand nucleotides from the 3' to 5' direction. Each linkage of a nucleoside monomer involved four steps of deprotection, coupling, capping, and oxidation or thiolation to synthesize 5 µmol oligonucleotide with the synthesis conditions as follows:
Nucleoside monomers were provided in a 0.05 mol/L acetonitrile solution. The conditions for each step were identical: 25°C; deprotection for 3 times using a 3% trichloroacetic acid-dichloromethane solution; coupling twice using a 0.25 mol/L ETT-acetonitrile solution as an activator; capping twice using 10% acetic anhydrideacetonitrile and pyridine/*N*-methylimidazole/acetonitrile (10:14:76, v/v/v); oxidation twice using 0.05 mol/L of iodine in tetrahydrofuran/pyridine/water (70:20:10, v/v/v); thiolation twice using 0.2 mol/L PADS in acetonitrile/3-methylpyridine (1:1, v/v).

### 1.2 Synthesis of Antisense Strand (AS)

Using the solid-phase phosphoramidite method, a blank CPG solid support was used as the starting cycle, and nucleoside monomers were linked one by one according to the arrangement of antisense strand nucleotides from the 3' to 5' direction. Each linkage of a nucleoside monomer involved four steps of deprotection, coupling, capping, and oxidation or thiolation. The conditions for the synthesis of a 5 *µ*mol oligonucleotide for the antisense strand were identical to those for the sense strand.

### 1.3 Purification and Annealing of Oligonucleotides

### 1.3.1 Ammonolysis

The synthesized solid support (sense or antisense strand) was transferred to a 5 mL centrifuge tube, followed by the addition of 3% diethylamine/ammonia (v/v). The mixture was allowed to react in a thermostatic water bath at 35°C (or 55°C) for 16 h (or 8 h), and then filtered. The solid support was washed three times with ethanol/water, 1 mL each time. The filtrate was concentrated by centrifugation, and the crude product was purified.

### 1.3.2 Purification

The methods for purification and desalting are well known to those skilled in the art. For example, a strong anionic packing column can be used; a sodium chloridesodium hydroxide system can be used for elution and purification. The product can be collected in tubes and desalted using a gel packing purification column. The elution system can be pure water.

### 1.3.3 Annealing

The sense strand (SS) was mixed with the antisense strand (AS) at a molar ratio (SS/AS = 1/1.05). The mixture was heated in a water bath to 70-95°C for 3-5 min, and then allowed to cool naturally to room temperature. The system was freeze-dried to obtain the product. Finally, double-stranded DR000001 to DR000400 were obtained.

### 2. Preparation of siRNA With Sense Strand Conjugated to Ligand

### 2.1 Conjugation of Compound E7 to CPG Carrier

Compound E7 (53 mg, 0.018 mmol) and HBTU (13.3 mg, 0.035 mmol) were mixed and dissolved in acetonitrile (5 mL) by shaking. DIEA (9.0 mg, 0.07 mmol) and DMAP (2.1 mg, 0.018 mmol) were then added and dissolved by shaking until clear. Blank carrier Resin (550 mg, CPG pore size 1000Å) was weighed and added to the reaction mixture, which was then placed on a shaker at 20°C overnight. A sample was taken and monitored until TLC (developing solvent: DCM/methanol = 4/1; color development: phosphomolybdic acid) showed that the reaction was complete. The reaction mixture was filtered through a sand core funnel. The filter cake was washed with anhydrous acetonitrile (20 mL*5), collected, and filtered under reduced pressure using an oil pump for 6 h to obtain 530 mg of off-white solid.

The resulting product (530 mg) was transferred to a 50 mL round-bottom flask. CapC (DMAP/acetonitrile), CapB (N-methylimidazole/pyridine/acetonitrile), and CapA (acetic anhydride/acetonitrile) were added in sequence. The mixture was then placed on a shaker at room temperature overnight, and filtered. The filter cake was washed with acetonitrile (20 mL*4), collected, and filtered under reduced pressure using an oil pump for 8 h to obtain 200 mg of off-white solid (GL6 solid support) for solid-phase synthesis.

### 2.2 Synthesis of Sense Strand (SS) Conjugated to a Ligand

Using the solid-phase phosphoramidite method, the GL6 solid support prepared above was used as the starting cycle, and nucleoside monomers were linked one by one according to the arrangement of sense strand nucleotides from the 3' to 5' direction. Each linkage of a nucleoside monomer involved four steps of deprotection, coupling, capping, and oxidation or thiolation to synthesize 5 µmol oligonucleotide with the synthesis conditions as follows:
Nucleoside monomers were provided in a 0.05 mol/L acetonitrile solution. The conditions for each step were identical: 25°C; deprotection for 3 times using a 3% trichloroacetic acid-dichloromethane solution; coupling twice using a 0.25 mol/L ETT-acetonitrile solution as an activator; capping twice using 10% acetic anhydrideacetonitrile and pyridine/N-methylimidazole/acetonitrile (10:14:76, v/v/v); oxidation twice using 0.05 mol/L of iodine in tetrahydrofuran/pyridine/water (70:20:10, v/v/v); thiolation twice using 0.2 mol/L PADS in acetonitrile/3-methylpyridine (1:1, v/v).

### 2.3 Synthesis of Antisense Strand (AS)

Using the solid-phase phosphoramidite method, a blank CPG solid support was used as the starting cycle, and nucleoside monomers were linked one by one according to the arrangement of antisense strand nucleotides from the 3' to 5' direction. Each linkage of a nucleoside monomer involved four steps of deprotection, coupling, capping, and oxidation or thiolation. The conditions for the synthesis of a 5 µmol oligonucleotide for the antisense strand were identical to those for the sense strand.

### 2.4 Purification and Annealing of Oligonucleotides

### 2.4.1 Ammonolysis

The synthesized solid support (sense or antisense strand) was transferred to a 5 mL centrifuge tube, followed by the addition of 3% diethylamine/ammonia (v/v). The mixture was allowed to react in a thermostatic water bath at 35°C (or 55°C) for 16 h (or 8 h), and then filtered. The solid support was washed three times with ethanol/water, 1 mL each time. The filtrate was concentrated by centrifugation, and the crude product was purified.

### 2.4.2 Purification

The methods for purification and desalting are well known to those skilled in the art. For example, a strong anionic packing column can be used; a sodium chloridesodium hydroxide system can be used for elution and purification. The product can be collected in tubes and desalted using a gel packing purification column. The elution system can be pure water.

### 2.4.3 Annealing

The sense strand (SS) was mixed with the antisense strand (AS) at a molar ratio (SS/AS = 111.05). The mixture was heated in a water bath to 70-95°C for 3-5 min, and then allowed to cool naturally to room temperature. The system was freeze-dried to obtain the product.

A siRNA conjugated to NAG37 or L96 was similarly obtained.

### Example 3 Activity Screening in Huh7 Cell Line

### Cell Transfection

On the first day, the Huh7 cell line (Nanjing Cobioer Biotechnology Co., Ltd., Cat#: CBP60202) was digested, resuspended, counted, and seeded into a 96-well plate at 100 µL/well (1×10⁴ cells/well) for transfection 18 hours later.

On the second day, 2 µL of 20 µM siRNA (DR000001 to DR000400) stock solution prepared in Example 2 was diluted with 198 µL of Opti-MEM and mixed well by pipetting up and down for later use. The dilution was performed according to the different experimental requirements in each experiment.

On the second day, 0.9 µL of RNAiMAX (Thermo, 13778150) was diluted with 14.1 µL of Opti-MEM, mixed well by gently pipetting up and down, and allowed to stand at room temperature for 5 min. Then, 15 µL of the prepared RNAi-MAX mixture and 15 µL of the diluted siRNA compound were mixed well by gently pipetting up and down without generating bubbles, allowed to stand at room temperature for 10 min, and transferred to a 96-well plate at 10 µL/well. RNA was extracted after incubation in a 5% CO₂ incubator at 37°C for 24 h.

### RNA Extraction

Cellular RNA was extracted using a nucleic acid extractor (Hangzhou Allsheng, Auto-pure96) according to the protocol of the high-throughput cellular RNA extraction kit (FireGen, FG0412).

### RNA Reverse Transcription

The denatured reaction mixture was prepared with reference to PrimeScript^{™} II 1st Strand cDNA Synthesis Kit (Takara, 6210B). Each well contained 1 µL of Oligo dT Primer, 1 µL of dNTP Mixture, and 12.5 µL of template RNA. The denaturation reaction was performed by incubation in a conventional PCR instrument at 65°C for 5 min. The mixture was quickly cooled on ice for 2 min.

The reverse transcription reaction mixture was prepared with reference to PrimeScript^{™} II 1st Strand cDNA Synthesis Kit (Takara, 6210B). Each well contained 4 µL of 5×Prime Script II Buffer, 0.5 µL of RNase Inhibitor, and 1 µL of PrimeScript II RTase.

The denatured reaction mixture (14.5 µL) was mixed with the reverse transcription reaction mixture, incubated in a conventional PCR instrument at 42°C for 45 min for reverse transcription, and incubated at 95°C for 5 min to inactivate the enzyme. The reverse transcription product (cDNA) was then cooled at 4°C.

After reverse transcription, 30 µL of DNase RNase-Free Distilled Water was added to each well of the cDNA sample.

### Fluorescence Quantitative PCR

Fluorescence quantitative PCR reaction was performed in a 20 µL system (ABI, QuantStudio3) with reference to the operating procedure of TaqMan^{™} Fast Advanced Master Mix (ABI, 4444965). The reaction program was: (50°C, 2 min) × 1 Cycle; (95°C, 20 s) × 1 Cycle; (95°C, 1 s; 60°C, 24 s) × 40 Cycles.

**Table 7 Primer Information**

| Primer Name | Sequence Information (5'-3') | Fluorophore |
|---|---|---|
| hACTB-PF | ACGTGGACATCCGCAAAGAC (SEQ ID NO: 1664) | / |
| hACTB-PR | TCTTCATTGTGCTGGGTGCC (SEQ ID NO: 1665) | / |
| hACTB-P | AACACAGTGCTGTCTGGCGGCACCA(S EQ ID NO: 1666) | 5'TET,3'BHQ2 |
| hAPOC3-PF | TGCCTCCCTTCTCAGCTTCA (SEQ ID NO: 1667) | / |
| hAPOC3-PR | GGGAACTGAAGCCATCGGTC (SEQ ID NO: 1668) | / |
| hAPOC3-P | ATGAAGCACGCCACCAAGACCGCCA (SEQ ID NO: 1669) | 5'6-FAM,3'BHQ1 |

### Data Statistics

The 2-^{△△Ct} value was calculated and converted into a percentage to obtain the residual inhibition. △△Ct = [(Cttarget gene of experimental group - Ctinternal reference of experimental group) - (Cttarget gene of control group - Ctinternal reference of control group)].

The final concentration of siRNA was 10 nM for high-throughput screening of the activity of siRNA compounds in the cell line. The experimental screening results are shown in Table 8.

**Table 8 Results of High-throughput Screening for Activity of 10 nM siRNA in Huh7 Cell Line**

| siRNA Compound No. | Residual Inhibition | STD |
|---|---|---|
| Mock | 100.1% | 6.8% |
| DR000060 | 8.0% | 1.4% |
| DR000199 | 3.4% | 0.8% |
| DR000277 | 3.6% | 1.1% |
| DR000344 | 8.4% | 0.2% |
| DR000348 | 10.9% | 1.4% |

### Example 4 Activity Screening of siRNA at Five 10-Fold Gradient Diluted Concentrations in Hep3B Cell Line

Similar to activity screening in the Huh7 cell line above, the activity screening was performed using the Hep3B cell line (Nanjing Cobioer Biotechnology Co., Ltd., Cat#: CBP60197).

The starting concentration of siRNA (DR000001 to DR000400) was 10 nM, and 5 concentration points (10 nM, 1 nM, 0.1 nM, 0.01 nM, 0.001 nM) were obtained by 10-fold gradient dilutions for screening the activity of siRNA in the Hep3B cell line. The screening results are shown in Table 9.

**Table 9 Activity Screening Results of siRNA in Hep3B Cell Line at five Concentration Points**

| siRNA No. | 10nM | 1nM | 0.1nM | 0.01nM | 0.001nM | IC₅₀ (nM) at Five Concentration Points |
|---|---|---|---|---|---|---|
| DR000060 | 2.7% | 3.0% | 13.2% | 56.2% | 64.9% | 0.0308 |
| DR000199 | 2.6% | 6.0% | 24.6% | 95.8% | 98.0% | 0.0417 |
| DR000277 | 2.5% | 5.9% | 40.0% | 86.6% | 103.0% | 0.0581 |
| DR000344 | 1.1% | 1.9% | 16.4% | 74.9% | 92.3% | 0.0275 |
| DR000348 | 1.5% | 1.5% | 14.8% | 64.1% | 91.5% | 0.0191 |

### Example 5 Activity Screening of siRNA at Eleven 3-Fold Gradient Diluted Concentrations in Hep3B Cell Line

The procedure of this example was similar to that of Example 4. The starting concentration of siRNA (DR000001 to DR000400) was 10 nM, and 11 concentration points (10 nM, 3.33 nM, 1.11 nM, 0.37 nM, 0.123 nM, 0.041 nM, 0.0136 nM, 0.0045 nM, 0.00152 nM, 0.000508 nM, 0.000169 nM) were obtained by 3-fold gradient dilutions for screening the activity of siRNA in the Hep3B cell line. The screening results are shown in Table 10.

**Table 10 Screening Results of siRNA in Hep3B Cell Line at 11 Concentration Points**

| siRNA No. | DR000060 | DR000199 | DR000277 | DR000344 | DR000348 |
|---|---|---|---|---|---|
| 10nM | 2.0% | 1.0% | 1.4% | 0.9% | 1.7% |
| 3.33nM | 2.3% | 1.4% | 1.9% | 1.3% | 1.2% |
| 1.11nM | 4.2% | 2.1% | 3.4% | 3.3% | 1.7% |
| 0.37nM | 8.6% | 5.6% | 10.1% | 10.1% | 4.3% |
| 0.123nM | 27.0% | 22.1% | 33.4% | 37.0% | 12.4% |
| 0.041nM | 55.3% | 40.6% | 68.0% | 65.3% | 40.2% |
| 0.0136nM | 99.0% | 70.0% | 97.0% | 83.5% | 68.9% |
| 0.0045nM | 103.9% | 89.0% | 89.5% | 86.5% | 73.3% |
| 0.00152nM | 105.8% | 86.8% | 109.5% | 81.9% | 99.5% |
| 0.000508nM | 112.4% | 97.9% | 106.8% | 77.4% | 101.9% |
| 0.000169nM | 93.9% | 99.6% | 114.8% | 95.3% | 83.2% |
| IC₅₀ (nM) | 0.0523 | 0.0321 | 0.0621 | 0.1013 | 0.0236 |

### Example 6 Activity Screening of siRNA at Eleven 3-Fold Gradient Diluted Concentrations in Huh7 Cell Line

The procedure of this example was similar to that of Example 3. The Huh7 cell line was selected. The starting concentration of siRNA (DR000001 to DR000400) was 10 nM, and 11 concentration points (10 nM, 3.33 nM, 1.11 nM, 0.37 nM, 0.123 nM, 0.041 nM, 0.0136 nM, 0.0045 nM, 0.00152 nM, 0.000508 nM, 0.000169 nM) were obtained by 3-fold gradient dilutions for screening the activity of siRNA in the Huh7 cell line. The screening results are shown in the table below.

**Table 11 Screening Results of siRNA in Huh7 Cell Line at 11 Concentration Points**

| siRNA Compound No. | DR000060 | DR000199 | DR000277 | DR000344 | DR000348 |
|---|---|---|---|---|---|
| 10nM | 9.0% | 5.3% | 4.4% | 4.4% | 5.6% |
| 3.33nM | 9.0% | 14.6% | 3.9% | 2.2% | 10.0% |
| 1.11nM | 38.2% | 30.0% | 6.7% | 13.6% | 3.0% |
| 0.37nM | 35.7% | 32.0% | 18.2% | 19.5% | 3.7% |
| 0.123nM | 93.9% | 54.8% | 39.3% | 38.9% | 14.9% |
| 0.041nM | 141.7% | 94.6% | 127.5% | 58.0% | 37.2% |
| 0.0136nM | 161.8% | 110.4% | 122.1% | 84.0% | 52.8% |
| 0.0045nM | 153.7% | 114.2% | 116.4% | 95.3% | 77.5% |
| 0.00152nM | 147.6% | 117.8% | 103.6% | 96.4% | 101.4% |
| 0.000508nM | 108.7% | 105.9% | 107.6% | 90.4% | 99.5% |
| 0.000169nM | 126.8% | 107.8% | 106.5% | 93.3% | 113.5% |
| IC₅₀ (nM) | 0.1354 | 0.1258 | 0.1056 | 0.0729 | 0.0113 |

### Example 7 Activity Screening of siRNA at Eleven 3-Fold Gradient Diluted Concentrations in Hep3B Cell Line

The procedure of this example was similar to that of Example 5. The Hep3B cell line was selected. The starting concentration of siRNA (DR002222 to DR02226, DR001478, DR002252, DR000344, DR000348, DR000277, DR000199, DR000060, DR001482) was 10 nM, and 11 concentration points (10 nM, 3.33 nM, 1.11 nM, 0.37 nM, 0.123 nM, 0.041 nM, 0.0136 nM, 0.0045 nM, 0.00152 nM, 0.000508 nM, 0.000169 nM) were obtained by 3-fold gradient dilutions for screening the activity of siRNA in the Hep3B cell line. The screening results are shown in Table 12.

**Table 12-1 Screening Results of siRNA in Hep3B Cell Line at 11 Concentration Points**

| siRNA No. | DR002222 | DR002223 | DR002224 | DR002225 | DR002226 | DR001478 | DR002252 |
|---|---|---|---|---|---|---|---|
| 10nM | 1.5% | 2.7% | 1.9% | 1.0% | 2.6% | 2.7% | 3.9% |
| 3.33nM | 2.1% | 2.7% | 2.1% | 1.8% | 3.2% | 3.3% | 6.8% |
| 1.11nM | 2.7% | 2.2% | 2.7% | 2.1% | 3.7% | 3.4% | 14.4% |
| 0.37nM | 4.8% | 2.4% | 6.2% | 4.3% | 7.1% | 4.5% | 41.3% |
| 0.123nM | 8.3% | 4.7% | 18.9% | 7.8% | 14.1% | 9.6% | 90.6% |
| 0.041nM | 19.5% | 9.0% | 40.4% | 15.9% | 33.0% | 23.0% | 126.3% |
| 0.0136nM | 43.1% | 22.8% | 83.4% | 33.4% | 68.3% | 58.7% | 152.2% |
| 0.0045nM | 83.3% | 55.9% | 111.4% | 63.8% | 105.6% | 99.5% | 147.0% |
| 0.00152nM | 100.8% | 85.7% | 111.7% | 92.7% | 122.5% | 121.3% | 141.7% |
| 0.000508nM | 105.0% | 101.3% | 107.2% | 98.2% | 112.0% | 128.0% | 130.0% |
| 0.000169nM | 83.2% | 92.8% | 99.2% | 80.7% | 88.1% | 116.8% | 112.1% |
| IC₅₀ (nM) | 0.0129 | 0.005 | 0.0321 | 0.007 | 0.0169 | 0.0123 | 0.2292 |

**Table 12-2 Screening Results of siRNA in Hep3B Cell Line at 11 Concentration Points**

| siRNA No. | DR00034 4 | DR00034 8 | DR00027 7 | DR00019 9 | DR00006 0 | DR00148 2 |
|---|---|---|---|---|---|---|
| 10nM | 1.6% | 3.2% | 3.1% | 1.3% | 2.7% | 1.9% |
| 3.33nM | 2.2% | 3.5% | 3.4% | 2.3% | 3.3% | 2.2% |
| 1.11nM | 2.9% | 2.9% | 5.0% | 2.6% | 4.3% | 3.7% |
| 0.37nM | 4.8% | 4.6% | 12.4% | 6.5% | 5.5% | 5.9% |
| 0.123nM | 9.2% | 6.3% | 34.1% | 16.9% | 16.3% | 9.8% |
| 0.041nM | 28.1% | 16.7% | 82.4% | 34.3% | 46.7% | 27.6% |
| 0.0136nM | 66.8% | 43.5% | 132.5% | 82.1% | 111.9% | 55.9% |
| 0.0045nM | 104.4% | 102.8% | 158.1% | 113.6% | 133.9% | 106.5% |
| 0.00152n M | 145.6% | 139.7% | 232.8% | 129.6% | 150.2% | 112.8% |
| 0.000508n M | 152.9% | 154.7% | 155.7% | 123.1% | 152.3% | 104.4% |
| 0.000169n M | 122.0% | 134.5% | 109.2% | 117.6% | 105.9% | 84.4% |
| IC₅₀ (nM) | 0.0083 | 0.0073 | 0.0512 | 0.0215 | 0.0305 | 0.0131 |

### Example 8 Off-target Activity Screening in psiCHECK2 GSSM-5Hits

### Plasmid Preparation

The corresponding antisense strand off-target plasmid was designed according to the siRNA sequence. The recombinant plasmid psiCHECK2 GSSM-5Hits was prepared by Sangon Biotech (Shanghai) Co., Ltd. and diluted to 1,000 ng/µL for later use.

### Cell Transfection

HEK293A cells (Nanjing Cobioer Biotechnology Co., Ltd., Cat#: CBP60436) were used, and 100 µL of cell suspension was seeded into a 96-well plate at 8×10³ cells/well.

On the following day, the complete medium in the wells was discarded and replaced with Opti-MEM medium at 80 µL/well. The cells were starved for about 1.5 h.

Preparation of siRNA: siRNA was diluted 3-fold starting from the final concentration of 40 nM to obtain 11 concentration points (40 nM, 13.3 nM, 4.44 nM, 1.48 nM, 0.493 nM, 0.164 nM, 0.0548 nM, 0.0182 nM, 0.00609 nM, 0.00203 nM, 0.000677 nM).

Preparation of plasmid mixture: plasmid: 0.01 µL/well, Opti-MEM: 8.99 µL/well.

Preparation of Lipo mixture: 0.2 µL of Lipo 2000 (Lipofectamine^{™} 2000 transfection reagent, Thermo, 11668019) was diluted with 9.8 µL of Opti-MEM in each well and allowed to stand at room temperature for 5 min.

Subsequently, 22 µL of the prepared Lipo mixture, 2.2 µL of siRNA, and 19.8 µL of plasmid mixture were loaded into the same well (designated as Well A mixture), mixed well by pipetting up and down, and incubated at room temperature for 20 min before co-transfection. Well A mixture was then added to each well of cells at 20 µL/well to obtain a final volume of 100 µL/well (20 µL of Well A mixture + existing 80 µL of Opti-MEM). After incubation in a 5% CO₂ incubator at 37°C for 4 h, 100 µL of DMEM containing 20% fetal bovine serum was added to each well. Testing was performed after further incubation in a 5% CO₂ incubator at 37°C for 24 h.

### Testing

The mixed Dual-Glo^{®} Luciferase (Dual-Glo^{®} Luciferase Assay System, Promega, E2940) was thawed and equilibrated to room temperature before testing. DMEM was then added to each tube at a 1:1 ratio to prepare substrate I immediately before use. Similarly, the Dual-Glo^{®} Stop & Glo^{®} Buffer was thawed, equilibrated to room temperature, and then mixed with the Dual-Glo^{®} Stop & Glo^{®} Substrate at a 100:1 ratio to prepare substrate II immediately before use. The existing culture medium in the 96-well plate was aspirated using a vacuum pump. 150 µL of substrate I was added to each well and incubated on a shaker at room temperature for 10 min. 120 µL of substrate I was transferred to a 96-well microplate and the Firefly chemiluminescence value was read on a microplate reader (Tecan, Infinite 200). Another 60 µL of substrate II was added to each well and incubated on a shaker at room temperature for 10 min. The Renilla chemiluminescence value was read on a microplate reader.

### Data Analysis and Processing

The fluorescence activity was measured using a microplate reader. The collected Renilla signal was normalized to the Firefly signal standard. The inhibitory effect of siRNA was determined through comparison with unprocessed results (residual inhibitory activity). The calculation process is as follows: Normalized Ren/Fir ratio: Ratio = Renilla (Renilla luciferase)/Firefly (Firefly luciferase).

Residual inhibition = (Ratio_{siRNA}/ Ratio_{control}) * 100%, taking the mean of two replicate wells, where Ratio_{control} was the ratio value of the control well (without siRNA) (taking the mean of two replicate wells).

Plotting: Graphpad Prism was used for plotting.

Half maximal inhibitory concentration (IC₅₀): Top and Bottom were plotted in this experiment. The IC₅₀ value was calculated according to the formula Y=Bottom+(Top-Bottom)/(1+10^((LogIC₅₀-X)*HillSlope)), where Y = 50 and X = log(concentration).

The off-target activity screening results of siRNA in psiCHECK2 GSSM-5Hits are shown in Table 13.

**Table 13 Off-target Activity Screening Results in psiCHECK2 GSSM-5Hits**

| siRNA Compou nd No. | DR002 222 | DR002 223 | DR002 224 | DR002 225 | DR002 226 | DR001 478 | DR002 252 |
|---|---|---|---|---|---|---|---|
| 40nM | 10.6% | 36.3% | 36.4% | 47.6% | 33.8% | 75.2% | 55.5% |
| 13.3nM | 6.5% | 41.0% | 20.9% | 50.9% | 32.1% | 87.2% | 74.2% |
| 4.44nM | 4.4% | 44.9% | 11.0% | 49.9% | 24.3% | 94.9% | 72.1% |
| 1.48nM | 3.7% | 59.8% | 8.7% | 55.2% | 31.2% | 117.3% | 98.1% |
| 0.493nM | 9.4% | 76.6% | 11.5% | 79.4% | 39.7% | 103.1% | 84.1% |
| 0.164nM | 23.0% | 77.9% | 31.3% | 84.5% | 75.3% | 98.9% | 81.9% |
| 0.0548n M | 82.0% | 83.7% | 67.3% | 94.4% | 99.1% | 94.8% | 88.2% |
| 0.0182n M | 98.8% | 89.8% | 86.9% | 88.8% | 102.7% | 98.0% | 86.2% |
| 0.00609n M | 102.3% | 94.3% | 92.9% | 88.7% | 105.1% | 91.3% | 89.3% |
| 0.00203n M | 112.4% | 90.1% | 92.0% | 92.8% | 109.8% | 97.8% | 94.2% |
| 0.000677 nM | 101.9% | 90.4% | 93.1% | 90.0% | 93.0% | 83.9% | 85.1% |
| GSSM-5 Hits fitted IC₅₀ (nM) | 0.0964 | 3.5624 | 0.0859 | 3.8879 | 0.3222 | >40nM | >40nM |

### Example 9 Activity Screening in Primary Human Hepatocytes (PHHs)

### Cell Transfection

On the first day, 1.4 mL of rat tail collagen solution (Sigma, C3867) was added to 40.6 mL of DNase RNase-Free Distilled Water and mixed well. The mixture was added to each well of a 96-well culture plate at 40 µL/well, and coated overnight at 4°C. The coating medium was discarded the next day.

On the second day, prior to use, the coated cell plate was rinsed with DPBS, which was then aspirated. PHHs (Shanghai Xuanyi Biotechnology Co., Ltd., Cat#: QYLF-HPMC) were recovered at 37°C, transferred into the recovery medium, centrifuged, resuspended, and counted. PHHs were plated in a 96-well plate at 90 µL/well (2×10⁴ cells/well). The complete medium was replaced after 4 h, and transfection was performed after 18 h.

On the third day, 2 µL of 20 µM siRNA (DR002222, DR002223, DR002225, DR002226, DR001478, and DR002252) stock solution was diluted with 198 µL of Opti-MEM and mixed well by pipetting up and down as the first concentration point. The gradient dilution was performed according to the actual experimental requirements.

On the third day, 0.9 µL of RNAiMAX (Thermo, 13778150) was diluted with 14.1 µL of Opti-MEM, mixed well by gently pipetting up and down, and allowed to stand at room temperature for 5 min. Then, 15 µL of the prepared RNAi-MAX mixture and 15 µL of the diluted siRNA compound were mixed well by gently pipetting up and down without generating bubbles, allowed to stand at room temperature for 10 min, and transferred to a 96-well plate at 10 µL/well. RNA was extracted after incubation in a 5% CO₂ incubator at 37°C for 24 h.

### RNA Extraction

Cellular RNA was extracted using a nucleic acid extractor (Hangzhou Allsheng, Auto-pure96) according to the protocol of the high-throughput cellular RNA extraction kit (FireGen, FG0412).

### RNA Reverse Transcription

The denatured reaction mixture was prepared with reference to PrimeScript^{™} II 1st Strand cDNA Synthesis Kit (Takara, 6210B). Each well contained 1 µL of Oligo dT Primer, 1 µL of dNTP Mixture, and 12.5 µL of template RNA. The denaturation reaction was performed by incubation in a conventional PCR instrument at 65°C for 5 min. The mixture was quickly cooled on ice for 2 min.

The reverse transcription reaction mixture was prepared with reference to PrimeScript^{™} II 1st Strand cDNA Synthesis Kit (Takara, 6210B). Each well contained 4 µL of 5×Prime Script II Buffer, 0.5 µL of RNase Inhibitor, and 1 µL of PrimeScript II RTase.

The denatured reaction mixture (14.5 µL) was mixed with the reverse transcription reaction mixture, incubated in a conventional PCR instrument at 42°C for 45 min for reverse transcription, and incubated at 95°C for 5 min to inactivate the enzyme. The reverse transcription product (cDNA) was then cooled at 4°C.

After reverse transcription, 30 µL of DNase RNase-Free Distilled Water was added to each well of the cDNA sample.

### Fluorescence Quantitative PCR

Fluorescence quantitative PCR reaction was performed in a 20 µL system (ABI, QuantStudio3) with reference to the operating procedure of TaqMan^{™} Fast Advanced Master Mix (ABI, 4444965). The reaction program was: (50°C, 2 min) × 1 Cycle; (95°C, 20 s) × 1 Cycle; (95°C, 1 s; 60°C, 24 s) × 40 Cycles.

**Table 14 Primer Information**

| Primer Name | Sequence Information (5'-3') | Fluorophore |
|---|---|---|
| hACTB-PF | ACGTGGACATCCGCAAAGAC (SEQ ID NO: 1670) | / |
| hACTB-PR | TCTTCATTGTGCTGGGTGCC (SEQ ID NO: 1671) | / |
| hACTB-P | AACACAGTGCTGTCTGGCGGCACCA (SEQ ID NO: 1672) | 5'TET,3'BHQ2 |
| hAPOC3-PF | TGCCTCCCTTCTCAGCTTCA (SEQ ID NO: 1673) | / |
| hAPOC3-PR | GGGAACTGAAGCCATCGGTC (SEQ ID NO: 1674) | / |
| hAPOC3-P | ATGAAGCACGCCACCAAGACCGCCA (SEQ ID NO: 1675) | 5'6-FAM,3'BHQ1 |

### Data Statistics

The 2-^{△△Ct} value was calculated and converted into a percentage to obtain the residual inhibition. △△Ct = [(Cttarget gene of experimental group - Ctinternal reference of experimental group) - (Cttarget gene of control group - Ctinternal reference of control group)].

The starting concentration of siRNA was 10 nM, and 5 concentration points (10 nM, 1 nM, 0.1 nM, 0.01 nM, 0.001 nM) were obtained by 10-fold gradient dilutions for screening the activity of siRNA in PHHs. The screening results are shown in Table 15.

**Table 15 Screening Results of siRNA in PHHs at 5 Concentration Points**

| siRNA No. | 10nM | 1nM | 0.1nM | 0.01nM | 0.001nM | IC₅₀ (nM) |
|---|---|---|---|---|---|---|
| DR002222 | 12.9% | 15.0% | 17.1% | 34.0% | 67.3% | 0.0044 |
| DR002223 | 16.0% | 14.5% | 22.4% | 43.9% | 70.5% | 0.0090 |
| DR002225 | 15.8% | 21.8% | 38.5% | 85.2% | 109.5% | 0.0264 |
| DR002226 | 15.3% | 17.3% | 33.9% | 91.4% | 91.0% | 0.0450 |
| DR001478 | 17.3% | 15.6% | 23.3% | 57.4% | 87.2% | 0.0114 |
| DR002252 | 17.3% | 39.8% | 86.0% | 107.8% | 100.4% | 0.3989 |

### Example 10 Activity Screening in Mouse HDI Model

### HDI Animal Modeling

Using the tail vein high-pressure injection method, six- to eight-week-old female Balb/c mice were transfected *in vivo* with a dual-gene stable transfection system. The transfection was performed via the tail vein, injecting a TransIT^{®}-QR Delivery Solution (total volume: 10% of animal body weight, Mirusbio-MIR 5240) containing Piggy-Bac transposon plasmids (Suzhou Bangye) and Piggy-Bac helper plasmids (Suzhou Bangye) (mass ratio: 1:1, total plasmid amount: 50 µg) containing different mass ratios of target gene cDNA sequences into mice within 5-7 seconds using a 27-gauge needle. After injection, the mice were returned to their cage and observed for 30 minutes. The day of modeling was designated as Day 0. Serum samples were collected at various time points after modeling (Day 7 to Day 35) for the detection of SEAP expression levels.

The dual-gene stable transfection system comprised a Piggy-Bac helper plasmid and a Piggy-Bac transposon plasmid, wherein the Piggy-Bac helper plasmid provided the Piggy-Bac transposase, while the Piggy-Bac transposon plasmid was based on the Piggy-Bac transposon and contained a dual-gene expression element, which included the secreted alkaline phosphatase (SEAP) gene and the target gene (APOC3).

### Detection of SEAP Expression Levels

The standard provided with the kit (Phospha-Light^{™} SEAP Reporter Gene Assay System, Invitrogen, T1016) was diluted 2-fold starting from an initial concentration of 15 mU/mL to obtain 7 concentration points.

The CSPD substrate was mixed with the Reaction Buffer Diluent at a ratio of 1:20 to obtain the reaction mixture. The 5×Dilution Buffer was diluted to 1×Dilution Buffer with DNase RNase-Free Distilled Water. The serum was mixed with 1×Dilution Buffer in a centrifuge tube to prepare the sample dilution, which was then incubated at 65°C for 30 min and then cooled to room temperature. Approximately 50 µL of the sample dilution was added to a 96-well plate, followed by the addition of 50 µL of Assay Buffer per well, and incubated at room temperature for 5 min. Then, 50 µL of the reaction solution was added to each well and incubated at room temperature for 20 min. The SEAP chemiluminescence value was read using a microplate reader (Tecan, Infinite 200).

The prophylactic and/or therapeutic effect of the sample to be tested was evaluated by measuring the levels of SEAP expression in serum. A sample to be tested capable of inhibiting SEAP expression levels was selected as a nucleic acid drug.

At 15 days after modeling, each mouse was administered a single subcutaneous dose according to Table 16: 200 µL of normal saline containing 3 mg/kg (mpk) APOC3 RNAi reagent (DR002222 to DR002226, DR001478, DR002252); or 200 µL of normal saline without APOC3 RNAi reagent as a control. The HDI model screening results are shown in Table 17-1 and Table 17-2.

**Table 16 Dosage of RNAi Reagent for Single Subcutaneous Administration to Mice**

| No. | Group | Dose (mg/kg, mpk) |
|---|---|---|
| 1 | Control | / |
| 2 | DR002222 | 3 |
| 3 | DR002223 | 3 |
| 4 | DR002224 | 3 |
| 5 | DR002225 | 3 |
| 6 | DR002226 | 3 |
| 7 | DR001478 | 3 |
| 8 | DR002252 | 3 |

**Table 17-1 Experimental Results of HDI Stably Transfected Plasmid Mouse Model**

| Days | Mean SEAP (mU/mL) | | | | | | | |
|---|---|---|---|---|---|---|---|---|
| | Cont rol | DR002 222 | DR002 223 | DR002 224 | DR002 225 | DR002 226 | DR001 478 | DR002 252 |
| 7 | 1265 .5 | 207.2 | 246.5 | 404.5 | 231.2 | 177.0 | 113.2 | 403.0 |
| 14 | 1725 .0 | 311.3 | 355.4 | 698.6 | 429.4 | 368.0 | 145.3 | 523.1 |
| 21 | 851. 6 | 374.5 | 334.7 | 536.0 | 537.1 | 463.5 | 193.9 | 401.6 |

**Table 17-2 Experimental Results of HDI Stably Transfected Plasmid Mouse Model**

| Days | Mean SEAP (relative to control) | | | | | | | |
|---|---|---|---|---|---|---|---|---|
| | Cont rol | DR002 222 | DR002 223 | DR002 224 | DR002 225 | DR002 226 | DR001 478 | DR002 252 |
| 7 | 100 % | 16.4% | 19.5% | 32.0% | 18.3% | 14.0% | 8.9% | 31.8% |
| 14 | 100 % | 18.0% | 20.6% | 40.5% | 24.9% | 21.3% | 8.4% | 30.3% |
| 21 | 100 % | 44.0% | 39.3% | 62.9% | 63.1% | 54.4% | 22.8% | 47.2% |

### Example 11 Activity Screening in Primary Cynomolgus Monkey Hepatocyte (PCHs)

### Cell Transfection

To each well of a 96-well cell culture plate, 100 µL of Coating Medium was added. The plate was coated at 37°C for 30 min, and then the Coating Medium was discarded.

PCHs (Milestone Biotechnologies) were recovered at 37°C, resuspended with Thawing Medium, centrifuged, counted, and plated in a 96-well plate at 90 µL/well (2×10⁴ cells/well).

siRNA compound dilution: 2 µL of 20 µM siRNA compound stock solution was diluted with 198 µL of Opti-MEM and mixed well by pipetting up and down as the first concentration point. The dilution was performed according to the actual experimental requirements.

Transfection: 0.9 µL of RNAiMAX (Thermo, 13778150) was diluted with 14.1 µL of Opti-MEM, mixed well by gently pipetting up and down, and allowed to stand at room temperature for 5 min. Then, 15 µL of the prepared RNAi-MAX mixture and 15 µL of the diluted compound were mixed well by gently pipetting up and down without generating bubbles, allowed to stand at room temperature for 10 min, and transferred to a 96-well plate at 10 µL/well. After incubation in a 5% CO₂ incubator at 37°C for 4 h, the Culture Medium was replaced and RNA was extracted the next day.

### RNA Extraction

Cellular RNA was extracted using a nucleic acid extractor (Hangzhou Allsheng, Auto-pure96) according to the protocol of the high-throughput cellular RNA extraction kit (FireGen, FG0412).

### RNA Reverse Transcription

The denatured reaction mixture was prepared with reference to PrimeScript^{™} II 1st Strand cDNA Synthesis Kit (Takara, 6210B). The preparation volume per well was: 1 µL of Oligo dT Primer, 1 µL of dNTP Mixture, and 12.5 µL of template RNA. The mixture was incubated in a conventional PCR instrument at 65°C for 5 min and then quickly cooled on ice for 2 min.

The reverse transcription reaction mixture was prepared with reference to PrimeScript^{™} II 1st Strand cDNA Synthesis Kit (Takara, 6210B). The preparation volume per well was: 4 µL of 5×Prime Script II Buffer, 0.5 µL of RNase Inhibitor, 1 µL of PrimeScript II RTase, and 14.5 µL of the denatured reaction mixture prepared in the previous step. The mixture was slowly mixed well, incubated in a conventional PCR instrument at 42°C for 45 min for reverse transcription, and incubated at 95°C for 5 min to inactivate the enzyme. The reverse transcription product (cDNA) was then cooled at 4°C.

After reverse transcription, 30 µL of DNase RNase-Free Distilled Water was added to each well of the cDNA sample.

### Fluorescence Quantitative PCR

Fluorescence quantitative PCR reaction was performed in a 20 µL system (ABI, QuantStudio3) with reference to the operating procedure of TaqMan^{™} Fast Advanced Master Mix (ABI, 4444965). The reaction program was: (50°C, 2 min) × 1 Cycle; (95°C, 20 s) × 1 Cycle; (95°C, 1 s; 60°C, 24 s) × 40 Cycles.

**Table 18 Primer Information**

| Primer Name | Sequence Information (5'-3') | Fluorophore |
|---|---|---|
| mkGAPDH-PF | TCAAGATCGTCAGCAACGCC (SEQ ID NO: 1676) | / |
| mkGAPDH-PR | ACAGTCTTCTGGGTGGCAGT (SEQ ID NO: 1677) | / |
| mkGAPDH-P | ACCAACTGCTTAGCACCCCTGGCCA (SEQ ID NO: 1678) | 5'TET,3'BHQ2 |
| mkAPOC3-PF | GCCTGCCTGCTCTGTTCATC (SEQ ID NO: 1679) | / |
| mkAPOC3-PR | AAGCCAAGAAGGGAGGTGTCC (SEQ ID NO: 1680) | / |
| mkAPOC3-P | TTGTTGCTGCCGTGCTGTCACTCCTG G (SEQ ID NO: 1681) | 5'6-FAM,3'BHQ1 |

### Data Statistics

The 2-△△Ct value was calculated and converted into a percentage to obtain the residual inhibition. △△Ct = [(Cttarget gene of experimental group - Ctinternal reference of experimental group) - (Cttarget gene of control group - Ctinternal reference of control group)].

The starting concentration of the siRNA compound was 10 nM, and 11 concentration points (10 nM, 3.33 nM, 1.11 nM, 0.37 nM, 0.123 nM, 0.041 nM, 0.0136 nM, 0.0045 nM, 0.00152 nM, 0.000508 nM, 0.000169 nM) were obtained by 3-fold gradient dilutions for screening the activity of the siRNA compound in PCHs. The screening results are shown in Table 19.

**Table 19 IC₅₀ Screening Results in PCHs at 11 Different Concentrations**

| siRN A Com poun d No. | 10 n M | 3.3 3n M | 1.1 1n M | 0.3 7n M | 0.12 3n M | 0.04 1n M | 0.01 36n M | 0.00 45n M | 0.001 52n M | 0.000 508n M | 0.000 169n M | IC₅₀ (nM ) |
|---|---|---|---|---|---|---|---|---|---|---|---|---|
| DR00 7153 | 32. 5 % | 40. 2% | 49. 5% | 45. 5% | 47.5 % | 56.1 % | 71.7 % | 87.9 % | 99.7 % | 99.4 % | 97.8 % | 0.01 54 |
| DR00 7816 | 23. 1 % | 40. 4% | 23. 5% | 25. 2% | 28.1 % | 33.5 % | 40.3 % | 49.5 % | 70.4 % | 100.6 % | 98.0 % | 0.00 18 |
| DR00 7849 | 21. 7 % | 32. 5% | 27. 6% | 32. 8% | 38.2 % | 47.3 % | 58.3 % | 77.4 % | 98.2 % | 100.1 % | 98.1 % | 0.00 86 |
| DR00 7093 | 29. 8 % | 35. 5% | 46. 6% | 47. 1% | 46.1 % | 51.3 % | 63.7 % | 90.0 % | 97.0 % | 99.8 % | 101.2 % | 0.01 14 |
| DR00 7815 | 33. 6 % | 57. 6% | 30. 6% | 35. 4% | 35.5 % | 40.5 % | 50.9 % | 65.8 % | 88.7 % | 100.9 % | 100.8 % | 0.00 33 |
| DR00 7846 | 41. 6 % | 64. 7% | 69. 2% | 45. 8% | 50.8 % | 59.4 % | 69.5 % | 90.2 % | 102.6 % | 110.6 % | 105.2 % | 0.01 01 |
| DR00 7850 | 22. 5 % | 28. 8% | 36. 7% | 38. 5% | 47.9 % | 57.1 % | 68.1 % | 80.5 % | 93.5 % | 96.8 % | 96.5 % | 0.02 44 |
| DR00 7848 | 29. 4 % | 31. 8% | 42. 3% | 37. 0% | 45.1 % | 47.7 % | 77.1 % | 90.1 % | 91.1 % | 92.2 % | 96.0 % | 0.02 28 |
| DR00 1478 | 28. 7 % | 45. 4% | 35. 0% | 35. 7% | 42.3 % | 54.2 % | 72.5 % | 91.1 % | 102.3 % | 93.4 % | 96.8 % | 0.01 99 |

### Example 12 Activity Screening in Mouse HDI Model

According to the experimental method of Example 10, six- to eight-week-old female Balb/c mice were selected for HDI modeling and dosing in this experiment. For modeling, the mass ratio of Piggy-Bac helper plasmid to recombinant Piggy-Bac transposon plasmid was 1:1, with a total plasmid amount of 50 µg. At 14 days after modeling, serum was collected and the mice were blindly assigned to groups (N=6) and administered a single subcutaneous dose of either 200 µL of normal saline containing 3 mg/kg (mpk) Apoc3 RNAi reagent or 200 µL of normal saline without Apoc3 RNAi reagent as control according to Table 18. The screening results in the HDI model are shown in Table 20.

**Table 20 Experimental Results of RNAi Reagent in HDI Stably Transfected Plasmid Mouse Model**

| | Day7 | | Day14 | | Day21 | | Day28 | |
|---|---|---|---|---|---|---|---|---|
| siRNA Compound | Mean | SD | Mean | SD | Mean | SD | Mean | SD |
| Normal saline | 90.0% | 6.2% | 89.1% | 7.1% | 87.6% | 11.0% | 88.2% | 18.2% |
| DR001478 | 7.5% | 1.0% | 5.1% | 1.3% | 12.9% | 4.3% | 18.1% | 3.7% |
| DR007153 | 6.7% | 2.4% | 4.3% | 3.7% | 8.7% | 6.8% | 8.1% | 7.7% |
| DR007849 | 7.5% | 2.2% | 5.8% | 2.5% | 12.7% | 4.5% | 12.2% | 4.0% |
| DR007816 | 6.3% | 3.0% | 3.6% | 2.5% | 7.9% | 6.4% | 9.3% | 8.2% |
| DR007093 | 8.9% | 3.1% | 7.9% | 3.2% | 19.7% | 7.6% | ND | ND |
| DR007850 | 9.6% | 2.4% | 12.4% | 4.7% | 29.1% | 9.6% | ND | ND |
| DR007815 | 6.3% | 3.3% | 10.7% | 10.5% | 23.8% | 20.0% | ND | ND |
| DR007848 | 7.0% | 1.5% | 9.5% | 3.0% | 23.7% | 4.6% | ND | ND |
| DR007846 | 9.5% | 1.7% | 13.7% | 3.3% | 40.8% | 18.5% | ND | ND |

| | | | | | | | | |
|---|---|---|---|---|---|---|---|---|
| ND: not detected. | | | | | | | | |

### Example 13 Pharmacodynamic Validation in hAPOC3 Transgenic Mouse Model

hAPOC3 transgenic mice (B6-hAPOC3-Tg, T055510, male, 6-8 weeks old, GemPharmatech) were used to detect triglycerides (TG), low-density lipoprotein cholesterol (LDL-c), high-density lipoprotein cholesterol (HDL-c), and total cholesterol (TC). Mice were randomly divided into 5 groups according to TG levels, with 5 animals in each group. The dose for each animal was calculated based on body weight and administered subcutaneously as a single dose. The siRNA conjugate was administered as a 3 mg/mL solution (0.9% aqueous sodium chloride solution as solvent). Specifically, before the experiment, the siRNA conjugate was dissolved and diluted to the desired concentration and volume with 0.9% aqueous sodium chloride solution. The dose volume was 5 mL/kg for both the normal saline and siRNA conjugate.

Blood samples were collected before administration (designated as Day -3) for testing. Animals were grouped based on the TG levels on Day -3. On Days 7, 14, 28, 42, 56, and 70 after administration (with the day of administration designated as Day 0), blood samples were collected from the orbital venous plexus of mice (starved for 5 h before each blood collection) and centrifuged to collect serum for detecting TG, LDL-c, TC, and target protein by ELISA (Human APOC3 ELISA Kit, Abcam, ab154131). The experimental results are shown in Tables 21 to 24.

**Table 21 Pharmacodynamic Results in hAPOC3 Transgenic Mouse Model - TG Inhibition**

| TG Inhibition | Normal saline | | DR001478 | | DR007093 | | DR007153 | | DR005722 | |
|---|---|---|---|---|---|---|---|---|---|---|
| | Mean | SD | Mean | SD | Mean | SD | Mean | SD | Mean | SD |
| D7 | 5.6% | 6.0% | -91.2% | 2.5% | -93.7% | 1.7% | -93.2% | 1.5% | -92.1% | 1.3% |
| D14 | -2.1% | 14.3% | -93.8% | 1.7% | -94.1% | 1.9% | -94.1% | 1.1% | -91.8% | 2.9% |
| D28 | 5.5% | 13.3% | -88.5% | 5.1% | -90.2% | 3.9% | -90.3% | 2.1% | -87.4% | 3.0% |
| D42 | 35.2% | 14.4% | -74.5% | 7.3% | -82.1% | 8.6% | -84.2% | 3.9% | -66.7% | 11.3% |
| D56 | 21.0% | 10.0% | -36.2% | 18.2% | -48.6% | 21.6% | -74.2% | 7.5% | -20.3% | 21.3% |
| D70 | 23.8% | 12.1% | -3.0% | 24.6% | -24.6% | 19.4% | -38.6% | 8.0% | / | / |

**Table 22 Pharmacodynamic Results in hAPOC3 Transgenic Mouse Model - TC Inhibition**

| TC Inhibition | Normal saline | | DR001478 | | DR007093 | | DR007153 | | DR005722 | |
|---|---|---|---|---|---|---|---|---|---|---|
| | Mean | SD | Mean | SD | Mean | SD | Mean | SD | Mean | SD |
| D7 | 4.8% | 5.8% | -78.7% | 4.0% | -78.4% | 4.0% | -79.8% | 3.0% | -78.7% | 3.8% |
| D14 | 3.3% | 14.4% | -79.8% | 3.4% | -79.1% | 4.8% | -81.1% | 2.3% | -78.0% | 5.6% |
| D28 | 12.3% | 6.5% | -75.6% | 6.1% | -75.5% | 5.9% | -77.2% | 3.0% | -77.0% | 4.7% |
| D42 | 22.9% | 8.3% | -67.2% | 6.2% | -72.6% | 8.5% | -74.5% | 3.2% | -62.7% | 6.3% |
| D56 | 19.0% | 4.7% | -34.4% | 12.6% | -41.6% | 21.3% | -63.7% | 5.8% | -17.9% | 16.4% |
| D70 | 20.0% | 13.0% | 1.6% | 19.2% | -18.9% | 16.3% | -31.9% | 9.0% | / | / |

**Table 23 Pharmacodynamic Results in hAPOC3 Transgenic Mouse Model - LDL-c Inhibition**

| LDL-c Inhibition | Normal saline | | DR001478 | | DR007093 | | DR007153 | | DR005722 | |
|---|---|---|---|---|---|---|---|---|---|---|
| | Mean | SD | Mean | SD | Mean | SD | Mean | SD | Mean | SD |
| D7 | 0.6% | 9.7% | -74.6% | 6.9% | -74.3% | 5.0% | -74.2% | 4.8% | -72.1% | 6.3% |
| D14 | 20.2% | 10.3% | -75.3% | 5.5% | -71.7% | 2.2% | -73.3% | 6.4% | -69.5% | 3.0% |
| D28 | 16.4% | 12.1% | -67.2% | 8.7% | -67.2% | 3.0% | -65.3% | 8.8% | -67.1% | 7.9% |
| D42 | 11.9% | 25.3% | -49.0% | 12.0% | -58.9% | 5.3% | -57.1% | 14.0% | -39.5% | 15.8% |
| D56 | 12.4% | 14.7% | -10.4% | 23.6% | -10.9% | 13.9% | -34.8% | 23.6% | 4.5% | 18.9% |

**Table 24 Pharmacodynamic Results in hAPOC3 Transgenic Mouse Model - Apoc3 Inhibition**

| Apoc3 Protein Inhibition | Normal saline | | DR001478 | | DR007093 | | DR007153 | | DR005722 | |
|---|---|---|---|---|---|---|---|---|---|---|
| | Mean | SD | Mean | SD | Mean | SD | Mean | SD | Mean | SD |
| D7 | -1.6% | 15.6% | -89.8% | 2.5% | -85.5% | 14.7% | -92.4% | 1.8% | -92.8% | 2.0% |
| D14 | 31.3% | 34.9% | -91.8% | 3.2% | -94.6% | 0.7% | -95.2% | 1.6% | -95.2% | 1.5% |
| D28 | -10.0% | 17.2% | -86.8% | 3.9% | -89.2% | 2.3% | -89.4% | 1.1% | -89.2% | 3.1% |
| D42 | -27.2% | 11.1% | -82.2% | 4.3% | -88.8% | 2.9% | -90.2% | 4.6% | -80.8% | 5.5% |
| D56 | -19.0% | 31.6% | -48.9% | 22.7% | -65.6% | 12.7% | -80.8% | 12.2% | -45.2% | 20.9% |
| D70 | 3.5% | 30.0% | -12.5% | 38.2% | -32.7% | 15.0% | -49.1% | 10.4% | / | / |

### Example 14 Off-target Activity Screening in psiCHECK2 GSSM-5Hits

With reference to Example 8, the HEK293A cell line was selected in this experiment. The starting concentration of the siRNA compound was 40 nM, and 11 concentration points (40 nM, 13.3 nM, 4.44 nM, 1.48 nM, 0.493 nM, 0.164 nM, 0.0548 nM, 0.0182 nM, 0.00609 nM, 0.00203 nM, 0.000677nM) were obtained by 3-fold gradient dilutions for screening the off-target activity of the siRNA compound in psiCHECK2 GSSM-5Hits. The screening results are shown in Table 25.

**Table 25 Off-target Activity Screening Results in psiCHECK2 GSSM-5Hits**

| siRNA Compo und No. | 40n M | 13.3 nM | 4.44 nM | 1.48 nM | 0.493 nM | 0.164 nM | 0.0548 nM | 0.0182 nM | 0.0060 9nM | 0.0020 3nM | 0.00067 7nM | Fitte d IC₅₀ (nM ) |
|---|---|---|---|---|---|---|---|---|---|---|---|---|
| DR007 848 | 67.8 % | 64.3 % | 68.5 % | 75.2 % | 82.8 % | 99.5 % | 99.0% | 100.3 % | 102.9% | 100.6% | 103.0% | >40 nM |
| DR007 850 | 51.6 % | 62.0 % | 63.2 % | 70.1 % | 83.3 % | 93.5 % | 92.9% | 103.5 % | 101.3% | 102.2% | 103.2% | >40 nM |
| DR007 846 | 72.7 % | 72.3 % | 75.6 % | 86.3 % | 93.5 % | 101.5 % | 111.6 % | 112.9 % | 111.3% | 118.6% | 103.2% | >40 nM |
| DR007 815 | 45.1 % | 49.9 % | 59.7 % | 60.5 % | 76.6 % | 83.7 % | 87.4% | 96.2% | 93.5% | 97.7% | 88.6% | 12.3 1 |
| DR007 849 | 2.6% | 2.8% | 2.9% | 3.4% | 3.2% | 3.8% | 9.2% | 37.6% | 88.4% | 108.3% | 101.0% | 0.01 44 |
| DR001 478 | 105. 9% | 100. 9% | 105. 3% | 112. 4% | 118.8 % | 122.3 % | 114.3 % | 108.6 % | 112.8% | 108.8% | 100.2% | >40 nM |

### Example 15 Preparation of Compound E2

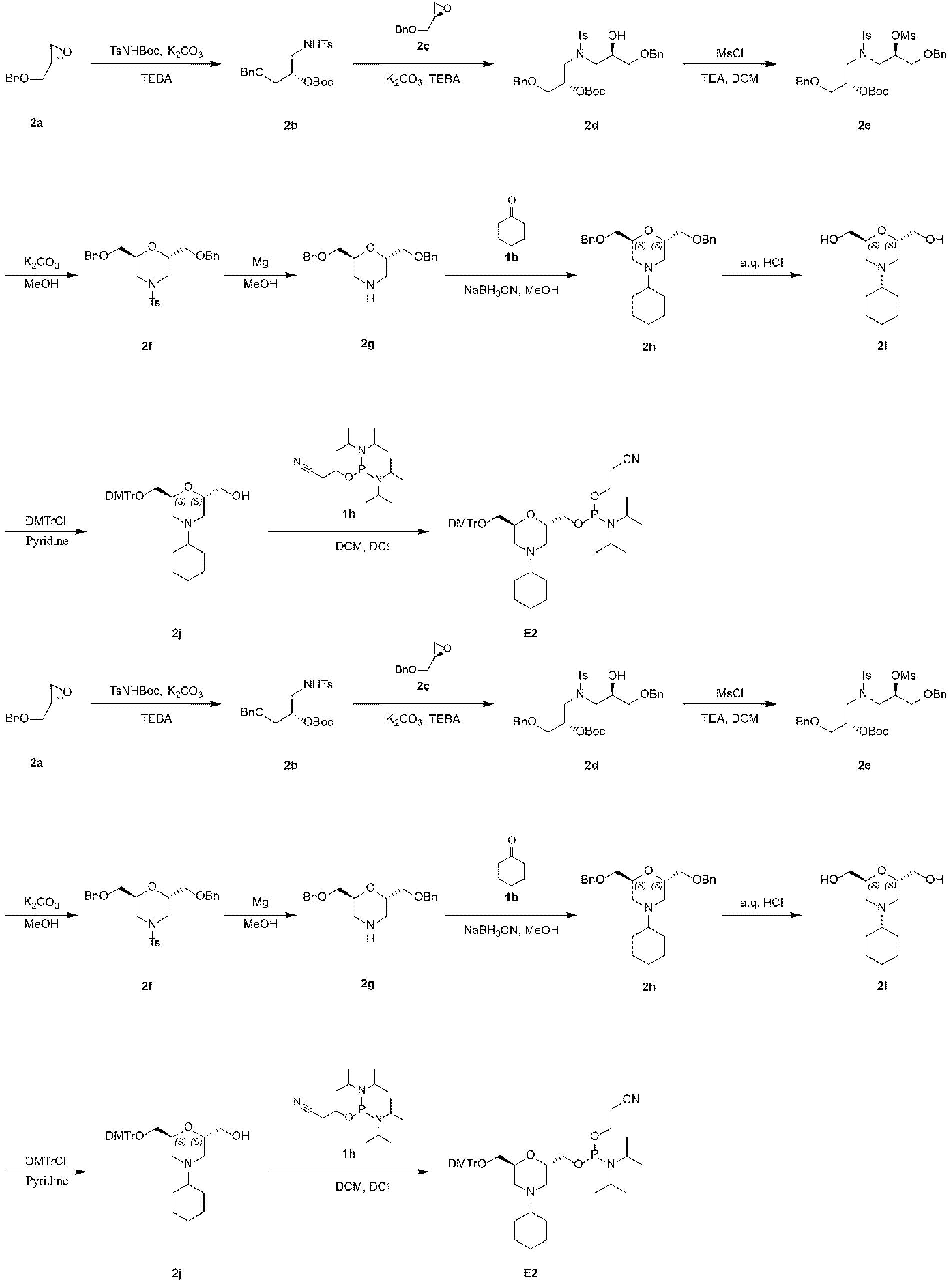

*1. Preparation of Compound 2b*

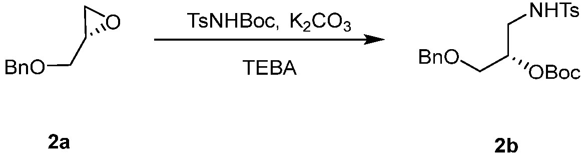

Compound 2a (38.0 g, 231 mmol), TsNHBoc (75.4 g, 278 mmol), K₂CO₃ (6.40 g, 46.3 mmol), and TEBA (5.27 g, 23.1 mmol) were added to a three-necked flask at room temperature. The reaction was performed at 95°C for 2 h, and then the temperature was cooled to 25°C until TLC (PE/EA = 2/1, UV 254 nm) showed that the reaction was complete. The reaction mixture was diluted with water (500 mL), and extracted with dichloromethane (200 mL × 3). The organic phase was concentrated under reduced pressure, and the crude product was purified by column separation (PE/ EA = 10/1-3/1) to obtain the title compound **2b** (40.0 g).
¹H NMR (400 MHz, CDCl₃) δ 7.72 (d, *J =* 8.4 Hz, 2 H), 7.28 - 7.39 (m, 7 H), 4.72 - 4.86 (m, 2 H), 4.42 - 4.56 (m, 2 H), 3.52 - 3.61 m, 2 H), 3.14 - 3.39 (m, 2 H), 2.43 (s, 3 H), 1.47 (s, 9 H)

### 2. Preparation of Compound 2d

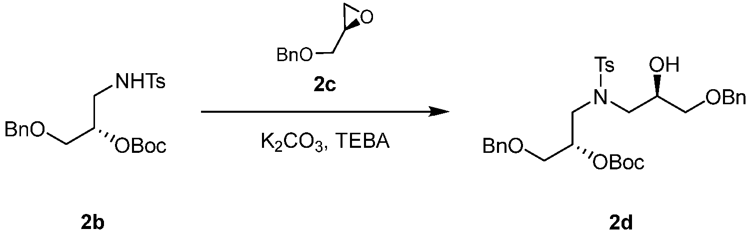

Compound **2b** (79.0 g, 181 mmol), compound **2c** (24.7 g, 150.8 mmol), potassium carbonate (4.21 g, 30.5 mmol), and TEBA (3.47 g, 15.2 mmol) were reacted at 95°C for 3 h until TLC (PE/EA = 2/1, UV 254 nm) showed that the reaction was complete. The reaction mixture was diluted with water (500 mL), and extracted with dichloromethane (200 mL × 3). The organic phase was concentrated under reduced pressure, and the crude product was purified by column separation (PE/EA= 10/1-3/1) to obtain compound **2d** (40.0 g, yield 43.8%).
¹H NMR (400 MHz, CDCl₃) δ 7.64 - 7.77 (m, 2 H), 7.27 - 7.38 (m, 13 H), 5.05 - 5.16 (m, 1 H), 4.50 - 4.59 (m, 4 H), 4.03 - 4.11 (m, 1 H), 3.62 - 3.76 (m, 2 H), 3.44 - 3.58 (m, 3 H), 3.25 - 3.33 (m, 2 H), 3.15 - 3.20 (m, 1 H), 2.42 (s, 3 H), 1.46 (s, 9 H)

### 3. Preparation of Compound 2e

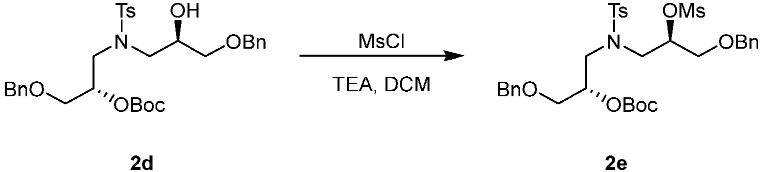

Compound **2d** (77.0 g, 128 mmol) and triethylamine (28.6 mL, 205 mmol) were sequentially added to dichloromethane (800 mL) in an ice-water bath, and methanesulfonyl chloride (24.2 g, 212 mmol) was slowly added dropwise. The reaction was performed at 25°C for 2 h until LCMS showed that the reaction was complete. The reaction mixture was washed with water (800 mL), and the organic phase was concentrated under reduced pressure to obtain crude compound **2e** (90.0 g).
m/z: ES+ [M+Na]+ 700.1
¹H NMR (400 MHz, CDCl₃) δ 7.68 (d, *J* = 8.4 Hz, 2 H), 7.28 - 7.39 (m, 12 H), 5.10 - 5.20 (m, 1 H), 4.93 - 5.02 (m, 1 H), 4.52 - 4.61 (m, 4 H), 3.72 - 3.88 (m, 2 H), 3.52 - 3.67 (m, 4 H), 3.27 - 3.36 (m, 1 H), 3.15 - 3.22 (m, 1 H), 3.04 (s, 3 H), 2.43 (s, 3 H), 1.43 (s, 9 H).

### 4. Preparation of Compound 2f

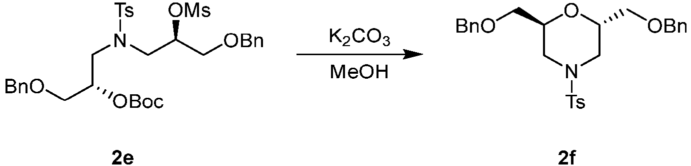

Compound **2e** (90.0 g, 133 mmol) and potassium carbonate (91.8 g, 664 mmol) were added to methanol (900 mL) at room temperature. The reaction was performed at 66°C for 2 h until TLC (PE/EA = 2/1, UV 254 nm) showed the formation of new spots. The organic phase was concentrated under reduced pressure. The reaction mixture was diluted with water (200 mL), and extracted with dichloromethane (200 mL × 3). The organic phase was concentrated under reduced pressure, and the crude product was purified by column separation (PE/EA = 30/1-2/1) to obtain compound **2f** (64.0 g, yield 99.9%).
¹H NMR (400 MHz, CDCl₃) δ 7.62 (d, *J =* 8.4 Hz, 2 H), 7.27 - 7.39 (m, 12 H), 4.48 - 4.62 (m, 4 H), 3.93 - 4.08 (m, 2 H), 3.55 - 3.69 (m, 4 H), 2.84 - 3.10 (m, 4 H), 2.44 (s, 3 H)

### 5. Preparation of Compound 2g

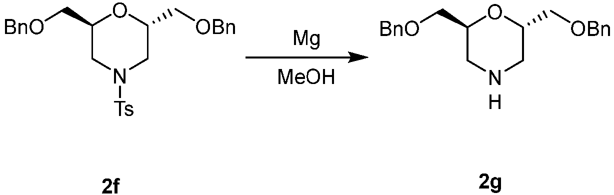

Compound **2f** (69.0 g, 143 mmol) and magnesium turnings (54.7 g, 2.28 mol) were added to methanol (400 mL) at room temperature. The reaction was performed at 66°C for 1 h until TLC (DCM/MeOH = 10/1, UV 254 nm) showed that the starting material reaction was complete and new spots were formed. The reaction mixture was diluted with water (3000 mL) and saturated aqueous ammonium chloride solution (3000 mL), and extracted with dichloromethane (1000 mL × 3). The organic phase was washed with saturated sodium bicarbonate (300 mL × 3) and concentrated under reduced pressure to obtain crude compound **2g** (32.0 g).
¹H NMR (400 MHz, CDCl₃) δ 7.27 - 7.40 (m, 10 H), 4.57 (s, 4 H), 3.90 - 4.00 (m, 2 H), 3.59 - 3.69 (m, 4 H), 2.77 - 3.04 (m, 4 H)

### 6. Preparation of Compound 2h

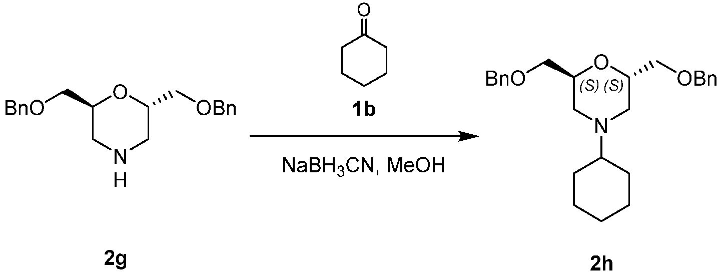

Compound **2g** (3.00 g, 9.16 mmol), compound **1b** (1.90 mL, 18.3 mmol), and acetic acid (1.01 mL, 18.3 mmol) were added to methanol (30 mL) at room temperature. The reaction was performed at 25°C for 18 h, followed by the addition of sodium cyanoborohydride (2.30 g, 36.7 mmol) for further reaction at 50°C for 4 h until TLC (DCM/MeOH= 10/1) showed the formation of new spots. The reaction mixture was diluted with water (50 mL), and extracted with dichloromethane (30 mL × 3). The organic phase was concentrated under reduced pressure, and the crude product was purified by column chromatography (dichloromethane/methanol = 99/1-5/1) to obtain compound **2h** (3.00 g, yield 79.9%).
¹H NMR (400 MHz, CDCl₃) δ 7.28 - 7.41 (m, 10 H), 4.51 - 4.71 (m, 4 H), 4.00 - 4.35 (m, 2 H), 3.49 - 3.77 (m, 4 H), 2.69 - 2.98 (m, 2 H), 1.63 - 2.12 (m, 7 H), 1.16 - 1.44 (m, 6 H)

### 7. Preparation of Compound 2i

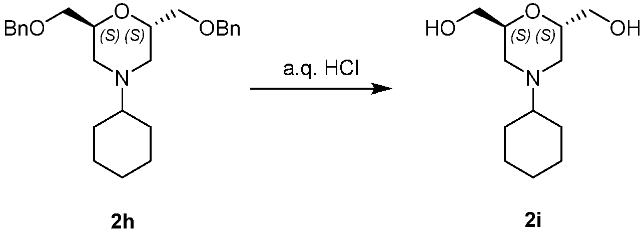

Compound **2h** (3.00 g, 7.32 mmol) was added to concentrated hydrochloric acid (10 mL, 12 M) at room temperature. The reaction was performed at 50°C for 18 h until TLC (DCM/MeOH = 10/1) showed the formation of new spots. The reaction mixture was concentrated under reduced pressure to obtain crude compound **2i** (2.00 g).
¹H NMR (400 MHz, CD₃OD) δ 4.27 - 4.38 (m, 1 H), 4.11 - 4.20 (m, 1 H), 3.96 - 4.04 (m, 2 H), 3.60 - 3.68 (m, 3 H), 3.36 - 3.43 (m, 1 H), 3.19 - 3.29 (m, 2 H), 3.11 (s, 1 H), 2.02 - 2.17 (m, 2 H), 1.90 - 2.00 (m, 2 H), 1.69 - 1.78 (m, 1 H), 1.57 - 1.69 (m, 1 H), 1.35 - 1.52 (m, 3 H), 1.20 - 1.31 (m, 1 H)

### 8. Preparation of Compound 2j

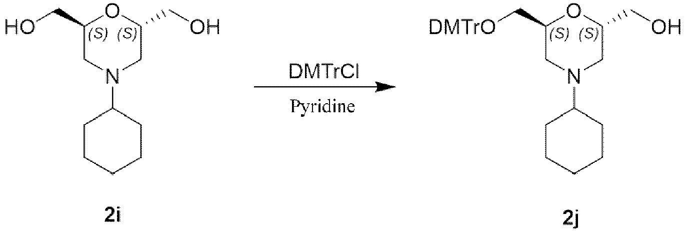

Compound **2i** (2.00 g, 8.72 mmol) was dissolved in pyridine (40 mL) at room temperature, and then DMTrCl (2.96 g, 8.72 mmol) was added. The reaction was performed at 25°C for 18 h until TLC (PE/EA = 1/1, UV 254 nm) showed that the reaction was complete. The reaction mixture was concentrated under reduced pressure, diluted with saturated aqueous ammonium chloride solution (200 mL), and extracted with DCM (100 mL × 3). The organic phase was concentrated under reduced pressure, and the crude product was purified by column chromatography (dichloromethane/methanol = 99/1-10/1) to obtain compound **2j** (1.40 g, yield 30.2%).

### 9. Preparation of Compound E2

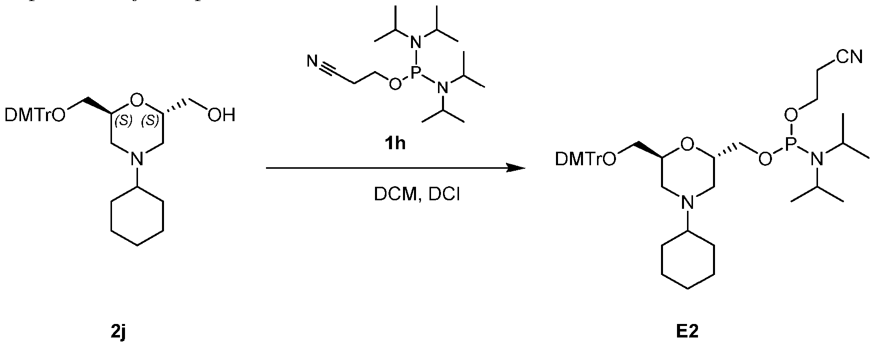

Compound **2j** (660 mg, 1.24 mmol), compound **1h** (374 mg, 1.24 mmol), and DCI (73.3 mg, 0.621 mmol) were added to DCM (10 mL). The reaction was performed at 25°C for 1 h until TLC (PE/EA = 5/1, PMA) showed that the starting material reaction was complete. The reaction mixture was diluted with saturated sodium bicarbonate (50 mL), and extracted with DCM (30 mL × 3). The organic phase was concentrated under reduced pressure, and the crude product was separated on a column (PE/EA = 99/1-30/1) to obtain compound **E2** (350 mg, yield 19.3%).
m/z: ES+ [M+H]+ 732.2
¹H NMR (400 MHz, CDCl₃) δ 7.40 - 7.50 (m, 2 H), 7.28 - 7.37 (m, 6 H), 7.21 (d, *J* = 7.2 Hz, 1 H), 6.77 - 6.89 (m, 4 H), 3.67 - 4.07 (m, 11 H), 3.56 - 3.66 (m, 2 H), 3.04 - 3.33 (m, 2 H), 2.30 - 2.96 (m, 6 H), 2.10 - 2.28 (m, 1 H), 1.63 - 1.88 (m, 4 H), 1.47 - 1.52 (m, 1 H), 1.24 - 1.31 (m, 3 H), 1.15 - 1.23 (m, 12 H), 1.11 - 1.14 (m, 1 H)

### Example 16 Preparation of Compound E1-1

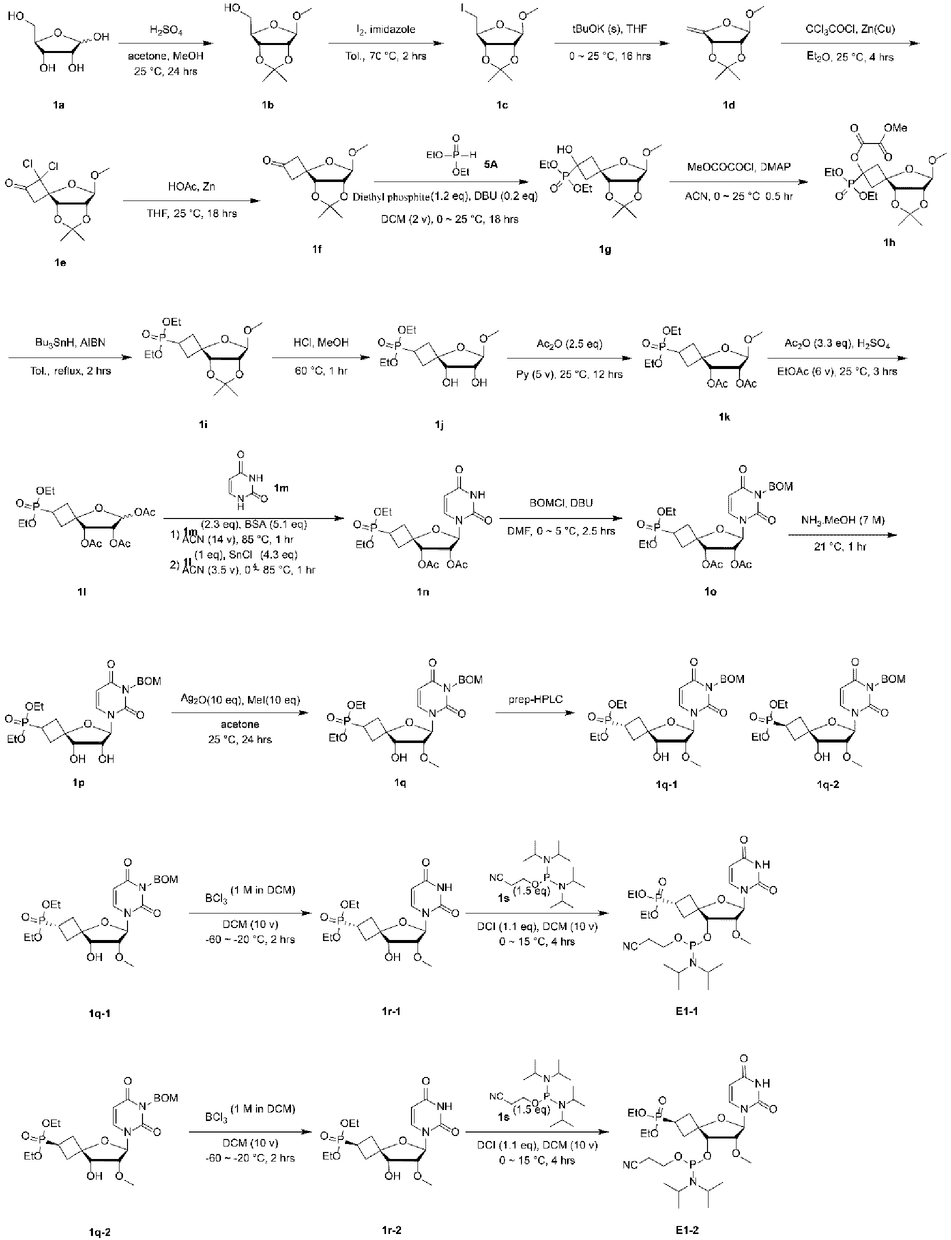

### 1. Preparation of Compound 1b

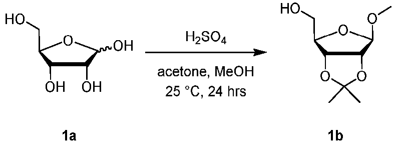

Compound **1a** (200 g, 1.33 mol, 1.00 eq) was suspended in a mixed solution of anhydrous acetone (1.00 L) and anhydrous methanol (1.00 L). Concentrated sulfuric acid (20.0 mL, 0.27 eq) was added dropwise, and the mixture was allowed to react at 25°C for 24 h. The reaction mixture was neutralized with saturated sodium bicarbonate and concentrated. The residue was dissolved in ethyl acetate and washed three times with saturated brine (500 mL). The organic phase was dried over anhydrous sodium sulfate and concentrated to obtain compound **1b** (242 g, 88.9%).

**¹H NMR:** 400 MHz CDCl₃ *δ* 4.97 (s, 1H), 4.84 (d, *J =* 6.0 Hz, 1H), 4.59 (d, *J =* 6.0 Hz, 1H), 4.44-4.43 (m, 1H), 3.72-3.59 (m, 2H), 3.44 (s, 3H), 1.49 (s, 3H), 1.32 (s, 3H).

### 2. Preparation of Compound 1c

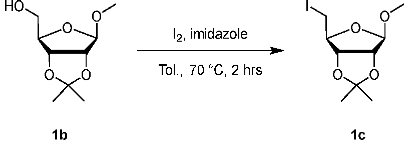

Compound **1b** (310 g, 1.52 mol, 1.00 eq), imidazole (206 g, 3.02 mol, 2.00 eq), and triphenylphosphine (476 g, 1.82 mol, 1.20 eq) were dissolved in toluene (2.1 L), and elemental iodine (446 g, 1.76 mmol, 1.16 eq) was added in portions at room temperature. The mixture was heated up to 70°C and stirred for 1.5 h until TLC showed that the reaction was complete. The reaction mixture was quenched with methanol (60 mL). After cooling, saturated aqueous sodium thiosulfate solution (2.1 L) was added. The organic phase was separated and collected, washed twice with saturated brine (1.5 L), dried over anhydrous sodium sulfate, filtered, and concentrated. The residue was pulped with methyl tert-butyl ether and filtered. The filtrate was concentrated, and the obtained crude product was purified by silica gel column chromatography (eluent: petroleum ether/ethyl acetate) to obtain compound **1c** (401 g, 1.27 mol, 91.1%).

**¹H NMR:** 400 MHz CDCl₃ *δ* 4.98 (s, 1H), 4.69 (d, J = 6.0 Hz, 1H), 4.56 (d, J = 6.0 Hz, 1H), 4.39-4.35 (m, 1H), 3.30 (s, 3H), 3.24-3.20 (m, 1H), 3.09 (t, J = 10.0 Hz, 1H) , 1.41 (s, 3H), 1.26 (s, 3H).

### 3. Preparation of Compound 1d

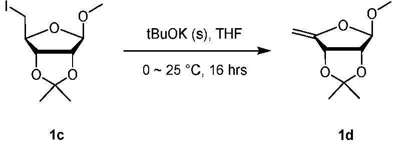

Compound **1c** (203 g, 646 mmol, 1.00 eq) was dissolved in tetrahydrofuran (2.03 L), and potassium tert-butoxide (145 g, 1.29 mol, 1.29 eq) was added in portions at 0°C. The mixture was stirred at 25°C for 16 h and then cooled to 5°C. The mixture was quenched with ice water (1.1 L), extracted with methyl tert-butyl ether (2.0 L), washed with saturated brine (2.0 L), dried over anhydrous sodium sulfate, filtered, and concentrated. The obtained crude product was purified by silica gel column chromatography (eluent: petroleum ether/ethyl acetate) to obtain compound 1d (160 g, 91.3%).

**¹H NMR:** 400 MHz CDCl₃ *δ* 5.11 (s, 1H), 5.02 (d, J = 5.6 Hz, 1H), 4.06 (d, J = 1.6 Hz, 1H), 4.50 (d, J = 5.6 Hz, 1H), 4.39 (d, J = 1.6 Hz, 1H), 3.41 (s, 3H), 3.24-3.20 (m, 1H), 3.09 (t, J = 10.0 Hz, 1H) , 1.47 (s, 3H), 1.35 (s, 3H).

### 4. Preparation of Compound 1e

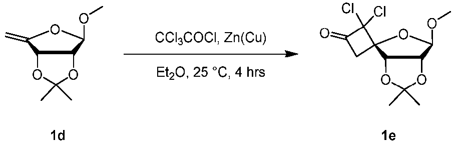

A zinc-copper couple (90.0 g, 1.37 mol, 5.50 eq) was dispersed in diethyl ether (200 mL). Compound **1d** (60 g, 241 mmol, 1.00 eq) was added at 25°C, and a solution of trichloroacetyl chloride (61.5 g, 338 mmol, 1.40 eq) in diethyl ether (200 mL) was added dropwise. The mixture was stirred for 1 h. After filtration, the filter cake was washed with methyl tert-butyl ether (500 mL), and the filtrate was poured into a saturated aqueous sodium bicarbonate solution (2.50 L) and filtered. The filtrate was washed three times with saturated brine (500 mL), dried over anhydrous sodium sulfate, filtered, and concentrated to obtain crude compound **1e** (71.8 g).

**¹H NMR:** 400 MHz CDCl₃ *δ* 5.12 (d, J = 5.6 Hz, 1H), 5.09 (s, 1H), 4.70 (d, J = 6.0 Hz, 1H), 3.70-3.55 (m, 2H), 3.54 (s, 3H), 1.45 (s, 3H), 1.36 (s, 3H).

### 5. Preparation of Compound 1f

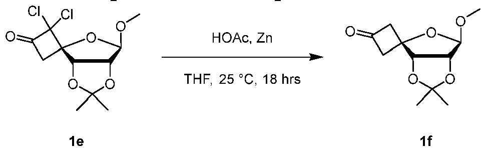

Compound **1e** (71.8 g, 242 mmol, 1.00 eq) was dissolved in tetrahydrofuran (1.60 L). Glacial acetic acid (69.1 mL, 1.20 mol, 5.00 eq) was added, and zinc powder (142 g, 2.17 mol, 9.00 eq) was added in portions. The mixture was stirred at 25°C for 18 h. After the reaction was complete, the reaction mixture was filtered, and the filtrate was concentrated to obtain a crude product. The crude product was dissolved in methyl tert-butyl ether, poured into a saturated aqueous sodium bicarbonate solution (2.0 L), and filtered. The filtrate was washed twice with saturated brine (500 mL), dried over anhydrous sodium sulfate, filtered, and concentrated. The obtained crude product was purified by silica gel column chromatography (eluent: petroleum ether/ethyl acetate/dichloromethane) to obtain compound **1f** (31 g, 56.3% yield in two steps).

**¹H NMR:** 400 MHz CDCl₃ *δ* 4.97 (s, 1H), 4.70-4.67 (m, 2H), 3.54-3.48 (m, 2H), 3.37-3.31 (m, 4H), 3.16-3.10 (m, 1H), 3.09-3.03 (m, 1H), 1.43 (s, 3H), 1.34 (s, 3H).

### 6. Preparation of Compound 1g

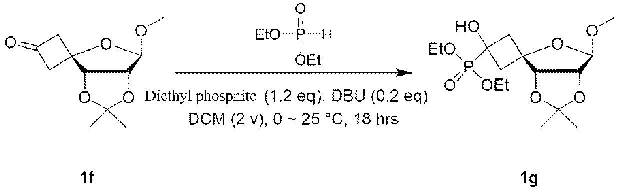

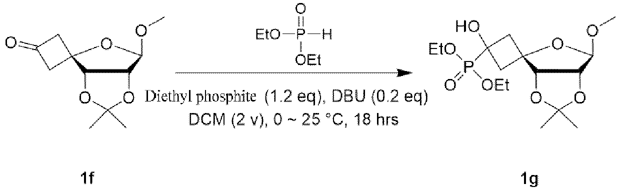

Compound **1f** (50 g, 219 mmol, 1.00 eq) and diethyl phosphite (33.2 g, 240 mmol, 1.20 eq) were dissolved in dichloromethane (100 mL); 1,8-diazabicycloundec-7-ene (DBU, 6.67 g, 43.8 mmol, 0.20 eq) was added at 0°C and stirred at 25°C for 18 h. The reaction mixture was washed three times with saturated NH₄Cl (100 mL) and once with saturated brine (100 mL), dried over anhydrous sodium sulfate, and concentrated. The obtained crude product was purified by silica gel column chromatography (eluent: petroleum ether/dichloromethane) to obtain compound **1g** (70 g, 87.2%).

**¹H NMR:** 400 MHz CDCl₃ *δ* 5.27 (s, 1H), 5.01 (brs, 1H), 4.78 (s, 1H), 4.65 (d, J = 5.6 Hz, 1H), 4.49 (d, J = 6.0 Hz, 1H), 4.20-4.05 (m, 5H), 3.31 (s, 1H), 3.29-3.05 (m, 1H), 2.75-2.68 (m, 1H), 2.50-2.40 (m, 1H), 2.35-2.25 (m, 1H), 1.36-1.25 (m, 12 H).

### 7. Preparation of Compound 1h

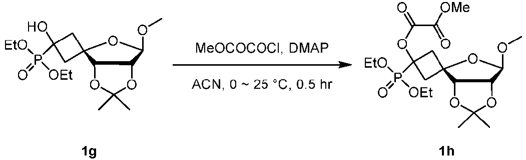

Compound **1g** (80.2 g, 219 mmol, 1.00 eq) and DMAP (40.1 g, 328 mmol, 1.50 eq) were dissolved in acetonitrile (562 mL). Methyl oxalyl chloride (40.2 g, 328 mmol, 1.50 eq) was added at 5°C. The mixture was stirred at 25°C for half an hour. The reaction mixture was concentrated. The obtained crude product was dissolved in ethyl acetate, washed five times with saturated ammonium chloride (300 mL), once with water (200 mL), and once with brine (200 mL), dried over anhydrous sodium sulfate, filtered, and concentrated to obtain crude compound **1h** (99 g).

**¹H NMR:** 400 MHz CDCl₃ *δ* 4.88-4.80 (m, 2H), 4.67-4.53 (m, 2H), 4.24-4.12 (m, 4H), 3.89 (s, 3H), 3.51-3.43 (m, 1H), 3.35-3.33 (m, 3H), 3.15-3.07 (m, 1H), 2.92-2.80 (m, 1H), 2.69-2.61 (m, 1H), 1.39-1.29 (m, 12H).

### 8. Preparation of Compound 1i

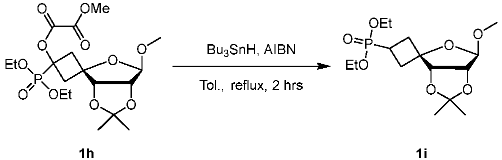

Compound **1h** (99 g, 218 mmol, 1.00 eq) was dissolved in anhydrous toluene (1.00 L). Tri-n-butyltin hydride (76.4 g, 262 mmol, 1.20 eq) and azobisisobutyronitrile (AIBN, 1.08 g, 6.56 mmol, 0.03 eq) were added. The mixture was refluxed for 2 h. The reaction mixture was concentrated. The obtained crude product was purified by silica gel column chromatography (eluent: petroleum ether/ethyl acetate) to obtain crude compound **1i** (146 g).

**¹H NMR:** 400 MHz CDCl₃ *δ* 4.75-4.49 (m, 3H), 4.05-3.95 (m, 4H), 3.26-3.24 (m, 3H), 2.76-2.07 (m, 5H), 1.39-1.29 (m, 12H).

### 9. Preparation of Compound 1j

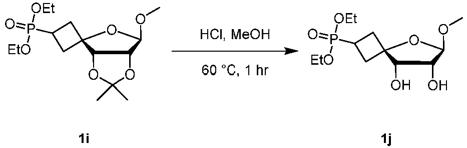

Compound **1i** (141 g, 201 mmol, 1.00 eq) was dissolved in methanol (1.41 L). An aqueous solution of HCl (704 mL, 1.40 mol, 2 M, 7.00 eq) was added and stirred at 60°C for 1 h. The reaction mixture was extracted with a mixture of methyl tert-butyl ether/petroleum ether. The aqueous phase was collected, adjusted to pH 8 with saturated sodium bicarbonate, and concentrated. Tetrahydrofuran was added to the residue and filtered. The filtrate was concentrated to obtain crude compound **1j** (62.4 g, 201 mmol).

### 10. Preparation of Compound 1k

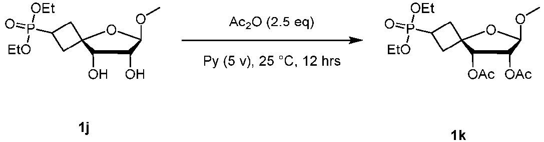

Compound **1j** (62.4 g, 201 mmol, 1.00 eq) was dissolved in pyridine (300 mL). Acetic anhydride (47.4 mL, 502 mmol, 2.50 eq) was added and stirred at 25°C for 12 h. The reaction mixture was diluted with saturated sodium bicarbonate (1.00 L), extracted twice with ethyl acetate (600 mL), dried over anhydrous sodium sulfate, filtered, and concentrated. The obtained crude product was purified by silica gel column chromatography (eluent: petroleum ether/ethyl acetate) to obtain compound **1k** (74 g, 93.3%)

**¹H NMR:** 400 MHz CDCl₃ *δ* 5.38-5.10 (m, 3H), 4.07-4.03 (m, 4H), 3.36-3.32 (m, 3H), 2.41-2.05 (m, 5H), 2.04-1.98 (m, 9H), 1.27-1.23 (m, 6H).

### 11. Preparation of Compound 1l

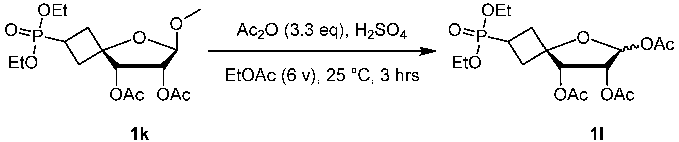

Compound **1k** (79.3 g, 201 mmol, 1.00 eq) was dissolved in ethyl acetate (476 mL). Acetic anhydride (62.6 mL, 663 mmol, 3.30 eq) and concentrated sulfuric acid (5.38 mL, 100 mmol, 0.50 eq) were added. The mixture was stirred at 25°C for 3 h. The reaction mixture was neutralized with saturated aqueous sodium bicarbonate solution, and extracted twice with ethyl acetate (1.00 L). The organic phases were combined, washed twice with saturated brine (500 mL), dried over anhydrous sodium sulfate, filtered, and concentrated. The crude product was purified by silica gel column chromatography to obtain compound **1l** (43 g, 50.6%).

**¹H NMR:** 400 MHz CDCl₃ *δ* 6.11 (d, J = 2.8 Hz, 1H), 5.44-5.39 (m, 1H), 5.33-5.32 (m, 1H), 4.10-4.05 (m, 5H), 2.80-2.35 (m, 4H), 2.12-2.02 (m, 9H), 1.32-1.23 (m, 6H).

### 12. Preparation of Compound 1n

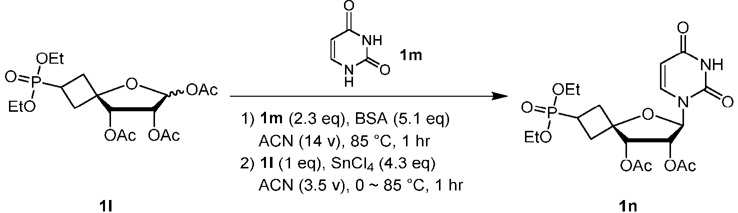

Compound **1m** (8.55 g, 76.2 mmol, 2.30 eq) and bis(trimethylsilyl)acetamide (BSA, 34.3 g, 169 mmol, 5.10 eq) were suspended in acetonitrile (200 mL) and stirred at 85°C for 1 h. After cooling, a solution of compound **1l** (14.0 g, 33.1 mmol, 1.00 eq) in acetonitrile (50.0 mL) was added, followed by tin tetrachloride (37.1 g, 142 mmol, 4.30 eq). The mixture was stirred at 25°C for 15 min and then stirred in an oil bath at 85°C for 45 min. After cooling, the reaction mixture was poured into a saturated aqueous sodium bicarbonate solution (1.50 L), and extracted three times with dichloromethane (700 mL). The organic phases were combined, washed with saturated brine (500 mL), dried over anhydrous sodium sulfate, filtered, and concentrated. The crude product was purified by silica gel column chromatography (eluent: dichloromethane/methanol) to obtain compound **1n** (14.4 g, 84.3%).

**¹H NMR:** 400 MHz CDCl₃ *δ* 9.70 (brs, 0.46H), 9.60 (brs, 0.60H), 7.17 (d, J = 8.0 Hz, 0.60H), 7.12 (d, J = 8.0 Hz, 0.49H), 5.98 (d, J = 6.4 Hz, 0.43H), 5.93 (d, J = 5.2 Hz, 0.55H), 5.78 (dt, J1 = 8.0 Hz, J2 = 2.0 Hz, 1H), 5.50-5.45 (m, 2H), 4.45-4.02 (m, 5H), 2.77-2.42 (m, 6H), 2.18 (s, 1.22H), 2.16 (s, 1.74 H), 2.04 (s, 1.31H), 2.02 (s, 1.67H), 1.32-1.28 (m, 6H).

### 13. Preparation of Compound 1o

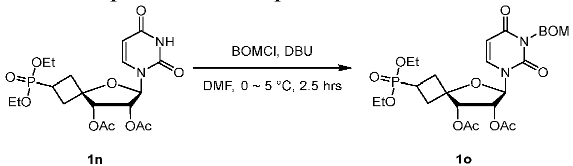

Compound **1n** (8.30 g, 17.4 mmol, 1.00 eq) was dissolved in DMF (40.0 mL). DBU (2.93 g, 19.2 mmol, 1.10 eq) was added, and benzyl chloromethyl ether (BOMCl, 3.01 g, 19.2 mmol, 1.10 eq) was added after cooling. The mixture was stirred at 0-5°C for 2.5 h. The reaction mixture was diluted with saturated ammonium chloride (160 mL), and extracted twice with dichloromethane (80.0 mL). The organic phases were combined, washed with saturated brine (80 mL), dried over anhydrous sodium sulfate, filtered, and concentrated. The crude product was purified by silica gel column chromatography (eluent: dichloromethane/methanol) to obtain compound **1o** (12.0 g, 92.3%).

**¹H NMR:** 400 MHz CDCl₃ *δ* 7.36-7.24 (m, 5H), 7.15-7.09 (m, 1H), 5.96-5.92 (m, 1H), 5.81-5.79 (m, 1H), 5.52-5.42 (m, 4H), 4.67 (s, 2H), 4.15-4.05 (m, 4H), 2.80-2.45 (m, 5H), 2.19-2.17 (m, 3H), 2.05-2.03 (m, 3H), 1.34-1.30 (m, 6H).

### 14. Preparation of Compound 1p

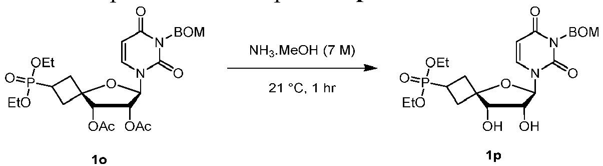

Compound **1o** (10.4 g, 17.4 mmol, 1.00 eq) was dissolved in ammonia methanol solution (29.9 mL, 7 M) and stirred at 21°C for 1 h. The crude product was obtained by concentration and purified by silica gel column chromatography (eluent dichloromethane/methanol) to obtain compound **1p** (8.00 g, 62.7%).

**¹H NMR:** 400 MHz CDCl₃ *δ* 7.34-7.06 (m, 5H), 5.83 (s, 0.52H), 5.71 (d, J = 8.4 Hz, 0.41H), 5.661 (d, J = 8.4 Hz, 0.53H), 5.60 (d, J = 2.0 Hz, 0.59H), 5.53 (d, J = 5.2 Hz, 0.41H), 5.42-5.36 (m, 2H), 4.62 (s, 2H), 4.51 (d, J = 3.6 Hz, 0.45H), 3.72 (d, J = 2.8 Hz, 0.36H), 3.54 (d, J = 2.0 Hz, 0.48H), 2.93-2.40 (m, 5H), 2.24-2.13 (m, 1H), 1.27-1.23 (m, 6H).

### 15. Preparation of Compound 1q

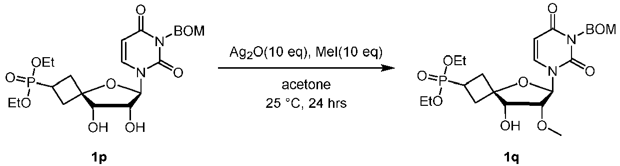

Compound **1p** (1 g, 1.95 mmol, 1.00 eq) was dissolved in acetone (19.6 mL). Silver oxide (3.63 g, 15.6 mmol, 10.0 eq) and methyl iodide (1.22 mL, 19.5 mmol, 10.0 eq) were added and stirred at 25°C for 24 h. The reaction mixture was filtered and concentrated to obtain crude compound **1q** (1.36 g).

**¹H NMR:** 400 MHz CDCl₃ *δ* 5.11 (s, 1H), 5.02 (d, J = 5.6 Hz, 1H), 4.06 (d, J = 1.6 Hz, 1H), 4.50 (d, J = 5.6 Hz, 1H), 4.39 (d, J = 1.6 Hz, 1H), 3.41 (s, 3H), 3.24-3.20 (m, 1H), 3.09 (t, J = 10.0 Hz, 1H) , 1.47 (s, 3H), 1.35 (s, 3H).

### 16. Preparation of Compounds 1q-1 and 1q-2

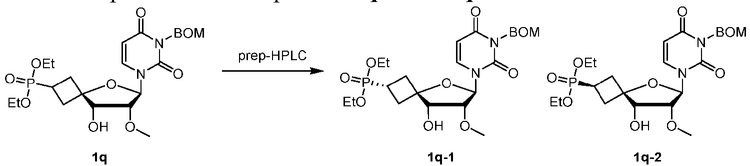

Crude compound **1q** (10.9 g, 20.7 mmol, 1.00 eq) was purified by C18 reversed-phase column chromatography (aqueous ammonium bicarbonate solution/acetonitrile) to obtain compound **1q-1** (1.50 g, 11.5%) and compound **1q-2** (1.50 g, 11.5%).

### Compound 1q-1:

**m/z:** ES+ [M+H]+ 525.2
**HPLC:** Retention time 0.820 min (Column: XBridge C18 2.1*50mm,5µm; Mobile phase: A: 10mM NH₄HCO₃ aqueous solution B: Acetonitrile; Gradient: 0-0.01 min 5% B, 0.01-0.7 min 5-95% B, 0.7-1.16 min 95% B, 1.16-1.5min, 95%-5% B; Flow rate: 1.5 mL/min; Column temp.: 40°C)
**¹H NMR:** 400 MHz CDCl₃ *δ* 7.37-7.08 (m, 5H), 7.09 (d, J = 8.0 Hz, 1H), 5.76-5.74 (m, 2H), 5.49-5.44 (m, 2H), 4.73-4.67 (m, 2H), 4.15-4.06 (m, 4H), 4.02 (d, J = 4.8 Hz, 1H), 3.87-3.85 (m, 1H), 3.56 (s, 3H), 2.81-2.64 (m, 2H), 2.50-2.39 (m, 2H), 2.36-2.27 (m, 1H), 1.34-1.30 (m, 6H).

### Compound 1q-2:

**m/z:** ES+ [M+H]+ 525.2
**HPLC:** Retention time 0.836 min (Column: XBridge C18 2.1*50mm, 5µm; Mobile phase: A: 10mM NH₄HCO₃ aqueous solution B: Acetonitrile; Gradient: 0-0.01 min 5% B, 0.01-0.7 min 5-95% B, 0.7-1.16 min 95% B, 1.16-1.5min, 95%-5% B; Flow rate: 1.5 mL/min; Column temp.: 40°C)
**¹H NMR:** 400 MHz CDCl₃ *δ* 7.37-7.27 (m, 5H), 7.08 (d, J = 8.0 Hz, 1H), 5.75-5.73 (m, 2H), 5.50-5.45 (m, 2H), 4.73-4.67 (m, 2H), 4.18-4.08 (m, 4H), 4.06 (d, J = 4.8 Hz, 1H), 3.88-3.86 (m, 1H), 3.56 (s, 3H), 3.06-2.95 (m, 1H), 2.83-2.41 (m, 5H), 1.34-1.31 (m, 6H).

### 17. Preparation of Compound 1r-1

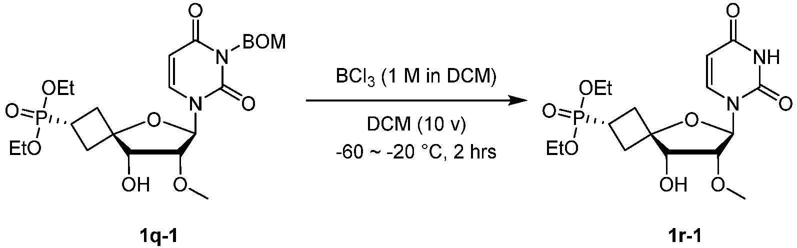

Compound **1q-1** (1.00 g, 1.90 mmol, 1.00 eq) was dissolved in anhydrous dichloromethane (DCM, 20.0 mL) and cooled to -60°C. Boron trichloride (7.62 mL, 7.62 mmol, 1 M in dichloromethane, 4.00 eq) was added and stirred at -20°C for 1 h. The reaction mixture was quenched with absolute ethanol (10.0 mL), neutralized to about pH 7 with concentrated ammonia, and concentrated. The crude product was added to a mixed solution of dichloromethane and ethanol and filtered. The filtrate was concentrated and the residue was purified by C18 reversed-phase column chromatography (aqueous ammonium bicarbonate solution/acetonitrile) to obtain compound **1r-1** (400 mg, 51.9%).
**m/z:** ES+ [M+H]+ 405.3
**¹H NMR:** 400 MHz CDCl₃ *δ* 8.51-8.43 (m, 1H), 7.15 (d, J = 8.4 Hz, 1H), 5.78 (d, J = 3.2 Hz, 1H), 5.76 (dd, J1 = 8.0 Hz, J2 = 2.4 Hz, 1H), 4.16-4.07 (m, 5H), 3.94 (dd, J1 = 4.8 Hz, J2 = 3.2 Hz, 1H), 3.55 (s, 3H), 2.96-2.94 (m, 1H),2.79-2.66 (m, 2H), 2.52-2.41 (m, 2H), 2.35-2.26 (m, 1H), 1.34-1.31 (m, 6H).

### 18. Preparation of Compound E1-1

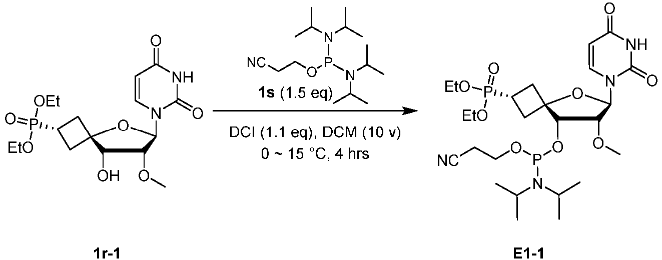

Compound **1r-1** (500 mg, 1.23 mmol, 1.00 eq) was dissolved in dichloromethane (5.00 mL). Compound **1s** (0.59 mL, 1.85 mmol, 1.50 eq) was added, and 4,5-dicyanoimidazole (160 mg, 1.36 mmol, 1.10 eq) was added after cooling to 0°C and stirred at 15°C for 4 h. The reaction mixture was quenched with saturated sodium bicarbonate (10.0 mL) and extracted twice with dichloromethane (10.0 mL). The crude product was obtained by concentration and purified by silica gel column chromatography (eluent: dichloromethane/acetone) to obtain compound **E1-1** (530 mg, 70.9%).

**¹H NMR:** 400 MHz CDCl₃ *δ* 9.17 (brs, 1H), 7.24 (d, J = 8.4 Hz, 1H), 5.83-5.79 (m, 1H), 5.64-5.61 (m, 1H), 4.43-4.33 (m, 1H), 4.09-3.62 (m, 9H), 3.43-3.38 (m, 3H), 2.86-2.66 (m, 3H), 2.57-2.21 (m, 4H), 1.28-1.18 (m, 18H).

### Example 17 Preparation of Compound E1

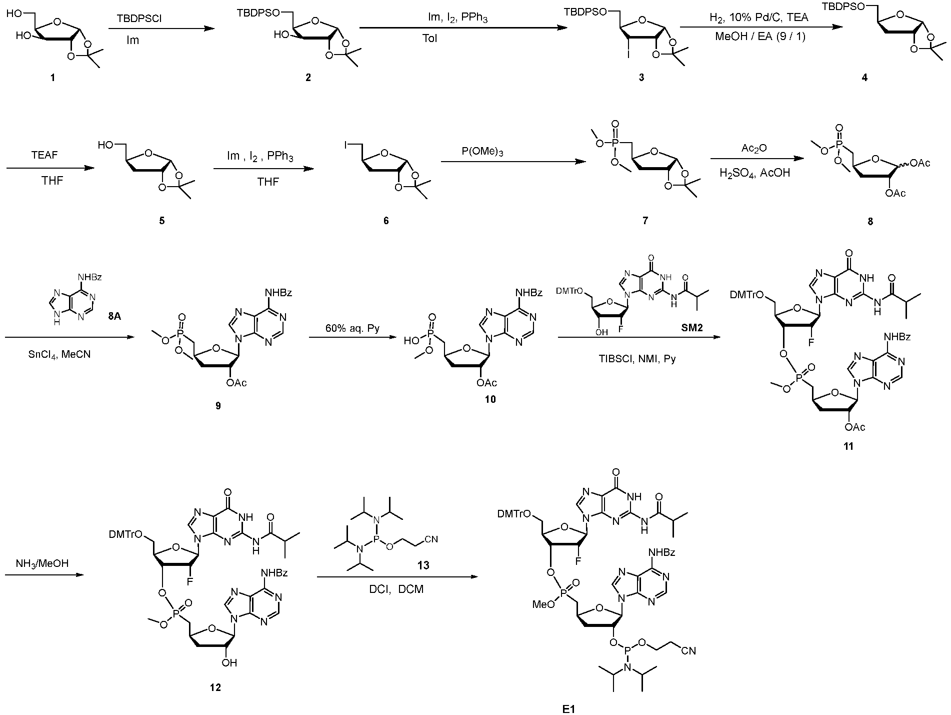

### 1. Preparation of Compound 2

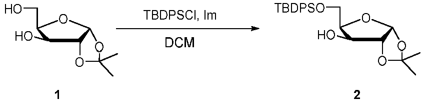

Compound **1** (50.0 g, 263 mmol) was dissolved in DCM (800 mL) at 25°C. Imidazole (26.9 g, 394 mmol) and TBDPSCl (75.2 mL, 289 mmol) were added in sequence. The reaction mixture was stirred at 25°C for 18 h until TLC (DCM/MeOH=1011, PE/EA=3/1) showed complete consumption of reactants and formation of new spots. The reaction mixture was spin-dried to obtain a crude product. The crude product was purified by MPLC (PE/EA=1/0-5/1) to obtain compound **2** (95.0 g, yield 84.31%) as a colorless oily liquid.

¹H NMR (400 MHz, CDCl₃) *δ* 7.70 (dd, *J =* 7.6, 1.6 Hz, 4 H), 7.35 - 7.46 (m, 6 H), 5.86 (d, *J =* 3.6 Hz, 1 H), 4.61 (dd, *J =* 4.8, 4.0 Hz, 1 H), 4.15 (td, *J* = 8.8, 5.2 Hz, 1 H), 3.93 - 4.01 (m, 1 H), 3.81 - 3.92 (m, 2 H), 1.60 (s, 3 H), 1.39 (s, 3 H), 1.06 (s, 9 H).

### 2. Preparation of Compound 3

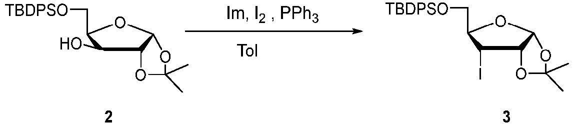

Compound **2** (95.0 g, 222 mmol) was dissolved in toluene (1.50 L) at 25°C. Imidazole (30.2 g, 443 mmol), triphenylphosphine (116 g, 443 mmol), and elemental iodine (84.4 g, 322 mmol) were added in sequence. The reaction mixture was stirred at 100°C for 18 h until TLC (PE/EA=5/1) showed complete consumption of reactants and formation of new spots. To the reaction mixture, 20.0 mL of saturated NaHSO₃ solution and 500 mL of water were added until the reaction mixture was separated into layers. The organic phase was washed with saturated NaCl solution (50.0 mL × 3), dried over anhydrous Na₂SO₄, and then spin-dried to obtain a crude product. The crude product was purified by MPLC (PE/EA=1/0-10/1) to obtain compound 3 (115 g, yield 96.35%) as a colorless oily liquid.

¹H NMR (400 MHz, CD₃OD) *δ* 7.64 - 7.72 (m, 4 H), 7.37 - 7.49 (m, 6 H), 5.95 (d, *J =* 3.6 Hz, 1 H), 5.06 (d, *J =* 3.6 Hz, 1 H), 4.42 (d, *J =* 3.2 Hz, 1 H), 3.87 (dd, *J* = 10.4, 5.6 Hz, 1 H), 3.66 (dd, *J* = 10.4, 6.4 Hz, 1 H), 3.53 (td, *J* = 6.0, 3.2 Hz, 1 H), 1.44 (s, 3 H), 1.29 (s, 3 H), 1.02 - 1.07 (m, 9 H).

### 3. Preparation of Compound 4

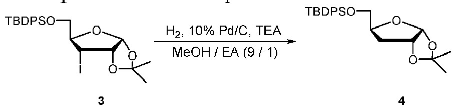

Compound 3 (12.5 g, 23.2 mmol) was dissolved in a mixed solvent of MeOH (225 mL) and EtOAc (25 mL) at 25°C. TEA (6.45 mL, 46.4 mmol) and Pd/C 10% (2.00 g, 18.8 mmol) were added, and the reaction mixture was stirred at 25°C under a hydrogen (14.696 psi) atmosphere for 14 h until TLC (PE/EA=10/1) showed complete consumption of reactants and formation of new spots. The reaction mixture was filtered, and the filtrate was spin-dried to obtain a crude product. The crude product was purified by MPLC (PE/EA=1/0-10/1) to obtain compound 4 (8.75 g, yield 83.06%) as a colorless oily liquid.

¹H NMR (400 MHz, CD₃OD) *δ* 7.64 - 7.72 (m, 4 H), 7.36 - 7.47 (m, 6 H), 5.80 (d, *J* = 3.6 Hz, 1 H), 4.77 (t, *J* = 4.4 Hz, 1 H), 4.25 - 4.32 (m, 1 H), 3.68 - 3.82 (m, 2 H), 2.00 (dd, J = 13.6, 4.8 Hz, 1 H), 1.78 - 1.87 (m, 1 H), 1.45 (s, 3 H), 1.30 (s, 3 H), 1.04 (s, 9 H).

### 4. Preparation of Compound 5

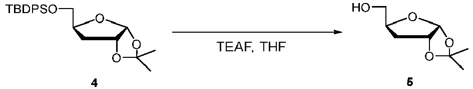

Compound **4** (35.0 g, 84.8 mmol) was dissolved in THF (500 mL) at 25°C. Tetraethylammonium fluoride (63.3 g, 424 mmol) was added, and the reaction mixture was stirred at 25°C for 18 h until TLC (PE/EA=10/1, DCM/MeOH=10/1) showed complete consumption of reactants and formation of new spots. The reaction mixture was concentrated under reduced pressure to obtain a crude product. The crude product was purified by MPLC (DCM/MeOH=1/0-10/1) to obtain compound **5** (12 g, yield 81.21%) as a white solid.

¹H NMR (400 MHz,CD₃OD) *δ* 5.79 (d, *J =* 3.6 Hz, 1 H), 4.77 (t, *J =* 4.0 Hz, 1 H), 4.21 - 4.28 (m, 1 H), 3.70 (dd, *J* = 12.0, 3.6 Hz, 1 H), 3.54 (dd, *J* = 12.0, 4.8 Hz, 1 H), 1.95 - 2.02 (m, 1 H), 1.74 (ddd, *J* = 13.2, 10.8, 4.8 Hz, 1 H), 1.46 (s, 3 H), 1.30 (s, 3 H).

### 5. Preparation of Compound 6

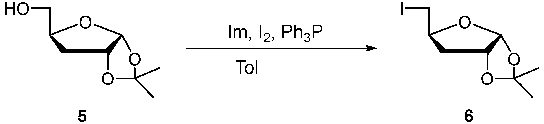

Compound **5** (12.0 g, 68.9 mmol) was dissolved in toluene (500 mL) at 25°C. Imidazole (9.38 g, 138 mmol), triphenylphosphine (36.1 g, 138 mmol), and elemental iodine (26.2 g, 103 mmol) were added in sequence. The reaction mixture was stirred at 100°C for 3 h until TLC (PE/EA=5/1) showed complete consumption of reactants and formation of new spots. To the reaction mixture, 100 mL of saturated NaHSO₃ solution and 100 mL of water were added until the reaction mixture was separated into layers. The organic phase was washed with saturated NaCl solution (100 mL × 3), dried over anhydrous Na₂SO₄, and then spin-dried to obtain a crude product. The crude product was purified by MPLC (PE/EA=1/0-10/1) to obtain compound **6** (16.6 g, yield 84.82%) as a white solid.

¹H NMR (400 MHz, CDCl₃) *δ* 5.88 (d, *J =* 3.6 Hz, 1 H), 4.77 (t, *J* = 4.2 Hz, 1 H), 4.13 - 4.21 (m, 1 H), 3.25 - 3.38 (m, 2 H), 2.31 (dd, *J* = 13.6, 4.4 Hz, 1 H), 1.61 - 1.67 (m, 1 H), 1.52 (s, 3 H), 1.33 (s, 3 H).

### 6. Preparation of Compound 7

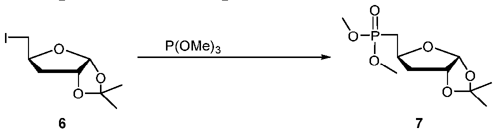

Compound 6 (40.0 g, 141 mmol) was dissolved in trimethyl phosphite (500 mL) at 25°C. The reaction mixture was stirred at 120°C for 11 h until TLC (ethyl acetate/acetone=3/1, PE/EA=10/1) showed that the starting material remained and new spots were formed. The reaction mixture was spin-dried to obtain a crude product. The crude product was purified by MPLC (ethyl acetate/acetone=1/0-20/1) to obtain compound 7 (8.90 g, yield 23.74%) as a pale yellow oily liquid and recover starting material compound 7 (30.0 g) as a white solid.

¹H NMR (400 MHz, CDCl₃) *δ* 5.81 (d, *J* = 4.0 Hz, 1 H), 4.74 (t, *J* = 4.4 Hz, 1 H), 4.40 - 4.52 (m, 1 H), 3.76 (dd, *J* = 10.8, 0.8 Hz, 6 H), 2.23 - 2.35 (m, 2 H), 1.95 - 2.07 (m, 1 H), 1.63 (ddd, *J* = 13.6, 10.8, 4.8 Hz, 1 H), 1.52 (s, 3 H), 1.32 (s, 3 H).

### 7. Preparation of Compound 8

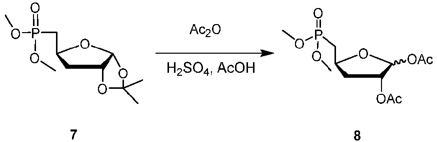

Compound **7** (13 g, 48.830 mmol), Ac₂O (23.036 mL, 244.150 mmol), and H₂SO₄ (2.615 mL, 48.830 mmol) were added in sequence to AcOH (260 mL). The reaction was allowed to proceed at 25°C for 6 h until TLC (ethyl acetate:acetone=3:1) detected new points. The reaction mixture was quenched with ice water, extracted with DCM (500 mL × 3), dried over anhydrous Na₂SO₄, filtered, and concentrated under reduced pressure to obtain a crude product. The crude product was purified by column chromatography (ethyl acetate:acetone=50:1-3:1) to obtain compound **8** (7.9 g, 25.464 mmol, 52.15%) as a yellow oil.

¹H NMR (400 MHz, CDCl₃) *δ* 6.10 (d, *J*=1.2 Hz, 1 H), 5.18 (d, *J*=5.2 Hz, 1 H), 4.58 - 4.72 (m, 1 H), 3.75 - 3.79 (m, 3 H), 3.71-3.74(m, 3H)2.16 - 2.38 (m, 3 H), 2.07 (s, 3 H), 2.05 (s, 3 H), 1.96 - 2.04 (m, 1 H).

### 8. Preparation of Compound 9

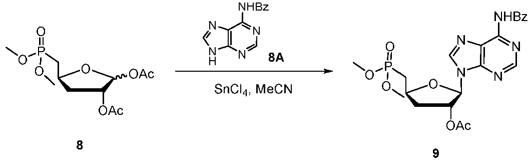

Compound **8** (7.9 g, 25.464 mmol) and compound **8A** (6.09 g, 25.464 mmol) were dissolved in CH₃CN (316 mL), followed by the slow dropwise addition of SnCl₄ (8.781 mL, 76.392 mmol) at 0°C. The reaction was allowed to proceed at 25°C for 2 h until TLC (ethyl acetate:acetone=10:1, PMA) showed that the starting material compound **8** disappeared and new spots were formed and LCMS (RW0006-267-P1A) showed 31.3% product generation. The reaction mixture was cooled to 0°C and adjusted to pH 8 with saturated aqueous NaHCO₃ solution. The aqueous phase was extracted with DCM (200 mL × 3). The organic phase was dried over anhydrous Na₂SO₄, filtered, and spin-dried to obtain a crude product. The crude product was purified by column chromatography (ethyl acetate:acetone=20:1-3:1) to obtain compound **9** (6.1 g, 12.464 mmol, 48.95%) as a yellow oil.

¹H NMR (400 MHz, CDCl₃) *δ* 8.75 - 8.84 (m, 1 H), 8.17 (s, 1 H), 8.01 - 8.10 (m, 2 H), 7.59 - 7.70 (m, 1 H), 7.50 - 7.57 (m, 2 H), 6.07 (s, 1 H), 5.66 - 5.73 (m, 1 H), 4.98 - 5.12 (m, 1 H), 4.70 - 4.81 (m, 1 H), 3.71 - 3.82 (m, 12 H), 2.70 - 2.81 (m, 1 H), 2.34 - 2.52 (m, 2 H), 2.21 - 2.27 (m, 1 H), 2.16 (s, 3 H), 2.06 (d, *J*=4.4 Hz, 4 H).

### 9. Preparation of Compound 10

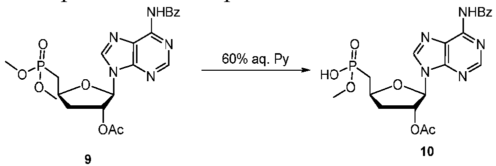

Compound **9** (6.1 g, 12.259 mmol) was dissolved in pyridine (30 mL) and water (20 mL). The reaction was allowed to proceed at 60°C for 12 h until TLC (DCM:MeOH=10:1) showed that the starting material disappeared. The reaction mixture was spin-dried to obtain a crude product. The crude product was purified by Prep-HPLC (MeCN/H₂O=30/1-80/1; flow rate: 30 mL/min) to obtain compound **10** (3.85 g, 8.098 mmol, 66.06%) as a yellow oil.

### 10. Preparation of Compound 11

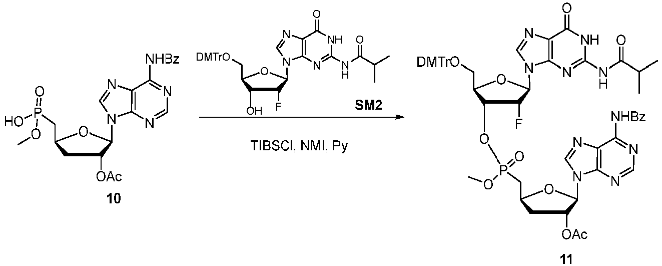

Compound **10** (3.7 g, 7.783 mmol) and compound **SM2** (10.24 g, 15.566 mmol) were dissolved in pyridine (37 mL). 2,4,6-Triisopropylbenzenesulfonyl chloride (14.14 g, 46.698 mmol) was added at 0°C, and *N*-methylimidazole (5.11 g, 62.263 mmol) was slowly added dropwise to the reaction mixture after reaction for 60 min at 0°C. The reaction was allowed to proceed at 25°C for 13 h until LCMS (RW0006-284-P1C) showed product generation and TLC (DCM:MeOH=10:1, UV) showed new points. The reaction mixture was cooled to 0°C, quenched with 50 mL of saturated sodium carbonate solution, and separated into layers. The organic phase was diluted with 300 mL of DCM, washed with water (50 mL × 3) and saturated aqueous NaCl solution (50 mL × 1), dried over anhydrous Na₂SO₄, filtered, and spin-dried to obtain a crude product. The crude product was purified by column chromatography (DCM:MeOH=2%-4%, TEA) to obtain compound **11** (2100 mg, 1.883 mmol, 24.20%) as a brown oil.

### 11. Preparation of Compound 12

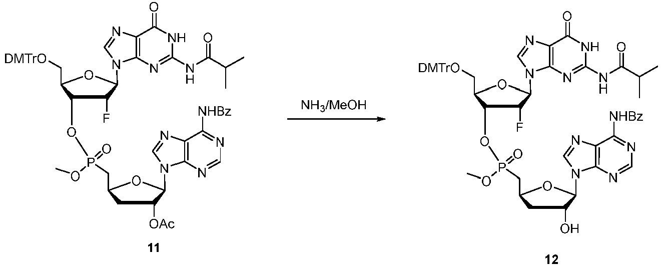

Compound **11** (2.4 g, 2.152 mmol) was dissolved in MeOH (36 mL). NH₃/MeOH (12.299 mL, 7M) was added and reacted at 0°C for 3 h until LCMS (RW0006-290-P1A) showed product generation. The reaction mixture was diluted with 150 mL of DCM and washed with 30 mL of water. The organic phase was dried over anhydrous Na₂SO₄, filtered, and spin-dried to obtain a crude product. The crude product was purified by Prep-HPLC (column: 01-Waters Xbridge BEH C18 19*150mm; mobile phase: TEAA-ACN; gradient: 33%-58.5%/15 min; flow rate: 15 mL/min; ) to obtain compound **12** (380 mg, 0.354 mmol, 16.45%) as a yellow solid.

### 12. Preparation of E1

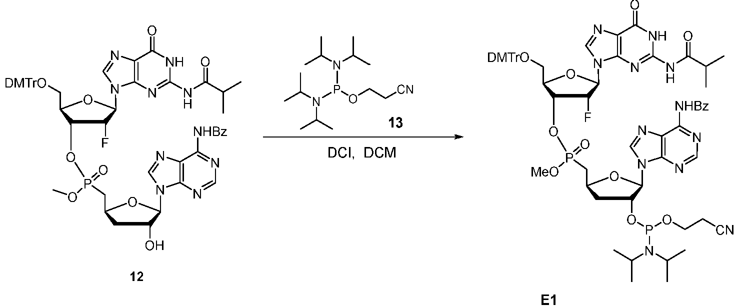

Compound **12** (380 mg, 0.354 mmol) was washed three times with acetonitrile, and dissolved in DCM (6.0 mL). 5A molecular sieve, DCI (41.82 mg, 0.354 mmol), and compound **13** (426.95 mg, 1.417 mmol) were added in sequence. The mixture was replaced with nitrogen three times and allowed to react at 25°C for 1 h until LCMS (RW00006-291-P1A) showed that 5.4% of the starting material remained. The reaction mixture was quenched with saturated aqueous NaHCO₃ solution containing 20 mL of ice, diluted with 30 mL of DCM, and separated into layers. The organic phase was washed with 20 mL of saturated aqueous NaHCO₃ solution and 20 mL of saturated aqueous NaCl solution. The organic phase was dried over anhydrous Na₂SO₄, filtered, and spin-dried to obtain a crude product. The crude product was purified by reversed-phase chromatography (acetonitrile/water: 20-80%, 30 min, 20 mL/min) to obtain **E1** (210 mg, 0.165 mmol, 46.57%) as a yellow solid.

¹H NMR (400 MHz, CDCl₃) *δ* 11.96 - 12.03(m, 1H), 10.07 - 10.20 (m, 1 H), 8.99 - 9.12 (m, 1 H), 8.62 - 8.80 (m, 1 H), 8.07 - 8.16 (m, 1 H), 8.01 - 8.05 (m, 2 H), 7.78 (d, *J*=1.2 Hz, 1 H), 7.60 - 7.66 (m, 1 H), 7.51 - 7.58 (m, 2 H), 7.21 - 7.26 (m, 2 H), 7.09 - 7.20 (m, 7 H), 6.66 - 6.75 (m, 4 H), 6.37 - 6.56 (m, 1 H), 5.75 - 6.11 (m, 3 H), 5.07 - 5.19 (m, 1 H), 4.60 - 4.74 (m, 1 H), 4.16 - 4.29 (m, 1 H), 3.81 - 3.89 (m, 1 H), 3.76 - 3.81 (m, 3 H), 3.70 (d, *J*=1.6 Hz, 7 H), 3.54 - 3.69 (m, 3 H), 3.05 - 3.15 (m, 1 H), 2.54 - 2.65 (m, 2 H), 2.03-2.53 (m, 5 H), 1.43 - 1.50 (m, 1 H), 1.14 - 1.21 (m, 11 H), 1.01 - 1.13 (m, 6 H) .

## Claims

1. A small interfering RNA (siRNA) for inhibiting the expression of apolipoprotein C3 (APOC3) in cells, the siRNA comprising a sense strand and an antisense strand forming a double-stranded region, wherein the length of the sense strand and the antisense strand is each independently 15-30 nucleotides, and the antisense strand comprises at least 15 contiguous nucleotides from a nucleotide sequence as set forth in any one of SEQ ID NOs: 414-826.

2. A siRNA according to claim 1, wherein the sense strand comprises at least 15 contiguous nucleotides from a nucleotide sequence as set forth in any one of SEQ ID NOs: 1-413.

3. A siRNA according to claim 1 or 2, wherein the double-stranded region is 15-25 base pairs in length, preferably 17-21 base pairs, more preferably 19 base pairs.

4. A siRNA according to any one of claims 1 to 3, wherein the siRNA comprises paired sense and antisense strand sequences as shown in Table 3.

5. A siRNA according to any one of claims 1 to 4, wherein the antisense strand comprises at least 15 contiguous nucleotides, at least 16 contiguous nucleotides, at least 17 contiguous nucleotides, at least 18 contiguous nucleotides, at least 19 contiguous nucleotides, or at least 20 contiguous nucleosides from a nucleotide sequence set forth in any one of SEQ ID NOs: 473, 612, 690, 757, 761, 816, 817, 818, 819, 820, 814, and 815; preferably, the antisense strand comprises a nucleotide sequence as set forth in any one of SEQ ID NOs: 473, 612, 690, 757, 761, 816, 817, 818, 819, 820, 814 and 815.

6. A siRNA according to any one of claims 1 to 3 and 5, wherein the sense strand comprises at least 15 contiguous nucleotides, at least 16 contiguous nucleotides, at least 17 contiguous nucleotides, at least 18 contiguous nucleotides, at least 19 contiguous nucleotides, or at least 20 contiguous nucleosides from a nucleotide sequence set forth in any one of SEQ ID NOs: 60, 199, 277, 344, 348, 403, 404, 405, 406, 407, 401, and 402, preferably the sense strand comprises a nucleotide sequence as set forth in any one of SEQ ID NOs: 60, 199, 277, 344, 348, 403, 404, 405, 406, 407, 401, and 402.

7. A siRNA according to any one of claims 1 to 6, wherein:
(a) the sense strand comprises a nucleotide sequence shown in SEQ ID NO: 60, and the antisense strand comprises a nucleotide sequence shown in SEQ ID NO: 473;
(b) the sense strand comprises a nucleotide sequence shown in SEQ ID NO: 199, and the antisense strand comprises a nucleotide sequence shown in SEQ ID NO: 612
(c) the sense strand comprises a nucleotide sequence shown in SEQ ID NO: 277, and the antisense strand comprises a nucleotide sequence shown in SEQ ID NO:690;
(d) the sense strand comprises a nucleotide sequence shown in SEQ ID NO: 344, and the antisense strand comprises a nucleotide sequence shown in SEQ ID NO: 757;
(e) the sense strand comprises a nucleotide sequence shown in SEQ ID NO: 348, and the antisense strand comprises a nucleotide sequence shown in SEQ ID NO: 761;
(f) the sense strand comprises a nucleotide sequence shown in SEQ ID NO: 403, and the antisense strand comprises a nucleotide sequence shown in SEQ ID NO: 816;
(g) the sense strand comprises a nucleotide sequence shown in SEQ ID NO: 404, and the antisense strand comprises a nucleotide sequence shown in SEQ ID NO: 817;
(h) the sense strand comprises a nucleotide sequence shown in SEQ ID NO: 405, and the antisense strand comprises a nucleotide sequence shown in SEQ ID NO: 818;
(i) the sense strand comprises a nucleotide sequence shown in SEQ ID NO: 406, and the antisense strand comprises a nucleotide sequence shown in SEQ ID NO: 819;
(j) the sense strand comprises a nucleotide sequence shown in SEQ ID NO: 407, and the antisense strand comprises a nucleotide sequence shown in SEQ ID NO: 820;
(k) the sense strand comprises a nucleotide sequence shown in SEQ ID NO: 401, and the antisense strand comprises a nucleotide sequence shown in SEQ ID NO: 814; or
(l) the sense strand comprises a nucleotide sequence shown in SEQ ID NO: 402, and the antisense strand comprises a nucleotide sequence shown in SEQ ID NO: 815.

8. A siRNA according to any one of claims 1 to 7, wherein substantially all nucleotides of the sense strand and substantially all nucleotides of the antisense strand are modified nucleotides, preferably all nucleotides of the sense strand and all nucleotides of the antisense strand are modified nucleotides.

9. A siRNA according to claim 8, wherein the sense strand and the antisense strand each independently comprise one or more nucleotide modifications selected from the group consisting of: a 2'-O-methyl modified nucleotide, a 2'-fluoro modified nucleotide, a 2'-deoxy modified nucleotide, an inosine ribonucleotide, an abasic nucleotide, an inverted abasic deoxyribonucleotide, a phosphorothioate internucleotide linkage modification, a vinylphosphonate modified nucleotide, a locked nucleotide, a 2'-amino modified nucleotide, a 2'-alkyl modified nucleotide, a morpholino nucleotide, a phosphoramidate, a non-natural base comprising nucleotide, and a terminal nucleotide linked to a cholesteryl derivative or a dodecanoic acid bisdecylamide group, and a deoxyribonucleotide.

10. A siRNA according to claim 8, wherein the sense strand and the antisense strand each independently comprise one or more nucleotide modifications selected from the group consisting of: a 2'-O-methyl modified nucleotide, a 2'-fluoro modified nucleotide, a 2'-deoxy modified nucleotide, an inosine ribonucleotide, an abasic nucleotide, an inverted abasic deoxyribonucleotide, and a phosphorothioate internucleotide linkage modification.

11. A siRNA according to claim 8, wherein the antisense strand comprises a modified nucleotide sequence set forth in any one of Table 5, and/or the sense strand comprises a modified nucleotide sequence set forth in any one of Table 4 of the specification.

12. A siRNA according to claim 8, wherein the siRNA comprises a paired modified sense strand sequence and modified antisense strand sequence set forth in any one of Table 6 of the specification.

13. A siRNA according to claim 8, wherein:
(1) the sense strand comprises 
STM1s-AmsAmAmAmGmGmGmAmCfAfGfUmAmUmUmCmUmCmAmGmAms-
STM1(SEQ ID NO: 1655),
 and the antisense strand comprises
(CP1a-U)sCfsUmGfAmGfAmAfUmAfCmUfGmUfCmCfCmUfUmsUfsUm(SEQ ID NO: 1651);
(2) the sense strand comprises 
STM1s-AmsAmUmUmAmAmAmAmGfGfGfAmCmAmGmUmAmUmUmCmAms-STM1
(SEQ ID NO: 1656),
 and the antisense strand comprises
(CP1a-U)sGfsAmAfUmAfCmUfGmUfCmCfCmUfUmUfUmAfAmsUfsUm (SEQ ID NO: 1650);
(3) the sense strand comprises 
STM1s-AmsAmUmUmAmAmAmAmGfGfGfAmCmAmGmUmAmUmUmCmAms-
STM1(SEQ ID NO: 1657),
 and the antisense strand comprises
(VPUm)sGfsAmAfUmAfCmUfGmUfCmCfCmUfUmUfUmAfAmsUfsUm (SEQ ID NO: 1649);
(4) the sense strand comprises IBs-AmsAmUmUmAmAmAmAmGfGfGfAmCmAmGmUmAmUmUmCmAms-IB (SEQ ID NO: 1658),
and the antisense strand comprises
(CP1a-U)sGfsAmAfUmAfCmUfGmUfCmCfCmUfUmUfUmAfAmsUfsUm(SEQ ID NO: 1650);
(5) the sense strand comprises UmsUmsAmAmAmAmGfGfGfAmCmAmGmUmAmUmUmCmsAm (SEQ ID NO: 1659), and the antisense strand comprises
(CP1a-U)sGfsAmAfUmAfCmUfGmUfCmCfCmUfUmUfUmAfAmsUfsUm(SEQ ID NO: 1650);
(6) the sense strand comprises
IBs-AmsAmAmAmGmGmGmAmCfAfGfUmAmUmUmCmUmCmAmGmAms-IB (SEQ ID NO: 1660),
and the antisense strand comprises
(CP1a-U)sCfsUmGfAmGfAmAfUmAfCmUfGmUfCmCfCmUfUmsUfsUm(SEQ ID NO: 1651);
(7) the sense strand comprises
AmsAmsGmGmGmAmCfAfGfUmAmUmUmCmUmCmAmGmsAm (SEQ ID NO: 1661),
and the antisense strand comprises
(CP1a-U)sCfsUmGfAmGfAmAfUmAfCmUfGmUfCmCfCmUfUmsUfsUm(SEQ ID NO: 1651);
(8) the sense strand comprises 
IBs-AmsAmAmAmGmGmGmAmCfAfGfUmAmUmUmCmUmCmAmGmAms-IBs-
GL6(SEQ ID NO: 1662),
 and the antisense strand comprises
(CP1a-U)sCfsUmGfAmGf(PCN-A)AfUmAfCmUfGmUfCmCfCmUfUmsUfsUm(SEQ ID NO:1652); or
(9) the sense strand comprises
AmsAmsGmGmGmAmCfAfGfUmAmUmUmCmUmCmAmGmsAm (SEQ ID NO: 1663),
and the antisense strand comprises
(CP1a-U)sCfsUmGfAmGf(PCN-A)AfUmAfCmUfGmUfCmCfCmUfUmsUfsUm(SEQ ID NO: 1652);
(a) the sense strand comprises CmsGmsAmGmGmAmUfGfCfCmUmCmCmCmUmUmCmUmUm, and the antisense strand comprises AmsAfsGmAfAmGfGmGfAmGfGmCfAmUfCmCfUmCfGmsUfsUm;
(b) the sense strand comprises CmsCmsGmUmUmAmAfGfGfAmCmAmAmGmUmUmCmUmUm, and the antisense strand comprises AmsAfsGmAfAmCfUmUfGmUfCmCfUmUfAmAfCmGfGmsUfsUm;
(c) the sense strand comprises CmsCmsGmUmUmAmAfGfGfAmCmAmAmGmUmUmCmUmUm, and the antisense strand comprises AmsAfsGmAfAmCfUmUfGmUfCmCfUmUfAmAfCmGfGmsUfsUm;
(d) the sense strand comprises CmsCmsAmAmGmUmCfCfAfCmCmUmGmCmCmUmAmUmUm, and the antisense strand comprises AmsAfsUmAfGmGfCmAfGmGfUmGfGmAfCmUfUmGfGmsUfsUm;
(e) the sense strand comprises AmsAmsGmGmGmAmCfAfGfUmAmUmUmCmUmCmAmGmUm , and the antisense strand comprises AmsCfsUmGfAmGfAmAfAfCmUfGmUfCmCfCmUfUmsUfsUm;
(f) the sense strand comprises UmsUmsAmAmAmAmGfGfGfAmCmAmGmUmAmUmUmCmUm, and the antisense strand comprises AmsGfsAmAfUmAfCmUfGmUfCmCfCmUfUmUfUmAfAmsUfsUm;
(g) the sense strand comprises AmsAmsGmGmGmAmCfAfGfUmAmUmUmCmUmCmAmGmUm, and the antisense strand comprises AmsCfsUmGfAmGfAmAfCTmAfCmUfGmUfCmCfCmUfUmsUfsUm;
(h) the sense strand comprises CmsCmsAmAmGmUmCfCfAfCmCmUmGmCmCmUmAmUmUm, and the antisense strand comprises AmsAfsUmAfGmGfCmAfGmGfUmGfGmAfCmUfUmGfGmsUfsUm;
(i) the sense strand comprises CmsCmsGmUmUmAmAfGfGfAmCmAmAmGmUmUmCmUmUm, and the antisense strand comprises AmsAfsGmAfAmCfUmUfGmUfCmCfUmUfAmAfCmGfGmsUfsUm;
(j) the sense strand comprises CmsGmsAmGmGmAmUfGfCfCmUmCmCmCmUmUmCmUmUm, and the antisense strand comprises AmsAfsGmAfAmGfGmGfAmGfGmCfAmUfCmCfUmCfGmsUfsUm;
(k) the sense strand comprises IBs-AmCmGmGmGmAmCmAmGfUfAfUmUmCmUmCmAmGmUmimAms-IB(SEQ ID NO: 1014), and the antisense strand comprises UmsCfsAmsCfUmGfAmGmAmAmUmAfCmUfGmUfCmCfCmGfsUm(SEQ ID NO: 1174); or
(l) the sense strand comprises AmsAmsGmGmGmAmCfAmGfUfAfCTmUmCmUmCmAmGmUmsGmsCm, and the antisense strand comprises GmsCfsAmCmUmGfAmGmAmAmUmAmCmUfGmUfCmCmCmUmUmsUmsUm.

14. A siRNA according to any one of claims 1 to 13, wherein the siRNA is further conjugated to a ligand moiety comprising N-acetylgalactosamine via a phosphate group or a phosphorothioate group, preferably the sense strand of the siRNA is conjugated to the ligand moiety via a phosphate group or a phosphorthioate group.

15. A siRNA according to claim 14, wherein the 3' end of the sense strand is conjugated to the ligand moiety via a phosphate group or a phosphorothioate group.

16. A siRNA according to claim 14 or 15, wherein the ligand moiety comprises a conjugation group of formula (X'): 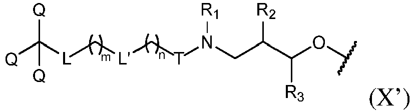 wherein, 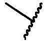 represents the position of attachment to siRNA; Q is independently H, 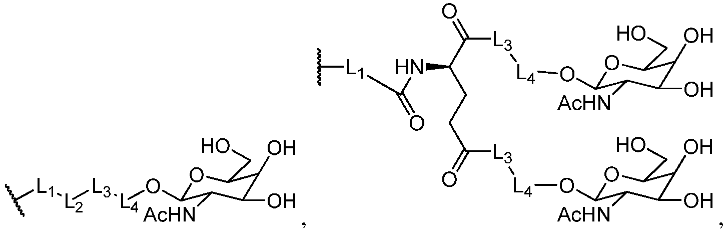 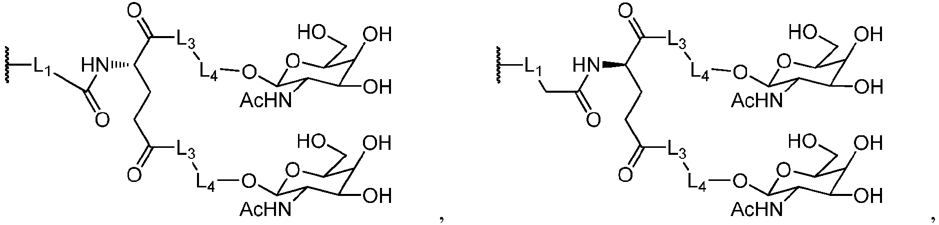 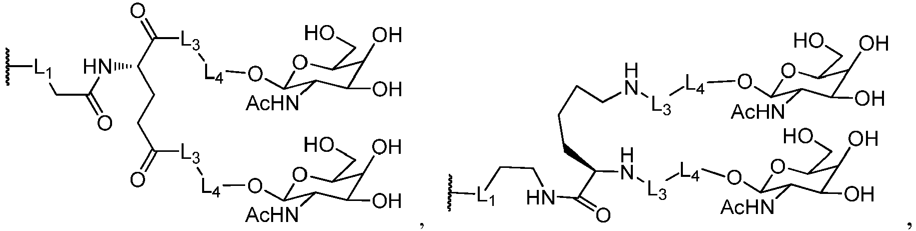 or 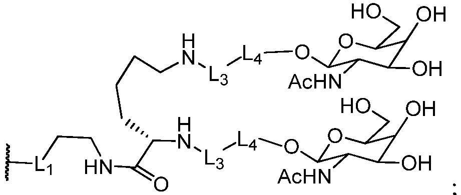
wherein L₁ is a bond, -CH₂-, -CH₂CH₂-, -C(O)-, -CH₂O-, -CH₂O-CH₂CH₂O-, or - NHC(O)-(CH2NHC(O))ₐ-;
L₂ is a bond or -CH₂CH₂C(O)-;
L₃ is a bond, -(NHCH₂CH₂)_{b}-, -(NHCH₂CH₂CH₂)_{b}-, or -C(O)CH₂-;
L₄ is -(OCH₂CH₂)_{c}-, -(OCH₂CH₂CH₂)_{c}-, -(OCH₂CH₂CH₂CH₂)_{c}-, - (OCH₂CH₂CH₂CH₂CH₂)_{c}-, or -NHC(O)-(CH₂)_{d}-;
wherein a = 0, 1, 2, or 3;
b = 1, 2, 3, 4 or 5;
c = 1, 2, 3, 4 or 5;
d = 1, 2, 3, 4, 5, 6, 7, or 8;
L is a bond, -CH₂O-, or -NHC(O)-;
L' is a bond, -C(O)NH-, -NHC(O)-, or -O(CH₂CH₂O)ₑ-;
wherein e is 1, 2, 3, 4, or 5;
T is a bond, -CH₂-, -C(O)-, -M-, -CH₂-M-, or -C(O)-M-;
wherein M is 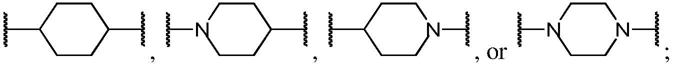
R₁ and R₂ together form -CH₂CH₂O- or -CH₂CH(R)-O-, and R₃ is H;
or R₁ and R₃ together form -C₁₋₂ alkylene-, and R₂ is H;
wherein R is -OR', -CH₂OR', or -CH₂CH₂OR'; wherein R' is H, a hydroxy-protecting group, or a solid support, and the hydroxy-protecting group is preferably - C(O)CH₂CH₂C(O)OH or 4,4'-dimethoxytrityl;
m = 0, 1, 2, 3, 4, 5, 6, 7, 8, 9 or 10;
n = 0, 1, 2, 3, 4, 5, 6, 7, 8, 9 or 10.

17. A siRNA according to claim 16, wherein the conjugation group is as shown in formula (I'): 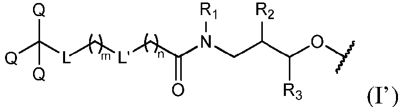 wherein, 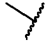 represents the position of attachment to siRNA; Q is independently H, 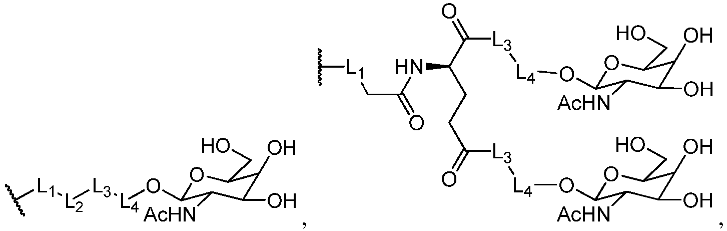 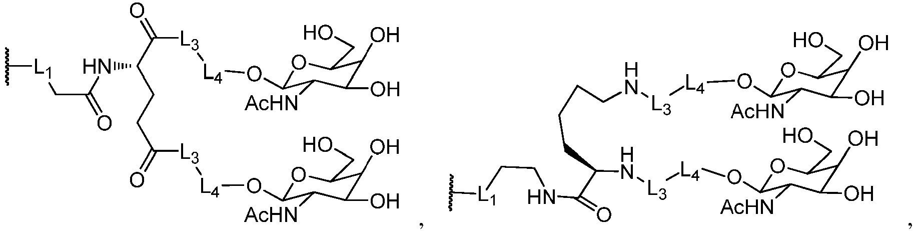 or 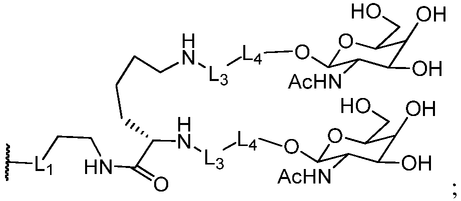
wherein L₁ is a bond, -CH₂-, -CH₂CH₂-, -C(O)-, -CH₂O-, -CH₂O-CH₂CH₂O-, or - NHC(O)-(CH2NHC(O))ₐ-;
L₂ is a bond or -CH₂CH₂C(O)-;
L₃ is a bond, -(NHCH₂CH₂)_{b}-, -(NHCH₂CH₂CH₂)_{b}-, or -C(O)CH₂-;
L₄ is -(OCH₂CH₂)_{c}-, -(OCH₂CH₂CH₂)_{c}-, -(OCH₂CH₂CH₂CH₂)_{c}-, - (OCH₂CH₂CH₂CH₂CH₂)_{c}-, or -NHC(O)-(CH₂)_{d}-;
wherein a = 0, 1, 2, or 3;
b = 1, 2, 3, 4 or 5;
c = 1, 2, 3, 4 or 5;
d = 1, 2, 3, 4, 5, 6, 7, or 8;
L is -CH₂O- or -NHC(O)-;
L' is a bond, -C(O)NH- or -NHC(O)-;
R₁ and R₂ together form -CH₂CH₂O- or -CH₂CH(R)-O-, and R₃ is H;
or R₁ and R₃ together form -C₁₋₂ alkylene-, and R₂ is H;
wherein R is -OR', -CH₂OR', or -CH₂CH₂OR'; wherein R' is H, a hydroxy-protecting group, or a solid support, and the hydroxy-protecting group is preferably - C(O)CH₂CH₂C(O)OH or 4,4'-dimethoxytrityl;
m 0, 1, 2, 3, 4, 5, 6,7, 8, 9 or 10;
n 0, 1, 2, 3, 4, 5, 6,7, 8, 9 or 10.

18. A siRNA according to claim 17, wherein: Q is independently H or 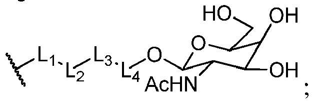
wherein L₁ is -CH₂O- or -NHC(O)-(CH₂NHC(O))ₐ-;
L₂ is -CH₂CH₂C(O)-;
L₃ is -(NHCH₂CH₂)_{b}- or -(NHCH₂CH₂CH₂)_{b}-;
L₄ is -(OCH₂CH₂)_{c}- or -NHC(O)-(CH₂)_{d}-;
wherein a = 0, 1, 2, or 3;
b = 1, 2, 3, 4 or 5;
c = 1, 2, 3, 4 or 5;
d = 1, 2, 3, 4, 5, 6, 7, or 8;
L is -CH₂O-;
L' is a bond;
R₁ and R₂ together form -CH₂CH₂O- or -CH₂CH(R)-O-, and R₃ is H;
or R₁ and R₃ together form -C₁₋₂ alkylene-, and R₂ is H;
wherein R is -OR', -CH₂OR', or -CH₂CH₂OR'; wherein R' is H, a hydroxy-protecting group, or a solid support, and the hydroxy-protecting group is preferably - C(O)CH₂CH₂C(O)OH or 4,4'-dimethoxytrityl;
m = 0, 1, 2, 3, 4, 5, 6, 7, 8, 9 or 10;
n = 0, 1, 2, 3, 4, 5, 6, 7, 8, 9 or 10.

19. A siRNA according to claim 18, wherein the conjugation group is as shown in formula (I'-1), formula (I'-2), or formula (I'-3): 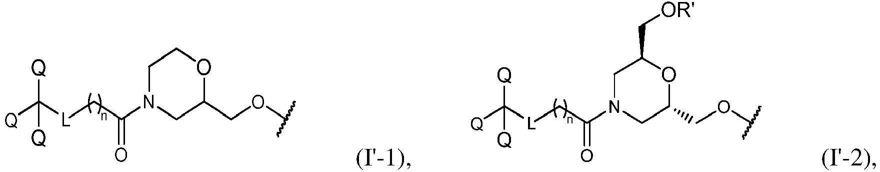 or 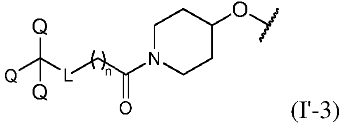 wherein, 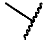 represents the position of attachment to siRNA;
Q is 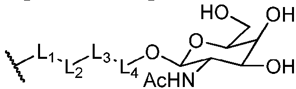
wherein L₁ is -CH₂O- or -NHC(O)-;
L₂ is -CH₂CH₂C(O)-;
L₃ is -(NHCH₂CH₂)_{b}- or -(NHCH₂CH₂CH₂)_{b}-;
L₄ is -(OCH₂CH₂)_{c}- or -NHC(O)-(CH₂)_{d}-;
wherein b=1, 2, 3, 4, or 5;
c = 1, 2, 3, 4 or 5;
d = 1, 2, 3, 4, 5, 6, 7, or 8;
L is -CH₂O-;
R' is H, a hydroxy-protecting group, or a solid support, wherein the hydroxy-protecting group is preferably -C(O)CH₂CH₂C(O)OH or 4,4'-dimethoxytrityl;
n = 0, 1, 2, 3, 4, 5, 6, 7, 8, 9 or 10.

20. A siRNA according to claim 17, wherein:
Q is independently H, 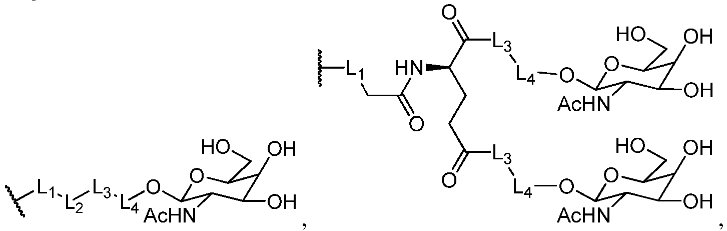 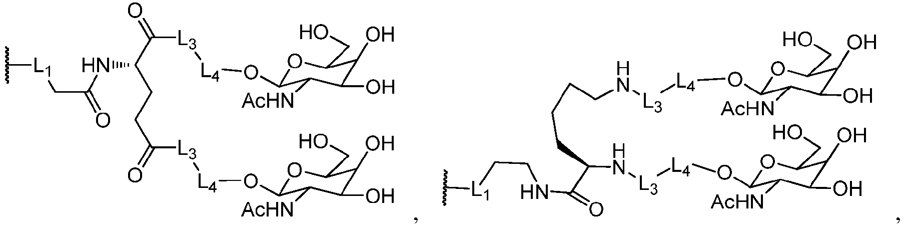 or 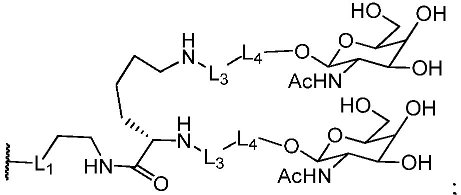
wherein L₁ is -CH₂O-, -CH₂O-CH₂CH₂O-, or -NHC(O)-(CH₂NHC(O))ₐ-;
L₂ is -CH₂CH₂C(O)-;
L₃ is -(NHCH₂CH₂)_{b}-, -(NHCH₂CH₂CH₂)_{b}-, or -C(O)CH₂-;
L₄ is -(OCH₂CH₂)_{c}- or -NHC(O)-(CH₂)_{d}-;
wherein a = 0, 1, 2, or 3;
b = 1, 2, 3, 4 or 5;
c = 1, 2, 3, 4 or 5;
d = 1, 2, 3, 4, 5, 6, 7, or 8;
L is -CH₂O- or -NHC(O)-;
L' is a bond or -C(O)NH-;
R₁ and R₂ together form -CH₂CH₂O- or -CH₂CH(R)-O-, and R₃ is H;
or R₁ and R₃ together form -C₁₋₂ alkylene-, and R₂ is H;
wherein R is -OR', -CH₂OR', or -CH₂CH₂OR'; wherein R' is H, a hydroxy-protecting group, or a solid support, and the hydroxy-protecting group is preferably - C(O)CH₂CH₂C(O)OH or 4,4'-dimethoxytrityl;
m = 0, 1, 2, 3, 4, 5, 6, 7, 8, 9 or 10;
n = 0, 1, 2, 3, 4, 5, 6, 7, 8, 9 or 10.

21. A siRNA according to claim 20, wherein the conjugation group is as shown in formula (II'-1) or formula (II'-2): 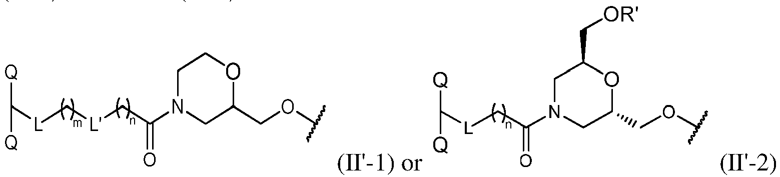 wherein, 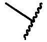 represents the position of attachment to siRNA; Q is independently 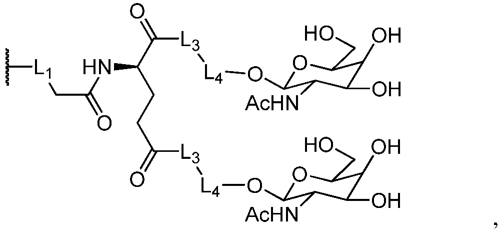 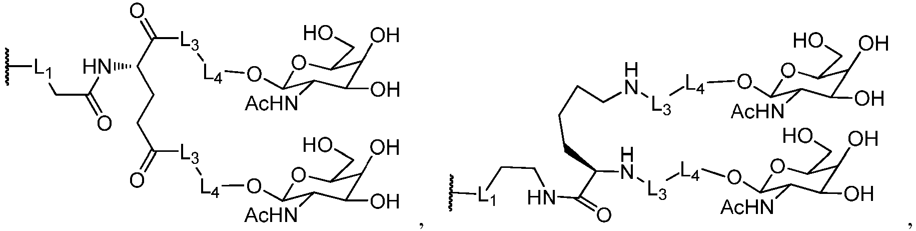 or 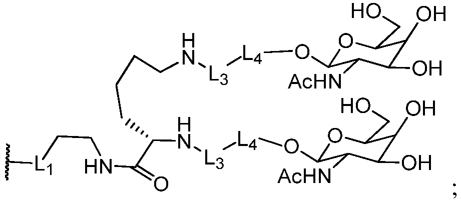
wherein L₁ is -CH₂O- or -CH₂O-CH₂CH₂O-;
L₃ is -(NHCH₂CH₂)_{b}-, -(NHCH₂CH₂CH₂)_{b}-, or -C(O)CH₂-;
L₄ is -(OCH₂CH₂)_{c}- or -NHC(O)-(CH₂)_{d}-;
wherein b=1, 2, 3, 4, or 5;
c = 1, 2, 3, 4 or 5;
d = 1, 2, 3, 4, 5, 6, 7, or 8;
L is -NHC(O)-;
L' is a bond or -C(O)NH-;
R' is H, a hydroxy-protecting group, or a solid support, wherein the hydroxy-protecting group is preferably -C(O)CH₂CH₂C(O)OH or 4,4'-dimethoxytrityl;
m = 0, 1, 2, 3, 4, 5, 6, 7, 8, 9 or 10;
n = 0, 1, 2, 3, 4, 5, 6, 7, 8, 9 or 10.

22. A siRNA according to claim 17, wherein:
Q is independently H, 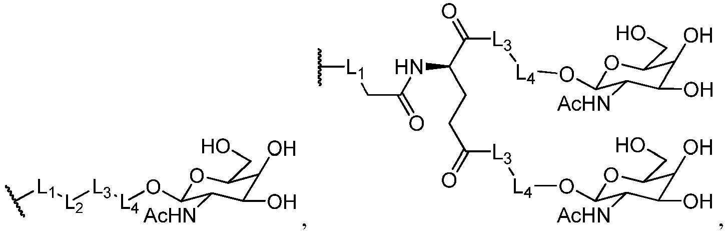 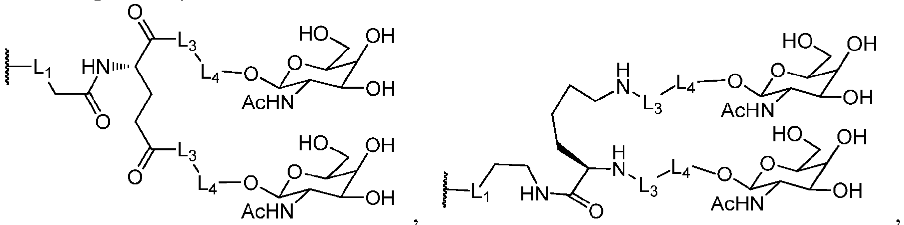 or 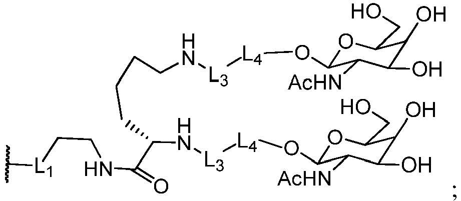
wherein L₁ is -CH₂-, -C(O)-, -CH₂O-, -CH₂O-CH₂CH₂O-, or -NHC(O)-(CH₂NHC(O))ₐ-;
L₂ is a bond;
L₃ is -(NHCH₂CH₂)_{b}-, -(NHCH₂CH₂CH₂)_{b}-, or -C(O)CH₂-;
L₄ is -(OCH₂CH₂)_{c}- or -NHC(O)-(CH₂)_{d}-;
wherein a = 0, 1, 2, or 3;
b = 1, 2, 3, 4 or 5;
c = 1, 2, 3, 4 or 5;
d = 1, 2, 3, 4, 5, 6, 7, or 8;
L is -CH₂O- or -NHC(O)-;
L' is a bond or -C(O)NH-;
R₁ and R₂ together form -CH₂CH₂O- or -CH₂CH(R)-O-, and R₃ is H;
or R₁ and R₃ together form -C₁₋₂ alkylene-, and R₂ is H;
wherein R is -OR', -CH₂OR', or -CH₂CH₂OR'; wherein R' is H, a hydroxy-protecting group, or a solid support, and the hydroxy-protecting group is preferably - C(O)CH₂CH₂C(O)OH or 4,4'-dimethoxytrityl;
m = 0, 1, 2, 3, 4, 5, 6, 7, 8, 9 or 10;
n = 0, 1, 2, 3, 4, 5, 6, 7, 8, 9 or 10.

23. A siRNA according to claim 22, wherein the conjugation group is as shown in formula (II'-2): 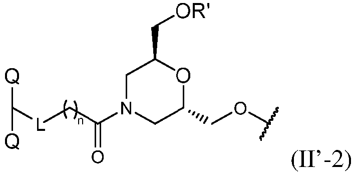 wherein, 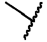 represents the position of attachment to siRNA; Q is independently 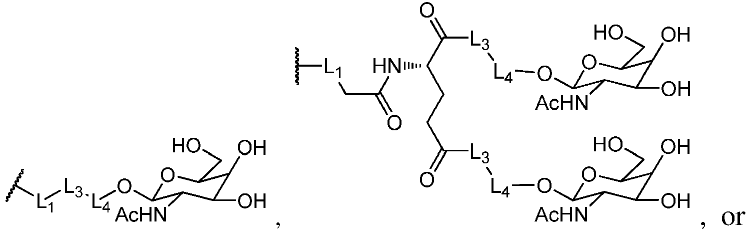 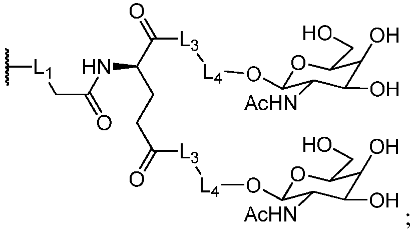
wherein L₁ is -CH₂- or -C(O)-;
L₃ is -(NHCH₂CH₂)_{b}-;
L₄ is -(OCH₂CH₂)_{c}-;
wherein b=1, 2, 3, 4, or 5;
c = 1, 2, 3, 4 or 5;
L is -CH₂O- or -NHC(O)-;
R' is H, a hydroxy-protecting group, or a solid support, wherein the hydroxy-protecting group is preferably -C(O)CH₂CH₂C(O)OH or 4,4'-dimethoxytrityl;
n = 0, 1, 2, 3, 4, 5, 6, 7, 8, 9 or 10.

24. A siRNA according to claim 16, wherein:
Q is independently H, 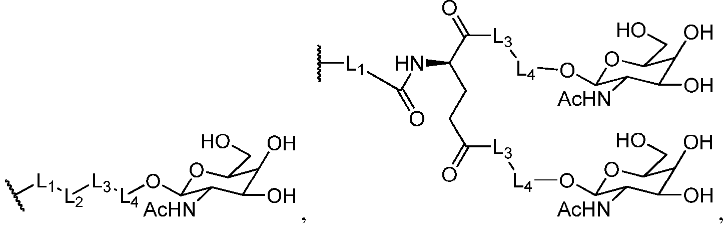 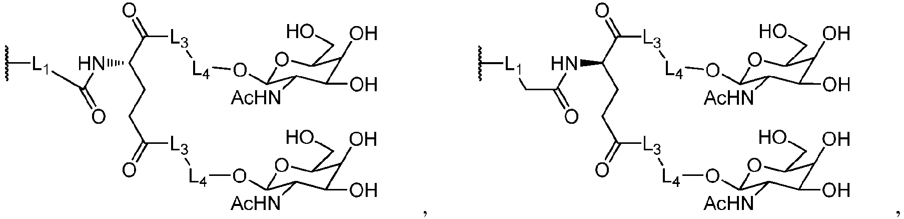 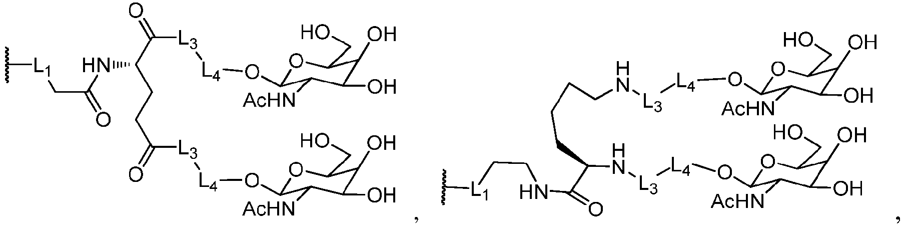 or 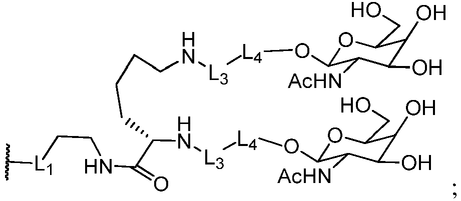
wherein L₁ is a bond, -CH₂-, -CH₂CH₂-, -C(O)-, -CH₂O-, -CH₂O-CH₂CH₂O-, or - NHC(O)-(CH2NHC(O))ₐ-;
L₂ is a bond or -CH₂CH₂C(O)-;
L₃ is a bond, -(NHCH₂CH₂)_{b}-, -(NHCH₂CH₂CH₂)_{b}-, or -C(O)CH₂-;
L₄ is -(OCH₂CH₂)_{c}-, -(OCH₂CH₂CH₂)_{c}-, -(OCH₂CH₂CH₂CH₂)_{c}-, - (OCH₂CH₂CH₂CH₂CH₂)_{c}-, or -NHC(O)-(CH₂)_{d}-;
wherein a = 0, 1, 2, or 3;
b = 1, 2, 3, 4 or 5;
c = 1, 2, 3, 4 or 5;
d = 1, 2, 3, 4, 5, 6, 7, or 8;
L is a bond, -CH₂O-, or -NHC(O)-;
L' is a bond, -C(O)NH-, -NHC(O)-, or -O(CH₂CH₂O)ₑ-;
wherein e is 1, 2, 3, 4, or 5;
T is a bond, -CH₂-, -M-, -CH₂-M-, or -C(O)-M-;
wherein M is 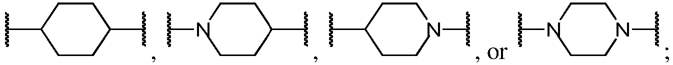
R₁ and R₂ together form -CH₂CH₂O- or -CH₂CH(R)-O-, and R₃ is H;
or R₁ and R₃ together form -C₁₋₂ alkylene-, and R₂ is H;
wherein R is -OR', -CH₂OR', or -CH₂CH₂OR'; wherein R' is H, a hydroxy-protecting group, or a solid support, and the hydroxy-protecting group is preferably - C(O)CH₂CH₂C(O)OH or 4,4'-dimethoxytrityl;
m = 0, 1, 2, 3, 4, 5, 6, 7, 8, 9 or 10;
n = 0, 1, 2, 3, 4, 5, 6, 7, 8, 9 or 10.

25. A siRNA according to claim 24, wherein, T is -M-, -CH₂-M-, or -C(O)-M-, wherein M is

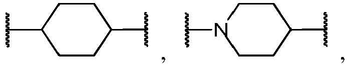

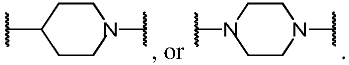

26. A siRNA according to claim 24 or 25, wherein,
Q is independently H or 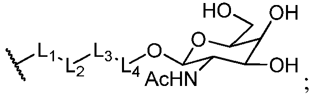
wherein L₁ is -CH₂O- or -NHC(O)-(CH₂NHC(O))ₐ-;
L₂ is -CH₂CH₂C(O)-;
L₃ is -(NHCH₂CH₂)_{b}- or -(NHCH₂CH₂CH₂)_{b}-;
L₄ is -(OCH₂CH₂)_{c}- or -NHC(O)-(CH₂)_{d}-;
wherein a = 0, 1, 2, or 3;
b = 1, 2, 3, 4 or 5;
c = 1, 2, 3, 4 or 5;
d = 1, 2, 3, 4, 5, 6, 7, or 8;
L is a bond or -CH₂O-;
L' is a bond or -O(CH₂CH₂O)ₑ-;
wherein e is 1, 2, 3, 4, or 5;
R₁ and R₂ together form -CH₂CH₂O- or -CH₂CH(R)-O-, and R₃ is H;
or R₁ and R₃ together form -C₁₋₂ alkylene-, and R₂ is H;
wherein R is -OR', -CH₂OR', or -CH₂CH₂OR'; wherein R' is H, a hydroxy-protecting group, or a solid support, and the hydroxy-protecting group is preferably - C(O)CH₂CH₂C(O)OH or 4,4'-dimethoxytrityl;
m = 0, 1, 2, 3, 4, 5, 6, 7, 8, 9 or 10;
n = 0, 1, 2, 3, 4, 5, 6, 7, 8, 9 or 10;
wherein T is as defined in claim 24 or 25.

27. A siRNA according to claim 26, wherein the conjugation group is as shown in formula (III'-1), formula (III'-2), or formula (III'-3): 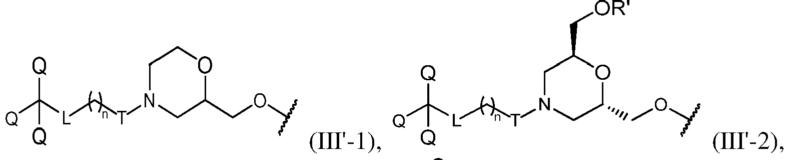 or 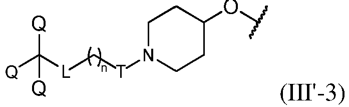 wherein,
Q is 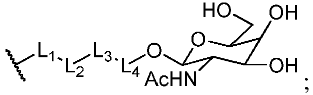
wherein L₁ is -CH₂O- or -NHC(O)-;
L₂ is -CH₂CH₂C(O)-;
L₃ is -(NHCH₂CH₂)_{b}- or -(NHCH₂CH₂CH₂)_{b}-;
L₄ is -(OCH₂CH₂)_{c}- or -NHC(O)-(CH₂)_{d}-;
wherein b=1, 2, 3, 4, or 5;
c = 1, 2, 3, 4 or 5;
d = 1, 2, 3, 4, 5, 6, 7, or 8;
L is a bond or -CH₂O-;
wherein R' is H, a hydroxy-protecting group, or a solid support, wherein the hydroxy-protecting group is preferably -C(O)CH₂CH₂C(O)OH or 4,4'-dimethoxytrityl;
n = 0, 1, 2, 3, 4, 5, 6, 7, 8, 9 or 10;
wherein T is as defined in claim 24 or 25.

28. A siRNA according to claim 24 or 25, wherein,
Q is independently H, 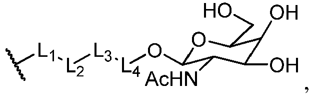 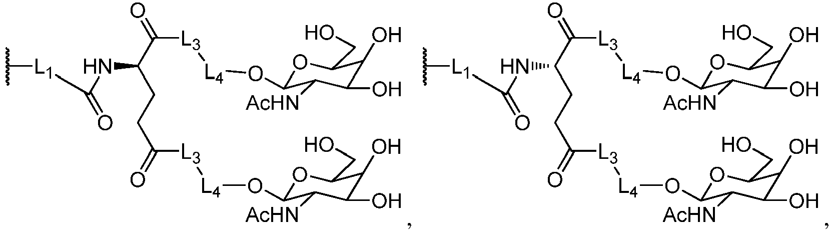 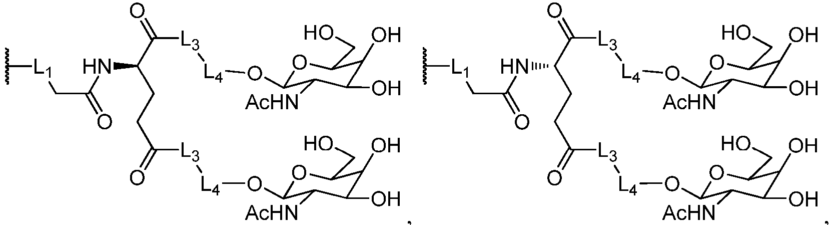 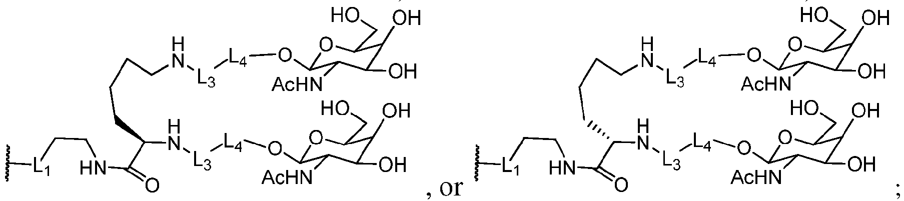
wherein L₁ is -CH₂-, -CH₂O-, or -C(O)-;
L₂ is a bond;
L₃ is -(NHCH₂CH₂)_{b}-, -(NHCH₂CH₂CH₂)_{b}-, or -C(O)CH₂-;
L₄ is -(OCH₂CH₂)_{c}- or -NHC(O)-(CH₂)_{d}-;
wherein b=1, 2, 3, 4, or 5;
c = 1, 2, 3, 4 or 5;
d = 1, 2, 3, 4, 5, 6, 7, or 8;
L is a bond or -NHC(O)-;
L' is a bond;
R₁ and R₂ together form -CH₂CH₂O- or -CH₂CH(R)-O-, and R₃ is H;
or R₁ and R₃ together form -C₁₋₂ alkylene-, and R₂ is H;
wherein R is -OR', -CH₂OR', or -CH₂CH₂OR'; wherein R' is H, a hydroxy-protecting group, or a solid support, and the hydroxy-protecting group is preferably - C(O)CH₂CH₂C(O)OH or 4,4'-dimethoxytrityl;
m = 0, 1, 2, 3, 4, 5, 6, 7, 8, 9 or 10;
n = 0, 1, 2, 3, 4, 5, 6, 7, 8, 9 or 10;
wherein T is as defined in claim 24 or 25.

29. A siRNA according to claim 24 or 25, wherein the conjugation group is as shown in formula (IV-1) or formula (IV-2): 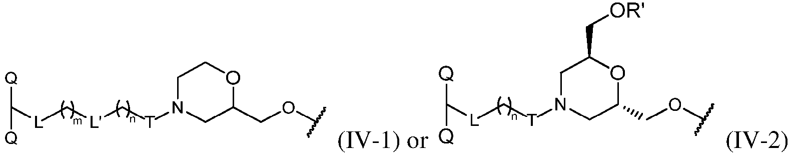 wherein, Q is independently 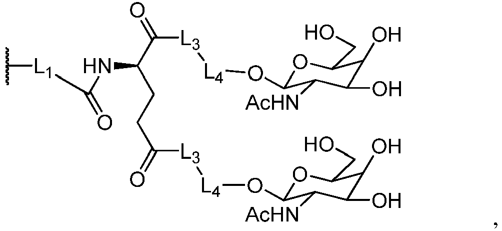 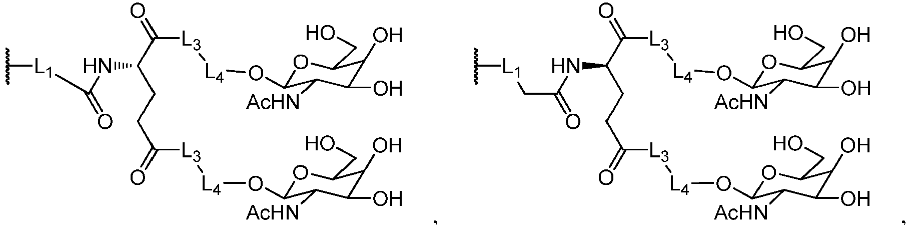 or 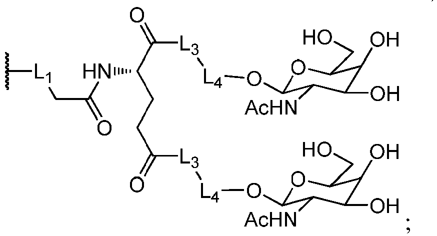
wherein L₁ is -CH₂-, -CH₂O-, or -C(O)-;
L₃ is -(NHCH₂CH₂)_{b}-, -(NHCH₂CH₂CH₂)_{b}-, or -C(O)CH₂-;
L₄ is -(OCH₂CH₂)_{c}- or -NHC(O)-(CH₂)_{d}-;
wherein b=1, 2, 3, 4, or 5;
c = 1, 2, 3, 4 or 5;
d = 1, 2, 3, 4, 5, 6, 7, or 8;
L is a bond or -NHC(O)-;
L' is a bond;
wherein R' is H, a hydroxy-protecting group, or a solid support, wherein the hydroxy-protecting group is preferably -C(O)CH₂CH₂C(O)OH or 4,4'-dimethoxytrityl;
m 0, 1, 2, 3, 4, 5, 6, 7, 8, 9 or 10;
n = 0, 1, 2, 3, 4, 5, 6, 7, 8, 9 or 10;
wherein T is as defined in claim 24 or 25.

30. A siRNA according to claim 16, wherein:
Q is independently H, 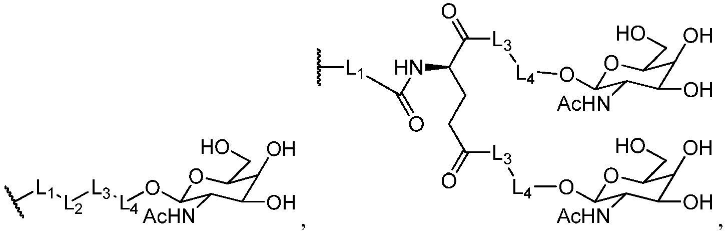 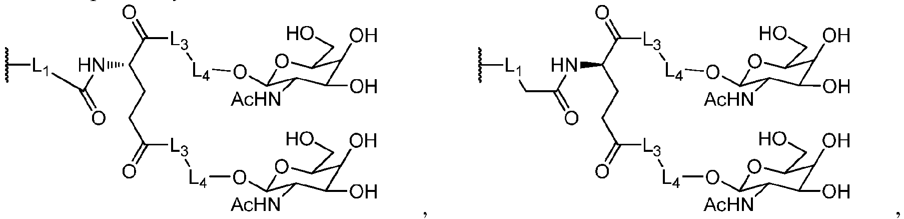 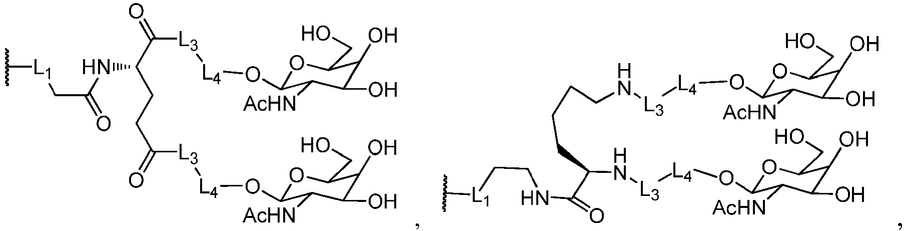 or 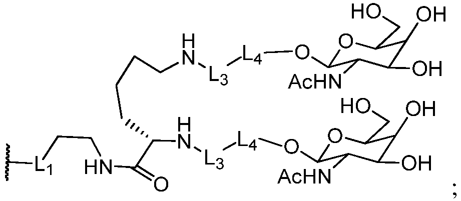
wherein L₁ is a bond, -CH₂-, -CH₂CH₂-, -C(O)-, -CH₂O-, -CH₂O-CH₂CH₂O-, or - NHC(O)-(CH2NHC(O))ₐ-;
L₂ is a bond or -CH₂CH₂C(O)-;
L₃ is a bond, -(NHCH₂CH₂)_{b}-, -(NHCH₂CH₂CH₂)_{b}-, or -C(O)CH₂-;
L₄ is -(OCH₂CH₂)_{c}-, -(OCH₂CH₂CH₂)_{c}-, -(OCH₂CH₂CH₂CH₂)_{c}-, - (OCH₂CH₂CH₂CH₂CH₂)_{c}-, or -NHC(O)-(CH₂)_{d}-;
wherein a = 0, 1, 2, or 3;
b = 1, 2, 3, 4 or 5;
c = 1, 2, 3, 4 or 5;
d = 1, 2, 3, 4, 5, 6, 7, or 8;
L is a bond, -CH₂O-, or -NHC(O)-;
L' is -O(CH₂CH₂O)ₑ-;
wherein e is 1, 2, 3, 4, or 5;
T is a bond, -CH₂-, -C(O)-, -M-, -CH₂-M-, or -C(O)-M-;
wherein M is 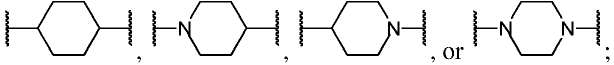
R₁ and R₂ together form -CH₂CH₂O- or -CH₂CH(R)-O-, and R₃ is H;
or R₁ and R₃ together form -C₁₋₂ alkylene-, and R₂ is H;
wherein R is -OR', -CH₂OR', or -CH₂CH₂OR'; wherein R' is H, a hydroxy-protecting group, or a solid support, and the hydroxy-protecting group is preferably - C(O)CH₂CH₂C(O)OH or 4,4'-dimethoxytrityl;
m = 0, 1, 2, 3, 4, 5, 6, 7, 8, 9 or 10;
n = 0, 1, 2, 3, 4, 5, 6, 7, 8, 9 or 10.

31. A siRNA according to claim 16, wherein the conjugation group is selected from:
| No. | Structure |
|---|---|
| 1 | 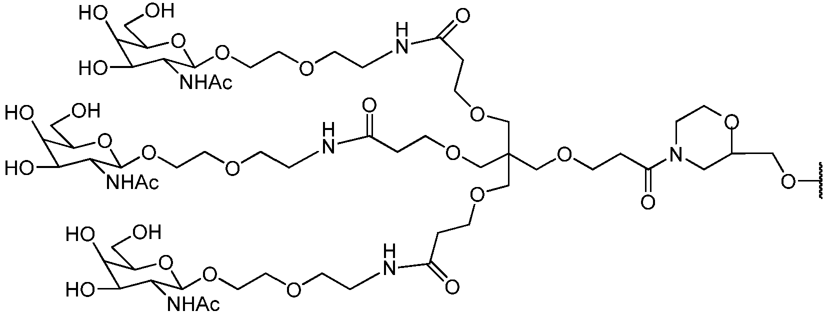 |
| 2 | 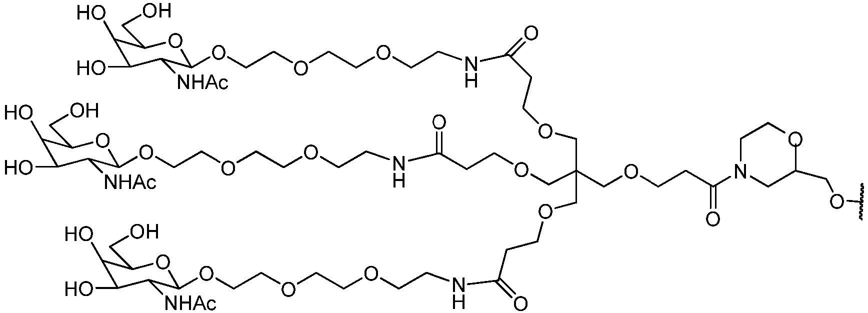 |
| 3 | 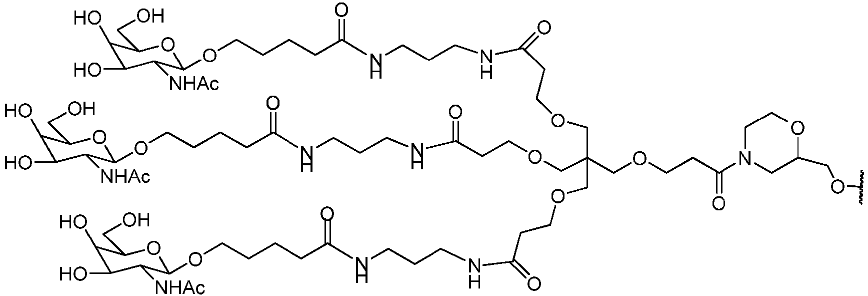 |
| 4 | 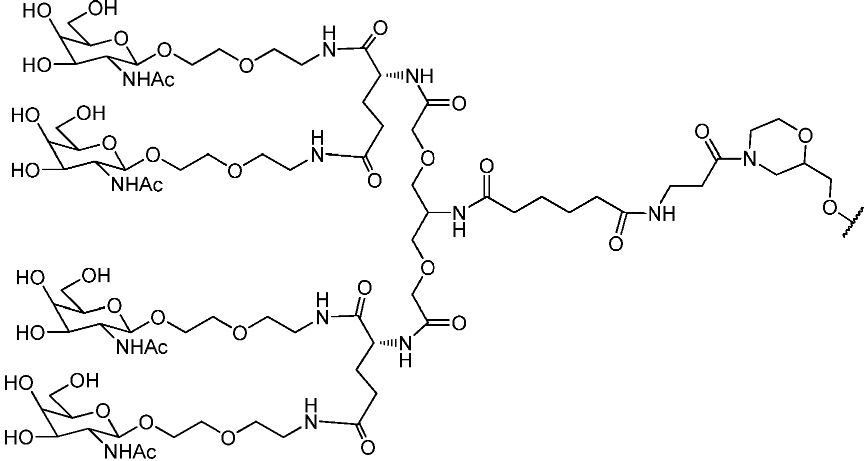 |
| 5 | 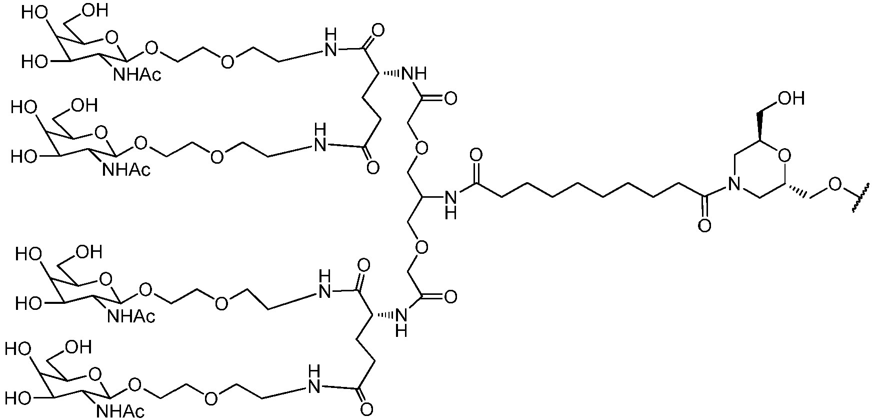 |
| 6 | 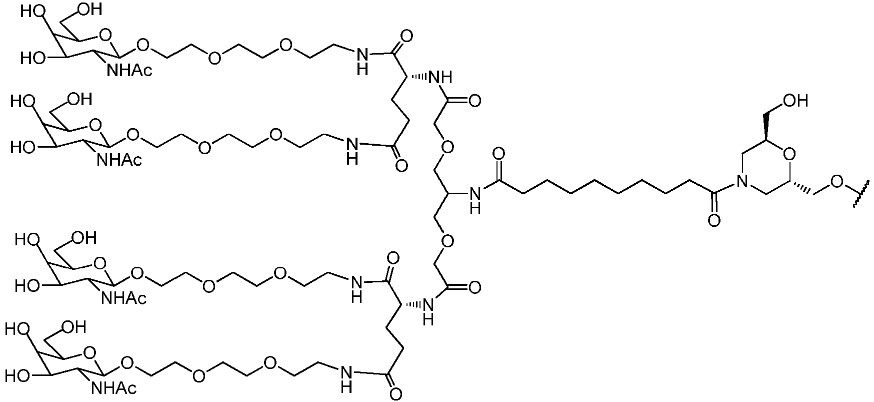 |
| 7 | 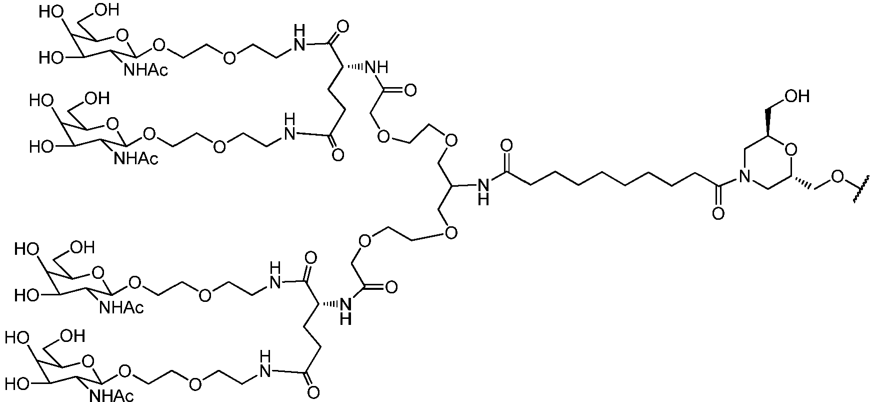 |
| 8 | 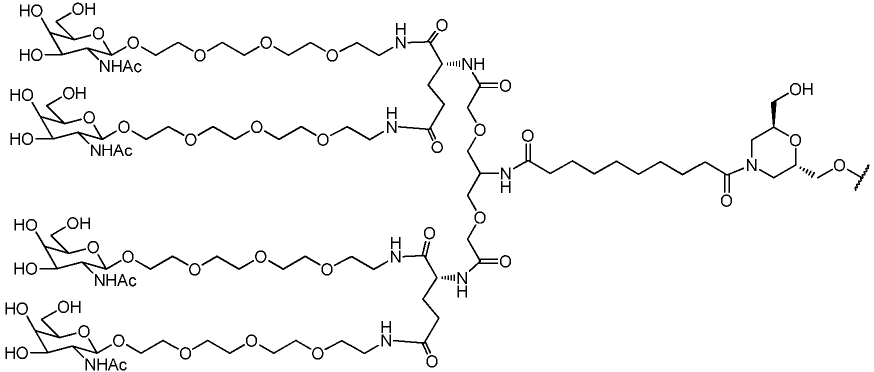 |
| 9 | 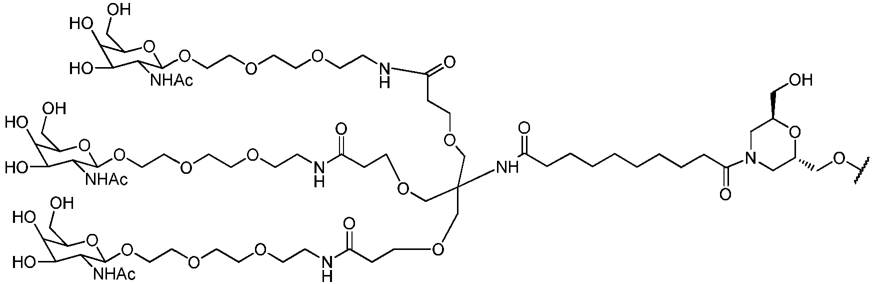 |
| 10 | 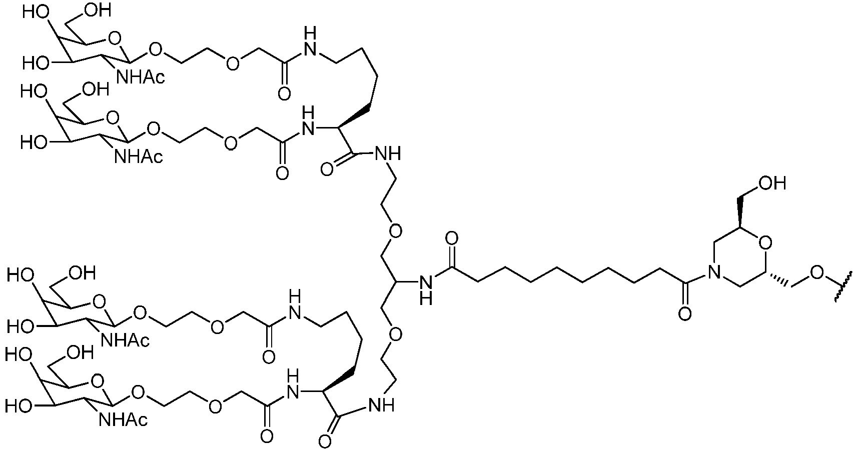 |
| 11 | 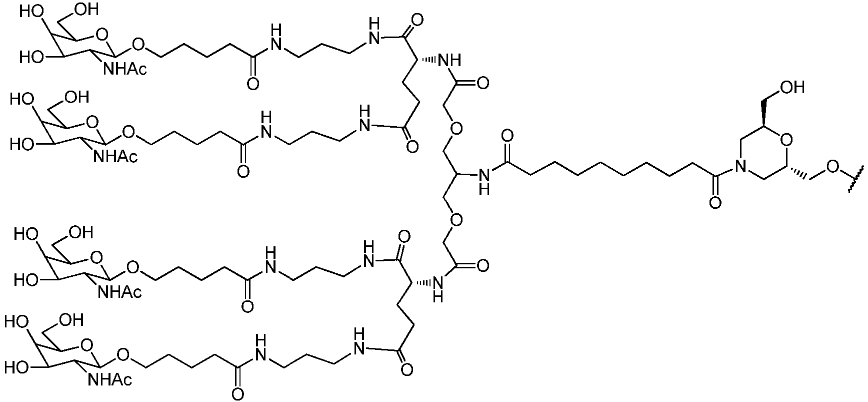 |
| 12 | 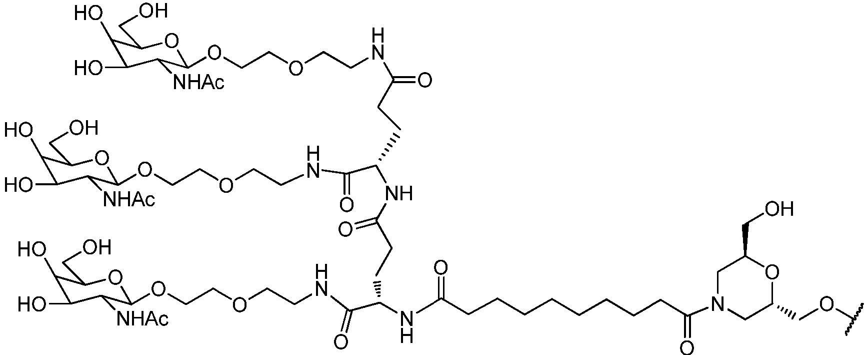 |
| 13 | 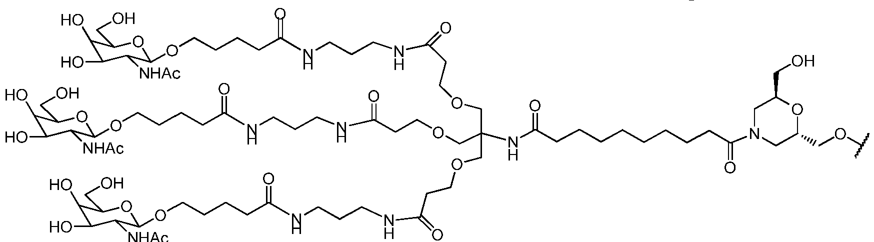 |
| 14 | 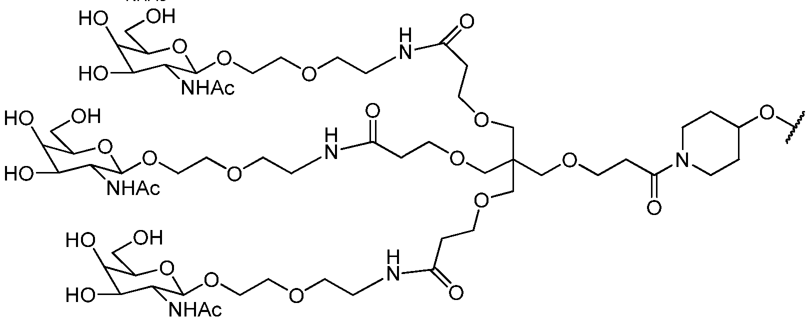 |
| 15 | 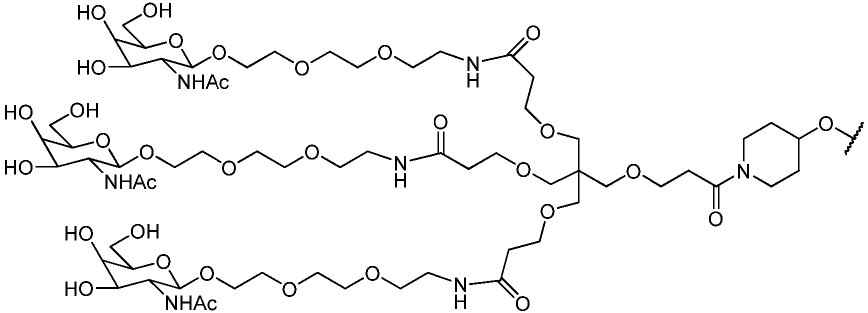 |
| 16 | 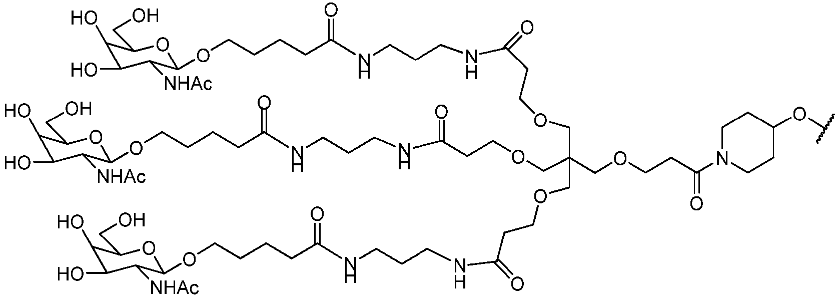 |

32. A siRNA according to claim 16, wherein the conjugation group is selected from:
| No. | Structure |
|---|---|
| 17 | 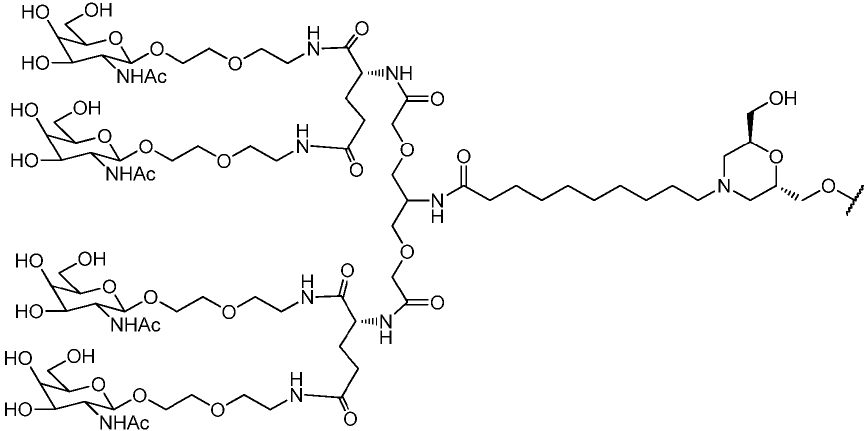 |
| 18 | 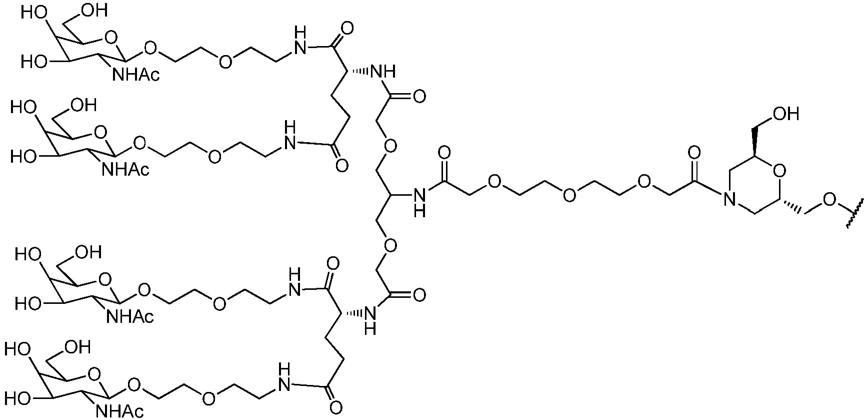 |
| 19 | 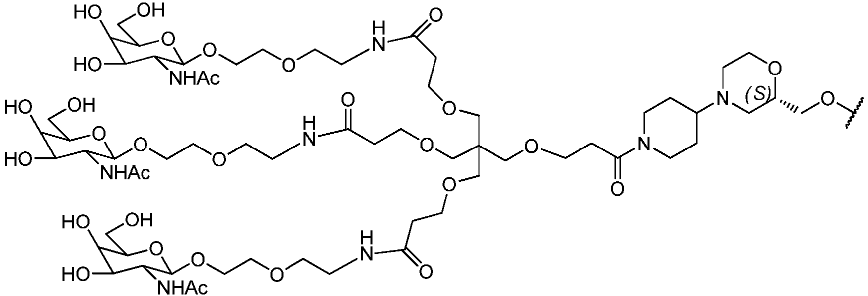 |
| 20 | 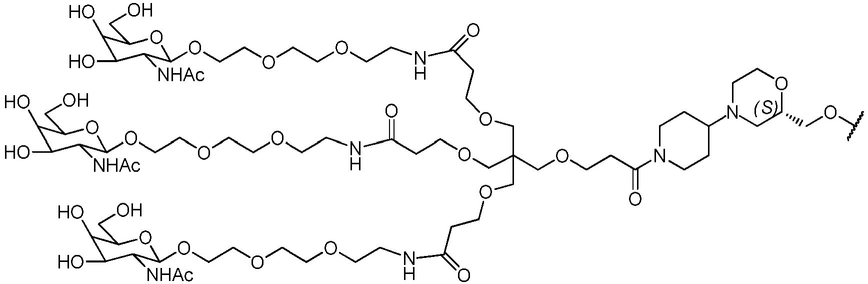 |
| 21 | 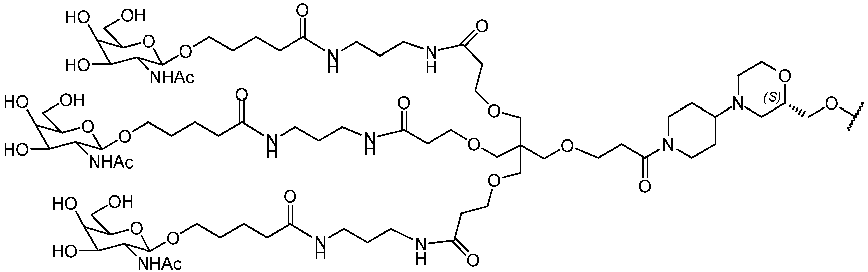 |
| 22 | 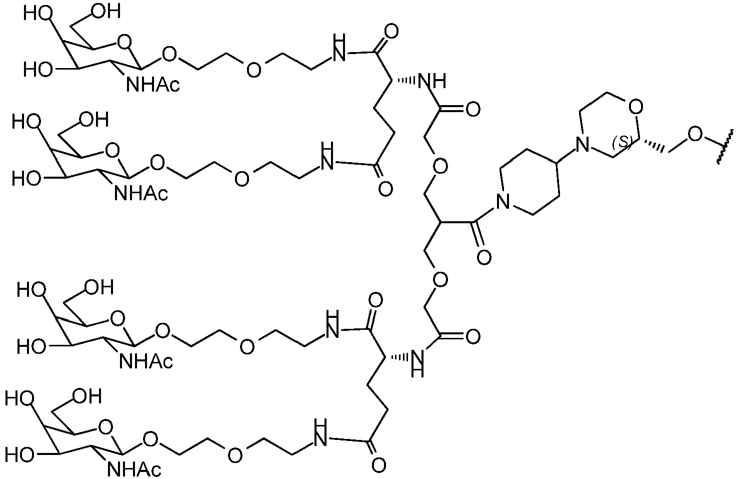 |
| 23-1 | 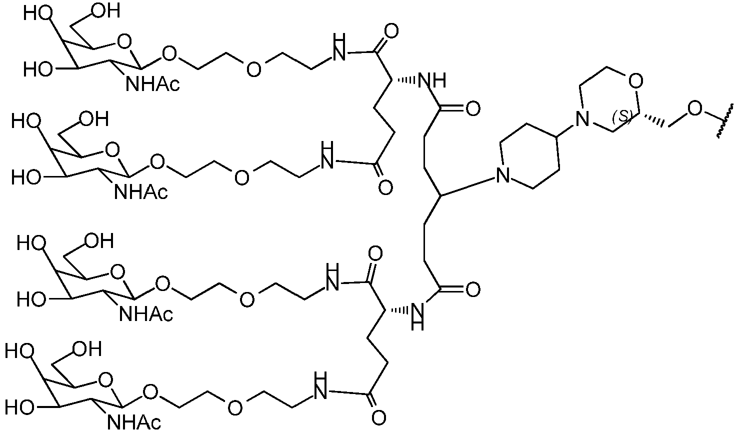 |
| 23-2 | 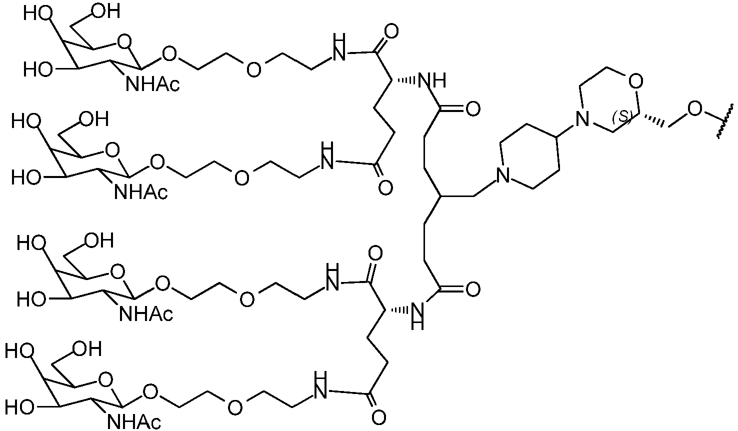 |
| 24 | 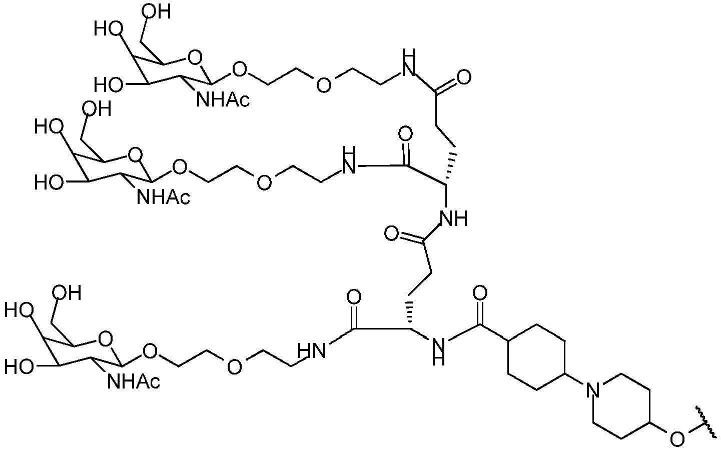 |
| 25 | 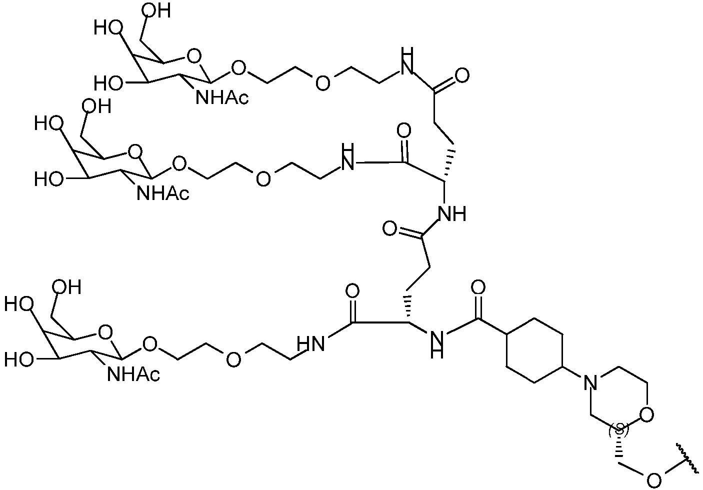 |
| 26 | 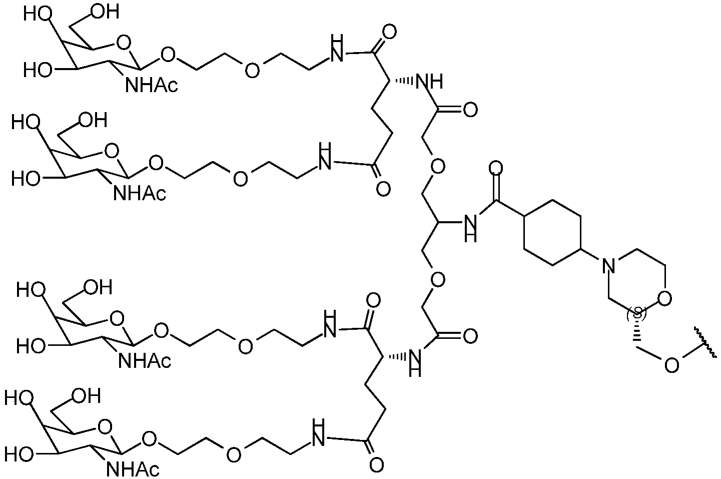 |
| 27 | 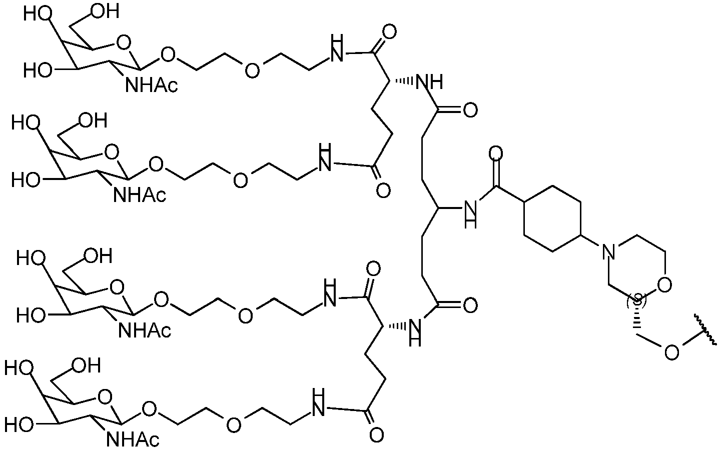 |
| 28 | 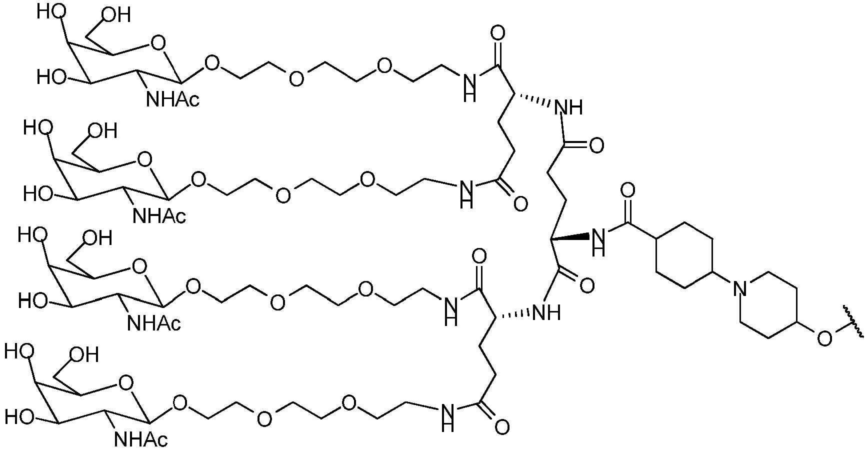 |
| 29 | 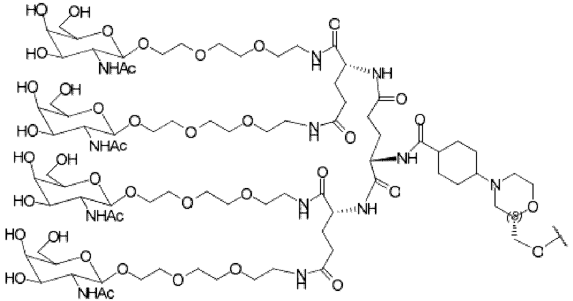 |

33. A siRNA according to any one of claims 1 to 32, wherein the ligand targets an asialoglycoprotein receptor (ASGPR).

34. A siRNA according to claim 14 or 15, wherein the ligand has the following structure: 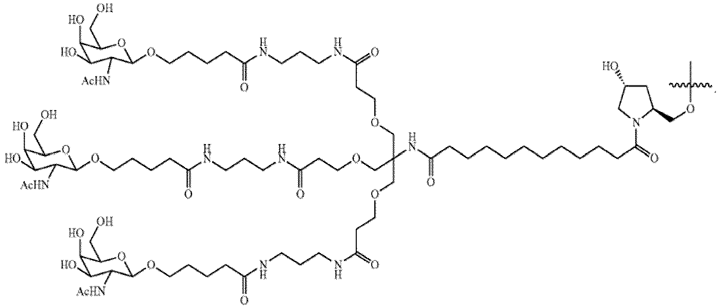 wherein 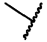 , represents the position of attachment to the sense strand of the siRNA via a phosphate group or a phosphorothioate group.

35. A siRNA according to any one of claim 14 or 15, wherein the ligand has the following structure: 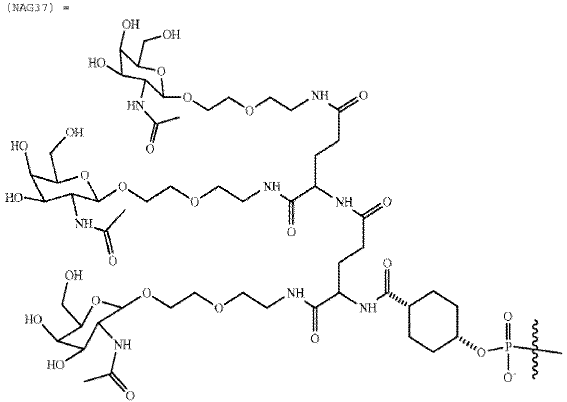 wherein 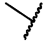 , represents the position of attachment to the sense strand of the siRNA via a phosphate group or a phosphorothioate group.

36. A siRNA according to claim 14 or 15, wherein the ligand has the following structure: 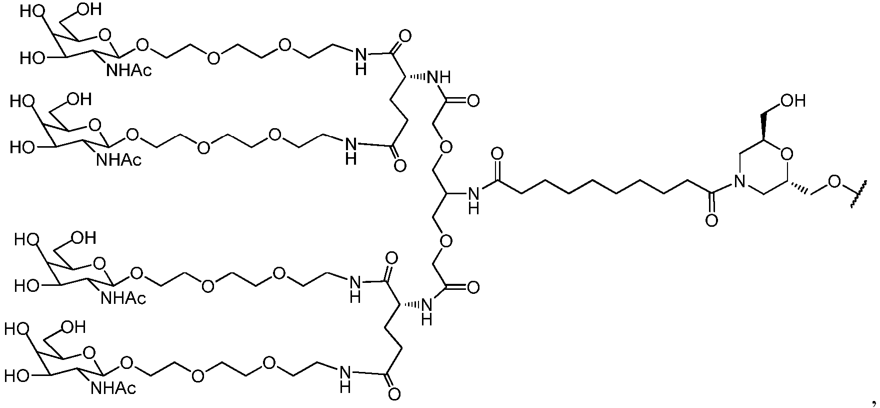 wherein 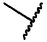 represents the position of attachment to the sense strand of the siRNA via a phosphate group or a phosphorothioate group.

37. A siRNA according to claim 13, wherein:
(1) the sense strand comprises 
STM1s-AmsAmAmAmGmGmGmAmCfAfGfUmAmUmUmCmUmCmAmGmAms-STM1s-
GL6 (SEQ ID NO: 1238),
 and the antisense strand comprises
(CP1a-U)sCfsUmGfAmGfAmAfUmAfCmUfGmUfCmCfCmUfUmsUfsUm (SEQ ID NO: 1651);
(2) the sense strand comprises 
STM1s-AmsAmUmUmAmAmAmAmGfGfGfAmCmAmGmUmAmUmUmCmAms-STM1s-
GL6 (SEQ ID NO: 1235),
 and the antisense strand comprises
(CP1a-U)sGfsAmAfUmAfCmUfGmUfCmCfCmUfUmUfUmAfAmsUfsUm (SEQ ID NO: 1650);
(3) the sense strand comprises 
STM1s-AmsAmUmUmAmAmAmAmGfGfGfAmCmAmGmUmAmUmUmCmAms-STM1s-
GL6 (SEQ ID NO: 1235),
 and the antisense strand comprises
(VPUm)sGfsAmAfUmAfCmUfGmUfCmCfCmUfUmUfUmAfAmsUfsUm (SEQ ID NO: 1649);
(4) the sense strand comprises
IBs-AmsAmUmUmAmAmAmAmGfGfGfAmCmAmGmUmAmUmUmCmAms-IBs-GL6 (SEQ ID NO: 1236),
and the antisense strand comprises
(CP1a-U)sGfsAmAfUmAfCmUfGmUfCmCfCmUfUmUfUmAfAmsUfsUm(SEQ ID NO: 1650);
(5) the sense strand comprises
UmsUmsAmAmAmAmGfGfGfAmCmAmGmUmAmUmUmCmsAms-GL6(SEQ ID NO: 1237),
and the antisense strand comprises
(CP1a-U)sGfsAmAfUmAfCmUfGmUfCmCfCmUfUmUfUmAfAmsUfsUm(SEQ ID NO: 1650);
(6) the sense strand comprises
IBs-AmsAmAmAmGmGmGmAmCfAfGfUmAmUmUmCmUmCmAmGmAms-IBs-GL6 (SEQ ID NO: 1239),
and the antisense strand comprises
(CP1a-U)sCfsUmGfAmGfAmAfUmAfCmUfGmUfCmCfCmUfUmsUfsUm(SEQ ID NO: 1651);
(7) the sense strand comprises
AmsAmsGmGmGmAmCfAfGfUmAmUmUmCmUmCmAmGmsAms-GL6(SEQ ID NO: 1240),
and the antisense strand comprises
(CP1a-U)sCfsUmGfAmGfAmAfUmAfCmUfGmUfCmCfCmUfUmsUfsUm(SEQ ID NO: 1651); or
(8) the sense strand comprises 
IBs-AmsAmAmAmGmGmGmAmCfAfGfUmAmUmUmCmUmCmAmGmAms-IBs-
GL6(SEQ ID NO: 1239),
 and the antisense strand comprises
(CP1a-U)sCfsUmGfAmGf(PCN-A)AfUmAfCmUfGmUfCmCfCmUfUmsUfsUm(SEQ ID NO: 1652); or
(9) the sense strand comprises
AmsAmsGmGmGmAmCfAfGfUmAmUmUmCmUmCmAmGmsAms-GL6(SEQ ID NO: 1240),
and the antisense strand comprises
(CP1a-U)sCfsUmGfAmGf(PCN-A)AfUmAfCmUfGmUfCmCfCmUfUmsUfsUm(SEQ ID NO: 1652).

38. A cell comprising a siRNA of any one of claims 1 to 37.

39. A pharmaceutical composition comprising a siRNA of any one of claims 1 to 37, or a cell of claim 38, and optionally a pharmaceutically acceptable vector or excipient.

40. A kit comprising a siRNA of any one of claims 1 to 37, a cell of claim 38, or a pharmaceutical composition of claim 39.

41. A method for treating diseases related to APOC3 in a subject, wherein the method comprises the step of administering to the subject a siRNA of any one of claims 1 to 37, a cell of claim 38, or a pharmaceutical composition of claim 39.

42. A method for reducing the risk of developing a disease related to APOC3 in a subject, wherein the method comprises the step of administering to the subject a siRNA of any one of claims 1 to 37, a cell of claim 38, or a pharmaceutical composition of claim 39.

43. A method according to claim 41 or 42, wherein the APOC3-related disease is selected from hyperlipidemia and hypertriglyceridemia.

44. A method according to claim 41 or 42, wherein the APOC3-related disease is a condition that can be caused by, associated with, or as a consequence of hypertriglyceridemia, such as non-alcoholic fatty liver disease, non-alcoholic steatohepatitis, polycystic ovary syndrome, kidney disease, obesity, type 2 diabetes mellitus, hypertension, atherosclerosis, cardiovascular disease, or pancreatitis.
